# EUROPEAN PATENT SPECIFICATION

(11) **EP 2 968 585 B1**
(45) Date of publication and mention of the grant of the patent: **18.07.2018**
(21) Application number: 14711942.4
(22) Date of filing: 13.03.2014
(51) Int. Cl.: A61K 47/68, A61P 35/00

(54) **PYRROLOBENZODIAZEPINES AND CONJUGATES THEREOF**
PYRROLOBENZODIAZEPINE UND KONJUGATE DAVON
PYRROLOBENZODIAZÉPINES ET LEURS CONJUGUÉS

(30) Priority: 13.03.2013 US 201361778752 P
(43) Date of publication of application: 20.01.2016
(73) Proprietor: MEDIMMUNE LIMITED, Cambridge Cambridgeshire CB21 6GH (GB)
(72) Inventor: HOWARD, Philip Wilson, London Greater London E1 2AX (GB)
(74) Representative: Watson, Robert James
(86) International application number: PCT/EP2014/054958
(87) International publication number: WO 2014/140174

(56) References cited:
- WO-A1-2007/085930
- WO-A1-2011/130598
- WO-A1-2011/130613
- WO-A1-2011/130616
- IIDA, HIROKAZU ET AL: "Design and synthesis of pyrrolo[2,1-c][1,4]benzodiazepine (PBD)-polyaminoalkyl conjugates by the use of SNAr reaction of 2-nitro-5-fluorobenzoate precursor as key reaction", HETEROCYCLES , 62, 693-711 CODEN: HTCYAM; ISSN: 0385-5414, 2004, XP008170094,
- MATSUMOTO, KIYOSHI ET AL: "Synthesis of polyaminoalkyl substituted conjugates of pyrrolo[2,1-c][1,4]benzodiazepine involving SNAr reaction of 2-nitro-5-fluorobenzoate precursors", HETEROCYCLES , 52(3), 1015-1020 CODEN: HTCYAM; ISSN: 0385-5414, 2000, XP008170093, DOI: 10.3987/COM-99-S116 10.3987/COM-99-S116
- JOHN A. FLYGARE ET AL: "Antibody-Drug Conjugates for the Treatment of Cancer", CHEMICAL BIOLOGY & DRUG DESIGN, vol. 81, no. 1, 17 January 2013 (2013-01-17), pages 113-121, XP055121985, ISSN: 1747-0277, DOI: 10.1111/cbdd.12085
- John A Hartley ET AL: "Abstract 2856: Pyrrolobenzodiazepine (PBD) dimers - potent next generation warheads in antibody drug conjugates (ADCs) targeted at both solid and haematological tumors. -- Hartley et al. 73 (1008): 2856 -- Cancer Research", Cancer Research: April 15, 2013; Volume 73, Issue 8, Supplement 1, 6 April 2013 (2013-04-06), XP055124634, Proceedings: AACR 104th Annual Meeting 2013; Apr 6-10, 2013; Washington, DC Retrieved from the Internet: URL:http://cancerres.aacrjournals.org/cgi/ content/meeting_abstract/73/8_MeetingAbstr acts/2856?sid=bc4882e4-e536-4b59-b7d7-ed2a dab7fe74 [retrieved on 2014-06-23]
- SCOTT C. JEFFREY ET AL: "A Potent Anti-CD70 Antibody-Drug Conjugate Combining a Dimeric Pyrrolobenzodiazepine Drug with Site-Specific Conjugation Technology", BIOCONJUGATE CHEMISTRY, vol. 24, no. 7, 17 July 2013 (2013-07-17), pages 1256-1263, XP055124636, ISSN: 1043-1802, DOI: 10.1021/bc400217g

## Description

The present invention relates to pyrrolobenzodiazepines (PBDs), in particular pyrrolobenzodiazepines having a linker group connected to a cell binding agent as defined in the claims.

### Background to the invention

### Pyrrolobenzodiazepines

Some pyrrolobenzodiazepines (PBDs) have the ability to recognise and bond to specific sequences of DNA; the preferred sequence is PuGPu. The first PBD antitumour antibiotic, anthramycin, was discovered in 1965 (Leimgruber, et al., J. Am. Chem. Soc., 87, 5793-5795 (1965); Leimgruber, et al., J. Am. Chem. Soc., 87, 5791-5793 (1965)). Since then, a number of naturally occurring PBDs have been reported, and over 10 synthetic routes have been developed to a variety of analogues (Thurston, et al., Chem. Rev. 1994, 433-465 (1994); Antonow, D. and Thurston, D.E., Chem. Rev. 2011 111 (4), 2815-2864). Family members include abbeymycin (Hochlowski, et al., J. Antibiotics, 40, 145-148 (1987)), chicamycin (Konishi, et al., J. Antibiotics, 37, 200-206 (1984)), DC-81 (Japanese Patent 58-180 487; Thurston, et al., Chem. Brit., 26, 767-772 (1990); Bose, et al., Tetrahedron, 48, 751-758 (1992)), mazethramycin (Kuminoto, et al., J. Antibiotics, 33, 665-667 (1980)), neothramycins A and B (Takeuchi, et al., J. Antibiotics, 29, 93-96 (1976)), porothramycin (Tsunakawa, et al., J. Antibiotics, 41, 1366-1373 (1988)), prothracarcin (Shimizu, et al, J. Antibiotics, 29, 2492-2503 (1982); Langley and Thurston, J. Org. Chem., 52, 91-97 (1987)), sibanomicin (DC-102)(Hara, et al., J. Antibiotics, 41, 702-704 (1988); Itoh, et al., J. Antibiotics, 41, 1281-1284 (1988)), sibiromycin (Leber, et al., J. Am. Chem. Soc., 110, 2992-2993 (1988)) and tomamycin (Arima, et al., J. Antibiotics, 25, 437-444 (1972)). PBDs are of the general structure:

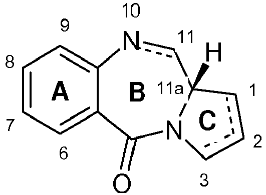

They differ in the number, type and position of substituents, in both their aromatic A rings and pyrrolo C rings, and in the degree of saturation of the C ring. In the B-ring there is either an imine (N=C), a carbinolamine(NH-CH(OH)), or a carbinolamine methyl ether (NH-CH(OMe)) at the N10-C11 position which is the electrophilic centre responsible for alkylating DNA. All of the known natural products have an (S)-configuration at the chiral C11a position which provides them with a right-handed twist when viewed from the C ring towards the A ring. This gives them the appropriate three-dimensional shape for isohelicity with the minor groove of B-form DNA, leading to a snug fit at the binding site (Kohn, In Antibiotics III. Springer-Verlag, New York, pp. 3-11 (1975); Hurley and Needham-VanDevanter, Acc. Chem. Res., 19, 230-237 (1986)). Their ability to form an adduct in the minor groove, enables them to interfere with DNA processing, hence their use as antitumour agents.

A particularly advantageous pyrrolobenzodiazepine compound is described by Gregson et al. (Chem. Commun. 1999, 797-798) as compound 1, and by Gregson et al. (J. Med. Chem. 2001, 44, 1161-1174) as compound **4a**. This compound, also known as SJG-136, is shown below:

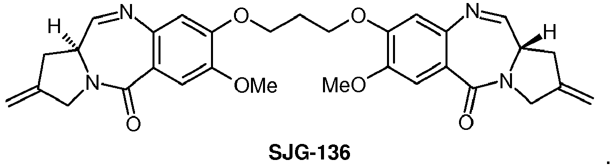

Other dimeric PBD compounds, such as those bearing C2 aryl substituents in WO 2005/085251, have been disclosed, an example being:

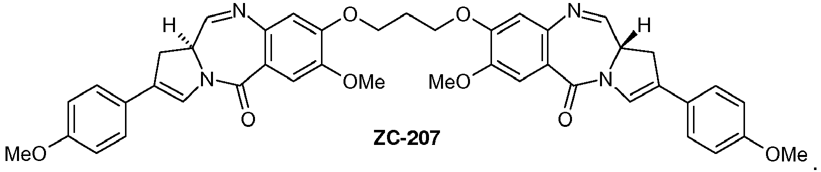

These compounds have been shown to be highly useful cytotoxic agents.

### Antibody-drug conjugates

Antibody therapy has been established for the targeted treatment of patients with cancer, immunological and angiogenic disorders (Carter, P. (2006) Nature Reviews Immunology 6:343-357). The use of antibody-drug conjugates (ADC), i.e. immunoconjugates, for the local delivery of cytotoxic or cytostatic agents, i.e. drugs to kill or inhibit tumor cells in the treatment of cancer, targets delivery of the drug moiety to tumors, and intracellular accumulation therein, whereas systemic administration of these unconjugated drug agents may result in unacceptable levels of toxicity to normal cells as well as the tumor cells sought to be eliminated (Xie et al (2006) Expert. Opin. Biol. Ther. 6(3):281-291; Kovtun et al (2006) Cancer Res. 66(6):3214-3121; Law et al (2006) Cancer Res. 66(4):2328-2337; Wu et al (2005) Nature Biotech. 23(9):1137-1145; Lambert J. (2005) Current Opin. in Pharmacol. 5:543-549; Hamann P. (2005) Expert Opin. Ther. Patents 15(9):1087-1103; Payne, G. (2003) Cancer Cell 3:207-212; Trail et al (2003) Cancer Immunol. Immunother. 52:328-337; Syrigos and Epenetos (1999) Anticancer Research 19:605-614).

Maximal efficacy with minimal toxicity is sought thereby. Efforts to design and refine ADC have focused on the selectivity of monoclonal antibodies (mAbs) as well as drug mechanism of action, drug-linking, drug/antibody ratio (loading), and drug-releasing properties (Junutula, et al., 2008b Nature Biotech., 26(8):925-932; Dornan et al (2009) Blood 114(13):2721-2729; US 7521541; US 7723485; WO2009/052249; McDonagh (2006) Protein Eng. Design & Sel. 19(7): 299-307; Doronina et al (2006) Bioconj. Chem. 17:114-124; Erickson et al (2006) Cancer Res. 66(8):1-8; Sanderson et al (2005) Clin. Cancer Res. 11:843-852; Jeffrey et al (2005) J. Med. Chem. 48:1344-1358; Hamblett et al (2004) Clin. Cancer Res. 10:7063-7070). Drug moieties may impart their cytotoxic and cytostatic effects by mechanisms including tubulin binding, DNA binding, or topoisomerase inhibition. Some cytotoxic drugs tend to be inactive or less active when conjugated to large antibodies or protein receptor ligands.

### PBDs in ADCs

Dimeric PBDs have been disclosed as the drugs in drug conjugates. For example, in WO 2011/130598, dimer PBD compounds having linker groups for connection to a cell binding agent, such as an antibody, are disclosed where the linker group is attached to one of the available N10 positions, and are generally cleaved by action of an enzyme on the linker group.

By contrast, in WO 2011/130613 and WO 2011/130616, dimer PBD compounds having linker groups for connection to a cell binding agent, such as an antibody, are disclosed where the linker group is attached via an aromatic group at one of the C2 postions, and are generally cleaved by action of an enzyme on the linker group. Such antibody drug conjugates are also described in Flyagre, J., et al, Chem. Biol. Drug Des. 81: 113-121 (2013), which also describes other types of antibody drug conjugates.

A further approach is described in WO 2007/085930, wherein tomamycin-like dimers have a linker group for connection to a cell binding agent, such as an antibody, where the linker group is attached to the tether between the tomamycin units, and are generally cleaved by action of an enzyme on the linker group.

The present inventors have developed a novel approach to forming PBD conjugates with cell binding agents, and in particular PBD antibody conjugates.

### Summary of the Invention

The invention is defined by the claims. Any subject-matter falling outside the scope of the claims is provided for information purposes only. In a general aspect the present invention provides a conjugate comprising a PBD compound with a linker for connecting to a cell binding agent, wherein the linker is attached in a non-cleavable manner to the C7 position of the one PBD units as described in the claims. The cell binding agent is preferably an antibody. The invention also provides the PBD compound with the linking unit attached, and intermediates for their synthesis.

In a first aspect, the present invention provides a conjugate of formula A: 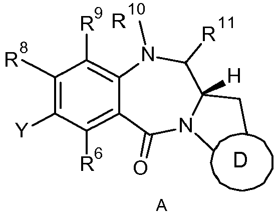 and salts and solvates thereof, wherein:
D represents either group D1 or D2: 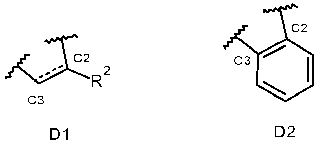 the dotted line indicates the optional presence of a double bond between C2 and C3; when there is a double bond present between C2 and C3, R² is selected from the group consisting of:
   (ia) C₅₋₁₀ aryl group, optionally substituted by one or more substituents selected from the group comprising: halo, nitro, cyano, ether, carboxy, ester, C₁₋₇ alkyl, C₃₋₇ heterocyclyl and bis-oxy-C₁₋₃ alkylene;
   (ib) C₁₋₅ saturated aliphatic alkyl;
   (ic) C₃₋₆ saturated cycloalkyl;
   (id) 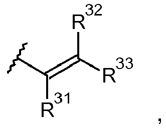 wherein each of R³¹, R³² and R³³ are independently selected from H, C₁₋₃ saturated alkyl, C₂₋₃ alkenyl, C₂₋₃ alkynyl and cyclopropyl, where the total number of carbon atoms in the R² group is no more than 5;
   (ie) 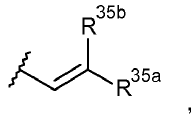 wherein one of R^{35a} and R^{35b} is H and the other is selected from: phenyl, which phenyl is optionally substituted by a group selected from halo, methyl, methoxy; pyridyl; and thiophenyl; and
   (if) 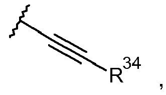 where R³⁴ is selected from: H; C₁₋₃ saturated alkyl; C₂₋₃ alkenyl; C₂₋₃ alkynyl; cyclopropyl; phenyl, which phenyl is optionally substituted by a group selected from halo, methyl, methoxy; pyridyl; and thiophenyl;
   (ig) halo;
      when there is a single bond present between C2 and C3,
R² is 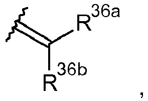 where R^{36a} and R^{36b} are independently selected from H, F, C₁₋₄ saturated alkyl, C₂₋₃ alkenyl, which alkyl and alkenyl groups are optionally substituted by a group selected from C₁₋₄ alkyl amido and C₁₋₄ alkyl ester; or, when one of R^{16a} and R^{16b} is H, the other is selected from nitrile and a C₁₋₄ alkyl ester;
R⁶ and R⁹ are independently selected from H, R, OH, OR, SH, SR, NH₂, NHR, NRR', NO₂, SnMe₃ and halo;
either
   (a) R¹⁰ is H, and R¹¹ is OH or OR^{A}, where R^{A} is C₁₋₄ alkyl; or
   (b) R¹⁰ and R¹¹ form a nitrogen-carbon double bond between the nitrogen and carbon atoms to which they are bound; or
   (c) R¹⁰ is H and R¹¹ is OSO_{z}M, where z is 2 or 3 and M is a monovalent pharmaceutically acceptable cation; or
   (d) R¹¹ is OH or OR^{A}, where R^{A} is C₁₋₄ alkyl and R¹⁰ is selected from:
      (d-i) 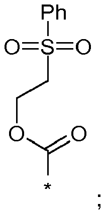
      (d-ii) 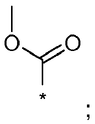
      (d-iii) 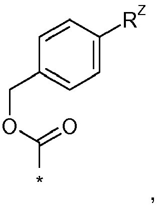
      where R^{Z} is selected from:
      (z-i) 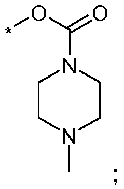
      (z-ii) OC(=O)CH₃;
      (z-iii) NO₂;
      (z-iv) OMe;
      (z-v) glucoronide;
      (z-vi) -C(=O)-X₁-NHC(=O)X₂-NH-R^{ZC}, where -C(=O)-X₁-NH- and-C(=O)-X₂-NH- represent natural amino acid residues and R^{ZC} is selected from Me, OMe, OCH₂CH₂OMe;
Y is selected from formulae A1 and A2 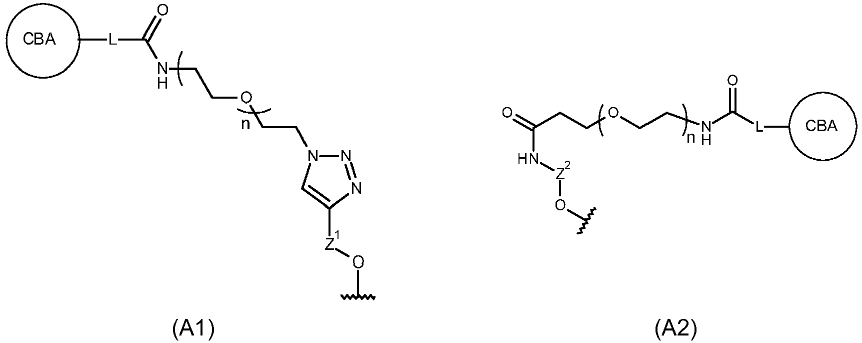
   Z¹ is a C₁₋₃ alkylene group;
   Z² is a C₁₋₃ alkylene group;
   L is a linker connected to a cell binding agent;
   CBA is the cell binding agent;
   n is an integer between 0 and 48;
R and R' are each independently selected from optionally substituted C₁₋₁₂ alkyl, C₃₋₂₀ heterocyclyl and C₅₋₂₀ aryl groups, and optionally in relation to the group NRR', R and R' together with the nitrogen atom to which they are attached form an optionally substituted 4-, 5-, 6- or 7-membered heterocyclic ring;
R⁸ is either:
   (a) independently selected from H, R, OH, OR, SH, SR, NH₂, NHR, NRR', NO₂, SnMe₃ and halo; or
   (b) of formula A*: 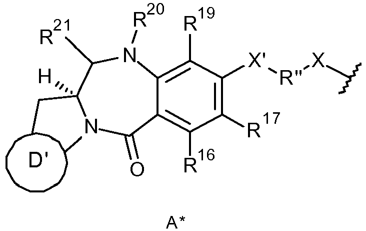 wherein:
      D' represents either group D'1 or D2: 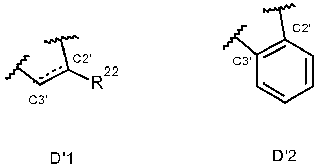 wherein the dotted line indicates the optional presence of a double bond between C2' and C3';
      R¹⁷ is independently selected from H, R, OH, OR, SH, SR, NH₂, NHR, NRR', NO₂, SnMe₃ and halo;
      R" is a C₃₋₁₂ alkylene group, which chain may be interrupted by one or more heteroatoms, e.g. O, S, N(H), NMe and/or aromatic rings, e.g. benzene or pyridine, which rings are optionally substituted; and
      X and X' are independently selected from O, S and N(H); and
      R²², R¹⁶, R¹⁹, R²⁰ and R²¹ are as defined for R², R⁶, R⁹, R¹⁰ and R¹¹ respectively.

Thus formula A is selected from the following formulae A-I, A-II and A-III, depending on Y:

| Y | A |
|---|---|
| (A1) | 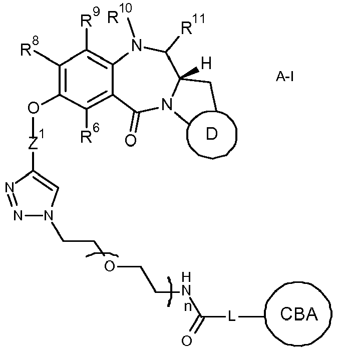 |
| (A2) | 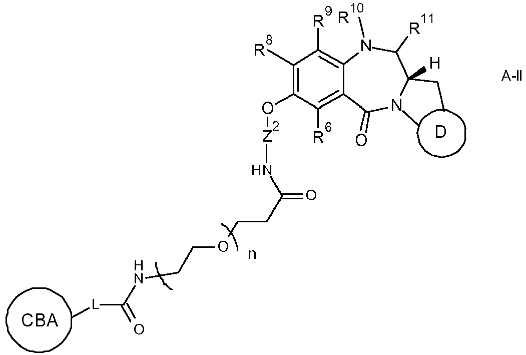 |
| (A3) | 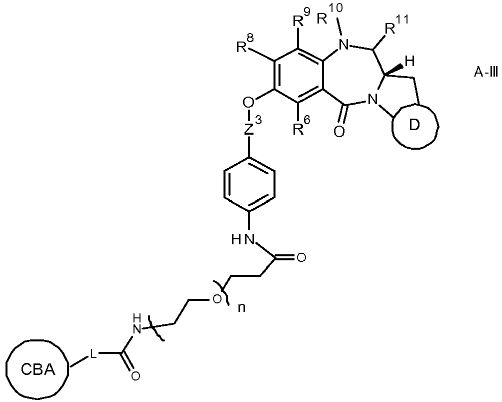 |

When R⁸ is A*, the compound is of the formula A*A:

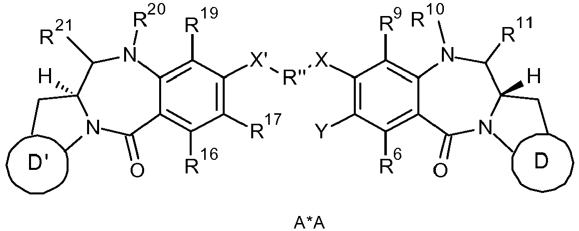

A second aspect of the present invention provides novel drug-linker compounds of formula (B): 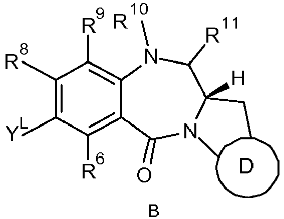 and salts and solvates thereof, wherein:
Y^{L} is selected from formulae B1 and B2: 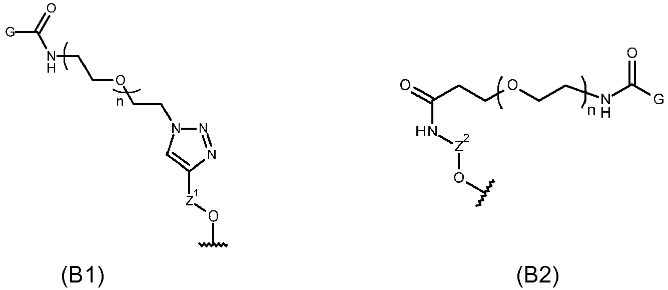
G is a linker for connecting to a cell binding agent; and
the remaining groups are as defined in the first aspect.

A third aspect of the present invention also provides compounds of formula (C), which may be used in the preparation of the drug-linkers and conjugates of the invention: 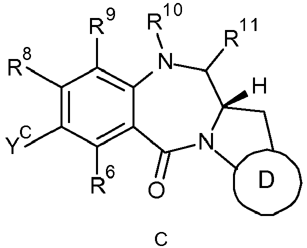 and salts and solvates thereof,
Y^{C} is selected from from formulae C1 and C2 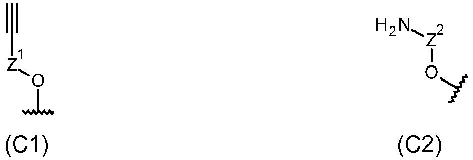 and the remaining groups are as defined in the first aspect.

A fourth aspect of the present invention provides the compound of the first aspect of the invention for use in a method of medical treatment. The fourth aspect also provides a pharmaceutical composition comprising a compound of the first aspect, and a pharmaceutically acceptable excipient.

A fifth aspect of the present invention provides a compound of the first aspect of the invention or a pharmaceutical composition of the fourth aspect of the invention for use in a method of treatment of a proliferative disease. The fifth aspect also provides the use of a compound of the first aspect in a method of manufacture of a medicament for the treatment of a proliferative disease, and a method of treating a mammal having a proliferative disease, comprising administering an effective amount of a compound of the first aspect or a pharmaceutical composition of the fourth aspect.

A sixth aspect of the present invention provides a method of synthesis of a compound of the first aspect of the present invention, comprising the step of conjugating a drug-linker of the second aspect with a cell-binding agent.

A seventh aspect of the present invention provides a method of synthesis of a drug-linke of the second aspect, comprising the step of reacting a compound of the third aspect with one or more suitable reagents.

### Detailed Description of the Invention

### Preferences

The following preferences may apply to all aspects of the invention as described above, or may relate to a single aspect. The preferences may be combined together in any combination.

### D

In some embodiments, D is D1.

In some embodiments, D is D2.

### R⁸

In some embodiments, R⁸ may be independently selected from H, OH, OR, SH, SR, NH₂, NHR, NRR', and halo.

In some embodiments, R⁸ may be independently selected from H, OH and OR, where R may be selected from optionally substituted C₁₋₇ alkyl, C₃₋₁₀ heterocyclyl and C₅₋₁₀ aryl groups. R in R⁸ may in some of these embodiments be a C₁₋₄ alkyl group, which may or may not be substituted. A substituent of interest is a C₅₋₆ aryl group (e.g. phenyl).

In some embodiments, R⁸ is selected from OMe and OCH₂Ph.

In some embodiments, R⁸ is of formula A*, such that the compound is a PBD dimer.

### Dimer link

X and X' are preferably O.

R" is a C₃₋₁₂ alkylene group, which chain may be interrupted by one or more heteroatoms, e.g. O, S, N(H), NMe and/or aromatic rings, e.g. benzene or pyridine, which rings are optionally substituted.

In some embodiments, R" may be C₃₋₁₂ alkylene group, which chain may be interrupted by one or more heteroatoms and/or aromatic rings, e.g. benzene or pyridine.
In some embodiments, R" may be C₃₋₁₂ alkylene group which is optionally interrupted by one or more heteroatoms selected from O, S, and NMe and/or aromatic rings, which rings are optionally substituted.
In some embodiments, the aromatic ring is a C₅₋₂₀ arylene group, where arylene pertains to a divalent moiety obtained by removing two hydrogen atoms from two aromatic ring atoms of an aromatic compound, which moiety has from 5 to 20 ring atoms.
In some embodiments, R" may be a C₃₋₁₂ alkylene group, which chain may be interrupted by one or more heteroatoms, e.g. O, S, N(H), NMe and/or aromatic rings, e.g. benzene or pyridine, which rings are optionally substituted by NH₂.
In some embodiments, R" may be C₃₋₁₂ alkylene group.
In some embodiments, R" may be selected from a C₃, C₅, C₇, C₉ and a C₁₁ alkylene group.
In some embodiments, R" may be selected from a C₃, C₅ and a C₇ alkylene group.
In some embodiments, R" may be selected from a C₃ and a C₅ alkylene group.
In some embodiments, R" is a C₃ alkylene group.
In some embodiments, R" is a C₅ alkylene group.
The alkylene groups listed above may be optionally interrupted by one or more heteroatoms and/or aromatic rings, e.g. benzene or pyridine, which rings are optionally substituted.
The alkylene groups listed above may be optionally interrupted by one or more heteroatoms and/or aromatic rings, e.g. benzene or pyridine.
The alkylene groups listed above may be unsubstituted linear aliphatic alkylene groups.
R" is preferably a C₃₋₇ alkylene group with no substituents. More preferably R" is a C₃, C₅ or C₇ alkylene. Most preferably, R" is a C₃ or C₅ alkylene.

### R⁶

In some embodiments, R⁶ may be independently selected from H, R, OH, OR, SH, SR, NH₂, NHR, NRR', NO₂, SnMe₃ and halo.

In some embodiments, R⁶ may be independently selected from H, OH, OR, SH, NH₂, NO₂ and halo.

In some embodiments, R⁶ is independently selected from H and halo.

In some embodiments, R⁶ is independently H.

These embodiments also apply to R¹⁶.

### R⁹

In some embodiments, R⁹ may be independently selected from H, R, OH, OR, SH, SR, NH₂, NHR, NRR', NO₂, SnMe₃ and halo.

In some embodiments, R⁹ is independently H.

These embodiments also apply to R¹⁹.

### R¹⁷

In some embodiments, R¹⁷ may be independently selected from H, OH, OR, SH, SR, NH₂, NHR, NRR', and halo.

In some embodiments, R¹⁷ may be independently selected from H, OH and OR, where R may be selected from optionally substituted C₁₋₇ alkyl, C₃₋₁₀ heterocyclyl and C₅₋₁₀ aryl groups. R in R¹⁷ may in some of these embodiments be a C₁₋₄ alkyl group, which may or may not be substituted. A substituent of interest is a C₅₋₆ aryl group (e.g. phenyl).

In some embodiments, R¹⁷ is selected from OMe and OCH₂Ph.

### R²

When R² is a C₅₋₁₀ aryl group, in some embodiments it may be a C₅₋₇ aryl group. A C₅₋₇ aryl group may be a phenyl group or a C₅₋₇ heteroaryl group, for example furanyl, thiophenyl and pyridyl. In some embodiments, R² may be phenyl. In other embodiments, R² may be thiophenyl, for example, thiophen-2-yl and thiophen-3-yl.

When R² is a C₅₋₁₀ aryl group, it some embodiments it may be a C₈₋₁₀ aryl, for example a quinolinyl or isoquinolinyl group. The quinolinyl or isoquinolinyl group may be bound to the PBD core through any available ring position. For example, the quinolinyl may be quinolin-2-yl, quinolin-3-yl, quinolin-4yl, quinolin-5-yl, quinolin-6-yl, quinolin-7-yl and quinolin-8-yl. Of these quinolin-3-yl and quinolin-6-yl may be preferred. The isoquinolinyl may be isoquinolin-1-yl, isoquinolin-3-yl, isoquinolin-4yl, isoquinolin-5-yl, isoquinolin-6-yl, isoquinolin-7-yl and isoquinolin-8-yl. Of these isoquinolin-3-yl and isoquinolin-6-yl may be preferred.

When R² is a C₅₋₁₀ aryl group, it may bear any number of substituent groups. In some embodiments, it may bear from 1 to 3 substituent groups. In some embodiments, it may bear 1 or 2 substituent groups. In some embodiments, it may bear a single substituent group. The substituents may be any position.

Where R² is C₅₋₇ aryl group, in some embodiments a single substituent may be on a ring atom that is not adjacent the bond to the remainder of the compound, i.e. it may be β or γ to the bond to the remainder of the compound. Therefore, in embodiments where the C₅₋₇ aryl group is phenyl, the substituent may be in the meta- or para- positions, or may be in the para- position.

Where R² is a C₈₋₁₀ aryl group, for example quinolinyl or isoquinolinyl, in some embodiments there may be any number of substituents at any position of the quinoline or isoquinoline rings. In some embodiments, it bears one, two or three substituents, and these may be on either the proximal and distal rings or both (if more than one substituent).

*R² substituents, when R² is a C₅₋₁₀ aryl group*
In embodiments where a substituent on R² when R² is a C₅₋₁₀ aryl group is halo, it may be F or Cl, and in some of these embodiments Cl.

In embodiments where a substituent on R² when R² is a C₅₋₁₀ aryl group is ether, it may in some embodiments be an alkoxy group, for example, a C₁₋₇ alkoxy group (e.g. methoxy, ethoxy) or it may in some embodiments be a C₅₋₇ aryloxy group (e.g phenoxy, pyridyloxy, furanyloxy). The alkoxy group may itself be further substituted, for example by an amino group (e.g. dimethylamino).

In embodiments where a substituent on R² when R² is is a C₅₋₁₀ aryl group is C₁₋₇ alkyl, it may be a C₁₋₄ alkyl group (e.g. methyl, ethyl, propryl, butyl).

In embodiments where a substituent on R² when R² is a C₅₋₁₀ aryl group is C₃₋₇ heterocyclyl, it may be C₆ nitrogen containing heterocyclyl group, e.g. morpholino, thiomorpholino, piperidinyl, piperazinyl. These groups may be bound to the rest of the PBD moiety via the nitrogen atom. These groups may be further substituted, for example, by C₁₋₄ alkyl groups. If the C₆ nitrogen containing heterocyclyl group is piperazinyl, the said further substituent may be on the second nitrogen ring atom.

In embodiments where a substituent on R² when R² is a C₅₋₁₀ aryl group is bis-oxy-C₁₋₃ alkylene, this may be bis-oxy-methylene or bis-oxy-ethylene.

In embodiments where a substituent on R² when R² is a C₅₋₁₀ aryl group is ester, this is preferably methyl ester or ethyl ester.

In some embodiments, substituents when R² is a C₅₋₁₀ aryl group may include methoxy, ethoxy, fluoro, chloro, cyano, bis-oxy-methylene, methyl-piperazinyl, morpholino, methylthiophenyl, dimethylaminopropyloxy and carboxy.

In some embodiments, R² may be selected from 4-methoxy-phenyl, 3-methoxyphenyl, 4-ethoxy-phenyl, 3-ethoxy-phenyl, 4-fluoro-phenyl, 4-chloro-phenyl, 3,4-bisoxymethylene-phenyl, 4-methylthiophenyl, 4-cyanophenyl, 4-phenoxyphenyl, quinolin-3-yl and quinolin-6-yl, isoquinolin-3-yl and isoquinolin-6-yl, 2-thienyl, 2-furanyl, methoxynaphthyl, naphthyl, 4-nitrophenyl, 4-(4-methylpiperazin-1-yl)phenyl and 3,4-bisoxymethylene-phenyl.

When R² is C₁₋₅ saturated aliphatic alkyl, it may be methyl, ethyl, propyl, butyl or pentyl. In some embodiments, it may be methyl, ethyl or propyl (n-pentyl or isopropyl). In some of these embodiments, it may be methyl. In other embodiments, it may be butyl or pentyl, which may be linear or branched.

When R² is C₃₋₆ saturated cycloalkyl, it may be cyclopropyl, cyclobutyl, cyclopentyl or cyclohexyl. In some embodiments, it may be cyclopropyl.

When R² is 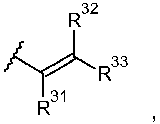 in some embodiments, the total number of carbon atoms in the R² group is no more than 4 or no more than 3.

In some embodiments, one of R³¹, R³² and R³³ is H, with the other two groups being selected from H, C₁₋₃ saturated alkyl, C₂₋₃ alkenyl, C₂₋₃ alkynyl and cyclopropyl.

In other embodiments, two of R³¹, R³² and R³³ are H, with the other group being selected from H, C₁₋₃ saturated alkyl, C₂₋₃ alkenyl, C₂₋₃ alkynyl and cyclopropyl.

In some embodiments, the groups that are not H are selected from methyl and ethyl. In some of these embodiments, the groups that are not H are methyl.

In some embodiments, R³¹ is H.

In some embodiments, R³² is H.

In some embodiments, R³³ is H.

In some embodiments, R³¹ and R³² are H.

In some embodiments, R³¹ and R³³ are H.

In some embodiments, R³² and R³³ are H.

A R² group of particular interest is:

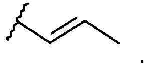

When R² is 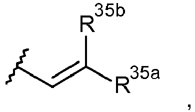 in some embodiments, the group (R^{35a} or R^{35b}) which is not H is optionally substituted phenyl. If the phenyl optional substituent is halo, it may be fluoro. In some embodiment, the phenyl group is unsubstituted.

When R² is 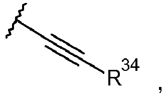 in some embodiments where R³⁴ is phenyl, it is unsubstituted. In other embodiments, the phenyl group bears a single fluoro substituent. In other emboidments, R¹⁴ is selected from H, methyl, ethyl, ethenyl and ethynyl. In some of these embodiments, R¹⁴ is selected from H and methyl.

When R² is halo, in some embodiments, it is fluoro.

When there is a single bond present between C2 and C3, R² is

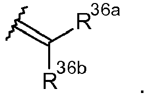

In some embodiments, R^{36a} and R^{36b} are both H.

In other embodiments, R^{36a} and R^{36b} are both methyl.

In further embodiments, one of R^{36a} and R^{36b} is H, and the other is selected from C₁₋₄ saturated alkyl, C₂₋₃ alkenyl, which alkyl and alkenyl groups are optionally substituted. In some of these further embodiment, the group which is not H may be selected from methyl and ethyl.

### R²²

The above preferences for R² when there is a double bond present between C2 and C3 apply equally to R²², when there is a double bond present between C2' and C3'.

The above preferences for R² when there is a single bond present between C2 and C3 apply equally to R²², when there is a single bond present between C2' and C3'.

### N10-C11

In some embodiment, R¹⁰ is H, and R¹¹ is OH, OR^{A}, where R^{A} is C₁₋₄ alkyl. In some of these embodiments, R¹¹ is OH. In others of these embodiments, R11 is OR^{A}, where R^{A} is C₁₋₄ alkyl. In some of these embodiments, R^{A} is methyl.

In some embodiments, R¹⁰ and R¹¹ form a nitrogen-carbon double bond between the nitrogen and carbon atoms to which they are bound.

In some embodiments, R¹⁰ is H and R¹¹ is OSO_{z}M, where z is 2 or 3 and M is a monovalent pharmaceutically acceptable cation. In some of these embodiments, M is a monovalent pharmaceutically acceptable cation, and may be Na⁺. Furthermore, in some embodiments z is 3.

In some embodiments where R¹⁰ is (d-iii), there may be an additional ntiro group on the benzene ring, e.g. ortho to R^{Z}.

In some embodiments, R¹¹ is OH or OR^{A}, where R^{A} is C₁₋₄ alkyl and R¹⁰ is selected from:

| | |
|---|---|
| R^{10a} | 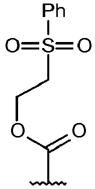 |
| R^{10b} | 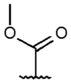 |
| R^{10c} | 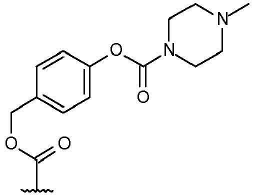 |
| R^{10d} | 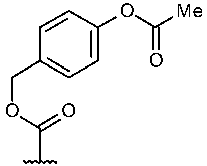 |
| R^{10e} | 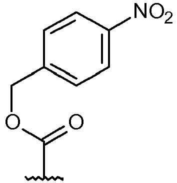 |
| R^{10f} | 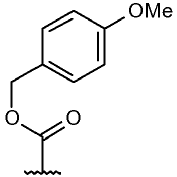 |
| R^{10g} | 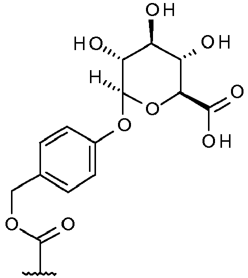 |
| R^{10h} | 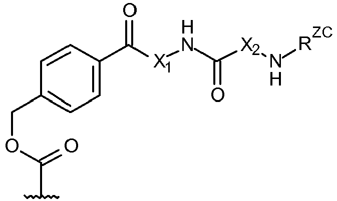 |

-C(=O)-X₁-NHC(=O)X₂-NH- represent a dipeptide. The amino acids in the dipeptide may be any combination of natural amino acids. The dipeptide may be the site of action for cathepsin-mediated cleavage.

In one embodiment, the dipeptide, -C(=O)-X₁-NHC(=O)X₂-NH-, is selected from:
-Phe-Lys-,
-Val-Ala-,
-Val-Lys-,
-Ala-Lys-,
-Val-Cit-,
-Phe-Cit-,
-Leu-Cit-,
-Ile-Cit-,
-Phe-Arg-,
-Trp-Cit-
where Cit is citrulline.

Preferably, the dipeptide, -C(=O)-X₁-NHC(=O)X₂-NH-, is selected from:
-Phe-Lys-,
-Val-Ala-,
-Val-Lys-,
-Ala-Lys-,
-Val-Cit-.

Most preferably, the dipeptide, -C(=O)-X₁-NHC(=O)X₂-NH-, is -Phe-Lys- or -Val-Ala-.

Other dipeptide combinations may be used, including those described by Dubowchik et al., Bioconjugate Chemistry, 2002, 13,855-869.

In one embodiment, the amino acid side chain is derivatised, where appropriate. For example, an amino group or carboxy group of an amino acid side chain may be derivatised. In one embodiment, an amino group NH₂ of a side chain amino acid, such as lysine, is a derivatised form selected from the group consisting of NHR and NRR'.

In one embodiment, a carboxy group COOH of a side chain amino acid, such as aspartic acid, is a derivatised form selected from the group consisting of COOR, CONH₂, CONHR and CONRR'.

In one embodiment, the amino acid side chain is chemically protected, where appropriate. The side chain protecting group may be a group as discussed above. The present inventors have established that protected amino acid sequences are cleavable by enzymes. For example, it has been established that a dipeptide sequence comprising a Boc side chain-protected Lys residue is cleavable by cathepsin.

Protecting groups for the side chains of amino acids are well known in the art and are described in the Novabiochem Catalog. Additional protecting group strategies are set out in Protective Groups in Organic Synthesis, Greene and Wuts.

Possible side chain protecting groups are shown below for those amino acids having reactive side chain functionality:
Arg: Z, Mtr, Tos;
Asn: Trt, Xan;
Asp: Bzl, t-Bu;
Cys: Acm, Bzl, Bzl-OMe, Bzl-Me, Trt;
Glu: Bzl, t-Bu;
Gln: Trt, Xan;
His: Boc, Dnp, Tos, Trt;
Lys: Boc, Z-Cl, Fmoc, Z, Alloc;
Ser: Bzl, TBDMS, TBDPS;
Thr: Bz;
Trp: Boc;
Tyr: Bzl, Z, Z-Br.

In one embodiment, the side chain protection is selected to be orthogonal to a group provided as, or as part of, a capping group, where present. Thus, the removal of the side chain protecting group does not remove the capping group, or any protecting group functionality that is part of the capping group.

In other embodiments of the invention, the amino acids selected are those having no reactive side chain functionality. For example, the amino acids may be selected from: Ala, Gly, Ile, Leu, Met, Phe, Pro, and Val.

It is particularly preferred in the present invention, that if L¹ comprises a dipeptide, then -C(=O)-X₁-NHC(=O)X₂-NH- is the same dipeptide.

Other preferred R¹⁰ groups include:

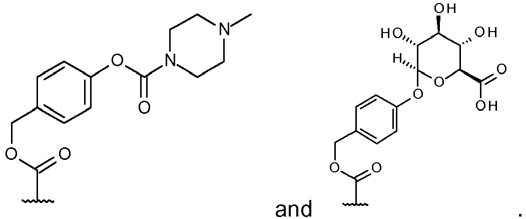

The above preferences apply equally to R²⁰ and R²¹.

### R and R'

In some embodiments, R is independently selected from optionally substituted C₁₋₁₂ alkyl, C₃₋₂₀ heterocyclyl and C₅₋₂₀ aryl groups. These groups are each defined in the substituents section below.

In some embodiments, R is independently optionally substituted C₁₋₁₂ alkyl. In other embodiments, R is independently optionally substituted C₃₋₂₀ heterocyclyl. In further embodiments, R is independently optionally substituted C₅₋₂₀ aryl. In further embodiments, R is independently optionally substituted C₁₋₁₂ alkyl.

Described above in relation to R² are various embodiments relating to preferred alkyl and aryl groups and the identity and number of optional substituents. The preferences set out for R² as it applies to R are applicable, where appropriate, to all other groups R.

The preferences for R apply also to R'.

In some embodiments of the invention there is provided a compound having a substituent group -NRR'. In one embodiment, R and R' together with the nitrogen atom to which they are attached form an optionally substituted 4-, 5-, 6- or 7-membered heterocyclic ring. The ring may contain a further heteroatom, for example N, O or S. In some of these embodiments, the heterocyclic ring is itself substituted with a group R. Where a further N heteroatom is present, the substituent may be on the N heteroatom.

### Dimers

In some embodiments, the groups X', D, R¹⁶, R¹⁹, R²⁰ and R²¹ are the same as the groups X, D', R⁶, R⁹, R¹⁰ and R¹¹ respectively. In these embodiments, the PBD monomer units have the same substituents except for at the 7 position.

Particularly preferred compounds of the first aspect of the present invention may be of formula Ia: 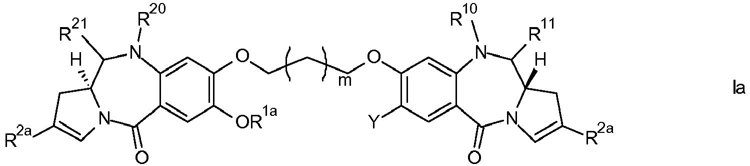 where
R¹⁰, R¹¹, R²⁰, R²¹ and Y are as defined above;
m is 1 or 3;
R^{1a} is methyl or phenyl; and
R^{2a} is selected from:
   (a) 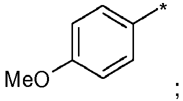
   (b) 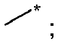
   (c) 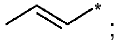
   (d) 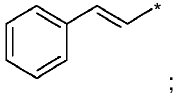
   (e) 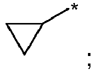
   (f) 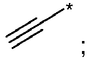
   (g) 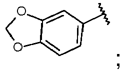 and
   (h)

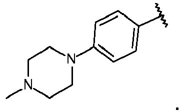

Particularly preferred compounds of the first aspect of the present invention may be of formula Ib: 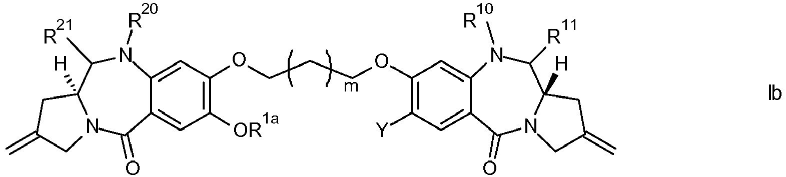 where
R¹⁰, R¹¹, R²⁰, R²¹ and Y are as defined above;
m is 1 or 3; and
R^{1a} is methyl or phenyl.

Particularly preferred compounds of the second aspect of the present invention may be of formula IIa: 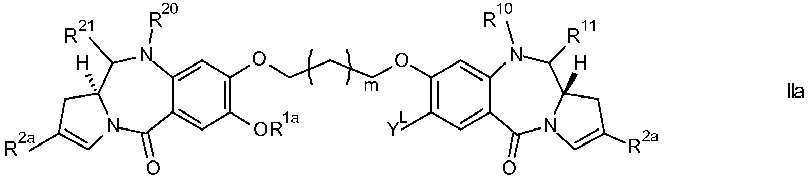 where
R¹⁰, R¹¹, R²⁰, R²¹ and Y^{L} are as defined above;
m is 1 or 3;
R^{1a} is methyl or phenyl; and
R^{2a} is selected from:
   (a) 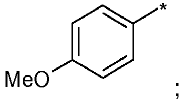
   (b) 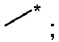
   (c) 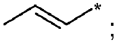
   (d) 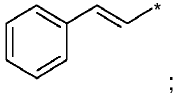
   (e) 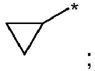
   (f) 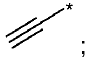
   (g) 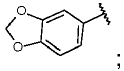 and
   (h)

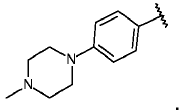

Particularly preferred compounds of the second aspect of the present invention may be of formula IIb: 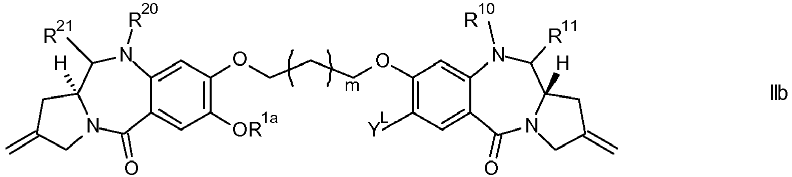 where
R¹⁰, R¹¹, R²⁰, R²¹ and Y^{L} are as defined above;
m is 1 or 3; and
R^{1a} is methyl or phenyl.

Particularly preferred compounds of the third aspect of the present invention may be of formula IIIa: 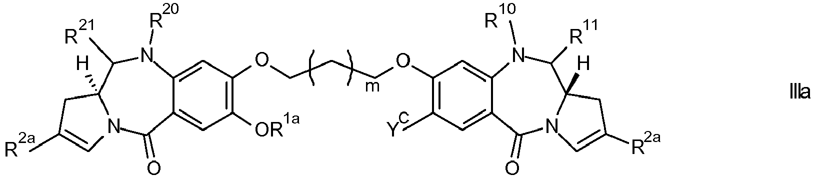 where
R¹⁰, R¹¹, R²⁰, R²¹ and Y^{C} are as defined above;
m is 1 or 3;
R^{1a} is methyl or phenyl; and
R^{2a} is selected from:
   (a) 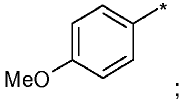
   (b) 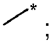
   (c) 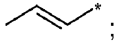
   (d) 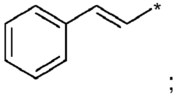
   (e) 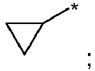
   (f) 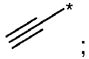
   (g) 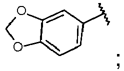 and
   (h)

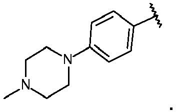

Particularly preferred compounds of the third aspect of the present invention may be of formula IIIb: 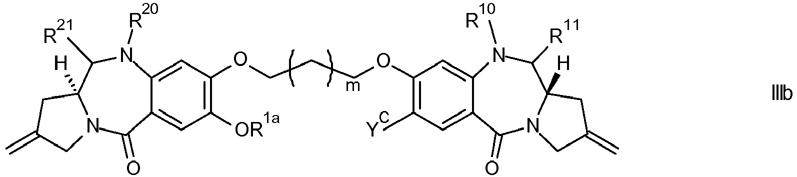 where
R¹⁰, R¹¹, R²⁰, R²¹ and Y^{C} are as defined above;
m is 1 or 3; and
R^{1a} is methyl or phenyl.

### Z¹, Z², Z³

In some embodiments, Z¹ is methylene. In some embodiments, Z¹ is ethylene. In some embodiments, Z¹ is propylene.

In some embodiments, Z² is methylene. In some embodiments, Z² is ethylene. In some embodiments, Z² is propylene.

In some embodiments, *Z*³ is methylene. In some embodiments, *Z*³ is ethylene. In some embodiments, *Z*³ is propylene.

### n(Y, Y^{L})

In some embodiments, n (in Y or Y^{L}) is an integer between 0 and 24.

In some embodiments, n (in Y or Y^{L}) is an integer between 0 and 12.

In some embodiments, n (in Y or Y^{L}) is an integer between 0 and 8.

In some embodiments, n (in Y or Y^{L}) is an integer between 0 and 6.

In some embodiments, n (in Y or Y^{L}) is 0.
In some embodiments, n (in Y or Y^{L}) is 1.
In some embodiments, n (in Y or Y^{L}) is 2.
In some embodiments, n (in Y or Y^{L}) is 3.
In some embodiments, n (in Y or Y^{L}) is 4.
In some embodiments, n (in Y or Y^{L}) is 5.
In some embodiments, n (in Y or Y^{L}) is 6.
In some embodiments, n (in Y or Y^{L}) is 7.
In some embodiments, n (in Y or Y^{L}) is 8.

In some embodiments, Z¹ is methylene and n is 3.
In some embodiments, Z² is propylene and n is 8.

### L and G

L is a linker connected to the cell binding agent in the conjugate conmpound. G is a linker for connecting the PBD dimer to the cell binding agent to form the conjugate compound.

Preferably, the linker contains an electrophilic functional group for reaction with a nucleophilic functional group on the cell binding agent. Nucleophilic groups on antibodies include, but are not limited to: (i) N-terminal amine groups, (ii) side chain amine groups, e.g. lysine, (iii) side chain thiol groups, e.g. cysteine, and (iv) sugar hydroxyl or amino groups where the antibody is glycosylated. Amine, thiol, and hydroxyl groups are nucleophilic and capable of reacting to form covalent bonds with electrophilic groups on linker moieties and linker reagents including: (i) maleimide groups (ii) activated disulfides, (iii) active esters such as NHS (N-hydroxysuccinimide) esters, HOBt (N-hydroxybenzotriazole) esters, haloformates, and acid halides; (iv) alkyl and benzyl halides such as haloacetamides; and (v) aldehydes, ketones, carboxyl, and, some of which are exemplified as follows:

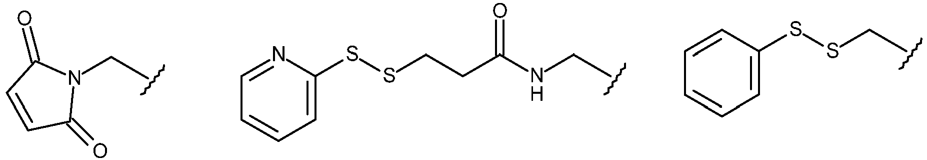

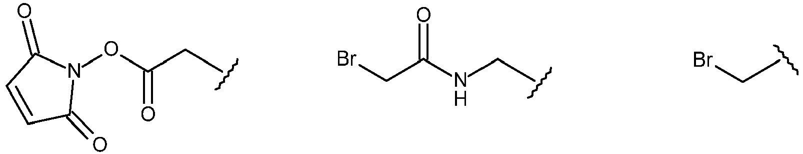

Certain antibodies have reducible interchain disulfides, i.e. cysteine bridges. Antibodies may be made reactive for conjugation with linker reagents by treatment with a reducing agent such as DTT (dithiothreitol). Each cysteine bridge will thus form, theoretically, two reactive thiol nucleophiles. Additional nucleophilic groups can be introduced into antibodies through the reaction of lysines with 2-iminothiolane (Traut's reagent) resulting in conversion of an amine into a thiol. Reactive thiol groups may be introduced into the antibody (or fragment thereof) by introducing one, two, three, four, or more cysteine residues (e.g., preparing mutant antibodies comprising one or more non-native cysteine amino acid residues). US 7521541 teaches engineering antibodies by introduction of reactive cysteine amino acids. In some embodiments, a Linker has a reactive nucleophilic group which is reactive with an electrophilic group present on an antibody. Useful electrophilic groups on an antibody include, but are not limited to, aldehyde and ketone carbonyl groups. The heteroatom of a nucleophilic group of a Linker can react with an electrophilic group on an antibody and form a covalent bond to an antibody unit. Useful nucleophilic groups on a Linker include, but are not limited to, hydrazide, oxime, amino, hydroxyl, hydrazine, thiosemicarbazone, hydrazine carboxylate, and arylhydrazide. The electrophilic group on an antibody provides a convenient site for attachment to a Linker.

In one embodiment, the group L is: 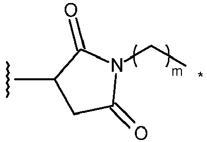 where the asterisk indicates the point of attachment to the rest of group Y, the wavy line indicates the point of attachment to the cell binding agent, and m is 0 to 6. In one embodiment, m is 5.

In one embodiment, the connection between the cell binding agent and L is through a thiol residue of the cell binding agent and a maleimide group of L.

In one embodiment, the connection between the cell binding agent and L is: 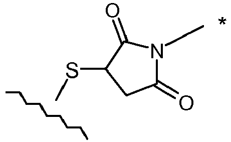 where the asterisk indicates the point of attachment to the remaining portion of the L group or the remaining portion of the Y group and the wavy line indicates the point of attachment to the remaining portion of the cell binding agent. In this embodiment, the S atom is typically derived from the cell binding agent.

In each of the embodiments above, an alternative functionality may be used in place of the maleimide-derived group shown below: 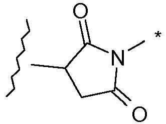 where the wavy line indicates the point of attachment to the cell binding agent as before, and the asterisk indicates the bond to the remaining portion of the L group or the remaining portion of the Y group.

In one embodiment, the maleimide-derived group is replaced with the group: 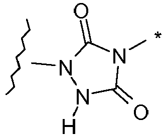 where the wavy line indicates point of attachment to the cell binding agent, and the asterisk indicates the bond to the remaining portion of the L group or the remaining portion of the Y group.

In one embodiment, the maleimide-derived group is replaced with a group, which optionally together with the cell binding agent, is selected from:
-C(=O)NH-,
-C(=O)O-,
-NHC(=O)-,
-OC(=O)-,
-OC(=O)O-,
-NHC(=O)O-,
-OC(=O)NH-,
-NHC(=O)NH-,
-NHC(=O)NH,
-C(=O)NHC(=O)-,
-S-,
-S-S-,
-CH₂C(=O)-
-C(=O)CH₂-,
=N-NH-, and
-NH-N=.

In one embodiment, the maleimide-derived group is replaced with a group, which optionally together with the cell binding agent, is selected from: 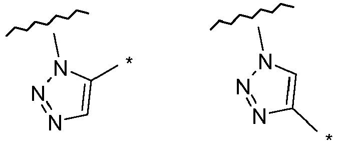 where the wavy line indicates either the point of attachment to the cell binding agent or the bond to the remaining portion of the L group or the remaining portion of the Y group, and the asterisk indicates the other of the point of attachment to the cell binding agent or the bond to the remaining portion of the L group or the remaining portion of the Y group.

Other groups that can be used as L for connecting the remaining portion of the Y group to the cell binding agent are described in WO 2005/082023.

Thus, in embodiments of the present invention, L is of formula:

-L^{A}-(CH₂)ₘ-

Where m is from 0 to 6; and
L^{A} is selected from:

| | | | |
|---|---|---|---|
| (L^{A1-1}) | 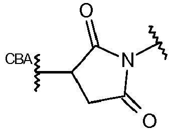 | (L^{A6}) | 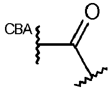 |
| (L^{A1-2}) | 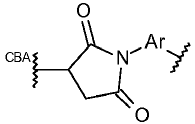 | (L^{A7}) | 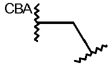 |
| (L^{A2}) | 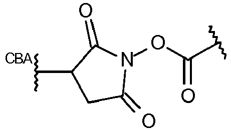 | (L^{A8-1}) | 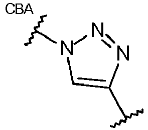 |
| (L^{A3-1}) | 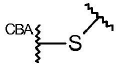 | (L^{A8-2}) | 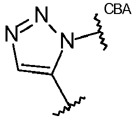 |
| (L^{A3-2}) | 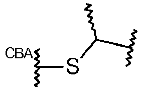 | (L^{A9-1}) | 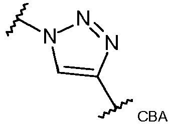 |
| (L^{A4}) | 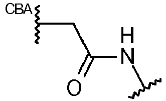 | (L^{A9-2}) | 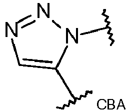 |
| (L^{A5}) | 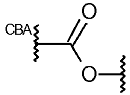 | | |

where Ar represents a C₅₋₆ arylene group, e.g. phenylene.

Thus, in embodiments of the present invention, G is of formula:

G^{A}-(CH₂)ₘ-

Where m is from 0 to 6; and
G^{A} is selected from:

| | | | |
|---|---|---|---|
| (G^{A1-1}) | 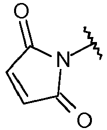 | (G^{A4}) | 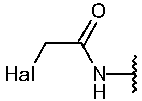 |
| | | | Where Hal = I, Br, Cl |
| (G^{A1-2}) | 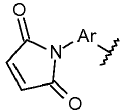 | | |
| (G^{A2}) | 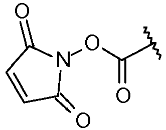 | (G^{A5}) | 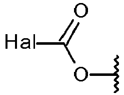 |
| (G^{A3-1}) | 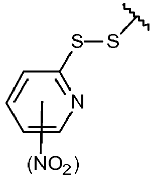 | (G^{A6}) | 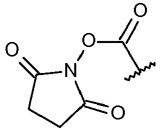 |
| | where the NO₂ group is optional | | |
| (G^{A3-2}) | 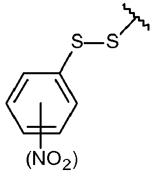 | (G^{A7}) | 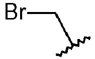 |
| | where the NO₂ group is optional | | |
| (G^{A3-3}) | 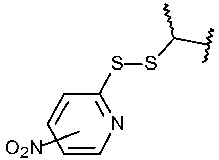 | (G^{A8}) | 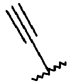 |
| | where the NO₂ group is optional | | |
| (G^{A3-4}) | 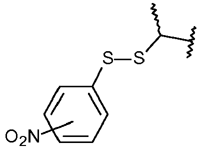 | (G^{A9}) | 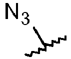 |
| | where the NO₂ group is optional | | |

where Ar represents a C₅₋₆ arylene group, e.g. phenylene.

In some embodiments, m may be 2 or 5.

### Cell Binding Agent

A cell binding agent may be of any kind, and include peptides and non-peptides. These can include antibodies or a fragment of an antibody that contains at least one binding site, lymphokines, hormones, hormone mimetics, vitamins, growth factors, nutrient-transport molecules, or any other cell binding molecule or substance.

### Peptides

In one embodiment, the cell binding agent is a linear or cyclic peptide comprising 4-30, preferably 6-20, contiguous amino acid residues. In this embodiment, it is preferred that one cell binding agent is linked to one monomer or dimer pyrrolobenzodiazepine compound.

In one embodiment the cell binding agent comprises a peptide that binds integrin αᵥβ₆. The peptide may be selective for αᵥβ₆ over XYS.

In one embodiment the cell binding agent comprises the A20FMDV-Cys polypeptide. The A20FMDV-Cys has the sequence: NAVPNLRGDLQVLAQKVARTC. Alternatively, a variant of the A20FMDV-Cys sequence may be used wherein one, two, three, four, five, six, seven, eight, nine or ten amino acid residues are substituted with another amino acid residue. Furthermore, the polypeptide may have the sequence NAVXXXXXXXXXXXXXXXRTC.

### Antibodies

The term "antibody" herein is used in the broadest sense and specifically covers monoclonal antibodies, polyclonal antibodies, dimers, multimers, multispecific antibodies (e.g., bispecific antibodies), and antibody fragments, so long as they exhibit the desired biological activity (Miller et al (2003) Jour. of Immunology 170:4854-4861). Antibodies may be murine, human, humanized, chimeric, or derived from other species. An antibody is a protein generated by the immune system that is capable of recognizing and binding to a specific antigen. (Janeway, C., Travers, P., Walport, M., Shlomchik (2001) Immuno Biology, 5th Ed., Garland Publishing, New York). A target antigen generally has numerous binding sites, also called epitopes, recognized by CDRs on multiple antibodies. Each antibody that specifically binds to a different epitope has a different structure. Thus, one antigen may have more than one corresponding antibody. An antibody includes a full-length immunoglobulin molecule or an immunologically active portion of a full-length immunoglobulin molecule, i.e., a molecule that contains an antigen binding site that immunospecifically binds an antigen of a target of interest or part thereof, such targets including but not limited to, cancer cell or cells that produce autoimmune antibodies associated with an autoimmune disease. The immunoglobulin can be of any type (e.g. IgG, IgE, IgM, IgD, and IgA), class (e.g. IgG1, IgG2, IgG3, IgG4, IgA1 and IgA2) or subclass of immunoglobulin molecule. The immunoglobulins can be derived from any species, including human, murine, or rabbit origin.

"Antibody fragments" comprise a portion of a full length antibody, generally the antigen binding or variable region thereof. Examples of antibody fragments include Fab, Fab', F(ab')₂, and scFv fragments; diabodies; linear antibodies; fragments produced by a Fab expression library, anti-idiotypic (anti-Id) antibodies, CDR (complementary determining region), and epitope-binding fragments of any of the above which immunospecifically bind to cancer cell antigens, viral antigens or microbial antigens, single-chain antibody molecules; and multispecific antibodies formed from antibody fragments.

The term "monoclonal antibody" as used herein refers to an antibody obtained from a population of substantially homogeneous antibodies, i.e. the individual antibodies comprising the population are identical except for possible naturally occurring mutations that may be present in minor amounts. Monoclonal antibodies are highly specific, being directed against a single antigenic site. Furthermore, in contrast to polyclonal antibody preparations which include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al (1975) Nature 256:495, or may be made by recombinant DNA methods (see, US 4816567). The monoclonal antibodies may also be isolated from phage antibody libraries using the techniques described in Clackson et al (1991) Nature, 352:624-628; Marks et al (1991) J. Mol. Biol., 222:581-597 or from transgenic mice carrying a fully human immunoglobulin system (Lonberg (2008) Curr. Opinion 20(4):450-459).

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (US 4816567; and Morrison et al (1984) Proc. Natl. Acad. Sci. USA, 81:6851-6855). Chimeric antibodies include "primatized" antibodies comprising variable domain antigen-binding sequences derived from a non-human primate (e.g. Old World Monkey or Ape) and human constant region sequences.

An "intact antibody" herein is one comprising a VL and VH domains, as well as a light chain constant domain (CL) and heavy chain constant domains, CH1, CH2 and CH3. The constant domains may be native sequence constant domains (e.g. human native sequence constant domains) or amino acid sequence variant thereof. The intact antibody may have one or more "effector functions" which refer to those biological activities attributable to the Fc region (a native sequence Fc region or amino acid sequence variant Fc region) of an antibody. Examples of antibody effector functions include C1q binding; complement dependent cytotoxicity; Fc receptor binding; antibody-dependent cell-mediated cytotoxicity (ADCC); phagocytosis; and down regulation of cell surface receptors such as B cell receptor and BCR.

Depending on the amino acid sequence of the constant domain of their heavy chains, intact antibodies can be assigned to different "classes." There are five major classes of intact antibodies: IgA, IgD, IgE, IgG, and IgM, and several of these may be further divided into "subclasses" (isotypes), e.g., IgG1, IgG2, IgG3, IgG4, IgA, and IgA2. The heavy-chain constant domains that correspond to the different classes of antibodies are called α, δ, ε, γ, and µ, respectively. The subunit structures and three-dimensional configurations of different classes of immunoglobulins are well known.

### Humanisation

Techniques to reduce the *in vivo* immunogenicity of a non-human antibody or antibody fragment include those termed "humanisation".

A "humanized antibody" refers to a polypeptide comprising at least a portion of a modified variable region of a human antibody wherein a portion of the variable region, preferably a portion substantially less than the intact human variable domain, has been substituted by the corresponding sequence from a non-human species and wherein the modified variable region is linked to at least another part of another protein, preferably the constant region of a human antibody. The expression "humanized antibodies" includes human antibodies in which one or more complementarity determining region ("CDR") amino acid residues and/or one or more framework region ("FW" or "FR") amino acid residues are substituted by amino acid residues from analogous sites in rodent or other non-human antibodies. The expression "humanized antibody" also includes an immunoglobulin amino acid sequence variant or fragment thereof that comprises an FR having substantially the amino acid sequence of a human immunoglobulin and a CDR having substantially the amino acid sequence of a non-human immunoglobulin.

"Humanized" forms of non-human (e.g., murine) antibodies are chimeric antibodies that contain minimal sequence derived from non-human immunoglobulin. Or, looked at another way, a humanized antibody is a human antibody that also contains selected sequences from non-human (e.g. murine) antibodies in place of the human sequences. A humanized antibody can include conservative amino acid substitutions or non-natural residues from the same or different species that do not significantly alter its binding and/or biologic activity. Such antibodies are chimeric antibodies that contain minimal sequence derived from non-human immunoglobulins.

There are a range of humanisation techniques, including 'CDR grafting', 'guided selection', 'deimmunization', 'resurfacing' (also known as 'veneering'), 'composite antibodies', 'Human String Content Optimisation' and framework shuffling.

### CDR grafting

In this technique, the humanized antibodies are human immunoglobulins (recipient antibody) in which residues from a complementary-determining region (CDR) of the recipient antibody are replaced by residues from a CDR of a non-human species (donor antibody) such as mouse, rat, camel, bovine, goat, or rabbit having the desired properties (in effect, the non-human CDRs are 'grafted' onto the human framework). In some instances, framework region (FR) residues of the human immunoglobulin are replaced by corresponding non-human residues (this may happen when, for example, a particular FR residue has significant effect on antigen binding).

Furthermore, humanized antibodies can comprise residues that are found neither in the recipient antibody nor in the imported CDR or framework sequences. These modifications are made to further refine and maximize antibody performance. Thus, in general, a humanized antibody will comprise all of at least one, and in one aspect two, variable domains, in which all or all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), or that of a human immunoglobulin.

### Guided selection

The method consists of combining the V_{H} or V_{L} domain of a given non-human antibody specific for a particular epitope with a human V_{H} or V_{L} library and specific human V domains are selected against the antigen of interest. This selected human VH is then combined with a VL library to generate a completely human VHxVL combination. The method is described in Nature Biotechnology (N.Y.) 12, (1994) 899-903.

### Composite antibodies

In this method, two or more segments of amino acid sequence from a human antibody are combined within the final antibody molecule. They are constructed by combining multiple human VH and VL sequence segments in combinations which limit or avoid human T cell epitopes in the final composite antibody V regions. Where required, T cell epitopes are limited or avoided by, exchanging V region segments contributing to or encoding a T cell epitope with alternative segments which avoid T cell epitopes. This method is described in US 2008/0206239 A1.

### Deimmunization

This method involves the removal of human (or other second species) T-cell epitopes from the V regions of the therapeutic antibody (or other molecule). The therapeutic antibodies V-region sequence is analysed for the presence of MHC class II- binding motifs by, for example, comparison with databases of MHC-binding motifs (such as the "motifs" database hosted at www.wehi.edu.au). Alternatively, MHC class II- binding motifs may be identified using computational threading methods such as those devised by Altuvia et al. (J. Mol. Biol. 249 244-250 (1995)); in these methods, consecutive overlapping peptides from the V-region sequences are testing for their binding energies to MHC class II proteins. This data can then be combined with information on other sequence features which relate to successfully presented peptides, such as amphipathicity, Rothbard motifs, and cleavage sites for cathepsin B and other processing enzymes.

Once potential second species (e.g. human) T-cell epitopes have been identified, they are eliminated by the alteration of one or more amino acids. The modified amino acids are usually within the T-cell epitope itself, but may also be adjacent to the epitope in terms of the primary or secondary structure of the protein (and therefore, may not be adjacent in the primary structure). Most typically, the alteration is by way of substitution but, in some circumstances amino acid addition or deletion will be more appropriate.

All alterations can be accomplished by recombinant DNA technology, so that the final molecule may be prepared by expression from a recombinant host using well established methods such as Site Directed Mutagenesis. However, the use of protein chemistry or any other means of molecular alteration is also possible.

### Resurfacing

This method involves:
(a) determining the conformational structure of the variable region of the non-human (e.g. rodent) antibody (or fragment thereof) by constructing a three-dimensional model of the non-human antibody variable region;
(b) generating sequence alignments using relative accessibility distributions from x-ray crystallographic structures of a sufficient number of non-human and human antibody variable region heavy and light chains to give a set of heavy and light chain framework positions wherein the alignment positions are identical in 98% of the sufficient number of non-human antibody heavy and light chains;
(c) defining for the non-human antibody to be humanized, a set of heavy and light chain surface exposed amino acid residues using the set of framework positions generated in step (b);
(d) identifying from human antibody amino acid sequences a set of heavy and light chain surface exposed amino acid residues that is most closely identical to the set of surface exposed amino acid residues defined in step (c), wherein the heavy and light chain from the human antibody are or are not naturally paired;
(e) substituting, in the amino acid sequence of the non-human antibody to be humanized, the set of heavy and light chain surface exposed amino acid residues defined in step (c) with the set of heavy and light chain surface exposed amino acid residues identified in step (d);
(f) constructing a three-dimensional model of the variable region of the non-human antibody resulting from the substituting specified in step (e);
(g) identifying, by comparing the three-dimensional models constructed in steps (a) and (f), any amino acid residues from the sets identified in steps (c) or (d), that are within 5 Angstroms of any atom of any residue of the complementarity determining regions of the non-human antibodt to be humanized; and
(h) changing any residues identified in step (g) from the human to the original non-human amino acid residue to thereby define a non-human antibody humanizing set of surface exposed amino acid residues; with the proviso that step (a) need not be conducted first, but must be conducted prior to step (g).

### Superhumanization

The method compares the non-human sequence with the functional human germline gene repertoire. Those human genes encoding canonical structures identical or closely related to the non-human sequences are selected. Those selected human genes with highest homology within the CDRs are chosen as FR donors. Finally, the non-human CDRs are grafted onto these human FRs. This method is described in patent WO 2005/079479 A2.

### Human String Content Optimization

This method compares the non-human (e.g. mouse) sequence with the repertoire of human germline genes and the differences are scored as Human String Content (HSC) that quantifies a sequence at the level of potential MHC/T-cell epitopes. The target sequence is then humanized by maximizing its HSC rather than using a global identity measure to generate multiple diverse humanized variants (described in Molecular Immunology, 44, (2007) 1986-1998).

### Framework Shuffling

The CDRs of the non-human antibody are fused in-frame to cDNA pools encompassing all known heavy and light chain human germline gene frameworks. Humanised antibodies are then selected by e.g. panning of the phage displayed antibody library. This is described in Methods 36, 43-60 (2005).

Examples of cell binding agents include those agents described for use in WO 2007/085930, which is incorporated herein.

Tumour-associate antigens and cognate antibodies for use in embodiments of the present invention are listed below.

### TUMOR-ASSOCIATED ANTIGENS AND COGNATE ANTIBODIES

### (1) BMPR1B (bone morphogenetic protein receptor-type IB)

### Nucleotide

Genbank accession no. NM_001203
Genbank version no. NM_001203.2 GI:169790809
Genbank record update date: Sep 23, 2012 02:06 PM

### Polypeptide

Genbank accession no. NP_001194
Genbank version no. NP_001194.1 GI:4502431
Genbank record update date: Sep 23, 2012 02:06 PM

### Cross-references

ten Dijke,P., et al Science 264 (5155): 101-104 (1994), Oncogene 14 10 (11):1377-1382 (1997)); WO2004/063362 (Claim 2); WO2003/042661 (Claim 12);
US2003/134790-A1 (Page 38-39); WO2002/102235 (Claim 13; Page 296); WO2003/055443 (Page 91-92); WO2002/99122 (Example 2; Page 528-530); WO2003/029421 (Claim 6); WO2003/024392 (Claim 2; Fig 112); WO2002/98358 (Claim 1; Page 183); WO2002/54940 (Page 100-101); WO2002/59377(Page 349-350); WO2002/30268 (Claim 27; Page 376); 15 WO2001/48204 (Example; Fig 4); NP_001194 bone morphogenetic protein receptor, type IB/pid=NP_001194.1.; MIM:603248; AY065994

### (2) E16 (LAT1, SLC7A5)

### Nucleotide

Genbank accession no. NM_003486
Genbank version no. NM_003486.5 GI:71979931
Genbank record update date: Jun 27, 2012 12:06 PM

### Polypeptide

Genbank accession no. NP_003477
Genbank version no. NP_003477.4 GI:71979932
Genbank record update date: Jun 27, 2012 12:06 PM

### Cross references

Biochem. Biophys. Res. Commun. 255 (2), 283-288 (1999), Nature 395 (6699):288-291 (1998), Gaugitsch, H.W., et 20 al (1992) J. Biol. Chem. 267 (16):11267-11273); WO2004/048938 (Example 2); WO2004/032842 (Example IV); WO2003/042661 (Claim 12); WO2003/016475 (Claim 1); WO2002/78524 (Example 2); WO2002/99074 (Claim 19; Page 127-129); WO2002/86443 (Claim 27; Pages 222, 393); WO2003/003906 (Claim 10; Page 293); WO2002/64798 (Claim 33; Page 93-95); WO2000/14228 (Claim 5; Page 133-136); US2003/224454 (Fig 3); 25 WO2003/025138 (Claim 12; Page 150); NP_003477 solute carrier family 7 (cationic amino
acid transporter, y+system), member 5 /pid=NP_003477.3 - Homo sapiens;
MIM:600182;; NM_015923.

### (3) STEAP1 (six transmembrane epithelial antigen of prostate)

### Nucleotide

Genbank accession no. NM_012449
Genbank version no. NM_012449.2 GI:22027487
Genbank record update date: Sep 9, 2012 02:57 PM

### Polypeptide

Genbank accession no. NP_036581
Genbank version no. NP_036581.1 GI:9558759
Genbank record update date: Sep 9, 2012 02:57 PM

### Cross references

Cancer Res. 61 (15), 5857-5860 (2001), Hubert, R.S., et al (1999) Proc. Natl. Acad. Sci. U.S.A. 96 (25):14523-14528); WO2004/065577 (Claim 6); WO2004/027049 (Fig 1L); EP1394274 (Example 11); WO2004/016225 (Claim 2); WO2003/042661 (Claim 12); US2003/157089 (Example 5); US2003/185830 (Example 5); US2003/064397 (Fig 2); WO2002/89747 (Example 5; Page 618-619); WO2003/022995 (Example 9; Fig 13A, 35 Example 53; Page 173, Example 2; Fig 2A); six transmembrane epithelial antigen of the prostate; MIM:604415.

### (4) 0772P (CA125, MUC16)

### Nucleotide

Genbank accession no. AF361486
Genbank version no. AF361486.3 GI:34501466
Genbank record update date: Mar 11, 2010 07:56 AM

### Polypeptide

Genbank accession no. AAK74120
Genbank version no. AAK74120.3 GI:34501467
Genbank record update date: Mar 11, 2010 07:56 AM

### Cross references

J. Biol. Chem. 276 (29):27371-27375 (2001)); WO2004/045553 (Claim 14); WO2002/92836 (Claim 6; Fig 12); WO2002/83866 (Claim 15; Page 116-121); US2003/124140 (Example 16); GI:34501467;

### (5) MPF (MPF, MSLN, SMR, megakaryocyte potentiating factor, mesothelin)

### Nucleotide

Genbank accession no. NM_005823
Genbank version no. NM_005823.5 GI:293651528
Genbank record update date: Sep 2, 2012 01:47 PM

### Polypeptide

Genbank accession no. NP_005814
Genbank version no. NP_005814.2 GI:53988378
Genbank record update date: Sep 2, 2012 01:47 PM

### Cross references

Yamaguchi, N., et al Biol. Chem. 269 (2), 805-808 (1994), Proc. Natl. Acad. Sci. U.S.A. 96 (20):11531-11536 (1999), Proc. Natl. Acad. Sci. U.S.A. 93 10 (1):136-140 (1996), J. Biol. Chem. 270 (37):21984-21990 (1995)); WO2003/101283 (Claim 14); (WO2002/102235 (Claim 13; Page 287-288); WO2002/101075 (Claim 4; Page 308- 309); WO2002/71928 (Page 320-321); WO94/10312 (Page 52-57); IM:601051.

### (6) Napi3b (NAPI-3B, NPTIIb, SLC34A2, solute carrier family 34 (sodium phosphate), member 2, type II sodium-dependent phosphate transporter 3b)

### Nucleotide

Genbank accession no. NM_006424
Genbank version no. NM_006424.2 GI:110611905
Genbank record update date: Jul 22, 2012 03:39 PM

### Polypeptide

Genbank accession no. NP_006415
Genbank version no. NP_006415.2 GI:110611906
Genbank record update date: Jul 22, 2012 03:39 PM

### Cross references

J. Biol. Chem. 277 (22):19665-19672 (2002), Genomics 62 (2):281-284 (1999), Feild, J.A., et al (1999) Biochem. Biophys. Res. Commun. 258 (3):578-582); WO2004/022778 (Claim 2); EP1394274 (Example 11); WO2002/102235 (Claim 13; Page 20 326); EP0875569 (Claim 1; Page 17-19); WO2001/57188 (Claim 20; Page 329); WO2004/032842 (Example IV); WO2001/75177 (Claim 24; Page 139-140); MIM:604217.

### (7) Sema 5b (FLJ10372, KIAA1445, Mm.42015, SEMA5B, SEMAG, Semaphorin 5b Hlog, 25 sema domain, seven thrombospondin repeats (type 1 and type 1-like), transmembrane domain (TM) and short cytoplasmic domain, (semaphorin) 5B)

### Nucleotide

Genbank accession no. AB040878
Genbank version no. AB040878.1 GI:7959148
Genbank record update date: Aug 2, 2006 05:40 PM

### Polypeptide

Genbank accession no. BAA95969
Genbank version no. BAA95969.1 GI:7959149
Genbank record update date: Aug 2, 2006 05:40 PM

### Cross references

Nagase T., et al (2000) DNA Res. 7 (2):143-150); WO2004/000997 (Claim 1); WO2003/003984 (Claim 1); WO2002/06339 (Claim 1; Page 50); WO2001/88133 (Claim 1; Page 41-43, 48-58); WO2003/054152 (Claim 20); WO2003/101400 (Claim 11); Accession: 30 Q9P283; Genew; HGNC:10737

### (8) PSCA hlg (2700050C12Rik, C530008O16Rik, RIKEN cDNA 2700050C12, RIKEN cDNA 2700050C12 gene)

### Nucleotide

Genbank accession no. AY358628
Genbank version no. AY358628.1 GI:37182377
Genbank record update date: Dec 1, 2009 04:15 AM

### Polypeptide

Genbank accession no. AAQ88991
Genbank version no. AAQ88991.1 GI:37182378
Genbank record update date: Dec 1, 2009 04:15 AM

### Cross references

Ross et al (2002) Cancer Res. 62:2546-2553; US2003/129192 (Claim 2); US2004/044180 (Claim 12); US2004/044179 35 (Claim 11); US2003/096961 (Claim 11); US2003/232056 (Example 5); WO2003/105758 16 (Claim 12); US2003/206918 (Example 5); EP1347046 (Claim 1); WO2003/025148 (Claim 20); GI:37182378.

### (9) ETBR (Endothelin type B receptor)

### Nucleotide

Genbank accession no. AY275463
Genbank version no. AY275463.1 GI:30526094
Genbank record update date: Mar 11, 2010 02:26 AM

### Polypeptide

Genbank accession no. AAP32295
Genbank version no. AAP32295.1 GI:30526095
Genbank record update date: Mar 11, 2010 02:26 AM

### Cross references

Nakamuta M., et al Biochem. Biophys. Res. Commun. 177, 34-39, 1991; Ogawa Y., et al Biochem. Biophys. Res. Commun. 178, 248-255, 1991; Arai H., et al Jpn. Circ. J. 56, 1303-1307, 1992; Arai H., et al J. Biol. Chem. 268, 3463-3470, 1993; Sakamoto A., Yanagisawa M., et al Biochem. Biophys. Res. Commun. 178, 656-663, 1991; Elshourbagy N.A., et al J. Biol. Chem. 268, 3873-3879, 1993; Haendler B., et al J. Cardiovasc. Pharmacol. 20, s1-S4, 1992; Tsutsumi M., et al Gene 228, 43-49, 1999; Strausberg R.L., et al Proc. Natl. Acad. Sci. U.S.A. 99, 16899-16903, 2002; Bourgeois C., et al J. Clin. Endocrinol. Metab. 82, 3116-3123, 1997;

Okamoto Y., et al Biol. Chem. 272, 21589-21596, 1997; Verheij J.B., et al Am. J. Med. Genet. 108, 223-225, 2002; Hofstra R.M.W., et al Eur. J. Hum. Genet. 5, 180-185, 1997; Puffenberger E.G., et al Cell 79, 1257-1266, 1994; Attie T., et al, Hum. Mol. Genet. 4, 2407-15 2409, 1995; Auricchio A., et al Hum. Mol. Genet. 5:351-354, 1996; Amiel J., et al Hum. Mol. Genet. 5, 355-357, 1996; Hofstra R.M.W., et al Nat. Genet. 12, 445-447, 1996; Svensson P.J., et al Hum. Genet. 103, 145-148, 1998; Fuchs S., et al Mol. Med. 7, 115-124, 2001; Pingault V., et al (2002) Hum. Genet. 111, 198-206; WO2004/045516 (Claim 1); WO2004/048938 (Example 2); WO2004/040000 (Claim 151); WO2003/087768 (Claim 1); 20 WO2003/016475 (Claim 1); WO2003/016475 (Claim 1); WO2002/61087 (Fig 1); WO2003/016494 (Fig 6); WO2003/025138 (Claim 12; Page 144); WO2001/98351 (Claim 1; Page 124-125); EP0522868 (Claim 8; Fig 2); WO2001/77172 (Claim 1; Page 297-299); US2003/109676; US6518404 (Fig 3); US5773223 (Claim 1a; Col 31-34); WO2004/001004.

### (10) MSG783 (RNF124, hypothetical protein FLJ20315)

### Nucleotide

Genbank accession no. NM_017763
Genbank version no. NM_017763.4 GI:167830482
Genbank record update date: Jul 22, 2012 12:34 AM

### Polypeptide

Genbank accession no. NP_060233
Genbank version no. NP_060233.3 GI:56711322
Genbank record update date: Jul 22, 2012 12:34 AM

### Cross references

WO2003/104275 (Claim 1); WO2004/046342 (Example 2); WO2003/042661 (Claim 12); WO2003/083074 (Claim 14; Page 61); WO2003/018621 (Claim 1); WO2003/024392 (Claim 2; Fig 93); WO2001/66689 (Example 6); LocusID:54894.

### (11) STEAP2 (HGNC_8639, IPCA-1, PCANAP1, STAMP1, STEAP2, STMP, prostate cancer associated gene 1, prostate cancer associated protein 1, six transmembrane epithelial antigen of prostate 2, six transmembrane prostate protein)

### Nucleotide

Genbank accession no. AF455138
Genbank version no. AF455138.1 GI:22655487
Genbank record update date: Mar 11, 2010 01:54 AM

### Polypeptide

Genbank accession no. AAN04080
Genbank version no. AAN04080.1 GI:22655488
Genbank record update date: Mar 11, 2010 01:54 AM

### Cross references

Lab. Invest. 82 (11):1573-1582 (2002)); WO2003/087306; US2003/064397 (Claim 1; Fig 1); WO2002/72596 (Claim 13; Page 54-55); WO2001/72962 (Claim 1; Fig 4B); 35 WO2003/104270 (Claim 11); WO2003/104270 (Claim 16); US2004/005598 (Claim 22); WO2003/042661 (Claim 12); US2003/060612 (Claim 12; Fig 10); WO2002/26822 (Claim 23; Fig 2); WO2002/16429 (Claim 12; Fig 10); GI:22655488.

### (12) TrpM4 (BR22450, FLJ20041, TRPM4, TRPM4B, transient receptor potential cation 5 channel, subfamily M, member 4)

### Nucleotide

Genbank accession no. NM_017636
Genbank version no. NM_017636.3 GI:304766649
Genbank record update date: Jun 29, 2012 11:27 AM

### Polypeptide

Genbank accession no. NP_060106
Genbank version no. NP_060106.2 GI:21314671
Genbank record update date: Jun 29, 2012 11:27 AM

### Cross references

Xu, X.Z., et al Proc. Natl. Acad. Sci. U.S.A. 98 (19):10692-10697 (2001), Cell 109 (3):397-407 (2002), J. Biol. Chem. 278 (33):30813-30820 (2003)); US2003/143557 (Claim 4); WO2000/40614 (Claim 14; Page 100-103); WO2002/10382 (Claim 1; Fig 9A); WO2003/042661 (Claim 12); WO2002/30268 (Claim 27; Page 391); US2003/219806 (Claim 4); WO2001/62794 (Claim 10 14; Fig 1A-D); MIM:606936.

### (13) CRIPTO (CR, CR1, CRGF, CRIPTO, TDGF1, teratocarcinoma-derived growth factor) Nucleotide

Genbank accession no. NM_003212
Genbank version no. NM_003212.3 GI:292494881
Genbank record update date: Sep 23, 2012 02:27 PM

### Polypeptide

Genbank accession no. NP_003203
Genbank version no. NP_003203.1 GI:4507425
Genbank record update date: Sep 23, 2012 02:27 PM

### Cross references

Ciccodicola, A., et al EMBO J. 8 (7):1987-1991 (1989), Am. J. Hum. Genet. 49 (3):555-565 (1991)); US2003/224411 (Claim 1); WO2003/083041 (Example 1); WO2003/034984 (Claim 12); WO2002/88170 (Claim 2; Page 52-53); WO2003/024392 (Claim 2; Fig 58); WO2002/16413 (Claim 1; Page 94-95, 105); WO2002/22808 (Claim 2; Fig 1); US5854399 (Example 2; Col 17-18); US5792616 (Fig 2); MIM:187395.

### (14) CD21 (CR2 (Complement receptor 2) or C3DR (C3d/Epstein Barr virus receptor) or Hs.73792)

### Nucleotide

Genbank accession no M26004
Genbank version no. M26004.1 GI:181939
Genbank record update date: Jun 23, 2010 08:47 AM

### Polypeptide

Genbank accession no. AAA35786
Genbank version no. AAA35786.1 GI:181940
Genbank record update date: Jun 23, 2010 08:47 AM

### Cross references

Fujisaku et al (1989) J. Biol. Chem. 264 (4):2118-2125); Weis J.J., et al J. Exp. Med. 167, 1047-1066, 1988; Moore M., et al Proc. Natl. Acad. Sci. U.S.A. 84, 9194-9198, 1987; Barel M., et al Mol. Immunol. 35, 1025-1031, 1998; Weis J.J., et al Proc. Natl. Acad. Sci. U.S.A. 83, 5639-5643, 1986; Sinha S.K., et al (1993) J. Immunol. 150, 5311-5320; WO2004/045520 (Example 4); US2004/005538 (Example 1); WO2003/062401 (Claim 9); WO2004/045520 (Example 4); WO91/02536 (Fig 9.1-9.9); WO2004/020595 (Claim 1); Accession: P20023; Q13866; Q14212; EMBL; M26004; AAA35786.1.

### (15) CD79b (CD79B, CD79β, IGb (immunoglobulin-associated beta), B29)

### Nucleotide

Genbank accession no NM_000626
Genbank version no. NM_000626.2 GI:90193589
Genbank record update date: Jun 26, 2012 01:53 PM

### Polypeptide

Genbank accession no. NP_000617
Genbank version no. NP_000617.1 GI:11038674
Genbank record update date: Jun 26, 2012 01:53 PM

### Cross references

Proc. Natl. Acad. Sci. U.S.A. (2003) 100 (7):4126- 4131, Blood (2002) 100 (9):3068-3076, Muller et al (1992) Eur. J. Immunol. 22 (6):1621-1625); WO2004/016225 (claim 2, Fig 140); WO2003/087768, US2004/101874 (claim 1, page 102); WO2003/062401 (claim 9); WO2002/78524 (Example 2); US2002/150573 (claim 35 5, page 15); US5644033; WO2003/048202 (claim 1, pages 306 and 309); WO 99/58658, US6534482 (claim 13, Fig 17A/B); WO2000/55351 (claim 11, pages 1145-1146); MIM:147245

### (16) FcRH2 (IFGP4, IRTA4, SPAP1A (SH2 domain containing phosphatase anchor protein 5 1a), SPAP1B, SPAP1C)

### Nucleotide

Genbank accession no NM_030764
Genbank version no. NM_030764.3 GI:227430280
Genbank record update date: Jun 30, 2012 12:30 AM

### Polypeptide

Genbank accession no. NP_110391
Genbank version no. NP_110391.2 GI:19923629
Genbank record update date: Jun 30, 2012 12:30 AM

### Cross references

AY358130); Genome Res. 13 (10):2265-2270 (2003), Immunogenetics 54 (2):87-95 (2002), Blood 99 (8):2662-2669 (2002), Proc. Natl. Acad. Sci. U.S.A. 98 (17):9772-9777 (2001), Xu, M.J., et al (2001) Biochem. Biophys. Res. Commun. 280 (3):768-775; WO2004/016225 (Claim 2); WO2003/077836; WO2001/38490 (Claim 5; Fig 18D-1-18D-2); WO2003/097803 (Claim 12);
10 WO2003/089624 (Claim 25);: MIM:606509.

### (17) HER2 (ErbB2)

### Nucleotide

Genbank accession no M11730
Genbank version no. M11730.1 GI:183986
Genbank record update date: Jun 23, 2010 08:47 AM

### Polypeptide

Genbank accession no. AAA75493
Genbank version no. AAA75493.1 GI:306840
Genbank record update date: Jun 23, 2010 08:47 AM

### Cross references

Coussens L., et al Science (1985) 230(4730):1132-1139); Yamamoto T., et al Nature 319, 230-234, 1986; Semba K., et al Proc. Natl. Acad. Sci. U.S.A. 82, 6497-6501, 1985; Swiercz J.M., et al J. Cell Biol. 165, 869- 15 880, 2004; Kuhns J.J., et al J. Biol. Chem. 274, 36422-36427, 1999; Cho H.-S., et al Nature 421, 756-760, 2003; Ehsani A., et al (1993) Genomics 15, 426-429; WO2004/048938 (Example 2); WO2004/027049 (Fig 11); WO2004/009622; WO2003/081210;
WO2003/089904 (Claim 9); WO2003/016475 (Claim 1); US2003/118592; WO2003/008537 (Claim 1); WO2003/055439 (Claim 29; Fig 1A-B); WO2003/025228 (Claim 37; Fig 5C); 20 WO2002/22636 (Example 13; Page 95-107); WO2002/12341 (Claim 68; Fig 7); WO2002/13847 (Page 71-74); WO2002/14503 (Page 114-117); WO2001/53463 (Claim 2; Page 41-46); WO2001/41787 (Page 15); WO2000/44899 (Claim 52; Fig 7); WO2000/20579 (Claim 3; Fig 2); US5869445 (Claim 3; Col 31-38); WO9630514 (Claim 2; Page 56-61); EP1439393 (Claim 7); WO2004/043361 (Claim 7); WO2004/022709; WO2001/00244 25 (Example 3; Fig 4); Accession: P04626; EMBL; M11767; AAA35808.1. EMBL; M11761; AAA35808.1

### ANTIBODIES

Abbott: US20110177095
   For example, an antibody comprising CDRs having overall at least 80% sequence identity to CDRs having amino acid sequences of SEQ ID NO:3 (CDR-H1), SEQ ID NO:4 (CDR-H2), SEQ ID NO:5 (CDR-H3), SEQ ID NO:104 and/or SEQ ID NO:6 (CDR-L1), SEQ ID NO:7 (CDR-L2), and SEQ ID NO:8 (CDR-L3), wherein the anti-HER2 antibody or anti-HER2 binding fragment has reduced immunogenicity as compared to an antibody having a VH of SEQ ID NO:1 and a VL of SEQ ID NO:2.
Biogen: US20100119511
   For example, ATCC accession numbers: PTA-10355, PTA-10356, PTA-10357, PTA-10358
   For example, a purified antibody molecule that binds to HER2 comprising a all six CDR's from an antibody selected from the group consisting of BIIB71F10 (SEQ ID NOs:11, 13), BIIB69A09 (SEQ ID NOs:15, 17); BIIB67F10 (SEQ ID NOs:19, 21); BIIB67F11 (SEQ ID NOs:23, 25), BIIB66A12 (SEQ ID NOs:27, 29), BIIB66C01 (SEQ ID NOs:31, 33), BIIB65C10 (SEQ ID NOs:35, 37), BIIB65H09 (SEQ ID NOs:39, 41) and BIIB65B03 (SEQ ID NOs:43, 45), or CDRs which are identical or which have no more than two alterations from said CDRs.
Herceptin (Genentech) - US6,054,297; ATCC accession no. CRL-10463 (Genentech)
Pertuzumab (Genentech)
   US20110117097
      for example, see SEQ IDs No. 15&16, SEQ IDs No. 17&18, SEQ IDs No. 23&24 & ATCC accession numbers HB-12215, HB-12216, CRL 10463, HB-12697.
   US20090285837
   US20090202546
      for example, ATCC accession numbers: HB-12215, HB-12216, CRL 10463, HB-12698.
   US20060088523
      - for example, ATCC accession numbers: HB-12215, HB-12216
      - for example, an antibody comprising the variable light and variable heavy amino acid sequences in SEQ ID Nos. 3 and 4, respectively.
      - for example, an antibody comprising a light chain amino acid sequence selected from SEQ ID No. 15 and 23, and a heavy chain amino acid sequence selected from SEQ ID No. 16 and 24
   US20060018899
      - for example, ATCC accession numbers: (7C2) HB-12215, (7F3) HB-12216, (4D5) CRL-10463, (2C4) HB-12697.
      - for example, an antibody comprising the amino acid sequence in SEQ ID No. 23, or a deamidated and/or oxidized variant thereof.
   US2011/0159014
      - for example, an antibody having a light chain variable domain comprising the hypervariable regions of SEQ ID NO: 1".
      - For example, an antibody having a heavy chain variable domain comprising the hypervariable regions of SEQ ID NO: 2.
   US20090187007
Glycotope: TrasGEX antibody http://www.glycotope.com/pipeline
   For example, see International Joint Cancer Institute and Changhai
   Hospital Cancer Cent: HMTI-Fc Ab - Gao J., et al BMB Rep. 2009 Oct 31;42(10):636-41.
Symphogen: US20110217305
Union Stem Cell &Gene Engineering, China - Liu HQ., et al Xi Bao Yu Fen Zi Mian Yi Xue Za Zhi. 2010 May;26(5):456-8.

### (18) NCA (CEACAM6)

### Nucleotide

Genbank accession no M18728
Genbank version no. M18728.1 GI:189084
Genbank record update date: Jun 23, 2010 08:48 AM

### Polypeptide

Genbank accession no. AAA59907
Genbank version no. AAA59907.1 GI:189085
Genbank record update date: Jun 23, 2010 08:48 AM

### Cross references

Barnett T., et al Genomics 3, 59-66, 1988; Tawaragi Y., et al Biochem. Biophys. Res. Commun. 150, 89-96, 1988; Strausberg R.L., et al Proc. Natl. Acad. Sci. U.S.A. 99:16899-16903, 2002; WO2004/063709; EP1439393 (Claim 7); WO2004/044178 (Example 4); WO2004/031238; WO2003/042661 (Claim 12); WO2002/78524 (Example 2); WO2002/86443 (Claim 27; Page 427); WO2002/60317 (Claim 2); Accession: P40199; Q14920; EMBL; M29541; AAA59915.1.

EMBL; M18728.

### (19) MDP (DPEP1)

### Nucleotide

Genbank accession no BC017023
Genbank version no. BC017023.1 GI:16877538
Genbank record update date: Mar 6, 2012 01:00 PM

### Polypeptide

Genbank accession no. AAH17023
Genbank version no. AAH17023.1 GI:16877539
Genbank record update date: Mar 6, 2012 01:00 PM

### Cross references

Proc. Natl. Acad. Sci. U.S.A. 99 (26):16899-16903 (2002)); WO2003/016475 (Claim 1); WO2002/64798 (Claim 33; Page 85- 87); JP05003790 (Fig 6-8); WO99/46284 (Fig 9); MIM:179780.

### (20) IL20R-alpha (IL20Ra, ZCYTOR7)

### Nucleotide

Genbank accession no AF184971
Genbank version no. AF184971.1 GI:6013324
Genbank record update date: Mar 10, 2010 10:00 PM

### Polypeptide

Genbank accession no. AAF01320
Genbank version no. AAF01320.1 GI:6013325
Genbank record update date: Mar 10, 2010 10:00 PM

### Cross references

Clark H.F., et al Genome Res. 13, 2265-2270, 2003; Mungall A.J., et al Nature 425, 805-811, 2003; Blumberg H., et al Cell 104, 9-19, 2001; Dumoutier L., et al J. Immunol. 167, 3545-3549, 2001; Parrish-Novak J., et al J. Biol. Chem. 277, 47517-47523, 2002; Pletnev S., et al (2003) 10 Biochemistry 42:12617-12624; Sheikh F., et al (2004) J. Immunol. 172, 2006-2010; EP1394274 (Example 11); US2004/005320 (Example 5); WO2003/029262 (Page 74-75); WO2003/002717 (Claim 2; Page 63); WO2002/22153 (Page 45-47); US2002/042366 (Page 20-21); WO2001/46261 (Page 57-59); WO2001/46232 (Page 63-65); WO98/37193 (Claim 1; Page 55-59); Accession: Q9UHF4; Q6UWA9; Q96SH8; EMBL; AF184971; AAF01320.1.

### (21) Brevican (BCAN, BEHAB)

### Nucleotide

Genbank accession no AF229053
Genbank version no. AF229053.1 GI:10798902
Genbank record update date: Mar 11, 2010 12:58 AM

### Polypeptide

Genbank accession no. AAG23135
Genbank version no. AAG23135.1 GI:10798903
Genbank record update date: Mar 11, 2010 12:58 AM

### Cross references

Gary S.C., et al Gene 256, 139-147, 2000; Clark H.F., et al Genome Res. 13, 2265-2270, 2003; Strausberg R.L., et al Proc. Natl. Acad. Sci. U.S.A. 99, 16899-16903, 2002; US2003/186372 (Claim 11); US2003/186373 (Claim 11); US2003/119131 (Claim 1; Fig 52); US2003/119122 (Claim 1; 20 Fig 52); US2003/119126 (Claim 1); US2003/119121 (Claim 1; Fig 52); US2003/119129 (Claim 1); US2003/119130 (Claim 1); US2003/119128 (Claim 1; Fig 52); US2003/119125 (Claim 1); WO2003/016475 (Claim 1); WO2002/02634 (Claim 1)

### (22) EphB2R (DRT, ERK, Hek5, EPHT3, Tyro5)

### Nucleotide

Genbank accession no NM_004442
Genbank version no. NM_004442.6 GI:111118979
Genbank record update date: Sep 8, 2012 04:43 PM

### Polypeptide

Genbank accession no. NP_004433
Genbank version no. NP_004433.2 GI:21396504
Genbank record update date: Sep 8, 2012 04:43 PM

### Cross references

Chan,J. and Watt, V.M., Oncogene 6 (6), 1057-1061 (1991) Oncogene 10 (5):897-905 (1995), Annu. Rev. Neurosci. 21:309-345 (1998), Int. Rev. Cytol. 196:177-244 (2000)); WO2003042661 (Claim 12); WO200053216 (Claim 1; Page 41); WO2004065576 (Claim 1); WO2004020583 (Claim 9); WO2003004529 (Page 128-132); WO200053216 (Claim 1; Page 42); MIM:600997.

### (23) ASLG659 (B7h)

### Nucleotide

Genbank accession no. AX092328
Genbank version no. AX092328.1 GI:13444478
Genbank record update date: Jan 26, 2011 07:37 AM

### Cross references

US2004/0101899 (Claim 2); WO2003104399 (Claim 11); WO2004000221 (Fig 3); US2003/165504 (Claim 1); US2003/124140 (Example 2); US2003/065143 (Fig 60); WO2002/102235 (Claim 13; Page 299); US2003/091580 (Example 2); WO2002/10187 (Claim 6; Fig 10); WO2001/94641 (Claim 12; Fig 7b); WO2002/02624 (Claim 13; Fig 1A-1B); US2002/034749 (Claim 54; Page 45-46); WO2002/06317 (Example 2; Page 320-321, Claim 34; Page 321-322); WO2002/71928 (Page 468-469); WO2002/02587 (Example 1; Fig 1); WO2001/40269 (Example 3; Pages 190-192); WO2000/36107 (Example 2; Page 205-207); WO2004/053079 (Claim 12); WO2003/004989 (Claim 1); WO2002/71928 (Page 233-234, 452-453); WO 01/16318.

### (24) PSCA (Prostate stem cell antigen precursor)

### Nucleotide

Genbank accession no AJ297436
Genbank version no. AJ297436.1 GI:9367211
Genbank record update date: Feb 1, 2011 11:25 AM

### Polypeptide

Genbank accession no. CAB97347
Genbank version no. CAB97347.1 GI:9367212
Genbank record update date: Feb 1, 2011 11:25 AM

### Cross references

Reiter R.E., et al Proc. Natl. Acad. Sci. U.S.A. 95, 1735-1740, 1998; Gu Z., et al Oncogene 19, 1288-1296, 2000; Biochem. Biophys. Res. Commun. (2000) 275(3):783-788; WO2004/022709; EP1394274 (Example 11); US2004/018553 (Claim 17); WO2003/008537 (Claim 1); WO2002/81646 (Claim 1; Page 164); WO2003/003906 (Claim 10; Page 288); WO2001/40309 (Example 1; Fig 17); US2001/055751 (Example 1; Fig 1b); WO2000/32752 (Claim 18; Fig 1); WO98/51805 (Claim 17; Page 97); WO98/51824 (Claim 10; Page 94); WO98/40403 (Claim 2; Fig 1B); Accession: O43653; EMBL; AF043498; AAC39607.1

### (25) GEDA

### Nucleotide

Genbank accession no AY260763
Genbank version no. AY260763.1 GI:30102448
Genbank record update date: Mar 11, 2010 02:24 AM

### Polypeptide

Genbank accession no. AAP14954
Genbank version no. AAP14954.1 GI:30102449
Genbank record update date: Mar 11, 2010 02:24 AM

### Cross references

AP14954 lipoma HMGIC fusion-partnerlike protein /pid=AAP14954.1 - Homo sapiens (human); WO2003/054152 (Claim 20); WO2003/000842 (Claim 1); WO2003/023013 (Example 3, Claim 20); US2003/194704 (Claim 45); GI:30102449;

### (26) BAFF-R (B cell -activating factor receptor, BLyS receptor 3, BR3)

### Nucleotide

Genbank accession no AF116456
Genbank version no. AF116456.1 GI:4585274
Genbank record update date: Mar 10, 2010 09:44 PM

### Polypeptide

Genbank accession no. AAD25356
Genbank version no. AAD25356.1 GI:4585275
Genbank record update date: Mar 10, 2010 09:44 PM

### Cross references

BAFF receptor/pid=NP_443177.1 - Homo sapiens: Thompson, J.S., et al Science 293 (5537), 2108-2111 (2001); WO2004/058309; WO2004/011611; WO2003/045422 (Example; Page 32-33); WO2003/014294 (Claim 35; Fig 6B); WO2003/035846 (Claim 70; Page 615-616); WO2002/94852 (Col 136-137); WO2002/38766 25 (Claim 3; Page 133); WO2002/24909 (Example 3; Fig 3); MIM:606269; NP_443177.1; NM_052945_1; AF132600

### (27) CD22 (B-cell receptor CD22-B isoform, BL-CAM, Lyb-8, Lyb8, SIGLEC-2, FLJ22814) Nucleotide

Genbank accession no AK026467
Genbank version no. AK026467.1 GI:10439337
Genbank record update date: Sep 11, 2006 11:24 PM

### Polypeptide

Genbank accession no. BAB15489
Genbank version no. BAB15489.1 GI:10439338
Genbank record update date: Sep 11, 2006 11:24 PM

### Cross references

Wilson et al (1991) J. Exp. Med. 173:137-146; 30 WO2003/072036 (Claim 1; Fig 1); IM:107266; NP_001762.1; NM_001771_1.

### (27a) CD22 (CD22 molecule)

### Nucleotide

Genbank accession no X52785
Genbank version no. X52785.1 GI:29778
Genbank record update date: Feb 2, 2011 10:09 AM

### Polypeptide

Genbank accession no. CAA36988
Genbank version no. CAA36988.1 GI:29779
Genbank record update date: Feb 2, 2011 10:09 AM

### Cross references

Stamenkovic I. et al., Nature 345 (6270), 74-77 (1990)

### Other information

Official Symbol: CD22
Other Aliases: SIGLEC-2, SIGLEC2
Other Designations: B-cell receptor CD22; B-lymphocyte cell adhesion molecule; BL-
CAM; CD22 antigen; T-cell surface antigen Leu-14; sialic acid binding Ig-like lectin 2; sialic acid-binding Ig-like lectin 2

### ANTIBODIES

G5/44 (Inotuzumab): DiJoseph JF.,et al Cancer Immunol Immunother. 2005 Jan;54(1):11-24.

Epratuzumab- Goldenberg DM., et al Expert Rev Anticancer Ther. 6(10): 1341-53, 2006.

### (28) CD79a (CD79A, CD79alpha), immunoglobulin-associated alpha, a B cell-specific protein that covalently interacts with Ig beta (CD79B) and forms a complex on the surface with Ig M

*35 molecules, transduces a signal involved in B-cell differentiation), pl: 4.84, MW: 25028 TM: 2*
*[P] Gene Chromosome: 19q13.2).*

### Nucleotide

Genbank accession no NM_001783
Genbank version no. NM_001783.3 GI:90193587
Genbank record update date: Jun 26, 2012 01:48 PM

### Polypeptide

Genbank accession no. NP_001774
Genbank version no. NP_001774.1 GI:4502685
Genbank record update date: Jun 26, 2012 01:48 PM

### Cross references

WO2003/088808, US2003/0228319; WO2003/062401 (claim 9); US2002/150573 (claim 4, pages 13-14); WO99/58658 (claim 13, Fig 16); WO92/07574 (Fig 1); US5644033; Ha et al (1992) J. Immunol. 148(5):1526-1531; Müller et al (1992) Eur. J. Immunol.. 22:1621-1625; Hashimoto et al (1994) Immunogenetics 40(4):287-295; Preud'homme et al (1992) Clin. Exp. 5 Immunol. 90(1):141-146; Yu et al (1992) J. Immunol. 148(2) 633-637; Sakaguchi et al (1988) EMBO J. 7(11):3457-3464

### (29) CXCR5 (Burkitt's lymphoma receptor 1, a G protein-coupled receptor that is activated by the CXCL13 chemokine, functions in lymphocyte migration and humoral defense, plays a 10 role in HIV-2 infection and perhaps development of AIDS, lymphoma, myeloma, and leukemia); 372 aa, pl: 8.54 MW: 41959 TM: 7 [P] Gene Chromosome: 11q23.3,

### Nucleotide

Genbank accession no NM_001716
Genbank version no. NM_001716.4 GI:342307092
Genbank record update date: Sep 30, 2012 01:49 PM

### Polypeptide

Genbank accession no. NP_001707
Genbank version no. NP_001707.1 GI:4502415
Genbank record update date: Sep 30, 2012 01:49 PM

### Cross references

WO2004/040000; WO2004/015426; US2003/105292 (Example 2); US6555339 (Example 2); WO2002/61087 (Fig 1); WO2001/57188 (Claim 20, page 269); WO2001/72830 (pages 12-13); WO2000/22129 (Example 1, pages 152-153, 15 Example 2, pages 254-256); WO99/28468 (claim 1, page 38); US5440021 (Example 2, col 49-52); WO94/28931 (pages 56-58); WO92/17497 (claim 7, Fig 5); Dobner et al (1992) Eur. J. Immunol. 22:2795-2799; Barella et al (1995) Biochem. J. 309:773-779

### (30) HLA-DOB (Beta subunit of MHC class II molecule (Ia antigen) that binds peptides and 20 presents them to CD4+ T lymphocytes); 273 aa, pl: 6.56, MW: 30820. TM: 1 [P] Gene Chromosome: 6p21.3)

### Nucleotide

Genbank accession no NM_002120
Genbank version no. NM_002120.3 GI:118402587
Genbank record update date: Sep 8, 2012 04:46 PM

### Polypeptide

Genbank accession no. NP_002111
Genbank version no. NP_002111.1 GI:4504403
Genbank record update date: Sep 8, 2012 04:46 PM

### Cross references

Tonnelle et al (1985) EMBO J. 4(11):2839-2847; Jonsson et al (1989) Immunogenetics 29(6):411-413; Beck et al (1992) J. Mol. Biol. 228:433-441; Strausberg et al (2002) Proc. Natl. Acad. Sci USA 99:16899- 16903; Servenius et al (1987) J. Biol. Chem. 262:8759-8766; Beck et al (1996) J. Mol. Biol. 25 255:1-13; Naruse et al (2002) Tissue Antigens 59:512-519; WO99/58658 (claim 13, Fig 15); US6153408 (Col 35-38); US5976551 (col 168-170); US6011146 (col 145-146); Kasahara et al (1989) Immunogenetics 30(1):66-68; Larhammar et al (1985) J. Biol. Chem. 260(26):14111-14119

### (31) P2X5 (Purinergic receptor P2X ligand-gated ion channel 5, an ion channel gated by extracellular ATP, may be involved in synaptic transmission and neurogenesis, deficiency may contribute to the pathophysiology of idiopathic detrusor instability); 422 aa), pl: 7.63, MW: 47206 TM: 1 [P] Gene Chromosome: 17p13.3).

### Nucleotide

Genbank accession no NM_002561
Genbank version no. NM_002561.3 GI:325197202
Genbank record update date: Jun 27, 2012 12:41 AM

### Polypeptide

Genbank accession no. NP_002552
Genbank version no. NP_002552.2 GI:28416933
Genbank record update date: Jun 27, 2012 12:41 AM

### Cross references

Le et al (1997) FEBS Lett. 418(1-2):195-199; WO2004/047749; WO2003/072035 (claim 10); Touchman et al (2000) Genome Res. 10:165-173; WO2002/22660 (claim 20); WO2003/093444 (claim 1); WO2003/087768 (claim 1); WO2003/029277 (page 82)

### (32) CD72 (B-cell differentiation antigen CD72, Lyb-2); 359 aa, pl: 8.66, MW: 40225, TM: 1 5 [P] Gene Chromosome: 9p13.3).

### Nucleotide

Genbank accession no NM_001782
Genbank version no. NM_001782.2 GI:194018444
Genbank record update date: Jun 26, 2012 01:43 PM

### Polypeptide

Genbank accession no. NP_001773
Genbank version no. NP_001773.1 GI:4502683
Genbank record update date: Jun 26, 2012 01:43 PM

### Cross references

WO2004042346 (claim 65); WO2003/026493 (pages 51-52, 57-58); WO2000/75655 (pages 105-106); Von Hoegen et al (1990) J. Immunol. 144(12):4870-4877; Strausberg et al (2002) Proc. Natl. Acad. Sci USA 99:16899-16903.

### (33) LY64 (Lymphocyte antigen 64 (RP105), type I membrane protein of the leucine rich repeat (LRR) family, regulates B-cell activation and apoptosis, loss of function is associated with increased disease activity in patients with systemic lupus erythematosis); 661 aa, pl: 6.20, MW: 74147 TM: 1 [P] Gene Chromosome: 5q12).

### Nucleotide

Genbank accession no NM_005582
Genbank version no. NM_005582.2 GI:167555126
Genbank record update date: Sep 2, 2012 01:50 PM

### Polypeptide

Genbank accession no. NP_005573
Genbank version no. NP_005573.2 GI:167555127
Genbank record update date: Sep 2, 2012 01:50 PM

### Cross references

US2002/193567; WO97/07198 (claim 11, pages 39-42); Miura et al (1996) 15 Genomics 38(3):299-304; Miura et al (1998) Blood 92:2815-2822; WO2003/083047; WO97/44452 (claim 8, pages 57-61); WO2000/12130 (pages 24-26).

### (34) FcRH1 (Fc receptor-like protein 1, a putative receptor for the immunoglobulin Fc domain that contains C2 type Ig-like and ITAM domains, may have a role in B-lymphocyte 20 differentiation); 429 aa, pl: 5.28, MW: 46925 TM: 1 [P] Gene Chromosome: 1q21-1q22)

### Nucleotide

Genbank accession no NM_052938
Genbank version no. NM_052938.4 GI:226958543
Genbank record update date: Sep 2, 2012 01:43 PM

### Polypeptide

Genbank accession no. NP_443170
Genbank version no. NP_443170.1 GI:16418419
Genbank record update date: Sep 2, 2012 01:43 PM

### Cross references

WO2003/077836; WO2001/38490 (claim 6, Fig 18E-1-18-E-2); Davis et al (2001) Proc. Natl. Acad. Sci USA 98(17):9772-9777; WO2003/089624 (claim 8); EP1347046 (claim 1); WO2003/089624 (claim 7).

### (35) IRTA2 (Immunoglobulin superfamily receptor translocation associated 2, a putative immunoreceptor with possible roles in B cell development and lymphomagenesis; deregulation of the gene by translocation occurs in some B cell malignancies); 977 aa, pl: 6.88, MW: 106468, TM: 1 [P] Gene Chromosome: 1q21)

### Nucleotide

Genbank accession no AF343662
Genbank version no. AF343662.1 GI:13591709
Genbank record update date: Mar 11, 2010 01:16 AM

### Polypeptide

Genbank accession no. AAK31325
Genbank version no. AAK31325.1 GI:13591710
Genbank record update date: Mar 11, 2010 01:16 AM

### Cross references

AF343663, AF343664, AF343665, AF369794, AF397453, AK090423, AK090475, AL834187, AY358085; Mouse:AK089756, AY158090, AY506558; NP_112571.1; WO2003/024392 (claim 2, Fig 97); Nakayama et al (2000) Biochem. Biophys. Res. Commun. 277(1):124-127; WO2003/077836; WO2001/38490 (claim 3, Fig 18B-1-18B-2).

### (36) TENB2 (TMEFF2, tomoregulin, TPEF, HPP1, TR, putative transmembrane 35 proteoglycan, related to the EGF/heregulin family of growth factors and follistatin); 374 aa)

### Nucleotide

Genbank accession no AF179274
Genbank version no. AF179274.2 GI:12280939
Genbank record update date: Mar 11, 2010 01:05 AM

### Polypeptide

Genbank accession no. AAD55776
Genbank version no. AAD55776.2 GI:12280940
Genbank record update date: Mar 11, 2010 01:05 AM

### Cross references

NCBI Accession: AAD55776, AAF91397, AAG49451, NCBI RefSeq: NP_057276; NCBI Gene: 23671; OMIM: 605734; SwissProt Q9UIK5; AY358907, CAF85723, CQ782436; WO2004/074320; JP2004113151; WO2003/042661; WO2003/009814; EP1295944 (pages 69-70); WO2002/30268 (page 329); WO2001/90304; US2004/249130; US2004/022727; WO2004/063355; US2004/197325; US2003/232350; 5 US2004/005563; US2003/124579; Horie et al (2000) Genomics 67:146-152; Uchida et al (1999) Biochem. Biophys. Res. Commun. 266:593-602; Liang et al (2000) Cancer Res. 60:4907-12; Glynne-Jones et al (2001) Int J Cancer. Oct 15; 94(2):178-84.

### (37) PSMA - FOLH1 (Folate hydrolase (prostate-specific membrane antigen) 1)

### Nucleotide

Genbank accession no M99487
Genbank version no. M99487.1 GI:190663
Genbank record update date: Jun 23, 2010 08:48 AM

### Polypeptide

Genbank accession no. AAA60209
Genbank version no. AAA60209.1 GI:190664
Genbank record update date: Jun 23, 2010 08:48 AM

### Cross references

Israeli R.S., et al Cancer Res. 53 (2), 227-230 (1993)

### Other information

Official Symbol: FOLH1
Other Aliases: GIG27, FGCP, FOLH, GCP2, GCPII, NAALAD1, NAALAdase, PSM, PSMA, mGCP
Other Designations: N-acetylated alpha-linked acidic dipeptidase 1; N-acetylated-alpha-linked acidic dipeptidase I; NAALADase I; cell growth-inhibiting gene 27 protein; folylpoly-gamma-glutamate carboxypeptidase; glutamate carboxylase II; glutamate carboxypeptidase 2; glutamate carboxypeptidase II; membrane glutamate carboxypeptidase; prostate specific membrane antigen variant F; pteroylpoly-gamma-glutamate carboxypeptidase

### ANTIBODIES

US 7,666,425:
   Antibodies produces by Hybridomas having the following ATCC references:ATCC accession No. HB-12101, ATCC accession No. HB-12109, ATCC accession No. HB-12127 and ATCC accession No. HB-12126.
Proscan: a monoclonal antibody selected from the group consisting of 8H12, 3E11, 17G1, 29B4, 30C1 and 20F2 (US 7,811,564; Moffett S., et al Hybridoma (Larchmt). 2007 Dec;26(6):363-72).
Cytogen: monoclonal antibodies 7E11-C5 (ATCC accession No. HB 10494) and 9H10-A4 (ATCC accession No. HB11430) - US 5,763,202
GlycoMimetics: NUH2 - ATCC accession No. HB 9762 (US 7,135,301)
Human Genome Science: HPRAJ70 - ATCC accession No. 97131 (US 6,824,993); Amino acid sequence encoded by the cDNA clone (HPRAJ70) deposited as American Type Culture Collection ("ATCC") Deposit No. 97131
Medarex: Anti-PSMA antibodies that lack fucosyl residues - US 7,875,278

Mouse anti-PSMA antibodies include the 3F5.4G6, 3D7.1.1, 4E10-1.14, 3E11, 4D8, 3E6, 3C9, 2C7, 1G3, 3C4, 3C6, 4D4, 1G9, 5C8B9, 3G6, 4C8B9, and monoclonal antibodies. Hybridomas secreting 3F5.4G6, 3D7.1.1, 4E10-1.14, 3E11, 4D8, 3E6, 3C9, 2C7, 1G3, 3C4, 3C6, 4D4, 1G9, 5C8B9, 3G6 or 4C8B9 have been publicly deposited and are described in U.S. Pat. No. 6,159,508. Relevant hybridomas have been publicly deposited and are described in U.S. Pat. No. 6,107,090. Moreover, humanized anti-PSMA antibodies, including a humanized version of J591, are described in further detail in PCT Publication WO 02/098897.

Other mouse anti-human PSMA antibodies have been described in the art, such as mAb 107-1A4 (Wang, S. et al. (2001) Int. J. Cancer 92:871-876) and mAb 2C9 (Kato, K. et al. (2003) Int. J. Urol. 10:439-444).

Examples of human anti-PSMA monoclonal antibodies include the 4A3, 7F12, 8C12, 8A11, 16F9, 2A10, 2C6, 2F5 and 1C3 antibodies, isolated and structurally characterized as originally described in PCT Publications WO 01/09192 and WO 03/064606 and in U.S. Provisional Application Ser. No. 60/654,125, entitled "Human Monoclonal Antibodies to Prostate Specific Membrane Antigen (PSMA)", filed on Feb. 18, 2005. The V.sub.H amino acid sequences of 4A3, 7F12, 8C12, 8A11, 16F9, 2A10, 2C6, 2F5 and 1C3 are shown in SEQ ID NOs: 1-9, respectively. The V.sub.L amino acid sequences of 4A3, 7F12, 8C12, 8A11, 16F9, 2A10, 2C6, 2F5 and 1C3 are shown in SEQ ID NOs: 10-18, respectively.

Other human anti-PSMA antibodies include the antibodies disclosed in PCT Publication WO 03/034903 and US Application No. 2004/0033229.

NW Biotherapeutics: A hybridoma cell line selected from the group consisting of 3F5.4G6 having ATCC accession number HB12060, 3D7-1.I. having ATCC accession number HB12309, 4E10-1.14 having ATCC accession number HB12310, 3E11 (ATCC HB12488), 4D8 (ATCC HB12487), 3E6 (ATCC HB12486), 3C9 (ATCC HB12484), 2C7 (ATCC HB12490), 1G3 (ATCC HB12489), 3C4 (ATCC HB12494), 3C6 (ATCC HB12491), 4D4 (ATCC HB12493), 1G9 (ATCC HB12495), 5C8B9 (ATCC HB12492) and 3G6 (ATCC HB12485) - see US 6,150,508

PSMA Development Company / Progenics / Cytogen - Seattle Genetics: mAb 3.9, produced by the hybridoma deposited under ATCC Accession No. PTA-3258 or mAb 10.3, produced by the hybridoma deposited under ATCC Accession No. PTA-3347 - US 7,850,971

PSMA Development Company- Compositions of PSMA antibodies (US 20080286284, Table 1)
This application is a divisional of U.S. patent application Ser. No. 10/395,894, filed on Mar. 21, 2003 (US 7,850,971)

University Hospital Freiburg, Germany - mAbs 3/A12, 3/E7, and 3/F11 (Wolf P., et al Prostate. 2010 Apr 1;70(5):562-9).

### (38) SST (Somatostatin Receptor; note that there are5 subtypes)

### (38.1) SSTR2 (Somatostatin receptor 2)

### Nucleotide

Genbank accession no NM_001050
Genbank version no. NM_001050.2 GI:44890054
Genbank record update date: Aug 19, 2012 01:37 PM

### Polypeptide

Genbank accession no. NP_001041
Genbank version no. NP_001041.1 GI:4557859
Genbank record update date: Aug 19, 2012 01:37 PM

### Cross references

Yamada Y., et al Proc. Natl. Acad. Sci. U.S.A. 89 (1), 251-255 (1992); Susini C., et al Ann Oncol. 2006 Dec; 17(12):1733-42

### Other information

Official Symbol: SSTR2
Other Designations: SRIF-1; SS2R; somatostatin receptor type 2

### (38.2) SSTR5 (Somatostatin receptor 5)

### Nucleotide

Genbank accession no D16827
Genbank version no. D16827.1 GI:487683
Genbank record update date: Aug 1, 2006 12:45 PM

### Polypeptide

Genbank accession no. BAA04107
Genbank version no. BAA04107.1 GI:487684
Genbank record update date: Aug 1, 2006 12:45 PM

### Cross references

Yamada,Y., et al Biochem. Biophys. Res. Commun. 195 (2), 844-852 (1993)

### Other information

Official Symbol: SSTR5
Other Aliases: SS-5-R
Other Designations: Somatostatin receptor subtype 5; somatostatin receptor type 5

(38.3) SSTR1
(38.4)SSTR3
(38.5) SSTR4

### AvB6 - Both subunits (39+40)

### (39) ITGAV (Integrin, alpha V;

### Nucleotide

Genbank accession no M14648 J02826 M18365
Genbank version no. M14648.1 GI:340306
Genbank record update date: Jun 23, 2010 08:56 AM

### Polypeptide

Genbank accession no. AAA36808
Genbank version no. AAA36808.1 GI:340307
Genbank record update date: Jun 23, 2010 08:56 AM

### Cross references

Suzuki S., et al Proc. Natl. Acad. Sci. U.S.A. 83 (22), 8614-8618 (1986)

### Other information

Official Symbol: ITGAV
Other Aliases: CD51, MSK8, VNRA, VTNR
Other Designations: antigen identified by monoclonal antibody L230; integrin alpha-V; integrin alphaVbeta3; integrin, alpha V (vitronectin receptor, alpha polypeptide, antigen CD51); vitronectin receptor subunit alpha

### (40) ITGB6 (Integrin, beta 6)

### Nucleotide

Genbank accession no NM_000888
Genbank version no. NM_000888.3 GI:9966771
Genbank record update date: Jun 27, 2012 12:46 AM

### Polypeptide

Genbank accession no. NP_000879
Genbank version no. NP_000879.2 GI:9625002
Genbank record update date: Jun 27, 2012 12:46 AM

### Cross references

Sheppard D.J., et al Biol. Chem. 265 (20), 11502-11507 (1990)

### Other information

Official Symbol: ITGB6
Other Designations: integrin beta-6

### ANTIBODIES

Biogen: US 7,943,742 - Hybridoma clones 6.3G9 and 6.8G6 were deposited with the ATCC, accession numbers ATCC PTA-3649 and -3645, respectively.
Biogen: US7,465,449 - In some embodiments, the antibody comprises the same heavy and light chain polypeptide sequences as an antibody produced by hybridoma 6.1A8, 6.3G9, 6.8G6, 6.2B1, 6.2B10, 6.2A1, 6.2E5, 7.1G10, 7.7G5, or7.1C5.
Centocor (J&J): US7,550,142; US7,163,681
   For example in US 7,550,142 - an antibody having human heavy chain and human light chain variable regions comprising the amino acid sequences shown in SEQ ID NO: 7 and SEQ ID NO: 8.
Seattle Genetics: 15H3 (Ryan MC., et al Cancer Res April 15, 2012; 72(8 Supplement): 4630)

### (41) CEACAM5 (Carcinoembryonic antigen-related cell adhesion molecule 5)

### Nucleotide

Genbank accession no M17303
Genbank version no. M17303.1 GI:178676
Genbank record update date: Jun 23, 2010 08:47 AM

### Polypeptide

Genbank accession no. AAB59513
Genbank version no. AAB59513.1 GI:178677
Genbank record update date: Jun 23, 2010 08:47 AM

### Cross references

Beauchemin N., et al Mol. Cell. Biol. 7 (9), 3221-3230 (1987)

### Other information

Official Symbol: CEACAM5
Other Aliases: CD66e, CEA
Other Designations: meconium antigen 100

### ANTIBODIES

AstraZeneca-Medlmmune:US 20100330103; US20080057063;
   US20020142359
      - for example an antibody having complementarity determining regions (CDRs) with the following sequences: heavy chain; CDR1 - DNYMH, CDR2 - WIDPENGDTE YAPKFRG, CDR3 - LIYAGYLAMD Y; and light chain CDR1 - SASSSVTYMH, CDR2 - STSNLAS, CDR3 - QQRSTYPLT.
      - Hybridoma 806.077 deposited as European Collection of Cell Cultures (ECACC) deposit no. 96022936.
Research Corporation Technologies, Inc.:US5,047,507
Bayer Corporation: US6,013,772
BioAlliance: US7,982,017; US7,674,605
   - US 7,674,605
      - an antibody comprising the heavy chain variable region sequence from the amino acid sequence of SEQ ID NO: 1, and the light chain variable region sequence from the amino acid sequence of SEQ ID NO:2.
      - an antibody comprising the heavy chain variable region sequence from the amino acid sequence of SEQ ID NO:5, and the light chain variable region sequence from the amino acid sequence of SEQ ID NO:6.
Celltech Therapeutics Limited: US5,877,293
The Dow Chemical Company: US5,472,693; US6,417,337; US6,333,405
   US5,472,693 - for example, ATCC No. CRL-11215
   US6,417,337 - for example, ATCC CRL-12208
   US6,333,405 - for example, ATCC CRL-12208
Immunomedics, Inc: US7,534,431; US7,230,084; US7,300,644; US6,730,300;
   US20110189085
      - an antibody having CDRs of the light chain variable region comprise: CDR1 comprises KASQDVGTSVA (SEQ ID NO: 20); CDR2 comprises WTSTRHT (SEQ ID NO: 21); and CDR3 comprises QQYSLYRS (SEQ ID NO: 22);
         and the CDRs of the heavy chain variable region of said anti-CEA antibody comprise: CDR1 comprises TYWMS (SEQ ID NO: 23); CDR2 comprises EIHPDSSTINYAPSLKD (SEQ ID NO: 24); and CDR3 comprises LYFGFPWFAY (SEQ ID NO: 25).
   US20100221175; US20090092598; US20070202044; US20110064653;
   US20090185974; US20080069775.

### (42) MET (met proto-oncogene; hepatocyte growth factor receptor)

### Nucleotide

Genbank accession no M35073
Genbank version no. M35073.1 GI:187553
Genbank record update date: Mar 6, 2012 11:12 AM

### Polypeptide

Genbank accession no. AAA59589
Genbank version no. AAA59589.1 GI:553531
Genbank record update date: Mar 6, 2012 11:12 AM

### Cross references

Dean M., et al Nature 318 (6044), 385-388 (1985)

### Other information

Official Symbol: MET
Other Aliases: AUTS9, HGFR, RCCP2, c-Met
Other Designations: HGF receptor; HGF/SF receptor; SF receptor; hepatocyte growth factor receptor; met proto-oncogene tyrosine kinase; proto-oncogene c-Met; scatter factor receptor; tyrosine-protein kinase Met

### ANTIBODIES

Abgenix/Pfizer: US20100040629
   for example, the antibody produced by hybridoma 13.3.2 having American Type Culture Collection (ATCC) accession number PTA-5026; the antibody produced by hybridoma 9.1.2 having ATCC accession number PTA-5027; the antibody produced by hybridoma 8.70.2 having ATCC accession number PTA-5028; or the antibody produced by hybridoma 6.90.3 having ATCC accession number PTA-5029.
Amgen/Pfizer: US20050054019
   for example, an antibody comprising a heavy chain having the amino acid sequences set forth in SEQ ID NO: 2 where X2 is glutamate and X4 is serine and a light chain having the amino acid sequence set forth in SEQ ID NO: 4 where X8 is alanine, without the signal sequences; an antibody comprising a heavy chain having the amino acid sequences set forth in SEQ ID NO: 6 and a light chain having the amino acid sequence set forth in SEQ ID NO: 8, without the signal sequences; an antibody comprising a heavy chain having the amino acid sequences set forth in SEQ ID NO: 10 and a light chain having the amino acid sequence set forth in SEQ ID NO: 12, without the signal sequences; or an antibody comprising a heavy chain having the amino acid sequences set forth in SEQ ID NO: 14 and a light chain having the amino acid sequence set forth in SEQ ID NO: 16, without the signal sequences.
Agouron Pharmaceuticals (Now Pfizer): US20060035907
Eli Lilly: US20100129369
Genentech: US5,686,292; US20100028337; US20100016241; US20070129301; US20070098707; US20070092520, US20060270594; US20060134104; US20060035278; US20050233960; US20050037431
   US 5,686,292 - for example, ATCC HB-11894 and ATCC HB-11895
   US 20100016241 - for example, ATCC HB-11894 (hybridoma 1A3.3.13) or HB-11895 (hybridoma 5D5.11.6)
National Defense Medical Center, Taiwan: Lu RM., et al Biomaterials. 2011 Apr;32(12):3265-74.
Novartis: US20090175860
   - for example, an antibody comprising the sequences of CDR1, CDR2 and CDR3 of heavy chain 4687, wherein the sequences of CDR1, CDR2, and CDR3 of heavy chain 4687 are residues 26-35, 50-65, and 98-102, respectively, of SEQ ID NO: 58; and the sequences of CDR1, CDR2, and CDR3 of light chain 5097, wherein the sequences of CDR1, CDR2, and CDR3 oflight chain 5097 are residues 24-39,55-61, and 94-100 of SEQ ID NO: 37.
Pharmacia Corporation: US20040166544
Pierre Fabre: US20110239316, US20110097262, US20100115639
Sumsung: US 20110129481 - for example a monoclonal antibody produced from a hybridoma cell having accession number KCLRF-BP-00219 or accession number of KCLRF-BP-00223.
Samsung: US 20110104176 - for example an antibody produced by a hybridoma cell having Accession Number: KCLRF-BP-00220.
University of Turin Medical School: DN-30 Pacchiana G., et al J Biol Chem. 2010 Nov 12;285(46):36149-57
Van Andel Research Institute: Jiao Y., et al Mol Biotechnol. 2005 Sep;31(1):41-54.

### (43) MUC1 (Mucin 1, cell surface associated)

### Nucleotide

Genbank accession no J05581
Genbank version no. J05581.1 GI:188869
Genbank record update date: Jun 23, 2010 08:48 AM

### Polypeptide

Genbank accession no. AAA59876
Genbank version no. AAA59876.1 GI:188870
Genbank record update date: Jun 23, 2010 08:48 AM

### Cross references

Gendler S.J., et al J. Biol. Chem. 265 (25), 15286-15293 (1990)

### Other information

Official Symbol: MUC1
Other Aliases: RP11-263K19.2, CD227, EMA, H23AG, KL-6, MAM6, MUC-1, MUC-1/SEC,
MUC-1/X, MUC1/ZD, PEM, PEMT, PUM
Other Designations: DF3 antigen; H23 antigen; breast carcinoma-associated antigen DF3; carcinoma-associated mucin; episialin; krebs von den Lungen-6; mucin 1, transmembrane; mucin-1; peanut-reactive urinary mucin; polymorphic epithelial mucin; tumor associated epithelial mucin; tumor-associated epithelial membrane antigen; tumor-associated mucin

### ANTIBODIES

AltaRex- Quest Pharma Tech: US 6,716,966 - for example an Alt-1 antibody produced by the hybridoma ATCC No PTA-975.
AltaRex- Quest Pharma Tech: US7,147,850
CRT: 5E5 - Sorensen AL., et al Glycobiology vol. 16 no. 2 pp. 96-107, 2006; HMFG2 - Burchell J., et al Cancer Res., 47, 5476-5482 (1987)
Glycotope GT-MAB: GT-MAB 2.5-GEX (Website:
   http://www.glycotope.com/pipeline/pankomab-gex)
Immunogen: US7,202,346
   - for example, antibody MJ-170: hybridoma cell line MJ-170 ATCC accession no. PTA-5286Monoclonal antibody MJ-171: hybridoma cell line MJ-171 ATCC accession no. PTA-5287; monoclonal antibody MJ-172: hybridoma cell line MJ-172 ATCC accession no. PTA-5288; or monoclonal antibody MJ-173: hybridoma cell line MJ-173 ATCC accession no. PTA-5302
Immunomedics: US 6,653,104
Ramot Tel Aviv Uni: US7,897,351
Regents Uni. CA: US 7,183,388; US20040005647; US20030077676.
Roche GlycArt: US8,021,856
Russian National Cancer Research Center: Imuteran- Ivanov PK., et al Biotechnol J. 2007 Jul;2(7):863-70
Technische Univ Braunschweig: (IIB6, HT186-B7, HT186-D11, HT186-G2, HT200-3A-C1, HT220-M-D1, HT220-M-G8) - Thie H., et al PLoS One. 2011 Jan 14;6(1):e15921

### (44) CA9 (Carbonic anhydrase IX)

### Nucleotide

Genbank accession no . X66839
Genbank version no. X66839.1 GI:1000701
Genbank record update date: Feb 2, 2011 10:15 AM

### Polypeptide

Genbank accession no. CAA47315
Genbank version no. CAA47315.1 GI:1000702
Genbank record update date: Feb 2, 2011 10:15 AM

### Cross references

Pastorek J., et al Oncogene 9 (10), 2877-2888 (1994)

### Other information

Official Symbol: CA9
Other Aliases: CAIX, MN
Other Designations: CA-IX; P54/58N; RCC-associated antigen G250; RCC-associated protein G250; carbonate dehydratase IX; carbonic anhydrase 9; carbonic dehydratase; membrane antigen MN; pMW1; renal cell carcinoma-associated antigen G250

### ANTIBODIES

Abgenix/Amgen: US20040018198
Affibody: Anti-CAIX Affibody molecules (http://www.affibody.com/en/Product-Portfolio/Pipeline/)
Bayer: US7,462,696
Bayer/Morphosys: 3ee9 mAb - Petrul HM., et al Mol Cancer Ther. 2012 Feb;11(2):340-9
Harvard Medical School: Antibodies G10, G36, G37, G39, G45, G57, G106, G119, G6, G27, G40 and G125. Xu C., et al PLoS One. 2010 Mar 10;5(3):e9625
Institute of Virology, Slovak Academy of Sciences (Bayer) - US5,955,075
   - for example, M75-ATCC Accession No. HB 11128 or MN12 - ATCC Accession No. HB 11647
Institute of Virology, Slovak Academy of Sciences: US7,816,493
   - for example the M75 monoclonal antibody that is secreted from the hybridoma VU-M75, which was deposited at the American Type Culture Collection under ATCC No. HB 11128; or the V/10 monoclonal antibody secreted from the hybridoma V/10-VU, which was deposited at the International Depository Authority of the Belgian Coordinated Collection of Microorganisms (BCCM) at the Laboratorium voor Moleculaire Bioloqie-Plasmidencollectie (LMBP) at the Universeit Gent in Gent, Belgium, under Accession No. LMBP 6009CB.
Institute of Virology, Slovak Academy of Sciences US20080177046; US20080176310; US20080176258; US20050031623
Novartis: US20090252738
Wilex: US7,691,375 - for example the antibody produced by the hybridoma cell line DSM ASC 2526.
Wilex: US20110123537; Rencarex: Kennett RH., et al Curr Opin Mol Ther. 2003 Feb;5(1):70-5
Xencor: US20090162382

### (45) EGFRvIII (Epidermal growth factor receptor (EGFR), transcript variant 3,

### Nucleotide

Genbank accession no. NM_201283
Genbank version no. NM_201283.1 GI:41327733
Genbank record update date: Sep 30, 2012 01:47 PM

### Polypeptide

Genbank accession no. NP_958440
Genbank version no. NP_958440.1 GI:41327734
Genbank record update date: Sep 30, 2012 01:47 PM

### Cross-references

Batra SK., et al Cell Growth Differ 1995;6:1251-1259.

### ANTIBODIES:

US7,628,986 and US7,736,644 (Amgen)
   For example, a heavy chain variable region amino acid sequence selected from the group consisting of SEQ ID NO: 142 and variants & a light chain variable region amino acid sequence selected from the group consisting of: SEQ ID NO: 144 and variants.
US20100111979 (Amgen)
   For example, an antibody comprising a heavy chain amino acid sequence comprising:
   CDR1 consisting of a sequence selected from the group consisting of the amino acid sequences for the CDR1 region of antibodies 13.1.2 (SEQ ID NO: 138), 131 (SEQ ID NO: 2), 170 (SEQ ID NO: 4), 150 (SEQ ID NO: 5), 095 (SEQ ID NO: 7), 250 (SEQ ID NO: 9), 139 (SEQ ID NO: 10), 211 (SEQ ID NO: 12), 124 (SEQ ID NO: 13), 318 (SEQ ID NO: 15), 342 (SEQ ID NO: 16), and 333 (SEQ ID NO: 17);
   CDR2 consisting of a sequence selected from the group consisting of the amino acid sequences for the CDR2 region of antibodies 13.1.2 (SEQ ID NO: 138), 131 (SEQ ID NO: 2), 170 (SEQ ID NO: 4), 150 (SEQ ID NO: 5), 095 (SEQ ID NO: 7), 250 (SEQ ID NO: 9), 139 (SEQ ID NO: 10), 211 (SEQ ID NO: 12), 124 (SEQ ID NO: 13), 318 (SEQ ID NO: 15), 342 (SEQ ID NO: 16), and 333 (SEQ ID NO: 17); and CDR3 consisting of a sequence selected from the group consisting of the amino acid sequences for the CDR3 region of antibodies 13.1.2 (SEQ ID NO: 138), 131 (SEQ ID NO: 2), 170 (SEQ ID NO: 4), 150 (SEQ ID NO: 5), 095 (SEQ ID NO: 7), 250 (SEQ ID NO: 9), 139 (SEQ ID NO: 10), 211 (SEQ ID NO: 12), 124 (SEQ ID NO: 13), 318 (SEQ ID NO: 15), 342 (SEQ ID NO: 16), and 333 (SEQ ID NO: 17).
US20090240038 (Amgen)
   For example, an antibody having at least one of the heavy or light chain polypeptides comprises an amino acid sequence that is at least 90% identical to the amino acid sequence selected from the group consisting of: SEQ ID NO: 2, SEQ ID NO: 19, SEQ ID NO: 142, SEQ ID NO: 144, and any combination thereof.
US20090175887 (Amgen)
   For example, an antibody having a heavy chain amino acid sequence selected from the group consisting of the heavy chain amino acid sequence of antibody 13.1.2 (SEQ ID NO: 138), 131 (SEQ ID NO: 2), 170 (SEQ ID NO: 4), 150 (SEQ ID NO: 5), 095 (SEQ ID NO: 7), 250 (SEQ ID NO: 9), 139 (SEQ ID NO: 10), 211 (SEQ ID NO: 12), 124 (SEQ ID NO: 13), 318 (SEQ ID NO: 15), 342 (SEQ ID NO: 16), and 333 (SEQ ID NO: 17).
US20090156790 (Amgen)
   For example, antibody having heavy chain polypeptide and a light chain polypeptide, wherein at least one of the heavy or light chain polypeptides comprises an amino acid sequence that is at least 90% identical to the amino acid sequence selected from the group consisting of: SEQ ID NO: 2, SEQ ID NO: 19, SEQ ID NO: 142, SEQ ID NO: 144, and any combination thereof.
US20090155282, US20050059087 and US20050053608 (Amgen)
   For example, an antibody heavy chain amino acid sequence selected from the group consisting of the heavy chain amino acid sequence of antibody 13.1.2 (SEQ ID NO: 138), 131 (SEQ ID NO: 2), 170 (SEQ ID NO: 4), 150 (SEQ ID NO: 5), 095 (SEQ ID NO: 7), 250 (SEQ ID NO: 9), 139 (SEQ ID NO: 10), 211 (SEQ ID NO: 12), 124 (SEQ ID NO: 13), 318 (SEQ ID NO: 15), 342 (SEQ ID NO: 16), and 333 (SEQ ID NO: 17).
MR1-1 (US7,129,332; Duke)
   For example, a variant antibody having the sequence of SEQ ID NO.18 with the substitutions S98P-T99Y in the CDR3 VH, and F92W in CDR3 VL.
L8A4, H10, Y10 (Wikstrand CJ., et al Cancer Res. 1995 Jul 15;55(14):3140-8; Duke)
US20090311803 (Harvard University)
   For example, SEQ ID NO:9 for antibody heavy chain variable region, and SEQ ID NO: 3 for light chain variable region amino acid sequences
US20070274991 (EMD72000, also known as matuzumab; Harvard University)
   For example, SEQ ID NOs: 3 & 9 for light chain and heavy chain respectively
US6,129,915 (Schering)
   For example, SEQ. ID NOs: 1, 2, 3, 4, 5 and 6.
mAb CH12 - Wang H., et al FASEB J. 2012 Jan;26(1):73-80 (Shanghai Cancer Institute).
RAbDMvIII - Gupta P., et al BMC Biotechnol. 2010 Oct 7;10:72 (Stanford University Medical Center).
mAb Ua30 - Ohman L., et al Tumour Biol. 2002 Mar-Apr;23(2):61-9 (Uppsala University).
Han DG., et al Nan Fang Yi Ke Da Xue Xue Bao. 2010 Jan;30(1):25-9 (Xi'an Jiaotong University).

### (46) CD33 (CD33 molecule)

### Nucleotide

Genbank accession no. M_23197
Genbank version no. NM_23197.1 GI:180097
Genbank record update date: Jun 23, 2010 08:47 AM

### Polypeptide

Genbank accession no. AAA51948
Genbank version no. AAA51948.1 GI:188098
Genbank record update date: Jun 23, 2010 08:47 AM

### Cross-references

Simmons D., et al J. Immunol. 141 (8), 2797-2800 (1988)

### Other information

Official Symbol: CD33
Other Aliases: SIGLEC-3, SIGLEC3, p67
Other Designations: CD33 antigen (gp67); gp67; myeloid cell surface antigen CD33; sialic acid binding Ig-like lectin 3; sialic acid-binding Ig-like lectin

### ANTIBODIES

H195 (Lintuzumab)- Raza A., et al Leuk Lymphoma. 2009 Aug;50(8):1336-44; US6,759,045 (Seattle Genetics/Immunomedics)
mAb OKT9: Sutherland, D.R. et al. Proc Natl Acad Sci USA 78(7): 4515-4519 1981, Schneider,C., et al J Biol Chem 257, 8516-8522 (1982)
mAb E6: Hoogenboom,H.R., et al J Immunol 144, 3211-3217 (1990)
US6,590,088 (Human Genome Sciences)
   For example, SEQ ID NOs: 1 and 2 and ATCC accession no. 97521
US7,557,189 (Immunogen)
   For example, an antibody or fragment thereof comprising a heavy chain variable region which comprises three CDRs having the amino acid sequences of SEQ ID NOs:1-3 and a light chain variable region comprising three CDRs having the amino acid sequences of SEQ ID NOs:4-6.

### (47) CD19 (CD19 molecule)

### Nucleotide

Genbank accession no. NM_001178098
Genbank version no. NM_001178098.1 GI:296010920
Genbank record update date: Sep 10, 2012 12:43 AM

### Polypeptide

Genbank accession no. NP_001171569
Genbank version no. NP_001171569.1 GI:296010921
Genbank record update date: Sep 10, 2012 12:43 AM

### Cross-references

Tedder TF., et al J. Immunol. 143 (2): 712-7 (1989)

### Other information

Official Symbol: CD19
Other Aliases: B4, CVID3
Other Designations: B-lymphocyte antigen CD19; B-lymphocyte surface antigen B4; T-cell surface antigen Leu-12; differentiation antigen CD19

### ANTIBODIES

Immunogen: HuB4 - Al-Katib AM., et al Clin Cancer Res. 2009 Jun 15;15(12):4038-45.
4G7: Kügler M., et al Protein Eng Des Sel. 2009 Mar;22(3):135-47
   For example, sequences in Fig. 3 of of Knappik, A. et al. J Mol Biol 2000 Feb;296(1):57-86
AstraZeneca /Medlmmune: MEDI-551 - Herbst R., et al J Pharmacol Exp Ther. 2010 Oct;335(1):213-22
Glenmark Pharmaceuticals: GBR-401 - Hou S., et al Mol Cancer Ther November 2011 10 (Meeting Abstract Supplement) C164
US7,109,304 (Immunomedics)
   For example, an antibody comprising the sequence of hA19Vk (SEQ ID NO:7) and the sequence of hA19VH (SEQ ID NO:10)
US7,902,338 (Immunomedics)
   For example, an antibody or antigen-binding fragment thereof that comprises the light chain complementarity determining region CDR sequences CDR1 of SEQ ID NO: 16 (KASQSVDYDGDSYLN); CDR2 of SEQ ID NO: 17 (DASNLVS); and CDR3 of SEQ ID NO: 18 (QQSTEDPWT) and the heavy chain CDR sequences CDR1 of SEQ ID NO: 19 (SYWMN); CDR2 of SEQ ID NO: 20 (QIWPGDGDTNYNGKFKG) and CDR3 of SEQ ID NO: 21 (RETTTVGRYYYAMDY) and also comprises human antibody framework (FR) and constant region sequences with one or more framework region amino acid residues substituted from the corresponding framework region sequences of the parent murine antibody, and wherein said substituted FR residues comprise the substitution of serine for phenylalanine at Kabat residue 91 of the heavy chain variable region.
Medarex: MDX-1342 - Cardarelli PM., et al Cancer Immunol Immunother. 2010 Feb;59(2):257-65.
MorphoSys /Xencor: MOR-208/XmAb-5574 - Zalevsky J., et al Blood. 2009 Apr 16; 113(16):3735-43
US7,968,687 (Seattle Genetics)
   An antibody or antigen-binding fragment comprising a heavy chain variable domain comprising the amino acid sequence of SEQ ID NO:9 and a light chain variable domain comprising the amino acid sequence of SEQ ID NO: 24.
4G7 chim - Lang P., et al Blood. 2004 May 15;103(10):3982-5 (University of Tübingen)
   For example, fig. 6 and SEQ ID No: 80 of US20120082664
Zhejiang University School of Medicine: 2E8 - Zhang J., et al J Drug Target. 2010 Nov; 18(9):675-8

### (48) IL2RA (Interleukin 2 receptor, alpha); NCBI Reference Sequence: NM_000417.2);

### Nucleotide

Genbank accession no. NM_000417
Genbank version no. NM_000417.2 GI:269973860
Genbank record update date: Sep 09, 2012 04:59 PM

### Polypeptide

Genbank accession no. NP_000408
Genbank version no. NP_000408.1 GI:4557667
Genbank record update date: Sep 09, 2012 04:59 PM

### Cross-references

Kuziel W.A., et al J. Invest. Dermatol. 94 (6 SUPPL), 27S-32S (1990)

### Other information

Official Symbol: IL2RA
Other Aliases: RP11-536K7.1, CD25, IDDM10, IL2R, TCGFR
Other Designations: FIL-2 receptor subunit alpha; IL-2-RA; IL-2R subunit alpha; IL2-RA; TAC antigen; interleukin-2 receptor subunit alpha; p55

### ANTIBODIES

US6,383,487 (Novartis/UCL: Baxilisimab [Simulect])
US6,521,230 (Novartis/UCL: Baxilisimab [Simulect])
   For example, an antibody having an antigen binding site comprises at least one domain which comprises CDR1 having the amino acid sequence in SEQ. ID. NO: 7, CDR2 having the amino acid sequence in SEQ. ID. NO: 8, and CDR3 chaving the amino acid sequence in SEQ. ID. NO: 9; or said CDR1, CDR2 and CDR3 taken in sequence as a whole comprise an amino acid sequence which is at least 90% identical to SEQ. ID. NOs: 7, 8 and 9 taken in sequence as a whole.
Daclizumab - Rech AJ., et al Ann N Y Acad Sci. 2009 Sep; 1174:99-106 (Roche)

### (49) AXL (AXL receptor tyrosine kinase)

### Nucleotide

Genbank accession no. M76125
Genbank version no. M76125.1 GI:292869
Genbank record update date: Jun 23, 2010 08:53 AM

### Polypeptide

Genbank accession no. AAA61243
Genbank version no. AAA61243.1 GI:29870
Genbank record update date: Jun 23, 2010 08:53 AM

### Cross-references

O'Bryan J.P., et al Mol. Cell. Biol. 11 (10), 5016-5031 (1991); Bergsagel P.L., et al J. Immunol. 148 (2), 590-596 (1992)

### Other information

Official Symbol: AXL
Other Aliases: JTK11, UFO
Other Designations: AXL oncogene; AXL transforming sequence/gene; oncogene AXL; tyrosine-protein kinase receptor UFO

### ANTIBODIES

YW327.6S2 - Ye X., et al Oncogene. 2010 Sep 23;29(38):5254-64. (Genentech)
BergenBio: BGB324 (http://www.bergenbio.com/BGB324)

### (50) CD30 - TNFRSF8 (Tumor necrosis factor receptor superfamily, member 8)

### Nucleotide

Genbank accession no. M83554
Genbank version no. M83554.1 GI:180095
Genbank record update date: Jun 23, 2010 08:53 AM

### Polypeptide

Genbank accession no. AAA51947
Genbank version no. AAA51947.1 GI:180096
Genbank record update date: Jun 23, 2010 08:53 AM

### Cross-references

Durkop H., et al Cell 68 (3), 421-427 (1992)

### Other information

Official Symbol: TNFRSF8
Other Aliases: CD30, D1S166E, Ki-1
Other Designations: CD30L receptor; Ki-1 antigen; cytokine receptor CD30; lymphocyte activation antigen CD30; tumor necrosis factor receptor superfamily member 8

### (51) BCMA (B-cell maturation antigen) - TNFRSF17 (Tumor necrosis factor receptor superfamily, member 17)

### Nucleotide

Genbank accession no. Z29574
Genbank version no. Z29574.1 GI:471244
Genbank record update date: Feb 02, 2011 10:40 AM

### Polypeptide

Genbank accession no. CAA82690
Genbank version no. CAA82690.1 GI:471245
Genbank record update date: Feb 02, 2011 10:40 AM

### Cross-references

Laabi Y., et al Nucleic Acids Res. 22 (7), 1147-1154 (1994)

### Other information

Official Symbol: TNFRSF17
Other Aliases: BCM, BCMA, CD269
Other Designations: B cell maturation antigen; B-cell maturation factor; B-cell maturation protein; tumor necrosis factor receptor superfamily member 17

### (52) CT Ags - CTA (Cancer Testis Antigens)

### Cross-references

Fratta E., et al. Mol Oncol. 2011 Apr;5(2):164-82; Lim SH., at al Am J Blood Res. 2012;2(1):29-35.

### (53) CD174 (Lewis Y) - FUT3 (fucosyltransferase 3 (galactoside 3(4)-L-fucosyltransferase, Lewis blood group)

### Nucleotide

Genbank accession no. NM000149
Genbank version no. NM000149.3 GI:148277008
Genbank record update date: Jun 26, 2012 04:49 PM

### Polypeptide

Genbank accession no. NP_000140
Genbank version no. NP_000140.1 GI:4503809
Genbank record update date: Jun 26, 2012 04:49 PM

### Cross-references

Kukowska-Latallo,J.F., et al Genes Dev. 4 (8), 1288-1303 (1990)

### Other information

Official Symbol: FUT3
Other Aliases: CD174, FT3B, FucT-III, LE, Les
Other Designations: Lewis FT; alpha-(1,3/1,4)-fucosyltransferase; blood group Lewis alpha-4-fucosyltransferase; fucosyltransferase III; galactoside 3(4)-L-fucosyltransferase

### (54) CLEC14A (C-type lectin domain family 14, member A; Genbank accession no. NM175060)

### Nucleotide

Genbank accession no. NM175060
Genbank version no. NM175060.2 GI:371123930
Genbank record update date: Apr 01, 2012 03:34 PM

### Polypeptide

Genbank accession no. NP_778230
Genbank version no. NP_778230.1 GI:28269707
Genbank record update date: Apr 01, 2012 03:34 PM

### Other information

Official Symbol: CLEC14A
Other Aliases: UNQ236/PRO269, C14orf27, CEG1, EGFR-5
Other Designations: C-type lectin domain family 14 member A; CIECT and EGF-like domain containing protein; epidermal growth factor receptor 5

### (55) GRP78 - HSPA5 (heat shock 70kDa protein 5 (glucose-regulated protein, 78kDa)

### Nucleotide

Genbank accession no. NM005347
Genbank version no. NM005347.4 GI:305855105
Genbank record update date: Sep 30, 2012 01:42 PM

### Polypeptide

Genbank accession no. NP_005338
Genbank version no. NP_005338.1 GI:16507237
Genbank record update date: Sep 30, 2012 01:42 PM

### Cross-references

Ting J., et al DNA 7 (4), 275-286 (1988)

### Other infromation

Official Symbol: HSPA5
Other Aliases: BIP, GRP78, MIF2
Other Designations: 78 kDa glucose-regulated protein; endoplasmic reticulum lumenal Ca(2+)-binding protein grp78; immunoglobulin heavy chain-binding protein

### (56) CD70 (CD70 molecule) L08096

### Nucleotide

Genbank accession no. L08096
Genbank version no. L08096.1 GI:307127
Genbank record update date: Jun 23, 2012 08:54 AM

### Polypeptide

Genbank accession no. AAA36175
Genbank version no. AAA36175.1 GI:307128
Genbank record update date: Jun 23, 2012 08:54 AM

### Cross-references

Goodwin R.G., et al Cell 73 (3), 447-456 (1993)

### Other information

Official Symbol: CD70
Other Aliases: CD27L, CD27LG, TNFSF7
Other Designations: CD27 ligand; CD27-L; CD70 antigen; Ki-24 antigen; surface antigen CD70; tumor necrosis factor (ligand) superfamily, member 7; tumor necrosis factor ligand superfamily member 7

### ANTIBODIES

MDX-1411 against CD70 (Medarex)
h1F6 (Oflazoglu, E., et al, Clin Cancer Res. 2008 Oct 1;14(19):6171-80; Seattle Genetics)
   For example, see US20060083736 SEQ ID NOs: 1, 2, 11 and 12 and Fig. 1.

### (57) Stem Cell specific antigens. For example:

- 5T4 (see entry (63) below)
- CD25 (see entry (48) above)
- CD32
   ∘ Polypeptide
      ▪ Genbank accession no. ABK42161
      ▪ Genbank version no. ABK42161.1 GI:117616286
      ▪ Genbank record update date: Jul 25, 2007 03:00 PM
- LGR5/GPR49
   ∘ Nucleotide
      ▪ Genbank accession no. NM_003667
      ▪ Genbank version no. NM_003667.2 GI:24475886
      ▪ Genbank record update date: Jul 22, 2012 03:38 PM
   ∘ Polypeptide
      ▪ Genbank accession no. NP_003658
      ▪ Genbank version no. NP_003658.1 GI:4504379
      ▪ Genbank record update date: Jul 22, 2012 03:38 PM
- Prominin/CD133
   ∘ Nucleotide
      ▪ Genbank accession no. NM_006017
      ▪ Genbank version no. NM_006017.2 GI:224994187
      ▪ Genbank record update date: Sep 30, 2012 01:47 PM
   ∘ Polypeptide
      ▪ Genbank accession no. NP_006008
      ▪ Genbank version no. NP_006008.1 GI:5174387
      ▪ Genbank record update date: Sep 30, 2012 01:47 PM

### (58) ASG-5

### Cross-references

(Smith L.M., et.al AACR 2010 Annual Meeting (abstract #2590); Gudas J.M., et.al. AACR 2010 Annual Meeting (abstract #4393)

### ANTIBODIES

Anti- AGS-5 Antibody: M6.131 (Smith, L.M., et.al AACR 2010 Annual Meeting (abstract #2590)

### (59) ENPP3 (Ectonucleotide pyrophosphatase/phosphodiesterase 3)

### Nucleotide

Genbank accession no. AF005632
Genbank version no. AF005632.2 GI:4432589
Genbank record update date: Mar 10, 2010 09:41 PM

### Polypeptide

Genbank accession no. AAC51813
Genbank version no. AAC51813.1 GI:2465540
Genbank record update date: Mar 10, 2010 09:41 PM

### Cross-references

Jin-Hua P., et al Genomics 45 (2), 412-415 (1997)

### Other information

Official Symbol: ENPP3
Other Aliases: RP5-988G15.3, B10, CD203c, NPP3, PD-IBETA, PDNP3
Other Designations: E-NPP 3; dJ1005H11.3 (phosphodiesterase I/nucleotide pyrophosphatase 3); dJ914N13.3 (phosphodiesterase I/nucleotide pyrophosphatase 3); ectonucleotide pyrophosphatase/phosphodiesterase family member 3; gp130RB13-6; phosphodiesterase I beta; phosphodiesterase I/nucleotide pyrophosphatase 3; phosphodiesterase-I beta

### (60) PRR4 (Proline rich 4 (lacrimal))

### Nucleotide

Genbank accession no. NM_007244
Genbank version no. NM_007244.2 GI:154448885
Genbank record update date: Jun 28, 2012 12:39 PM

### Polypeptide

Genbank accession no. NP_009175
Genbank version no. NP_009175.2 GI:154448886
Genbank record update date: Jun 28, 2012 12:39 PM

### Cross-references

Dickinson D.P., et al Invest. Ophthalmol. Vis. Sci. 36 (10), 2020-2031 (1995)

### Other information

Official Symbol: PRR4
Other Aliases: LPRP, PROL4
Other Designations: lacrimal proline-rich protein; nasopharyngeal carcinoma-associated proline-rich protein 4; proline-rich polypeptide 4; proline-rich protein 4

### (61) GCC - GUCY2C (guanylate cyclase 2C (heat stable enterotoxin receptor)

### Nucleotide

Genbank accession no. NM_004963
Genbank version no. NM_004963.3 GI:222080082
Genbank record update date: Sep 02, 2012 01:50 PM

### Polypeptide

Genbank accession no. NP_004954
Genbank version no. NP_004954.2 GI:222080083
Genbank record update date: Sep 02, 2012 01:50 PM

### Cross-references

De Sauvage F.J., et al J. Biol. Chem. 266 (27), 17912-17918 (1991); Singh S., et al Biochem. Biophys. Res. Commun. 179 (3), 1455-1463 (1991)

### Other information

Official Symbol: GUCY2C
Other Aliases: DIAR6, GUC2C, MUCIL, STAR
Other Designations: GC-C; STA receptor; guanylyl cyclase C; hSTAR; heat-stable enterotoxin receptor; intestinal guanylate cyclase

### (62) Liv-1 - SLC39A6 (Solute carrier family 39 (zinc transporter), member 6)

### Nucleotide

Genbank accession no. U41060
Genbank version no. U41060.2 GI:12711792
Genbank record update date: Nov 30, 2009 04:35 PM

### Polypeptide

Genbank accession no. AAA96258
Genbank version no. AAA96258.2 GI:12711793
Genbank record update date: Nov 30, 2009 04:35 PM

### Cross-references

Taylor KM., et al Biochim Biophys Acta. 2003 Apr 1;1611(1-2):16-30

### Other information

Official Symbol: SLC39A6
Other Aliases: LIV-1
Other Designations: LIV-1 protein, estrogen regulated; ZIP-6; estrogen-regulated protein LIV-1; solute carrier family 39 (metal ion transporter), member 6; solute carrier family 39 member 6; zinc transporter ZIP6; zrt- and Irt-like protein 6

### (63) 5T4, Trophoblast glycoprotein, TPBG - TPBG (trophoblast glycoprotein)

### Nucleotide

Genbank accession no. AJ012159
Genbank version no. AJ012159.1 GI:3805946
Genbank record update date: Feb 01, 2011 10:27 AM

### Polypeptide

Genbank accession no. CAA09930
Genbank version no. CAA09930.1 GI:3805947
Genbank record update date: Feb 01, 2011 10:27 AM

### Cross-references

King K.W.,et al Biochim. Biophys. Acta 1445 (3), 257-270 (1999)

### Other information

- Official Symbol: TPBG
- Other Aliases: 5T4, 5T4AG, M6P1
- Other Designations: 5T4 oncofetal antigen; 5T4 oncofetal trophoblast glycoprotein; 5T4 oncotrophoblast glycoprotein

### (64) CD56 - NCMA1 (Neural cell adhesion molecule 1)

### Nucleotide

Genbank accession no. NM_000615
Genbank version no. NM_000615.6 GI:336285433
Genbank record update date: Sep 23, 2012 02:32 PM

### Polypeptide

Genbank accession no. NP_000606
Genbank version no. NP_000606.3 GI:94420689
Genbank record update date: Sep 23, 2012 02:32 PM

### Cross-references

Dickson, G., et al, Cell 50 (7), 1119-1130 (1987)

### Other information

Official Symbol: NCAM1
Other Aliases: CD56, MSK39, NCAM
Other Designations: antigen recognized by monoclonal antibody 5.1H11; neural cell adhesion molecule, NCAM

### ANTIBODIES

Immunogen: HuN901 (Smith SV., et al Curr Opin Mol Ther. 2005 Aug;7(4):394-401)
For example, see humanized from murine N901 antibody. See Fig. 1b and 1e of Roguska, M.A., et al. Proc Natl Acad Sci USA Feb 1994;91:969-973.

### (65) CanAg (Tumor associated antigen CA242)

### Cross-references

Haglund C., et al Br J Cancer 60:845-851, 1989;Baeckstrom D., et al J Biol Chem 266:21537-21547, 1991

### ANTIBODIES

huC242 (Tolcher AW et al., J Clin Oncol. 2003 Jan 15;21(2):211-22; Immunogen)
For example, see US20080138898A1 SEQ ID NO: 1 and 2

### (66) FOLR1 (Folate Receptor 1)

### Nucleotide

Genbank accession no. J05013
Genbank version no. J05013.1 GI:182417
Genbank record update date: Jun 23, 2010 08:47 AM

### Polypeptide

Genbank accession no. AAA35823
Genbank version no. AAA35823.1 GI:182418
Genbank record update date: Jun 23, 2010 08:47 AM

### Cross-references

Elwood P.C., et al J. Biol. Chem. 264 (25), 14893-14901 (1989)

### Other information

Official Symbol: FOLR1
Other Aliases: FBP, FOLR
Other Designations: FR-alpha; KB cells FBP; adult folate-binding protein; folate binding protein; folate receptor alpha; folate receptor, adult; ovarian tumor-associated antigen MOv18

### ANTIBODIES

M9346A - Whiteman KR., et al Cancer Res April 15, 2012; 72(8 Supplement): 4628 (Immunogen)

### (67) GPNMB (Glycoprotein (transmembrane) nmb)

### Nucleotide

Genbank accession no. X76534
Genbank version no. X76534.1 GI:666042
Genbank record update date: Feb 02, 2011 10:10 AM

### Polypeptide

Genbank accession no. CAA54044
Genbank version no. CAA54044.1 GI:666043
Genbank record update date: Feb 02, 2011 10:10 AM

### Cross-references

Weterman M.A., et al Int. J. Cancer 60 (1), 73-81 (1995)

### Other information

Official Symbol: GPNMB
Other Aliases: UNQ1725/PRO9925, HGFIN, NMB
Other Designations: glycoprotein NMB; glycoprotein nmb-like protein; osteoactivin; transmembrane glycoprotein HGFIN; transmembrane glycoprotein NMB

### ANTIBODIES

Celldex Therapeutics: CR011 (Tse KF., et al Clin Cancer Res. 2006 Feb 15;12(4):1373-82)
For example, see EP1827492B1 SEQ ID NO: 22, 24, 26, 31, 33 and 35

### (68) TIM-1 - HAVCR1 (Hepatitis A virus cellular receptor 1)

### Nucleotide

Genbank accession no. AF043724
Genbank version no. AF043724.1 GI:2827453
Genbank record update date: Mar 10, 2010 06:24 PM

### Polypeptide

Genbank accession no. AAC39862
Genbank version no. AAC39862.1 GI:2827454
Genbank record update date: Mar 10, 2010 06:24 PM

### Cross-references

Feigelstock D., et al J. Virol. 72 (8), 6621-6628 (1998)

### Other information

Official Symbol: HAVCR1
Other Aliases: HAVCR, HAVCR-1, KIM-1, KIM1, TIM, TIM-1, TIM1, TIMD-1, TIMD1
Other Designations: T cell immunoglobin domain and mucin domain protein 1; T-cell membrane protein 1; kidney injury molecule 1

### (69) RG-1/Prostate tumor target Mindin - Mindin/RG-1

### Cross-references

Parry R., et al Cancer Res. 2005 Sep 15;65(18):8397-405

### (70) B7-H4 - VTCN1 (V-set domain containing T cell activation inhibitor 1

### Nucleotide

Genbank accession no. BX648021
Genbank version no. BX648021.1 GI:34367180
Genbank record update date: Feb 02, 2011 08:40 AM

### Cross-references

Sica GL., et al Immunity. 2003 Jun;18(6):849-61

### Other information

Official Symbol: VTCN1
Other Aliases: RP11-229A19.4, B7-H4, B7H4, B7S1, B7X, B7h.5, PRO1291, VCTN1
Other Designations: B7 family member, H4; B7 superfamily member 1; T cell costimulatory molecule B7x; T-cell costimulatory molecule B7x; V-set domain-containing T-cell activation inhibitor 1; immune costimulatory protein B7-H4

### (71) PTK7 (PTK7 protein tyrosine kinase 7)

### Nucleotide

Genbank accession no. AF447176
Genbank version no. AF447176.1 GI:17432420
Genbank record update date: Nov 28, 2008 01:51 PM

### Polypeptide

Genbank accession no. AAL39062
Genbank version no. AAL39062.1 GI:17432421
Genbank record update date: Nov 28, 2008 01:51 PM

### Cross-references

Park S.K.,et al J. Biochem. 119 (2), 235-239 (1996)

### Other information

Official Symbol: PTK7
Other Aliases: CCK-4, CCK4
Other Designations: colon carcinoma kinase 4; inactive tyrosine-protein kinase 7; pseudo tyrosine kinase receptor 7; tyrosine-protein kinase-like 7

### (72) CD37 (CD37 molecule)

### Nucleotide

Genbank accession no. NM_001040031
Genbank version no. NM_001040031.1 GI:91807109
Genbank record update date: Jul 29, 2012 02:08 PM

### Polypeptide

Genbank accession no. NP_001035120
Genbank version no. NP_001035120.1 GI:91807110
Genbank record update date: Jul 29, 2012 02:08 PM

### Cross-references

Schwartz-Albiez R., et al J. Immunol. 140 (3), 905-914 (1988)

### Other information

Official Symbol: CD37
Other Aliases: GP52-40, TSPAN26
Other Designations: CD37 antigen; cell differentiation antigen 37; leukocyte antigen CD37; leukocyte surface antigen CD37; tetraspanin-26; tspan-26

### ANTIBODIES

Boehringer Ingelheim: mAb 37.1 (Heider KH., et al Blood. 2011 Oct 13;118(15):4159-68)
Trubion: CD37-SMIP (G28-1 scFv-Ig) ((Zhao X., et al Blood. 2007; 110: 2569-2577)
   For example, see US20110171208A1 SEQ ID NO: 253
Immunogen: K7153A (Deckert J., et al Cancer Res April 15, 2012; 72(8 Supplement): 4625)

### (73) CD138 - SDC1 (syndecan 1)

### Nucleotide

Genbank accession no. AJ551176
Genbank version no. AJ551176.1 GI:29243141
Genbank record update date: Feb 01, 2011 12:09 PM

### Polypeptide

Genbank accession no. CAD80245
Genbank version no. CAD80245.1 GI:29243142
Genbank record update date: Feb 01, 2011 12:09 PM

### Cross-references

O'Connell FP., et al Am J Clin Pathol. 2004 Feb;121(2):254-63

### Other information

Official Symbol: SDC1
Other Aliases: CD138, SDC, SYND1, syndecan
Other Designations: CD138 antigen; heparan sulfate proteoglycan fibroblast growth factor receptor; syndecan proteoglycan 1; syndecan-1

### ANTIBODIES

Biotest: chimerized MAb (nBT062) - (Jagannath S., et al Poster ASH #3060, 2010; WIPO
Patent Application WO/2010/128087)
   For example, see US20090232810 SEQ ID NO: 1 and 2
Immunogen: B-B4 (Tassone P., et al Blood 104_3688-3696)
   For example, see US20090175863A1 SEQ ID NO: 1 and 2

### (74) CD74 (CD74 molecule, major histocompatibility complex, class II invariant chain)

### Nucleotide

Genbank accession no. NM_004355
Genbank version no. NM_004355.1 GI:343403784
Genbank record update date: Sep 23, 2012 02:30 PM

### Polypeptide

Genbank accession no. NP_004346
Genbank version no. NP_004346.1 GI:10835071
Genbank record update date: Sep 23, 2012 02:30 PM

### Cross-references

Kudo,J., et al Nucleic Acids Res. 13 (24), 8827-8841 (1985)

### Other information

Official Symbol: CD74
Other Aliases: DHLAG, HLADG, II, Ia-GAMMA
Other Designations: CD74 antigen (invariant polypeptide of major histocompatibility complex, class II antigen-associated); HLA class II histocompatibility antigen gamma chain; HLA-DR antigens-associated invariant chain; HLA-DR-gamma; la-associated invariant chain; MHC HLA-DR gamma chain; gamma chain of class II antigens; p33

### ANTIBODIES

Immunomedics: hLL1 (Milatuzumab,) - Berkova Z., et al Expert Opin Investig Drugs. 2010 Jan;19(1):141-9)
   For example, see US20040115193 SEQ ID NOs: 19, 20, 21, 22, 23 and 24
Genmab: HuMax-CD74 (see website)

### (75) Claudins - CLs (Claudins)

### Cross-references

Offner S., et al Cancer Immunol Immunother. 2005 May; 54(5):431-45, Suzuki H., et al Ann N YAcad Sci. 2012 Jul;1258:65-70)

In humans, 24 members of the family have been described - see literature reference.

### (76) EGFR (Epidermal growth factor receptor)

### Nucleotide

Genbank accession no. NM_005228
Genbank version no. NM_005228.3 GI:41927737
Genbank record update date: Sep 30, 2012 01:47 PM

### Polypeptide

Genbank accession no. NP_005219
Genbank version no. NP_005219.2 GI:29725609
Genbank record update date: Sep 30, 2012 01:47 PM

### Cross-references

Dhomen NS., et al Crit Rev Oncog. 2012;17(1):31-50

### Other information

Official Symbol: EGFR
Other Aliases: ERBB, ERBB1, HER1, PIG61, mENA
Other Designations: avian erythroblastic leukemia viral (v-erb-b) oncogene homolog; cell growth inhibiting protein 40; cell proliferation-inducing protein 61; proto-oncogene c-ErbB-1; receptor tyrosine-protein kinase erbB-1

### ANTIBODIES

BMS: Cetuximab (Erbitux) - Broadbridge VT., et al Expert Rev Anticancer Ther. 2012 May;12(5):555-65.
   For example, see US6217866 - ATTC deposit No. 9764.
Amgen: Panitumumab (Vectibix) - Argiles G., et al Future Oncol. 2012 Apr;8(4):373-89
   For example, see US6235883 SEQ ID NOs: 23-38.
Genmab: Zalutumumab - Rivera F., et al Expert Opin Biol Ther. 2009 May;9(5):667-74.
YM Biosciences: Nimotuzumab - Ramakrishnan MS., et al MAbs. 2009 Jan-Feb;1(1):41-8.
   For example, see US5891996 SEQ ID NOs: 27-34.

### (77) Her3 (ErbB3) - ERBB3 (v-erb-b2 erythroblastic leukemia viral oncogene homolog 3 (avian))

### Nucleotide

Genbank accession no. M34309
Genbank version no. M34309.1 GI:183990
Genbank record update date: Jun 23, 2010 08:47 PM

### Polypeptide

Genbank accession no. AAA35979
Genbank version no. AAA35979.1 GI:306841
Genbank record update date: Jun 23, 2010 08:47 PM

### Cross-references

Plowman,G.D., et al., Proc. Natl. Acad. Sci. U.S.A. 87 (13), 4905-4909 (1990)

### Other information

Official Symbol: ERBB3
Other Aliases: ErbB-3, HER3, LCCS2, MDA-BF-1, c-erbB-3, c-erbB3, erbB3-S, p180-ErbB3, p45-sErbB3, p85-sErbB3
Other Designations: proto-oncogene-like protein c-ErbB-3; receptor tyrosine-protein kinase erbB-3; tyrosine kinase-type cell surface receptor HER3

### ANTIBODIES

Merimack Pharma : MM-121 (Schoeberl B., et al Cancer Res. 2010 Mar 15;70(6):2485-2494)
For example, see US2011028129 SEQ ID NOs: 1, 2, 3, 4, 5, 6, 7 and 8.

### (78) RON - MST1R (macrophage stimulating 1 receptor (c-met-related tyrosine kinase))

### Nucleotide

Genbank accession no. X70040
Genbank version no. X70040.1 GI:36109
Genbank record update date: Feb 02, 2011 10:17 PM

### Polypeptide

Genbank accession no. CCA49634
Genbank version no. CCA49634.1 GI:36110
Genbank record update date: Feb 02, 2011 10:17 PM

### Cross-references

Ronsin C., et al Oncogene 8 (5), 1195-1202 (1993)

### Other information

Official Symbol: MST1R
Other Aliases: CD136, CDw136, PTK8, RON
Other Designations: MSP receptor; MST1R variant RON30; MST1R variant RON62; PTK8 protein tyrosine kinase 8; RON variant E2E3; c-met-related tyrosine kinase; macrophage-stimulating protein receptor; p185-Ron; soluble RON variant 1; soluble RON variant 2; soluble RON variant 3; soluble RONvariant 4

### (79) EPHA2 (EPH receptor A2)

### Nucleotide

Genbank accession no. BC037166
Genbank version no. BC037166.2 GI:33879863
Genbank record update date: Mar 06, 2012 01:59 PM

### Polypeptide

Genbank accession no. AAH37166
Genbank version no. AAH37166.1 GI:22713539
Genbank record update date: Mar 06, 2012 01:59 PM

### Cross-references

Strausberg R.L., et al Proc. Natl. Acad. Sci. U.S.A. 99 (26), 16899-16903 (2002)

### Other information

Official Symbol: EPHA2
Other Aliases: ARCC2, CTPA, CTPP1, ECK
Other Designations: ephrin type-A receptor 2; epithelial cell receptor protein tyrosine kinase; soluble EPHA2 variant 1; tyrosine-protein kinase receptor ECK

### ANTIBODIES

Medimmune: 1C1 (Lee JW., et al Clin Cancer Res. 2010 May 1;16(9):2562-2570)
For example, see US20090304721A1 Fig. 7 and 8.

### (80) CD20 - MS4A1 (membrane-spanning 4-domains, subfamily A, member 1)

### Nucleotide

Genbank accession no. M27394
Genbank version no. M27394.1 GI:179307
Genbank record update date: Nov 30, 2009 11:16 AM

### Polypeptide

Genbank accession no. AAA35581
Genbank version no. AAA35581.1 GI:179308
Genbank record update date: Nov 30, 2009 11:16 AM

### Cross-references

Tedder T.F., et al Proc. Natl. Acad. Sci. U.S.A. 85 (1), 208-212 (1988)

### Other information

Official Symbol: MS4A1
Other Aliases: B1, Bp35, CD20, CVID5, LEU-16, MS4A2, S7
Other Designations: B-lymphocyte antigen CD20; B-lymphocyte cell-surface antigen B1; CD20 antigen; CD20 receptor; leukocyte surface antigen Leu-16

### ANTIBODIES

Genentech/Roche: Rituximab - Abdulla NE., et al BioDrugs. 2012 Apr 1;26(2):71-82.
   For example, see US5736137, ATCC deposit No. HB-69119.
GSK/Genmab: Ofatumumab - Nightingale G., et al Ann Pharmacother. 2011 Oct;45(10):1248-55.
   For example, see US20090169550A1 SEQ ID NOs: 2, 4 and 5.
Immunomedics: Veltuzumab - Goldenberg DM., et al Leuk Lymphoma. 2010 May;51(5):747-55.
   For example, see US7919273B2 SEQ ID NOs: 1, 2, 3, 4, 5 and 6.

### (81) Tenascin C - TNC (Tenascin C)

### Nucleotide

Genbank accession no. NM_002160
Genbank version no. NM_002160.3 GI:340745336
Genbank record update date: Sep 23, 2012 02:33 PM

### Polypeptide

Genbank accession no. NP_002151
Genbank version no. NP_002151.2 GI:153946395
Genbank record update date: Sep 23, 2012 02:33 PM

### Cross-references

Nies D.E., et al J. Biol. Chem. 266 (5), 2818-2823 (1991); Siri A., et al Nucleic Acids Res. 19 (3), 525-531 (1991)

### Other information

Official Symbol: TNC
Other Aliases: 150-225, GMEM, GP, HXB, JI, TN, TN-C
Other Designations: GP 150-225; cytotactin; glioma-associated-extracellular matrix antigen; hexabrachion (tenascin); myotendinous antigen; neuronectin; tenascin; tenascin-C isoform 14/AD1/16

### ANTIBODIES

Philogen : G11 (von Lukowicz T., et al J Nucl Med. 2007 Apr;48(4):582-7) and F16 (Pedretti M., et al Lung Cancer. 2009 Apr;64(1):28-33)
For example, see US7968685 SEQ ID NOs: 29, 35, 45 and 47.

### (82) FAP (Fibroblast activation protein, alpha)

### Nucleotide

Genbank accession no. U09278
Genbank version no. U09278.1 GI:1888315
Genbank record update date: Jun 23, 2010 09:22 AM

### Polypeptide

Genbank accession no. AAB49652
Genbank version no. AAB49652.1 GI:1888316
Genbank record update date: Jun 23, 2010 09:22 AM

### Cross-references

Scanlan,M.J.,et al Proc. Natl. Acad. Sci. U.S.A. 91 (12), 5657-5661 (1994)

### Other information

Official Symbol: FAP
Other Aliases: DPPIV, FAPA
Other Designations: 170 kDa melanoma membrane-bound gelatinase; integral membrane serine protease; seprase

### (83) DKK-1 (Dickkopf 1 homolog (Xenopus laevis)

### Nucleotide

Genbank accession no. NM_012242
Genbank version no. NM_012242.2 GI:61676924
Genbank record update date: Sep 30, 2012 01:48 PM

### Polypeptide

Genbank accession no. NP_036374
Genbank version no. NP_036374.1 GI:7110719
Genbank record update date: Sep 30, 2012 01:48 PM

### Cross-references

Fedi P. et al J. Biol. Chem. 274 (27), 19465-19472 (1999)

### Other information

Official Symbol: DKK1
Other Aliases: UNQ492/PRO1008, DKK-1, SK
Other Designations: dickkopf related protein-1; dickkopf-1 like; dickkopf-like protein 1; dickkopf-related protein 1; hDkk-1

### ANTIBODIES

Novartis: BHQ880 (Fulciniti M., et al Blood. 2009 Jul 9;114(2):371-379)
For example, see US20120052070A1 SEQ ID NOs: 100 and 108.

### (84) CD52 (CD52 molecule)

### Nucleotide

Genbank accession no. NM_001803
Genbank version no. NM_001803.2 GI:68342029
Genbank record update date: Sep 30, 2012 01:48 PM

### Polypeptide

Genbank accession no. NP_001794
Genbank version no. NP_001794.2 GI:68342030
Genbank record update date: Sep 30, 2012 01:48 PM

### Cross-references

Xia M.Q., et al Eur. J. Immunol. 21 (7), 1677-1684 (1991)

### Other information

Official Symbol: CD52
Other Aliases: CDW52
Other Designations: CAMPATH-1 antigen; CD52 antigen (CAMPATH-1 antigen); CDW52 antigen (CAMPATH-1 antigen); cambridge pathology 1 antigen; epididymal secretory protein E5; he5; human epididymis-specific protein 5

### ANTIBODIES

Alemtuzumab (Campath) - Skoetz N., et al Cochrane Database Syst Rev. 2012 Feb 15;2:CD008078.
For example, see Drugbank Acc. No. DB00087 (BIOD00109, BTD00109)

### (85) CS1 - SLAMF7 (SLAM family member 7)

### Nucleotide

Genbank accession no. NM_021181
Genbank version no. NM_021181.3 GI:1993571
Genbank record update date: Jun 29, 2012 11:24 AM

### Polypeptide

Genbank accession no. NP_067004
Genbank version no. NP_067004.3 Gi:19923572
Genbank record update date: Jun 29, 2012 11:24 AM

### Cross-references

Boles K.S., et al Immunogenetics 52 (3-4), 302-307 (2001)

### Other information

Official Symbol: SLAMF7
Other Aliases: UNQ576/PRO1138, 19A, CD319, CRACC, CS1
Other Designations: 19A24 protein; CD2 subset 1; CD2-like receptor activating cytotoxic cells; CD2-like receptor-activating cytotoxic cells; membrane protein FOAP-12; novel LY9 (lymphocyte antigen 9) like protein; protein 19A

### ANTIBODIES

BMS: elotuzumab/HuLuc63 (Benson DM., et al J Clin Oncol. 2012 Jun 1;30(16):2013-2015)
For example, see US20110206701 SEQ ID NOs: 9, 10, 11, 12, 13, 14, 15 and 16.

### (86) Endoglin - ENG (Endoglin)

### Nucleotide

Genbank accession no. AF035753
Genbank version no. AF035753.1 GI:3452260
Genbank record update date: Mar 10, 2010 06:36 PM

### Polypeptide

Genbank accession no. AAC32802
Genbank version no. AAC32802.1 GI:3452261
Genbank record update date: Mar 10, 2010 06:36 PM

### Cross-references

Rius C., et al Blood 92 (12), 4677-4690 (1998)
Official Symbol: ENG

### Other information

Other Aliases: RP11-228B15.2, CD105, END, HHT1, ORW, ORW1
Other Designations: CD105 antigen

### (87) Annexin A1 - ANXA1 (Annexin A 1)

### Nucleotide

Genbank accession no. X05908
Genbank version no. X05908.1 GI:34387
Genbank record update date: Feb 02, 2011 10:02 AM

### Polypeptide

Genbank accession no. CCA29338
Genbank version no. CCA29338.1 GI:34388
Genbank record update date: Feb 02, 2011 10:02 AM

### Cross-references

Wallner B.P.,et al Nature 320 (6057), 77-81 (1986)

### Other information

Official Symbol: ANXA1
Other Aliases: RP11-71A24.1, ANX1, LPC1
Other Designations: annexin I (lipocortin I); annexin-1; calpactin II; calpactin-2; chromobindin-9; lipocortin I; p35; phospholipase A2 inhibitory protein

### (88) V-CAM (CD106) - VCAM1 (Vascular cell adhesion molecule 1)

### Nucleotide

Genbank accession no. M60335
Genbank version no. M60335.1 GI:340193
Genbank record update date: Jun 23, 2010 08:56 AM

### Polypeptide

Genbank accession no. AAA61269
Genbank version no. AAA61269.1 GI:340194
Genbank record update date: Jun 23, 2010 08:56 AM

### Cross-references

Hession C., et al J. Biol. Chem. 266 (11), 6682-6685 (1991)

### Other information

Official Symbol VCAM1
Other Aliases: CD106, INCAM-100
Other Designations: CD106 antigen; vascular cell adhesion protein 1

### Antibody Sequences

### Anti-Integrin αvβ6

RHAB6.2 
QVQLVQSGSELKKPGASVKISCKASGFAFTDSYMHWVRQAPGQGLEWMGWIDPENGDTE
YAPKFQGRFVFSLDTSVSTAYLQISSLKAEDTAVYYCTRGTPTAVPNLRGDLQVLAQKVAG
PYPFDYWGQGTLVTVSS
RHCB6.2 
QVQLVQSGAEVKKPGASVKVSCKASGYTFIDSYMHWVRQAPGQRLEWMGWIDPENGDTE
YAPKFQGRVTITTDTSASTAYMELSSLRSEDTAVYYCARGTPTAVPNLRGDLQVLAQKVAG
PYPFDYWGQGTLVTVSS
RHF 
QVQLVQSGAEVKKPGASVKVSCKASGFNFIDSYMHWVRQAPGQRLEWMGWIDPENGDT
EYAPKFQGRVTFTTDTSASTAYMELSSLRSEDTAVYYCNEGTPTGPYYFDYWGQGTLVTV
SS
RHFB6 
QVQLVQSGAEVKKPGASVKVSCKASGFNFIDSYMHWVRQAPGQRLEWMGWIDPENGDT
EYAPKFQGRVTFTTDTSASTAYMELSSLRSEDTAVYYCNEGTPTAVPNLRGDLQVLAQKVA
GPYYFDYWGQGTLVTVSS
RHAY100bP 
QVQLVQSGSELKKPGASVKISCKASGFAFTDSYMHWVRQAPGQGLEWMGWIDPENGDTE
YAPKFQGRFVFSLDTSVSTAYLQISSLKAEDTAVYYCTRGTPTGPYPFDYWGQGTLVTVSS
RKF 
ENVLTQSPGTLSLSPGERATLSCSASSSVSYMHWFQQKPGQAPRLLIYSTSNLASGIPDRF
SGSGSGTDFTLTISRLEPEDFAVYYCQQRSSYPLTFGGGTKVEIK
RKFL36L50 
ENVLTQSPGTLSLSPGERATLSCSASSSVSYMHWLQQKPGQAPRLLIYLTSNLASGIPDRF
SGSGSGTDFTLTISRLEPEDFAVYYCQQRSSYPLTFGGGTKVEIK
RKC

EIVLTQSPGTLSLSPGERATLSCSASSSVSYMHWFQQKPGQAPRLLIYSTSNLASGIPDRFS
GSGSGTDFTLTISRLEPEDFAVYYCQQRSSYPLTFGGGTKVEIK

### Anti-CD33

CD33 Hum195 VH 
QVQLVQSGAEVKKPGSSVKVSCKASGYTFTDYNMHWVRQAPGQGLEWIGYIYPYNGGTG
YNQKFKSKATITADESTNTAYMELSSLRSEDTAVYYCARGRPAMDYWGQGTLVTVSS
CD33 Hum195 VK

DIQMTQSPSSLSASVGDRVTITCRASESVDNYGISFMNWFQQKPGKAPKLLIYAASNQGSG
VPSRFSGSGSGTDFTLTISSLQPDDFATYYCQQSKEVPWTFGQGTKVEIK

### Anti-CD19

CD19 B4 resurfaced VH 
QVQLVQPGAEVVKPGASVKLSCKTSGYTFTSNWMHWVKQRPGQGLEWIGEIDPSDSYTN
YNQNFKGKAKLTVDKSTSTAYMEVSSLRSDDTAVYYCARGSNPYYYAMDYWGQGTSVTV
SS
CD19 B4 resurfaced VK

EIVLTQSPAIMSASPGERVTMTCSASSGVNYMHWYQQKPGTSPRRWIYDTSKLASGVPAR
FSGSGSGTSYSLTISSMEPEDAATYYCHQRGSYTFGGGTKLEIK

### Anti-Her2

Herceptin VH chain
Herceptin VL chain

### Anti-CD25

Simulect VK (also known as Basiliximab)
Simulect VH

QLQQSGTVLARPGASVKMSCKASGYSFTRYWMHWIKQRPGQGLEWIGAIYPGNSDTSYN
QKFEGKAKLTAVTSASTAYMELSSLTHEDSAVYYCSRDYGYYFDFWGQGTTLTVSS

### Anti-PSMA

Deimmunised VH '1 
EVQLVQSGPEVKKPGATVKISCKTSGYTFTEYTIHWVKQAPGKGLEWIGNINPNNGGTTYN
QKFEDKATLTVDKSTDTAYMELSSLRSEDTAVYYCAAGWNFDYWGQGTLLTVSS
Deimmunised VK '1 
DIQMTQSPSSLSTSVGDRVTLTCKASQDVGTAVDWYQQKPGPSPKLLIYWASTRHTGIPSR
FSGSGSGTDFTLTISSLQPEDFADYYCQQYNSYPLTFGPGTKVDIK
Deimmunised VH1 '5 
EVKLVESGGGLVQPGGSMKLSCVASGFTFSNYWMNWVRQAPGKGLEWVAEIRSQSNNF
ATHYAESVKGRVTISRDDSKSIVYLQMNNLRAEDTGVYYCTRRWNNFWGQGTTVTVSS
Deimmunised VH2 '5 
EVKLVESGGGLVQPGGSLKLSCVASGFTFSNYWMNWVRQAPGKGLEWVAEIRSQSNNFA
THYAESVKGRVTISRDDSKSIVYLQMNNLRAEDTAVYYCTRRWNNFWGQGTTVTVSS
Deimmunised VH3 '5 
EVQLVESGGGLVQPGGSLKLSCVASGFTFSNYWMNWVRQAPGKGLEWVAEIRSQSNNFA
THYAESVKGRVTISRDDSKSIVYLQMNNLRAEDTAVYYCTRRWNNFWGQGTTVTVSS
Deimmunised VH4 '5 
EVQLVESGGGLVQPGGSLKLSCVASGFTFSNYWMNWVRQAPGKGLEWVAEIRSQSNNFA
THYAESVKGRFTISRDDSKSIVYLQMNNLRAEDTAVYYCTRRWNNFWGQGTTVTVSS
Deimmunised VK1 '5 
NIVMTQFPSSMSASVGDRVTITCKASENVGTYVSWYQQKPDQSPKMLIYGASNRFTGVPD
RFTGSGSATDFTLTISSLQTEDLADYYCGQSYTFPYTFGQGTKLEMK
Deimmunised VK2 '5 
NIVMTQFPSSMSASVGDRVTITCKASENVGTYVSWYQQKPDQSPKMLIYGASNRFTGVPD
RFSGSGSGTDFTLTISSLQAEDLADYYCGQSYTFPYTFGQGTKLEIK
Deimmunised VK3 '5 
NIQMTQFPSAMSASVGDRVTITCKASENVGTYVSWYQQKPDQSPKMLIYGASNRFTGVPD
RFSGSGSGTDFTLTISSLQAEDLADYYCGQSYTFPYTFGQGTKLEIK
Deimmunised VK4 '5 
NIQMTQFPSAMSASVGDRVTITCKASENVGTYVSWYQQKPDQSPKMLIYGASNRFTGVPD
RFSGSGSGTDFTLTISSLQAEDEADYYCGQSYTFPYTFGQGTKLEIK
Deimmunised VK DI '5 
NIVMTQFPKSMSASAGERMTLTCKASENVGTYVSWYQQKPTQSPKMLIYGASNRFTGVPD
RFSGSGSGTDFILTISSVQAEDLVDYYCGQSYTFPYTFGGGTKLEMK
Deimmunised VH DI '5 
EVKLEESGGGLVQPGGSMKISCVASGFTFSNYWMNWVRQSPEKGLEWVAEIRSQSNNFA
THYAESVKGRVIISRDDSKSSVYLQMNSLRAEDTAVYYCTRRWNNFWGQGTTVTVSS
Humanised R**H**A '5 
EVQLVESGGGLVQPGGSLKLSCAASGFTFSNYWMNWVRQASGKGLEWVGEIRSQSNNFA
THYAESVKGRFTISRDDSKNTAYLQMNSLKTEDTAVYYCTRRWNNFWGQGTTVTVSS
Humanised R**H**B '5 
EVKLVESGGGLVQPGGSLKLSCAASGFTFSNYWMNWVRQASGKGLEWVAEIRSQSNNFA
THYAESVKGRVIISRDDSKNTVYLQMNSLRTEDTAVYYCTRRWNNFWGQGTTVTVSS
Humanised R**H**C '5 
EVQLVESGGGLVQPGGSLKLSCAASGFTFSNYWMNWVRQASGKGLEWVAEIRSQSNNFA
THYAESVKGRVIISRDDSKNTVYLQMNSLRTEDTAVYYCTRRWNNFWGQGTTVTVSS
Humanised R**H**D '5 
EVKLVESGGGLVQPGGSLKLSCAASGFTFSNYWMNWVRQASGKGLEWVGEIRSQSNNFA
THYAESVKGRVIISRDDSKNTVYLQMNSLRTEDTAVYYCTRRWNNFWGQGTTVTVSS
Humanised R**H**E '5 
EVKLVESGGGLVQPGGSLKLSCAASGFTFSNYWMNWVRQASGKGLEWVAEIRSQSNNFA
THYAESVKGRFTISRDDSKNTVYLQMNSLRTEDTAVYYCTRRWNNFWGQGTTVTVSS
Humanised R**H**F '5 
EVKLVESGGGLVQPGGSLKLSCAASGFTFSNYWMNWVRQASGKGLEWVAEIRSQSNNFA
THYAESVKGRVIISRDDSKNTAYLQMNSLRTEDTAVYYCTRRWNNFWGQGTTVTVSS
Humanised R**H**G '5 
EVKLVESGGGLVQPGGSLKLSCAASGFTFSNYWMNWVRQASGKGLEWVAEIRSQSNNFA
THYAESVKGRVIISRDDSKNTAYLQMNSLRTEDTAVYYCTRRWNNFWGQGTTVTVSS
Humanised R**K**A '5 
DIQMTQSPSSVSASVGDRVTITCKASENVGTYVSWYQQKPGTAPKLLIYGASNRFTGVPSR
FSGSGSATDFTLTINNLQPEDFATYYCGQSYTFPYTFGQGTKVEIK
Humanised R**K**B '5 
DIQMTQSPSSVSASVGDRVTITCKASENVGTYVSWYQQKPGTAPKLLIYGASNRFTGVPSR
FSGSGSATDFTLTINNLQPEDFATYYCGQSYTFPYTFGQGTKVEIK
Humanised R**K**C '5 
DIQMTQSPSSVSASVGDRVTITCKASENVGTYVSWYQQKPGTAPKMLIYGASNRFTGVPS
RFSGSGSATDFTLTINNLQPEDFATYYCGQSYTFPYTFGQGTKVEIK
Humanised R**K**D '5 
DIQMTQSPSSVSASVGDRVTITCKASENVGTYVSWYQQKPGTAPKMLIYGASNRFTGVPS
RFSGSGSATDFTLTINNLQPEDFATYYCGQSYTFPYTFGQGTKVEIK
Humanised R**K**E '5 
NIVMTQSPSSVSASVGDRVTITCKASENVGTYVSWYQQKPGTAPKLLIYGASNRFTGVPDR
FTGSGSATDFILTINNLQPEDFATYYCGQSYTFPYTFGQGTKVEIK
Humanised R**K**F '5 
NIVMTQSPSSVSASVGDRVTITCKASENVGTYVSWYQQKPGTAPKMLIYGASNRFTGVPSR
FSGSGSATDFILTINNLQPEDFATYYCGQSYTFPYTFGQGTKVEIK
Humanised R**K**G '5

NIVMTQSPSSVSASVGDRVTITCKASENVGTYVSWYQQKPGTAPKMLIYGASNRFTGVPDR
FTGSGSATDFTLTINNLQPEDFATYYCGQSYTFPYTFGQGTKVEIK

The parent antibody may also be a fusion protein comprising an albumin-binding peptide (ABP) sequence (Dennis et al. (2002) "Albumin Binding As A General Strategy For Improving The Pharmacokinetics Of Proteins" J Biol Chem. 277:35035-35043; WO 01/45746). Antibodies of the invention include fusion proteins with ABP sequences taught by: (i) Dennis et al (2002) J Biol Chem. 277:35035-35043 at Tables III and IV, page 35038; (ii) US 2004/0001827 at [0076]; and (iii) WO 01/45746 at pages 12-13, and all of which are incorporated herein by reference.

In one embodiment, the antibody has been raised to target specific the tumour related antigen αᵥβ₆.

The cell binding agent may be labelled, for example to aid detection or purification of the agent either prior to incorporation as a conjugate, or as part of the conjugate. The label may be a biotin label. In another embodiment, the cell binding agent may be labelled with a radioisotope.

Embodiments of the present invention include ConjA wherein the cell binding agent is selected from an antibody to any of the antigens discussed above.

Embodiments of the present invention include ConjB wherein the cell binding agent is selected from an antibody to any of the antigens discussed above.

Embodiments of the present invention include ConjA wherein the cell binding agent is selected from any of the antibodies discussed above.

Embodiments of the present invention include ConjB wherein the cell binding agent is selected from any of the antibodies discussed above.

The present invention may also relate to conjugates where the cell binding agent is selected from an antibody to any of the antigens discussed above and any of the antibodies discussed above linked to different drugs.

### Drug loading

The drug loading is the average number of PBD drugs per cell binding agent, e.g. antibody. Where the compounds of the invention are bound to cysteines, drug loading may range from 1 to 8 drugs (D) per cell binding agent, i.e. where 1, 2, 3, 4, 5, 6, 7, and 8 drug moieties are covalently attached to the cell binding agent. Compositions of conjgates include collections of cell binding agents, e.g. antibodies, conjugated with a range of drugs, from 1 to 8.

Where the compounds of the invention are bound to lysines, drug loading may range from 1 to 80 drugs (D) per cell binding agent, although an upper limit of 40, 20, 10 or 8 may be preferred. Compositions of conjgates include collections of cell binding agents, e.g. antibodies, conjugated with a range of drugs, from 1 to 80, 1 to 40, 1 to 20, 1 to 10 or 1 to 8.

The average number of drugs per antibody in preparations of ADC from conjugation reactions may be characterized by conventional means such as UV, reverse phase HPLC, HIC, mass spectroscopy, ELISA assay, and electrophoresis. The quantitative distribution of ADC in terms of p may also be determined. By ELISA, the averaged value of p in a particular preparation of ADC may be determined (Hamblett et al (2004) Clin. Cancer Res. 10:7063-7070; Sanderson et al (2005) Clin. Cancer Res. 11:843-852). However, the distribution of p (drug) values is not discernible by the antibody-antigen binding and detection limitation of ELISA. Also, ELISA assay for detection of antibody-drug conjugates does not determine where the drug moieties are attached to the antibody, such as the heavy chain or light chain fragments, or the particular amino acid residues. In some instances, separation, purification, and characterization of homogeneous ADC where p is a certain value from ADC with other drug loadings may be achieved by means such as reverse phase HPLC or electrophoresis. Such techniques are also applicable to other types of conjugates.

For some antibody-drug conjugates, p may be limited by the number of attachment sites on the antibody. For example, an antibody may have only one or several cysteine thiol groups, or may have only one or several sufficiently reactive thiol groups through which a linker may be attached. Higher drug loading, e.g. p >5, may cause aggregation, insolubility, toxicity, or loss of cellular permeability of certain antibody-drug conjugates.

Typically, fewer than the theoretical maximum of drug moieties are conjugated to an antibody during a conjugation reaction. An antibody may contain, for example, many lysine residues that do not react with the drug-linker intermediate (D-L) or linker reagent. Only the most reactive lysine groups may react with an amine-reactive linker reagent. Also, only the most reactive cysteine thiol groups may react with a thiol-reactive linker reagent. Generally, antibodies do not contain many, if any, free and reactive cysteine thiol groups which may be linked to a drug moiety. Most cysteine thiol residues in the antibodies of the compounds exist as disulfide bridges and must be reduced with a reducing agent such as dithiothreitol (DTT) or TCEP, under partial or total reducing conditions. The loading (drug/antibody ratio) of an ADC may be controlled in several different manners, including: (i) limiting the molar excess of drug-linker intermediate (D-L) or linker reagent relative to antibody, (ii) limiting the conjugation reaction time or temperature, and (iii) partial or limiting reductive conditions for cysteine thiol modification.

Certain antibodies have reducible interchain disulfides, i.e. cysteine bridges. Antibodies may be made reactive for conjugation with linker reagents by treatment with a reducing agent such as DTT (dithiothreitol). Each cysteine bridge will thus form, theoretically, two reactive thiol nucleophiles. Additional nucleophilic groups can be introduced into antibodies through the reaction of lysines with 2-iminothiolane (Traut's reagent) resulting in conversion of an amine into a thiol. Reactive thiol groups may be introduced into the antibody (or fragment thereof) by engineering one, two, three, four, or more cysteine residues (e.g., preparing mutant antibodies comprising one or more non-native cysteine amino acid residues). US 7521541 teaches engineering antibodies by introduction of reactive cysteine amino acids.

Cysteine amino acids may be engineered at reactive sites in an antibody and which do not form intrachain or intermolecular disulfide linkages (Junutula, et al., 2008b Nature Biotech., 26(8):925-932; Dornan et al (2009) Blood 114(13):2721-2729; US 7521541; US 7723485; WO2009/052249). The engineered cysteine thiols may react with linker reagents or the drug-linker reagents of the present invention which have thiol-reactive, electrophilic groups such as maleimide or alpha-halo amides to form ADC with cysteine engineered antibodies and the PBD drug moieties. The location of the drug moiety can thus be designed, controlled, and known. The drug loading can be controlled since the engineered cysteine thiol groups typically react with thiol-reactive linker reagents or drug-linker reagents in high yield. Engineering an IgG antibody to introduce a cysteine amino acid by substitution at a single site on the heavy or light chain gives two new cysteines on the symmetrical antibody. A drug loading near 2 can be achieved with near homogeneity of the conjugation product ADC.

Where more than one nucleophilic or electrophilic group of the antibody reacts with a drug-linker intermediate, or linker reagent followed by drug moiety reagent, then the resulting product is a mixture of ADC compounds with a distribution of drug moieties attached to an antibody, e.g. 1, 2, 3, etc. Liquid chromatography methods such as polymeric reverse phase (PLRP) and hydrophobic interaction (HIC) may separate compounds in the mixture by drug loading value. Preparations of ADC with a single drug loading value (p) may be isolated, however, these single loading value ADCs may still be heterogeneous mixtures because the drug moieties may be attached, via the linker, at different sites on the antibody.

Thus the antibody-drug conjugate compositions of the invention include mixtures of antibody-drug conjugate compounds where the antibody has one or more PBD drug moieties and where the drug moieties may be attached to the antibody at various amino acid residues.

In one embodiment, the average number of dimer pyrrolobenzodiazepine groups per cell binding agent is in the range 1 to 20. In some embodiments the range is selected from 1 to 8, 2 to 8, 2 to 6, 2 to 4, and 4 to 8.

In some embodiments, there is one dimer pyrrolobenzodiazepine group per cell binding agent.

### Preferred Compounds

Particularly preferred compounds of the second aspect of the present invention include:

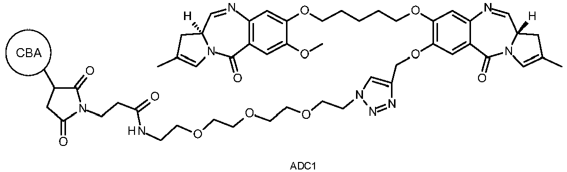

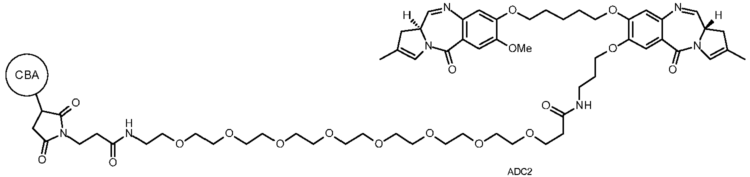

Particularly preferred compounds of the second aspect of the present invention include:

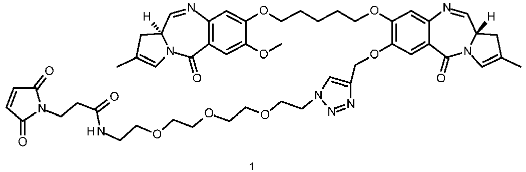

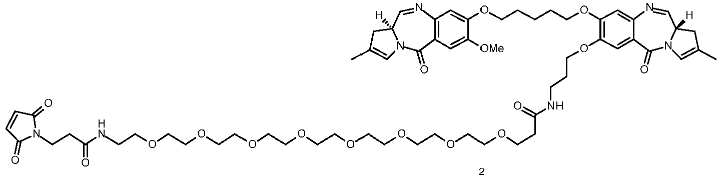

Particularly preferred compounds of the third aspect of the present invention include:

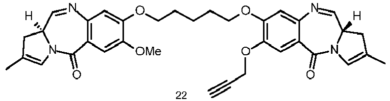

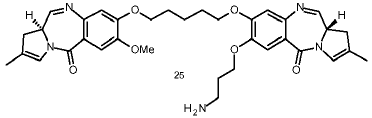

### Substituents

The phrase "optionally substituted" as used herein, pertains to a parent group which may be unsubstituted or which may be substituted.

Unless otherwise specified, the term "substituted" as used herein, pertains to a parent group which bears one or more substituents. The term "substituent" is used herein in the conventional sense and refers to a chemical moiety which is covalently attached to, or if appropriate, fused to, a parent group. A wide variety of substituents are well known, and methods for their formation and introduction into a variety of parent groups are also well known.

In a preferred embodiment, the substituents described herein (which include optional substituents) are limited to those groups that are not reactive to a cell binding agent. The link to the cell binding agent in the present case is formed from the bridge between the two PBD moieties through a linker group to the cell binding agent. Reactive functional groups located at other parts of the PBD structure may be capable of forming additional bonds to the cell binding agent (this may be referred to as crosslinking). These additional bonds may alter transport and biological activity of the conjugate. Therefore, in some embodiment, the additional substituents are limited to those lacking reactive functionality.

In one embodiment, the substituents are selected from the group consisting of R, OR, SR, NRR', NO₂, halo, CO₂R, COR, CONH₂, CONHR, and CONRR'.
In one embodiment, the substituents are selected from the group consisting of R, OR, SR, NRR', NO₂, CO₂R, COR, CONH₂, CONHR, and CONRR'.
In one embodiment, the substituents are selected from the group consisting of R, OR, SR, NRR', NO₂, and halo.

In one embodiment, the substituents are selected from the group consisting of R, OR, SR, NRR', and NO₂.
Any one of the embodiment mentioned above may be applied to any one of the substituents described herein. Alternatively, the substituents may be selected from one or more of the groups listed below.

Examples of substituents are described in more detail below.

C₁₋₁₂ alkyl: The term "C₁₋₁₂ alkyl" as used herein, pertains to a monovalent moiety obtained by removing a hydrogen atom from a carbon atom of a hydrocarbon compound having from 1 to 12 carbon atoms, which may be aliphatic or alicyclic, and which may be saturated or unsaturated (e.g. partially unsaturated, fully unsaturated). Thus, the term "alkyl" includes the sub-classes alkenyl, alkynyl, cycloalkyl, etc., discussed below.

Examples of saturated alkyl groups include, but are not limited to, methyl (C₁), ethyl (C₂), propyl (C₃), butyl (C₄), pentyl (C₅), hexyl (C₆) and heptyl (C₇).

Examples of saturated linear alkyl groups include, but are not limited to, methyl (C₁), ethyl (C₂), n-propyl (C₃), n-butyl (C₄), n-pentyl (amyl) (C₅), n-hexyl (C₆) and n-heptyl (C₇).

Examples of saturated branched alkyl groups include iso-propyl (C₃), iso-butyl (C₄), sec-butyl (C₄), tert-butyl (C₄), iso-pentyl (C₅), and neo-pentyl (C₅).

An alkyl group may optionally be interrupted by one or more heteroatoms selected from O, N(H) and S. Such groups may be referred to as "heteroalkyl".

C₂₋₁₂ Heteroalkyl: The term "C₂₋₁₂ heteroalkyl" as used herein, pertains to a monovalent moiety obtained by removing a hydrogen atom from a carbon atom of a hydrocarbon compound having from 2 to 12 carbon atoms, and one or more heteroatoms selected from O, N(H) and S, preferably O and S.

Examples of heteroalkyl groups include, but are not limited to those comprising one or more ethylene glycol units of the type -(OCH₂CH₂)-. The terminal of a heteroalkyl group may be the primary form of a heteroatom, e.g. -OH, -SH or -NH₂. In a preferred embodiment, the terminal is -CH₃.

C₂₋₁₂ Alkenyl: The term "C₂₋₁₂ alkenyl" as used herein, pertains to an alkyl group having one or more carbon-carbon double bonds.

Examples of unsaturated alkenyl groups include, but are not limited to, ethenyl (vinyl, -CH=CH₂), 1-propenyl (-CH=CH-CH₃), 2-propenyl (allyl, -CH-CH=CH₂), isopropenyl (1-methylvinyl, -C(CH₃)=CH₂), butenyl (C₄), pentenyl (C₅), and hexenyl (C₆).

C₂₋₁₂ alkynyl: The term "C₂₋₁₂ alkynyl" as used herein, pertains to an alkyl group having one or more carbon-carbon triple bonds.

Examples of unsaturated alkynyl groups include, but are not limited to, ethynyl (-C≡CH) and 2-propynyl (propargyl, -CH₂-C≡CH).

C₃₋₁₂ cycloalkyl: The term "C₃₋₁₂ cycloalkyl" as used herein, pertains to an alkyl group which is also a cyclyl group; that is, a monovalent moiety obtained by removing a hydrogen atom from an alicyclic ring atom of a cyclic hydrocarbon (carbocyclic) compound, which moiety has from 3 to 7 carbon atoms, including from 3 to 7 ring atoms.

Examples of cycloalkyl groups include, but are not limited to, those derived from:
saturated monocyclic hydrocarbon compounds:
   cyclopropane (C₃), cyclobutane (C₄), cyclopentane (C₅), cyclohexane (C₆), cycloheptane (C₇), methylcyclopropane (C₄), dimethylcyclopropane (C₅), methylcyclobutane (C₅), dimethylcyclobutane (C₆), methylcyclopentane (C₆), dimethylcyclopentane (C₇) and methylcyclohexane (C₇);
unsaturated monocyclic hydrocarbon compounds:
   cyclopropene (C₃), cyclobutene (C₄), cyclopentene (C₅), cyclohexene (C₆), methylcyclopropene (C₄), dimethylcyclopropene (C₅), methylcyclobutene (C₅), dimethylcyclobutene (C₆), methylcyclopentene (C₆), dimethylcyclopentene (C₇) and methylcyclohexene (C₇); and
saturated polycyclic hydrocarbon compounds:
   norcarane (C₇), norpinane (C₇), norbornane (C₇).

C₃₋₂₀ heterocyclyl: The term "C₃₋₂₀ heterocyclyl" as used herein, pertains to a monovalent moiety obtained by removing a hydrogen atom from a ring atom of a heterocyclic compound, which moiety has from 3 to 20 ring atoms, of which from 1 to 10 are ring heteroatoms. Preferably, each ring has from 3 to 7 ring atoms, of which from 1 to 4 are ring heteroatoms.

In this context, the prefixes (e.g. C₃₋₂₀, C₃₋₇, C₅₋₆, etc.) denote the number of ring atoms, or range of number of ring atoms, whether carbon atoms or heteroatoms. For example, the term "C₅₋₆heterocyclyl", as used herein, pertains to a heterocyclyl group having 5 or 6 ring atoms.

Examples of monocyclic heterocyclyl groups include, but are not limited to, those derived from:
N₁: aziridine (C₃), azetidine (C₄), pyrrolidine (tetrahydropyrrole) (C₅), pyrroline (e.g., 3-pyrroline, 2,5-dihydropyrrole) (C₅), 2H-pyrrole or 3H-pyrrole (isopyrrole, isoazole) (C₅), piperidine (C₆), dihydropyridine (C₆), tetrahydropyridine (C₆), azepine (C₇);
O₁: oxirane (C₃), oxetane (C₄), oxolane (tetrahydrofuran) (C₅), oxole (dihydrofuran) (C₅), oxane (tetrahydropyran) (C₆), dihydropyran (C₆), pyran (C₆), oxepin (C₇);
S₁: thiirane (C₃), thietane (C₄), thiolane (tetrahydrothiophene) (C₅), thiane (tetrahydrothiopyran) (C₆), thiepane (C₇);
O₂: dioxolane (C₅), dioxane (C₆), and dioxepane (C₇);
O₃: trioxane (C₆);
N₂: imidazolidine (C₅), pyrazolidine (diazolidine) (C₅), imidazoline (C₅), pyrazoline (dihydropyrazole) (C₅), piperazine (C₆);
N₁O₁: tetrahydrooxazole (C₅), dihydrooxazole (C₅), tetrahydroisoxazole (C₅), dihydroisoxazole (C₅), morpholine (C₆), tetrahydrooxazine (C₆), dihydrooxazine (C₆), oxazine (C₆);
N₁S₁: thiazoline (C₅), thiazolidine (C₅), thiomorpholine (C₆);
N₂O₁: oxadiazine (C₆);
O₁S₁: oxathiole (C₅) and oxathiane (thioxane) (C₆); and,
N₁O₁S₁: oxathiazine (C₆).

Examples of substituted monocyclic heterocyclyl groups include those derived from saccharides, in cyclic form, for example, furanoses (C₅), such as arabinofuranose, lyxofuranose, ribofuranose, and xylofuranse, and pyranoses (C₆), such as allopyranose, altropyranose, glucopyranose, mannopyranose, gulopyranose, idopyranose, galactopyranose, and talopyranose.

C₅₋₂₀ aryl: The term "C₅₋₂₀ aryl", as used herein, pertains to a monovalent moiety obtained by removing a hydrogen atom from an aromatic ring atom of an aromatic compound, which moiety has from 3 to 20 ring atoms. Preferably, each ring has from 5 to 7 ring atoms.

In this context, the prefixes (e.g. C₃₋₂₀, C₅₋₇, C₅₋₆, etc.) denote the number of ring atoms, or range of number of ring atoms, whether carbon atoms or heteroatoms. For example, the term "C₅₋₆ aryl" as used herein, pertains to an aryl group having 5 or 6 ring atoms.

The ring atoms may be all carbon atoms, as in "carboaryl groups".
Examples of carboaryl groups include, but are not limited to, those derived from benzene (i.e. phenyl) (C₆), naphthalene (C₁₀), azulene (C₁₀), anthracene (C₁₄), phenanthrene (C₁₄), naphthacene (C₁₈), and pyrene (C₁₆).

Examples of aryl groups which comprise fused rings, at least one of which is an aromatic ring, include, but are not limited to, groups derived from indane (e.g. 2,3-dihydro-1H-indene) (C₉), indene (C₉), isoindene (C₉), tetraline (1,2,3,4-tetrahydronaphthalene (C₁₀), acenaphthene (C₁₂), fluorene (C₁₃), phenalene (C₁₃), acephenanthrene (C₁₅), and aceanthrene (C₁₆).

Alternatively, the ring atoms may include one or more heteroatoms, as in "heteroaryl groups". Examples of monocyclic heteroaryl groups include, but are not limited to, those derived from:
N₁: pyrrole (azole) (C₅), pyridine (azine) (C₆);
O₁: furan (oxole) (C₅);
S₁: thiophene (thiole) (C₅);
N₁O₁: oxazole (C₅), isoxazole (C₅), isoxazine (C₆);
N₂O₁: oxadiazole (furazan) (C₅);
N₃O₁: oxatriazole (C₅);
N₁S₁: thiazole (C₅), isothiazole (C₅);
N₂: imidazole (1,3-diazole) (C₅), pyrazole (1,2-diazole) (C₅), pyridazine (1,2-diazine) (C₆), pyrimidine (1,3-diazine) (C₆) (e.g., cytosine, thymine, uracil), pyrazine (1,4-diazine) (C₆);
N₃: triazole (C₅), triazine (C₆); and,
N₄: tetrazole (C₅).

Examples of heteroaryl which comprise fused rings, include, but are not limited to:
C₉ (with 2 fused rings) derived from benzofuran (O₁), isobenzofuran (O₁), indole (N₁), isoindole (N₁), indolizine (N₁), indoline (N₁), isoindoline (N₁), purine (N₄) (e.g., adenine, guanine), benzimidazole (N₂), indazole (N₂), benzoxazole (N₁O₁), benzisoxazole (N₁O₁), benzodioxole (O₂), benzofurazan (N₂O₁), benzotriazole (N₃), benzothiofuran (S₁), benzothiazole (N₁S₁), benzothiadiazole (N₂S);
C₁₀ (with 2 fused rings) derived from chromene (O₁), isochromene (O₁), chroman (O₁), isochroman (O₁), benzodioxan (O₂), quinoline (N₁), isoquinoline (N₁), quinolizine (N₁), benzoxazine (N₁O₁), benzodiazine (N₂), pyridopyridine (N₂), quinoxaline (N₂), quinazoline (N₂), cinnoline (N₂), phthalazine (N₂), naphthyridine (N₂), pteridine (N₄);
C₁₁ (with 2 fused rings) derived from benzodiazepine (N₂);
C₁₃ (with 3 fused rings) derived from carbazole (N₁), dibenzofuran (O₁), dibenzothiophene (S₁), carboline (N₂), perimidine (N₂), pyridoindole (N₂); and,
C₁₄ (with 3 fused rings) derived from acridine (N₁), xanthene (O₁), thioxanthene (S₁), oxanthrene (O₂), phenoxathiin (O₁S₁), phenazine (N₂), phenoxazine (N₁O₁), phenothiazine (N₁S₁), thianthrene (S₂), phenanthridine (N₁), phenanthroline (N₂), phenazine (N₂).

The above groups, whether alone or part of another substituent, may themselves optionally be substituted with one or more groups selected from themselves and the additional substituents listed below.

Halo: -F, -Cl, -Br, and -I.

Hydroxy: -OH.

Ether: -OR, wherein R is an ether substituent, for example, a C₁₋₇ alkyl group (also referred to as a C₁₋₇ alkoxy group, discussed below), a C₃₋₂₀ heterocyclyl group (also referred to as a C₃₋₂₀ heterocyclyloxy group), or a C₅₋₂₀ aryl group (also referred to as a C₅₋₂₀ aryloxy group), preferably a C₁₋₇alkyl group.

Alkoxy: -OR, wherein R is an alkyl group, for example, a C₁₋₇ alkyl group. Examples of C₁₋₇ alkoxy groups include, but are not limited to, -OMe (methoxy), -OEt (ethoxy), -O(nPr) (n-propoxy), -O(iPr) (isopropoxy), -O(nBu) (n-butoxy), -O(sBu) (sec-butoxy), -O(iBu) (isobutoxy), and -O(tBu) (tert-butoxy).

Acetal: -CH(OR¹)(OR²), wherein R¹ and R² are independently acetal substituents, for example, a C₁₋₇ alkyl group, a C₃₋₂₀ heterocyclyl group, or a C₅₋₂₀ aryl group, preferably a C₁₋₇ alkyl group, or, in the case of a "cyclic" acetal group, R¹ and R², taken together with the two oxygen atoms to which they are attached, and the carbon atoms to which they are attached, form a heterocyclic ring having from 4 to 8 ring atoms. Examples of acetal groups include, but are not limited to, -CH(OMe)₂, -CH(OEt)₂, and -CH(OMe)(OEt).

Hemiacetal: -CH(OH)(OR¹), wherein R¹ is a hemiacetal substituent, for example, a C₁₋₇ alkyl group, a C₃₋₂₀ heterocyclyl group, or a C₅₋₂₀ aryl group, preferably a C₁₋₇ alkyl group. Examples of hemiacetal groups include, but are not limited to, -CH(OH)(OMe) and-CH(OH)(OEt).

Ketal: -CR(OR¹)(OR²), where R¹ and R² are as defined for acetals, and R is a ketal substituent other than hydrogen, for example, a C₁₋₇ alkyl group, a C₃₋₂₀ heterocyclyl group, or a C₅₋₂₀ aryl group, preferably a C₁₋₇ alkyl group. Examples ketal groups include, but are not limited to, -C(Me)(OMe)₂, -C(Me)(OEt)₂, -C(Me)(OMe)(OEt), -C(Et)(OMe)₂, -C(Et)(OEt)₂, and -C(Et)(OMe)(OEt).

Hemiketal: -CR(OH)(OR¹), where R¹ is as defined for hemiacetals, and R is a hemiketal substituent other than hydrogen, for example, a C₁₋₇ alkyl group, a C₃₋₂₀ heterocyclyl group, or a C₅₋₂₀ aryl group, preferably a C₁₋₇ alkyl group. Examples of hemiacetal groups include, but are not limited to, -C(Me)(OH)(OMe), -C(Et)(OH)(OMe), -C(Me)(OH)(OEt), and -C(Et)(OH)(OEt).

Oxo (keto, -one): =O.

Thione (thioketone): =S.

Imino (imine): =NR, wherein R is an imino substituent, for example, hydrogen, C₁₋₇ alkyl group, a C₃₋₂₀ heterocyclyl group, or a C₅₋₂₀ aryl group, preferably hydrogen or a C₁₋₇ alkyl group. Examples of ester groups include, but are not limited to, =NH, =NMe, =NEt, and =NPh.

Formyl (carbaldehyde, carboxaldehyde): -C(=O)H.

Acyl (keto): -C(=O)R, wherein R is an acyl substituent, for example, a C₁₋₇ alkyl group (also referred to as C₁₋₇ alkylacyl or C₁₋₇ alkanoyl), a C₃₋₂₀ heterocyclyl group (also referred to as C₃₋₂₀ heterocyclylacyl), or a C₅₋₂₀ aryl group (also referred to as C₅₋₂₀ arylacyl), preferably a C₁₋₇ alkyl group. Examples of acyl groups include, but are not limited to, -C(=O)CH₃ (acetyl), -C(=O)CH₂CH₃ (propionyl), -C(=O)C(CH₃)₃ (t-butyryl), and -C(=O)Ph (benzoyl, phenone).

Carboxy (carboxylic acid): -C(=O)OH.

Thiocarboxy (thiocarboxylic acid): -C(=S)SH.

Thiolocarboxy (thiolocarboxylic acid): -C(=O)SH.

Thionocarboxy (thionocarboxylic acid): -C(=S)OH.

Imidic acid: -C(=NH)OH.

Hydroxamic acid: -C(=NOH)OH.

Ester (carboxylate, carboxylic acid ester, oxycarbonyl): -C(=O)OR, wherein R is an ester substituent, for example, a C₁₋₇ alkyl group, a C₃₋₂₀ heterocyclyl group, or a C₅₋₂₀ aryl group, preferably a C₁₋₇ alkyl group. Examples of ester groups include, but are not limited to, -C(=O)OCH₃, -C(=O)OCH₂CH₃, -C(=O)OC(CH₃)₃, and -C(=O)OPh.

Acyloxy (reverse ester): -OC(=O)R, wherein R is an acyloxy substituent, for example, a C₁₋₇ alkyl group, a C₃₋₂₀ heterocyclyl group, or a C₅₋₂₀ aryl group, preferably a C₁₋₇ alkyl group. Examples of acyloxy groups include, but are not limited to, -OC(=O)CH₃ (acetoxy), -OC(=O)CH₂CH₃, -OC(=O)C(CH₃)₃, -OC(=O)Ph, and -OC(=O)CH₂Ph.

Oxycarboyloxy: -OC(=O)OR, wherein R is an ester substituent, for example, a C₁₋₇ alkyl group, a C₃₋₂₀ heterocyclyl group, or a C₅₋₂₀ aryl group, preferably a C₁₋₇ alkyl group. Examples of ester groups include, but are not limited to, -OC(=O)OCH₃, -OC(=O)OCH₂CH₃, -OC(=O)OC(CH₃)₃, and -OC(=O)OPh.

Amino: -NR¹R², wherein R¹ and R² are independently amino substituents, for example, hydrogen, a C₁₋₇ alkyl group (also referred to as C₁₋₇ alkylamino or di-C₁₋₇ alkylamino), a C₃₋₂₀ heterocyclyl group, or a C₅₋₂₀ aryl group, preferably H or a C₁₋₇ alkyl group, or, in the case of a "cyclic" amino group, R¹ and R², taken together with the nitrogen atom to which they are attached, form a heterocyclic ring having from 4 to 8 ring atoms. Amino groups may be primary (-NH₂), secondary (-NHR¹), or tertiary (-NHR¹R²), and in cationic form, may be quaternary (-⁺NR¹R²R³). Examples of amino groups include, but are not limited to, -NH₂, -NHCH₃, -NHC(CH₃)₂, -N(CH₃)₂, -N(CH₂CH₃)₂, and -NHPh. Examples of cyclic amino groups include, but are not limited to, aziridino, azetidino, pyrrolidino, piperidino, piperazino, morpholino, and thiomorpholino.

Amido (carbamoyl, carbamyl, aminocarbonyl, carboxamide): -C(=O)NR¹R², wherein R¹ and R² are independently amino substituents, as defined for amino groups. Examples of amido groups include, but are not limited to, -C(=O)NH₂, -C(=O)NHCH₃, -C(=O)N(CH₃)₂, -C(=O)NHCH₂CH₃, and -C(=O)N(CH₂CH₃)₂, as well as amido groups in which R¹ and R², together with the nitrogen atom to which they are attached, form a heterocyclic structure as in, for example, piperidinocarbonyl, morpholinocarbonyl, thiomorpholinocarbonyl, and piperazinocarbonyl.

Thioamido (thiocarbamyl): -C(=S)NR¹R², wherein R¹ and R² are independently amino substituents, as defined for amino groups. Examples of amido groups include, but are not limited to, -C(=S)NH₂, -C(=S)NHCH₃, -C(=S)N(CH₃)₂, and -C(=S)NHCH₂CH₃.

Acylamido (acylamino): -NR¹C(=O)R², wherein R¹ is an amide substituent, for example, hydrogen, a C₁₋₇ alkyl group, a C₃₋₂₀ heterocyclyl group, or a C₅₋₂₀ aryl group, preferably hydrogen or a C₁₋₇ alkyl group, and R² is an acyl substituent, for example, a C₁₋₇ alkyl group, a C₃₋₂₀ heterocyclyl group, or a C₅₋₂₀aryl group, preferably hydrogen or a C₁₋₇ alkyl group. Examples of acylamide groups include, but are not limited to, -NHC(=O)CH₃, -NHC(=O)CH₂CH₃, and -NHC(=O)Ph. R¹ and R² may together form a cyclic structure, as in, for example, succinimidyl, maleimidyl, and phthalimidyl:

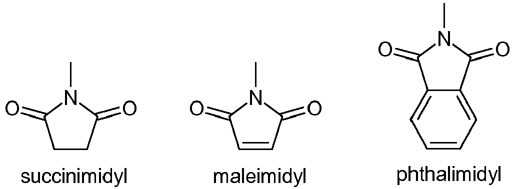

Aminocarbonyloxy: -OC(=O)NR¹R², wherein R¹ and R² are independently amino substituents, as defined for amino groups. Examples of aminocarbonyloxy groups include, but are not limited to, -OC(=O)NH₂, -OC(=O)NHMe, -OC(=O)NMe₂, and -OC(=O)NEt₂.

Ureido: -N(R¹)CONR²R³ wherein R² and R³ are independently amino substituents, as defined for amino groups, and R¹ is a ureido substituent, for example, hydrogen, a C₁₋₇ alkyl group, a C₃₋₂₀ heterocyclyl group, or a C₅₋₂₀ aryl group, preferably hydrogen or a C₁₋₇ alkyl group. Examples of ureido groups include, but are not limited to, -NHCONH₂, -NHCONHMe, -NHCONHEt, -NHCONMe₂, -NHCONEt₂, -NMeCONH₂, -NMeCONHMe, -NMeCONHEt,-NMeCONMe₂, and -NMeCONEt₂.

Guanidino: -NH-C(=NH)NH₂.

Tetrazolyl: a five membered aromatic ring having four nitrogen atoms and one carbon atom,

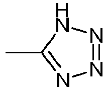

Imino: =NR, wherein R is an imino substituent, for example, for example, hydrogen, a C₁₋₇ alkyl group, a C₃₋₂₀ heterocyclyl group, or a C₅₋₂₀ aryl group, preferably H or a C₁₋₇alkyl group. Examples of imino groups include, but are not limited to, =NH, =NMe, and =NEt.

Amidine (amidino): -C(=NR)NR₂, wherein each R is an amidine substituent, for example, hydrogen, a C₁₋₇ alkyl group, a C₃₋₂₀ heterocyclyl group, or a C₅₋₂₀ aryl group, preferably H or a C₁₋₇ alkyl group. Examples of amidine groups include, but are not limited to, -C(=NH)NH₂, -C(=NH)NMe₂, and -C(=NMe)NMe₂.

Nitro: -NO₂.

Nitroso: -NO.

Azido: -N₃.

Cyano (nitrile, carbonitrile): -CN.

Isocyano: -NC.

Cyanato: -OCN.

Isocyanato: -NCO.

Thiocyano (thiocyanato): -SCN.

Isothiocyano (isothiocyanato): -NCS.

Sulfhydryl (thiol, mercapto): -SH.

Thioether (sulfide): -SR, wherein R is a thioether substituent, for example, a C₁₋₇ alkyl group (also referred to as a C₁₋₇alkylthio group), a C₃₋₂₀ heterocyclyl group, or a C₅₋₂₀ aryl group, preferably a C₁₋₇ alkyl group. Examples of C₁₋₇ alkylthio groups include, but are not limited to, -SCH₃ and -SCH₂CH₃.

Disulfide: -SS-R, wherein R is a disulfide substituent, for example, a C₁₋₇ alkyl group, a C₃₋₂₀ heterocyclyl group, or a C₅₋₂₀ aryl group, preferably a C₁₋₇ alkyl group (also referred to herein as C₁₋₇ alkyl disulfide). Examples of C₁₋₇ alkyl disulfide groups include, but are not limited to, -SSCH₃ and -SSCH₂CH₃.

Sulfine (sulfinyl, sulfoxide): -S(=O)R, wherein R is a sulfine substituent, for example, a C₁₋₇ alkyl group, a C₃₋₂₀ heterocyclyl group, or a C₅₋₂₀ aryl group, preferably a C₁₋₇ alkyl group. Examples of sulfine groups include, but are not limited to, -S(=O)CH₃ and -S(=O)CH₂CH₃.

Sulfone (sulfonyl): -S(=O)₂R, wherein R is a sulfone substituent, for example, a C₁₋₇ alkyl group, a C₃₋₂₀ heterocyclyl group, or a C₅₋₂₀ aryl group, preferably a C₁₋₇ alkyl group, including, for example, a fluorinated or perfluorinated C₁₋₇ alkyl group. Examples of sulfone groups include, but are not limited to, -S(=O)₂CH₃ (methanesulfonyl, mesyl), -S(=O)₂CF₃ (triflyl), -S(=O)₂CH₂CH₃ (esyl), -S(=O)₂C₄F₉ (nonaflyl), -S(=O)₂CH₂CF₃ (tresyl), -S(=O)₂CH₂CH₂NH₂ (tauryl), -S(=O)₂Ph (phenylsulfonyl, besyl), 4-methylphenylsulfonyl (tosyl), 4-chlorophenylsulfonyl (closyl), 4-bromophenylsulfonyl (brosyl), 4-nitrophenyl (nosyl), 2-naphthalenesulfonate (napsyl), and 5-dimethylamino-naphthalen-1-ylsulfonate (dansyl).

Sulfinic acid (sulfino): -S(=O)OH, -SO₂H.

Sulfonic acid (sulfo): -S(=O)₂OH, -SO₃H.

Sulfinate (sulfinic acid ester): -S(=O)OR; wherein R is a sulfinate substituent, for example, a C₁₋₇ alkyl group, a C₃₋₂₀ heterocyclyl group, or a C₅₋₂₀ aryl group, preferably a C₁₋₇ alkyl group. Examples of sulfinate groups include, but are not limited to, -S(=O)OCH₃ (methoxysulfinyl; methyl sulfinate) and -S(=O)OCH₂CH₃ (ethoxysulfinyl; ethyl sulfinate).

Sulfonate (sulfonic acid ester): -S(=O)₂OR, wherein R is a sulfonate substituent, for example, a C₁₋₇ alkyl group, a C₃₋₂₀ heterocyclyl group, or a C₅₋₂₀ aryl group, preferably a C₁₋₇ alkyl group. Examples of sulfonate groups include, but are not limited to, -S(=O)₂OCH₃ (methoxysulfonyl; methyl sulfonate) and -S(=O)₂OCH₂CH₃ (ethoxysulfonyl; ethyl sulfonate).

Sulfinyloxy: -OS(=O)R, wherein R is a sulfinyloxy substituent, for example, a C₁₋₇ alkyl group, a C₃₋₂₀ heterocyclyl group, or a C₅₋₂₀ aryl group, preferably a C₁₋₇ alkyl group. Examples of sulfinyloxy groups include, but are not limited to, -OS(=O)CH₃ and -OS(=O)CH₂CH₃.

Sulfonyloxy: -OS(=O)₂R, wherein R is a sulfonyloxy substituent, for example, a C₁₋₇ alkyl group, a C₃₋₂₀ heterocyclyl group, or a C₅₋₂₀ aryl group, preferably a C₁₋₇ alkyl group. Examples of sulfonyloxy groups include, but are not limited to, -OS(=O)₂CH₃ (mesylate) and -OS(=O)₂CH₂CH₃ (esylate).

Sulfate: -OS(=O)₂OR; wherein R is a sulfate substituent, for example, a C₁₋₇ alkyl group, a C₃₋₂₀ heterocyclyl group, or a C₅₋₂₀ aryl group, preferably a C₁₋₇ alkyl group. Examples of sulfate groups include, but are not limited to, -OS(=O)₂OCH₃ and -SO(=O)₂OCH₂CH₃.

Sulfamyl (sulfamoyl; sulfinic acid amide; sulfinamide): -S(=O)NR¹R², wherein R¹ and R² are independently amino substituents, as defined for amino groups. Examples of sulfamyl groups include, but are not limited to, -S(=O)NH₂, -S(=O)NH(CH₃), -S(=O)N(CH₃)₂, -S(=O)NH(CH₂CH₃), -S(=O)N(CH₂CH₃)₂, and -S(=O)NHPh.

Sulfonamido (sulfinamoyl; sulfonic acid amide; sulfonamide): -S(=O)₂NR¹R², wherein R¹ and R² are independently amino substituents, as defined for amino groups. Examples of sulfonamido groups include, but are not limited to, -S(=O)₂NH₂, -S(=O)₂NH(CH₃), -S(=O)₂N(CH₃)₂, -S(=O)₂NH(CH₂CH₃), -S(=O)₂N(CH₂CH₃)₂, and -S(=O)₂NHPh.

Sulfamino: -NR¹S(=O)₂OH, wherein R¹ is an amino substituent, as defined for amino groups. Examples of sulfamino groups include, but are not limited to, -NHS(=O)₂OH and -N(CH₃)S(=O)₂OH.

Sulfonamino: -NR¹S(=O)₂R, wherein R¹ is an amino substituent, as defined for amino groups, and R is a sulfonamino substituent, for example, a C₁₋₇ alkyl group, a C₃₋₂₀ heterocyclyl group, or a C₅₋₂₀ aryl group, preferably a C₁₋₇ alkyl group. Examples of sulfonamino groups include, but are not limited to, -NHS(=O)₂CH₃ and -N(CH₃)S(=O)₂C₆H₅.

Sulfinamino: -NR¹S(=O)R, wherein R¹ is an amino substituent, as defined for amino groups, and R is a sulfinamino substituent, for example, a C₁₋₇ alkyl group, a C₃₋₂₀ heterocyclyl group, or a C₅₋₂₀ aryl group, preferably a C₁₋₇ alkyl group. Examples of sulfinamino groups include, but are not limited to, -NHS(=O)CH₃ and -N(CH₃)S(=O)C₆H₅.

Phosphino (phosphine): -PR₂, wherein R is a phosphino substituent, for example, -H, a C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphino groups include, but are not limited to, -PH₂, -P(CH₃)₂, -P(CH₂CH₃)₂, -P(t-Bu)₂, and -P(Ph)₂.

Phospho: -P(=O)₂.

Phosphinyl (phosphine oxide): -P(=O)R₂, wherein R is a phosphinyl substituent, for example, a C₁₋₇ alkyl group, a C₃₋₂₀ heterocyclyl group, or a C₅₋₂₀ aryl group, preferably a C₁₋₇ alkyl group or a C₅₋₂₀ aryl group. Examples of phosphinyl groups include, but are not limited to, -P(=O)(CH₃)₂, -P(=O)(CH₂CH₃)₂, -P(=O)(t-Bu)₂, and -P(=O)(Ph)₂.

Phosphonic acid (phosphono): -P(=O)(OH)₂.

Phosphonate (phosphono ester): -P(=O)(OR)₂, where R is a phosphonate substituent, for example, -H, a C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphonate groups include, but are not limited to, -P(=O)(OCH₃)₂, -P(=O)(OCH₂CH₃)₂, -P(=O)(O-t-Bu)₂, and -P(=O)(OPh)₂.

Phosphoric acid (phosphonooxy): -OP(=O)(OH)₂.

Phosphate (phosphonooxy ester): -OP(=O)(OR)₂, where R is a phosphate substituent, for example, -H, a C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphate groups include, but are not limited to, -OP(=O)(OCH₃)₂, -OP(=O)(OCH₂CH₃)₂, -OP(=O)(O-t-Bu)₂, and -OP(=O)(OPh)₂.

Phosphorous acid: -OP(OH)₂.

Phosphite: -OP(OR)₂, where R is a phosphite substituent, for example, -H, a C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphite groups include, but are not limited to, -OP(OCH₃)₂, -OP(OCH₂CH₃)₂, -OP(O-t-Bu)₂, and -OP(OPh)₂.

Phosphoramidite: -OP(OR¹)-NR²₂, where R¹ and R² are phosphoramidite substituents, for example, -H, a (optionally substituted) C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphoramidite groups include, but are not limited to, -OP(OCH₂CH₃)-N(CH₃)₂, -OP(OCH₂CH₃)-N(i-Pr)₂, and -OP(OCH₂CH₂CN)-N(i-Pr)₂.

Phosphoramidate: -OP(=O)(OR¹)-NR²₂, where R¹ and R² are phosphoramidate substituents, for example, -H, a (optionally substituted) C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphoramidate groups include, but are not limited to, -OP(=O)(OCH₂CH₃)-N(CH₃)₂, -OP(=O)(OCH₂CH₃)-N(i-Pr)₂, and -OP(=O)(OCH₂CH₂CN)-N(i-Pr)₂.

### Alkylene

C₃₋₁₂ alkylene: The term "C₃₋₁₂ alkylene", as used herein, pertains to a bidentate moiety obtained by removing two hydrogen atoms, either both from the same carbon atom, or one from each of two different carbon atoms, of a hydrocarbon compound having from 3 to 12 carbon atoms (unless otherwise specified), which may be aliphatic or alicyclic, and which may be saturated, partially unsaturated, or fully unsaturated. Thus, the term "alkylene" includes the sub-classes alkenylene, alkynylene, cycloalkylene, etc., discussed below.

Examples of linear saturated C₃₋₁₂ alkylene groups include, but are not limited to, -(CH₂)ₙ-where n is an integer from 3 to 12, for example, -CH₂CH₂CH₂- (propylene), -CH₂CH₂CH₂CH₂- (butylene), -CH₂CH₂CH₂CH₂CH₂- (pentylene) and -CH₂CH₂CH₂CH-₂CH₂CH₂CH₂- (heptylene).

Examples of branched saturated C₃₋₁₂ alkylene groups include, but are not limited to, -CH(CH₃)CH₂-, -CH(CH₃)CH₂CH₂-, -CH(CH₃)CH₂CH₂CH₂-, -CH₂CH(CH₃)CH₂-, -CH₂CH(CH₃)CH₂CH₂-, -CH(CH₂CH₃)-, -CH(CH₂CH₃)CH₂-, and -CH₂CH(CH₂CH₃)CH₂-.

Examples of linear partially unsaturated C₃₋₁₂ alkylene groups (C₃₋₁₂ alkenylene, and alkynylene groups) include, but are not limited to, -CH=CH-CH₂-, -CH₂-CH=CH₂-, -CH=CH-CH₂-CH₂-, -CH=CH-CH₂-CH₂-CH₂-, -CH=CH-CH=CH-, -CH=CH-CH=CH-CH₂-,-CH=CH-CH=CH-CH₂-CH₂-, -CH=CH-CH₂-CH=CH-, -CH=CH-CH₂-CH₂-CH=CH-, and -CH₂-C=C-CH₂-.

Examples of branched partially unsaturated C₃₋₁₂ alkylene groups (C₃₋₁₂ alkenylene and alkynylene groups) include, but are not limited to, -C(CH₃)=CH-, -C(CH₃)=CH-CH₂-, -CH=CH-CH(CH₃)- and -C≡C-CH(CH₃)-.

Examples of alicyclic saturated C₃₋₁₂ alkylene groups (C₃₋₁₂ cycloalkylenes) include, but are not limited to, cyclopentylene (e.g. cyclopent-1,3-ylene), and cyclohexylene (e.g. cyclohex-1,4-ylene).

Examples of alicyclic partially unsaturated C₃₋₁₂ alkylene groups (C₃₋₁₂ cycloalkylenes) include, but are not limited to, cyclopentenylene (e.g. 4-cyclopenten-1,3-ylene), cyclohexenylene (e.g. 2-cyclohexen-1,4-ylene; 3-cyclohexen-1,2-ylene; 2,5-cyclohexadien-1,4-ylene).

### Includes Other Forms

Unless otherwise specified, included in the above are the well known ionic, salt, solvate, and protected forms of these substituents. For example, a reference to carboxylic acid (-COOH) also includes the anionic (carboxylate) form (-COO⁻), a salt or solvate thereof, as well as conventional protected forms. Similarly, a reference to an amino group includes the protonated form (-N⁺HR¹R²), a salt or solvate of the amino group, for example, a hydrochloride salt, as well as conventional protected forms of an amino group. Similarly, a reference to a hydroxyl group also includes the anionic form (-O⁻), a salt or solvate thereof, as well as conventional protected forms.

### Salts

It may be convenient or desirable to prepare, purify, and/or handle a corresponding salt of the active compound, for example, a pharmaceutically-acceptable salt. Examples of pharmaceutically acceptable salts are discussed in Berge, et al., J. Pharm. Sci., 66, 1-19 (1977).

For example, if the compound is anionic, or has a functional group which may be anionic (e.g. -COOH may be -COO⁻), then a salt may be formed with a suitable cation. Examples of suitable inorganic cations include, but are not limited to, alkali metal ions such as Na⁺ and K⁺, alkaline earth cations such as Ca²⁺ and Mg²⁺, and other cations such as Al⁺³. Examples of suitable organic cations include, but are not limited to, ammonium ion (i.e. NH₄⁺) and substituted ammonium ions (e.g. NH₃R⁺, NH₂R₂⁺, NHR₃⁺, NR₄⁺). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺.

If the compound is cationic, or has a functional group which may be cationic (e.g. -NH₂ may be -NH₃⁺), then a salt may be formed with a suitable anion. Examples of suitable inorganic anions include, but are not limited to, those derived from the following inorganic acids: hydrochloric, hydrobromic, hydroiodic, sulfuric, sulfurous, nitric, nitrous, phosphoric, and phosphorous.

Examples of suitable organic anions include, but are not limited to, those derived from the following organic acids: 2-acetyoxybenzoic, acetic, ascorbic, aspartic, benzoic, camphorsulfonic, cinnamic, citric, edetic, ethanedisulfonic, ethanesulfonic, fumaric, glucheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, trifluoroacetic acid and valeric. Examples of suitable polymeric organic anions include, but are not limited to, those derived from the following polymeric acids: tannic acid, carboxymethyl cellulose.

### Solvates

It may be convenient or desirable to prepare, purify, and/or handle a corresponding solvate of the active compound. The term "solvate" is used herein in the conventional sense to refer to a complex of solute (e.g. active compound, salt of active compound) and solvent. If the solvent is water, the solvate may be conveniently referred to as a hydrate, for example, a mono-hydrate, a di-hydrate, a tri-hydrate, etc.

The invention includes compounds where a solvent adds across the imine bond of the PBD moiety, which is illustrated below where the solvent is water or an alcohol (R^{A}OH, where R^{A} is C₁₋₄ alkyl):

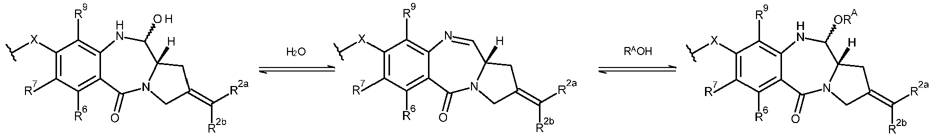

These forms can be called the carbinolamine and carbinolamine ether forms of the PBD (as described in the section relating to R¹⁰ above). The balance of these equilibria depend on the conditions in which the compounds are found, as well as the nature of the moiety itself.

These particular compounds may be isolated in solid form, for example, by lyophilisation.

### Isomers

Certain compounds of the invention may exist in one or more particular geometric, optical, enantiomeric, diasteriomeric, epimeric, atropic, stereoisomeric, tautomeric, conformational, or anomeric forms, including but not limited to, cis- and trans-forms; E- and Z-forms; c-, t-, and r- forms; endo- and exo-forms; R-, S-, and meso-forms; D- and L-forms; d- and I-forms; (+) and (-) forms; keto-, enol-, and enolate-forms; syn- and anti-forms; synclinal- and anticlinal-forms; α- and β-forms; axial and equatorial forms; boat-, chair-, twist-, envelope-, and halfchair-forms; and combinations thereof, hereinafter collectively referred to as "isomers" (or "isomeric forms").

The term "chiral" refers to molecules which have the property of non-superimposability of the mirror image partner, while the term "achiral" refers to molecules which are superimposable on their mirror image partner.

The term "stereoisomers" refers to compounds which have identical chemical constitution, but differ with regard to the arrangement of the atoms or groups in space.

"Diastereomer" refers to a stereoisomer with two or more centers of chirality and whose molecules are not mirror images of one another. Diastereomers have different physical properties, e.g. melting points, boiling points, spectral properties, and reactivities. Mixtures of diastereomers may separate under high resolution analytical procedures such as electrophoresis and chromatography.

"Enantiomers" refer to two stereoisomers of a compound which are non-superimposable mirror images of one another.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. The compounds of the invention may contain asymmetric or chiral centers, and therefore exist in different stereoisomeric forms. It is intended that all stereoisomeric forms of the compounds of the invention, including but not limited to, diastereomers, enantiomers and atropisomers, as well as mixtures thereof such as racemic mixtures, form part of the present invention. Many organic compounds exist in optically active forms, i.e., they have the ability to rotate the plane of plane-polarized light. In describing an optically active compound, the prefixes D and L, or *R* and *S*, are used to denote the absolute configuration of the molecule about its chiral center(s). The prefixes d and I or (+) and (-) are employed to designate the sign of rotation of plane-polarized light by the compound, with (-) or I meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The terms "racemic mixture" and "racemate" refer to an equimolar mixture of two enantiomeric species, devoid of optical activity.

Note that, except as discussed below for tautomeric forms, specifically excluded from the term "isomers", as used herein, are structural (or constitutional) isomers (i.e. isomers which differ in the connections between atoms rather than merely by the position of atoms in space). For example, a reference to a methoxy group, -OCH₃, is not to be construed as a reference to its structural isomer, a hydroxymethyl group, -CH₂OH. Similarly, a reference to ortho-chlorophenyl is not to be construed as a reference to its structural isomer, meta-chlorophenyl. However, a reference to a class of structures may well include structurally isomeric forms falling within that class (e.g. C₁₋₇ alkyl includes n-propyl and iso-propyl; butyl includes n-, iso-, sec-, and tert-butyl; methoxyphenyl includes ortho-, meta-, and para-methoxyphenyl).

The above exclusion does not pertain to tautomeric forms, for example, keto-, enol-, and enolate-forms, as in, for example, the following tautomeric pairs: keto/enol (illustrated below), imine/enamine, amide/imino alcohol, amidine/amidine, nitroso/oxime, thioketone/enethiol, N-nitroso/hyroxyazo, and nitro/aci-nitro.

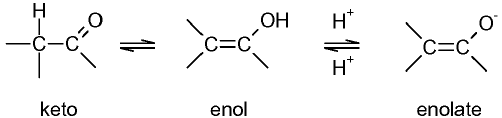

The term "tautomer" or "tautomeric form" refers to structural isomers of different energies which are interconvertible via a low energy barrier. For example, proton tautomers (also known as prototropic tautomers) include interconversions via migration of a proton, such as keto-enol and imine-enamine isomerizations. Valence tautomers include interconversions by reorganization of some of the bonding electrons.

Note that specifically included in the term "isomer" are compounds with one or more isotopic substitutions. For example, H may be in any isotopic form, including ¹H, ²H (D), and ³H (T); C may be in any isotopic form, including ¹²C, ¹³C, and ¹⁴C; O may be in any isotopic form, including ¹⁶O and ¹⁸O; and the like.

Examples of isotopes that can be incorporated into compounds of the invention include isotopes of hydrogen, carbon, nitrogen, oxygen, phosphorous, fluorine, and chlorine, such as, but not limited to ²H (deuterium, D), ³H (tritium), ¹¹C, ¹³C, ¹⁴C, ¹⁵N, ¹⁸F, ³¹P, ³²P, ³⁵S, ³⁶Cl, and ¹²⁵I. Various isotopically labeled compounds of the present invention, for example those into which radioactive isotopes such as 3H, 13C, and 14C are incorporated. Such isotopically labelled compounds may be useful in metabolic studies, reaction kinetic studies, detection or imaging techniques, such as positron emission tomography (PET) or single-photon emission computed tomography (SPECT) including drug or substrate tissue distribution assays, or in radioactive treatment of patients. Deuterium labelled or substituted therapeutic compounds of the invention may have improved DMPK (drug metabolism and pharmacokinetics) properties, relating to distribution, metabolism, and excretion (ADME). Substitution with heavier isotopes such as deuterium may afford certain therapeutic advantages resulting from greater metabolic stability, for example increased in vivo half-life or reduced dosage requirements. An 18F labeled compound may be useful for PET or SPECT studies. Isotopically labeled compounds of this invention and prodrugs thereof can generally be prepared by carrying out the procedures disclosed in the schemes or in the examples and preparations described below by substituting a readily available isotopically labeled reagent for a non-isotopically labeled reagent. Further, substitution with heavier isotopes, particularly deuterium (i.e., 2H or D) may afford certain therapeutic advantages resulting from greater metabolic stability, for example increased in vivo half-life or reduced dosage requirements or an improvement in therapeutic index. It is understood that deuterium in this context is regarded as a substituent. The concentration of such a heavier isotope, specifically deuterium, may be defined by an isotopic enrichment factor. In the compounds of this invention any atom not specifically designated as a particular isotope is meant to represent any stable isotope of that atom.

Unless otherwise specified, a reference to a particular compound includes all such isomeric forms, including (wholly or partially) racemic and other mixtures thereof. Methods for the preparation (e.g. asymmetric synthesis) and separation (e.g. fractional crystallisation and chromatographic means) of such isomeric forms are either known in the art or are readily obtained by adapting the methods taught herein, or known methods, in a known manner.

### Biological Activity

### In vitro cell proliferation assays

Generally, the cytotoxic or cytostatic activity of an antibody-drug conjugate (ADC) is measured by: exposing mammalian cells having receptor proteins, e.g. HER2, to the antibody of the ADC in a cell culture medium; culturing the cells for a period from about 6 hours to about 5 days; and measuring cell viability. Cell-based *in vitro* assays are used to measure viability (proliferation), cytotoxicity, and induction of apoptosis (caspase activation) of an ADC of the invention.

The *in vitro* potency of antibody-drug conjugates can be measured by a cell proliferation assay. The CellTiter-Glo® Luminescent Cell Viability Assay is a commercially available (Promega Corp., Madison, WI), homogeneous assay method based on the recombinant expression of *Coleoptera* luciferase (US Patent Nos. 5583024; 5674713 and 5700670). This cell proliferation assay determines the number of viable cells in culture based on quantitation of the ATP present, an indicator of metabolically active cells (Crouch et al (1993) J. Immunol. Meth. 160:81-88; US 6602677). The CellTiter-Glo® Assay is conducted in 96 well format, making it amenable to automated high-throughput screening (HTS) (Cree et al (1995) AntiCancer Drugs 6:398-404). The homogeneous assay procedure involves adding the single reagent (CellTiter-Glo® Reagent) directly to cells cultured in serum-supplemented medium. Cell washing, removal of medium and multiple pipetting steps are not required. The system detects as few as 15 cells/well in a 384-well format in 10 minutes after adding reagent and mixing. The cells may be treated continuously with ADC, or they may be treated and separated from ADC. Generally, cells treated briefly, i.e. 3 hours, showed the same potency effects as continuously treated cells.

The homogeneous "add-mix-measure" format results in cell lysis and generation of a luminescent signal proportional to the amount of ATP present. The amount of ATP is directly proportional to the number of cells present in culture. The CellTiter-Glo® Assay generates a "glow-type" luminescent signal, produced by the luciferase reaction, which has a half-life generally greater than five hours, depending on cell type and medium used. Viable cells are reflected in relative luminescence units (RLU). The substrate, Beetle Luciferin, is oxidatively decarboxylated by recombinant firefly luciferase with concomitant conversion of ATP to AMP and generation of photons.

The *in vitro* potency of antibody-drug conjugates can also be measured by a cytotoxicity assay. Cultured adherent cells are washed with PBS, detached with trypsin, diluted in complete medium, containing 10% FCS, centrifuged, re-suspended in fresh medium and counted with a haemocytometer. Suspension cultures are counted directly. Monodisperse cell suspensions suitable for counting may require agitation of the suspension by repeated aspiration to break up cell clumps.

The cell suspension is diluted to the desired seeding density and dispensed (100µl per well) into black 96 well plates. Plates of adherent cell lines are incubated overnight to allow adherence. Suspension cell cultures can be used on the day of seeding.

A stock solution (1ml) of ADC (20µg/ml) is made in the appropriate cell culture medium. Serial 10-fold dilutions of stock ADC are made in 15ml centrifuge tubes by serially transferring 100µl to 900µl of cell culture medium.

Four replicate wells of each ADC dilution (100µl) are dispensed in 96-well black plates, previously plated with cell suspension (100µl), resulting in a final volume of 200 µl. Control wells receive cell culture medium (100µl).

If the doubling time of the cell line is greater than 30 hours, ADC incubation is for 5 days, otherwise a four day incubation is done.

At the end of the incubation period, cell viability is assessed with the Alamar blue assay. AlamarBlue (Invitrogen) is dispensed over the whole plate (20µl per well) and incubated for 4 hours. Alamar blue fluorescence is measured at excitation 570nm, emission 585nm on the Varioskan flash plate reader. Percentage cell survival is calculated from the mean fluorescence in the ADC treated wells compared to the mean fluorescence in the control wells.

### In vivo efficacy

The *in vivo* efficacy of antibody-drug conjugates (ADC) of the invention can be measured by tumor xenograft studies in mice. For example, the in vivo efficacy of an anti-HER2 ADC of the invention can be measured by a high expressing HER2 transgenic explant mouse model. An allograft is propagated from the Fo5 mmtv transgenic mouse which does not respond to, or responds poorly to, HERCEPTIN® therapy. Subjects were treated once with ADC at certain dose levels (mg/kg) and PBD drug exposure (µg/m²); and placebo buffer control (Vehicle) and monitored over two weeks or more to measure the time to tumor doubling, log cell kill, and tumor shrinkage.

### Use

The conjugates of the invention may be used to provide a PBD conjugate at a target location.

The target location is preferably a proliferative cell population. The antibody is an antibody for an antigen present in a proliferative cell population.

In one embodiment the antigen is absent or present at a reduced level in a non-proliferative cell population compared to the amount of antigen present in the proliferative cell population, for example a tumour cell population.

The target location may be *in vitro, in vivo* or ex *vivo.*

The antibody-drug conjugate (ADC) compounds of the invention include those with utility for anticancer activity. In particular, the compounds include an antibody conjugated, i.e. covalently attached by a linker, to a PBD moiety.

At the target location the linker may not be cleaved. The antibody-drug conjugate (ADC compounds of the invention may have a cytotoxic effect without the cleavage of the linker to release a PBD drug moiety. The antibody-drug conjugates (ADC) of the invention selectively deliver cytotoxic agent to tumor tissue whereby greater selectivity, i.e. a lower efficacious dose, may be achieved.

Thus, in one aspect, the present invention provides a conjugate compound as described herein for use in therapy.

In a further aspect there is also provides a conjugate compound as described herein for use in the treatment of a proliferative disease. A second aspect of the present invention provides the use of a conjugate compound in the manufacture of a medicament for treating a proliferative disease.

One of ordinary skill in the art is readily able to determine whether or not a candidate conjugate treats a proliferative condition for any particular cell type. For example, assays which may conveniently be used to assess the activity offered by a particular compound are described in the examples below.

The term "proliferative disease" pertains to an unwanted or uncontrolled cellular proliferation of excessive or abnormal cells which is undesired, such as, neoplastic or hyperplastic growth, whether *in vitro* or *in vivo.*

Examples of proliferative conditions include, but are not limited to, benign, pre-malignant, and malignant cellular proliferation, including but not limited to, neoplasms and tumours (e.g. histocytoma, glioma, astrocyoma, osteoma), cancers (e.g. lung cancer, small cell lung cancer, gastrointestinal cancer, bowel cancer, colon cancer, breast carinoma, ovarian carcinoma, prostate cancer, testicular cancer, liver cancer, kidney cancer, bladder cancer, pancreas cancer, brain cancer, sarcoma, osteosarcoma, Kaposi's sarcoma, melanoma), leukemias, psoriasis, bone diseases, fibroproliferative disorders (e.g. of connective tissues), and atherosclerosis. Cancers of particular interest include, but are not limited to, leukemias and ovarian cancers.

Any type of cell may be treated, including but not limited to, lung, gastrointestinal (including, e.g. bowel, colon), breast (mammary), ovarian, prostate, liver (hepatic), kidney (renal), bladder, pancreas, brain, and skin.

In one embodiment, the treatment is of a pancreatic cancer.
In one embodiment, the treatment is of a tumour having αᵥβ₆ integrin on the surface of the cell.

It is contemplated that the antibody-drug conjugates (ADC) of the present invention may be used to treat various diseases or disorders, e.g. characterized by the overexpression of a tumor antigen. Exemplary conditions or hyperproliferative disorders include benign or malignant tumors; leukemia, haematological, and lymphoid malignancies. Others include neuronal, glial, astrocytal, hypothalamic, glandular, macrophagal, epithelial, stromal, blastocoelic, inflammatory, angiogenic and immunologic, including autoimmune, disorders.

Generally, the disease or disorder to be treated is a hyperproliferative disease such as cancer. Examples of cancer to be treated herein include, but are not limited to, carcinoma, lymphoma, blastoma, sarcoma, and leukemia or lymphoid malignancies. More particular examples of such cancers include squamous cell cancer (e.g. epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer.

Autoimmune diseases for which the ADC compounds may be used in treatment include rheumatologic disorders (such as, for example, rheumatoid arthritis, Sjögren's syndrome, scleroderma, lupus such as SLE and lupus nephritis, polymyositis/dermatomyositis, cryoglobulinemia, anti-phospholipid antibody syndrome, and psoriatic arthritis), osteoarthritis, autoimmune gastrointestinal and liver disorders (such as, for example, inflammatory bowel diseases (e.g. ulcerative colitis and Crohn's disease), autoimmune gastritis and pernicious anemia, autoimmune hepatitis, primary biliary cirrhosis, primary sclerosing cholangitis, and celiac disease), vasculitis (such as, for example, ANCA-associated vasculitis, including Churg-Strauss vasculitis, Wegener's granulomatosis, and polyarteriitis), autoimmune neurological disorders (such as, for example, multiple sclerosis, opsoclonus myoclonus syndrome, myasthenia gravis, neuromyelitis optica, Parkinson's disease, Alzheimer's disease, and autoimmune polyneuropathies), renal disorders (such as, for example, glomerulonephritis, Goodpasture's syndrome, and Berger's disease), autoimmune dermatologic disorders (such as, for example, psoriasis, urticaria, hives, pemphigus vulgaris, bullous pemphigoid, and cutaneous lupus erythematosus), hematologic disorders (such as, for example, thrombocytopenic purpura, thrombotic thrombocytopenic purpura, post-transfusion purpura, and autoimmune hemolytic anemia), atherosclerosis, uveitis, autoimmune hearing diseases (such as, for example, inner ear disease and hearing loss), Behcet's disease, Raynaud's syndrome, organ transplant, and autoimmune endocrine disorders (such as, for example, diabetic-related autoimmune diseases such as insulin-dependent diabetes mellitus (IDDM), Addison's disease, and autoimmune thyroid disease (e.g. Graves' disease and thyroiditis)). More preferred such diseases include, for example, rheumatoid arthritis, ulcerative colitis, ANCA-associated vasculitis, lupus, multiple sclerosis, Sjögren's syndrome, Graves' disease, IDDM, pernicious anemia, thyroiditis, and glomerulonephritis.

### Treatment

The conjugates of the present invention may be used in a method of therapy. Also provided is a conjugate as defined in the claims for use in a method of treatment, comprising administering to a subject in need of treatment a therapeutically-effective amount of a conjugate compound of the invention. The term "therapeutically effective amount" is an amount sufficient to show benefit to a patient. Such benefit may be at least amelioration of at least one symptom. The actual amount administered, and rate and time-course of administration, will depend on the nature and severity of what is being treated. Prescription of treatment, e.g. decisions on dosage, is within the responsibility of general practitioners and other medical doctors.

A compound of the invention may be administered alone or in combination with other treatments, either simultaneously or sequentially dependent upon the condition to be treated. Examples of treatments and therapies include, but are not limited to, chemotherapy (the administration of active agents, including, e.g. drugs, such as chemotherapeutics); surgery; and radiation therapy.

A "chemotherapeutic agent" is a chemical compound useful in the treatment of cancer, regardless of mechanism of action. Classes of chemotherapeutic agents include, but are not limited to: alkylating agents, antimetabolites, spindle poison plant alkaloids, cytotoxic/antitumor antibiotics, topoisomerase inhibitors, antibodies, photosensitizers, and kinase inhibitors. Chemotherapeutic agents include compounds used in "targeted therapy" and conventional chemotherapy.

Examples of chemotherapeutic agents include: erlotinib (TARCEVA®, Genentech/OSI Pharm.), docetaxel (TAXOTERE®, Sanofi-Aventis), 5-FU (fluorouracil, 5-fluorouracil, CAS No. 51-21-8), gemcitabine (GEMZAR®, Lilly), PD-0325901 (CAS No. 391210-10-9, Pfizer), cisplatin (cis-diamine, dichloroplatinum(II), CAS No. 15663-27-1), carboplatin (CAS No. 41575-94-4), paclitaxel (TAXOL®, Bristol-Myers Squibb Oncology, Princeton, N.J.), trastuzumab (HERCEPTIN®, Genentech), temozolomide (4-methyl-5-oxo- 2,3,4,6,8-pentazabicyclo [4.3.0] nona-2,7,9-triene- 9-carboxamide, CAS No. 85622-93-1, TEMODAR®, TEMODAL®, Schering Plough), tamoxifen ((Z)-2-[4-(1,2-diphenylbut-1-enyl)phenoxy]-*N*,*N*-dimethylethanamine, NOLVADEX®, ISTUBAL®, VALODEX®), and doxorubicin (ADRIAMYCIN®), Akti-1/2, HPPD, and rapamycin.
More examples of chemotherapeutic agents include: oxaliplatin (ELOXATIN®, Sanofi), bortezomib (VELCADE®, Millennium Pharm.), sutent (SUNITINIB®, SU11248, Pfizer), letrozole (FEMARA®, Novartis), imatinib mesylate (GLEEVEC®, Novartis), XL-518 (Mek inhibitor, Exelixis, WO 2007/044515), ARRY-886 (Mek inhibitor, AZD6244, Array BioPharma, Astra Zeneca), SF-1126 (PI3K inhibitor, Semafore Pharmaceuticals), BEZ-235 (PI3K inhibitor, Novartis), XL-147 (PI3K inhibitor, Exelixis), PTK787/ZK 222584 (Novartis), fulvestrant (FASLODEX®, AstraZeneca), leucovorin (folinic acid), rapamycin (sirolimus, RAPAMUNE®, Wyeth), lapatinib (TYKERB®, GSK572016, Glaxo Smith Kline), lonafarnib (SARASAR™, SCH 66336, Schering Plough), sorafenib (NEXAVAR®, BAY43-9006, Bayer Labs), gefitinib (IRESSA®, AstraZeneca), irinotecan (CAMPTOSAR®, CPT-11, Pfizer), tipifarnib (ZARNESTRA™, Johnson & Johnson), ABRAXANE™ (Cremophor-free), albumin-engineered nanoparticle formulations of paclitaxel (American Pharmaceutical Partners, Schaumberg, II), vandetanib (rINN, ZD6474, ZACTIMA®, AstraZeneca), chloranmbucil, AG1478, AG1571 (SU 5271; Sugen), temsirolimus (TORISEL®, Wyeth), pazopanib (GlaxoSmithKline), canfosfamide (TELCYTA®, Telik), thiotepa and cyclosphosphamide (CYTOXAN®, NEOSAR®); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); a camptothecin (including the synthetic analog topotecan); bryostatin; callystatin; CC-1065 (including its adozelesin, carzelesin and bizelesin synthetic analogs); cryptophycins (particularly cryptophycin 1 and cryptophycin 8); dolastatin; duocarmycin (including the synthetic analogs, KW-2189 and CB1-TM1); eleutherobin; pancratistatin; a sarcodictyin; spongistatin; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e.g. calicheamicin, calicheamicin gamma1I, calicheamicin omegaI1 (Angew Chem. Intl. Ed. Engl. (1994) 33:183-186); dynemicin, dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, morpholino-doxorubicin, cyanomorpholino-doxorubicin, 2-pyrrolino-doxorubicin and deoxydoxorubicin), epirubicin, esorubicin, idarubicin, nemorubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogs such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; 6-thioguanine; mercaptopurine; methotrexate; platinum analogs such as cisplatin and carboplatin; vinblastine; etoposide (VP-16); ifosfamide; mitoxantrone; vincristine; vinorelbine (NAVELBINE®); novantrone; teniposide; edatrexate; daunomycin; aminopterin; capecitabine (XELODA®, Roche); ibandronate; CPT-11; topoisomerase inhibitor RFS 2000; difluoromethylornithine (DMFO); retinoids such as retinoic acid; and pharmaceutically acceptable salts, acids and derivatives of any of the above.

Also included in the definition of "chemotherapeutic agent" are: (i) anti-hormonal agents that act to regulate or inhibit hormone action on tumors such as anti-estrogens and selective estrogen receptor modulators (SERMs), including, for example, tamoxifen (including NOLVADEX®; tamoxifen citrate), raloxifene, droloxifene, 4-hydroxytamoxifen, trioxifene, keoxifene, LY117018, onapristone, and FARESTON® (toremifine citrate); (ii) aromatase inhibitors that inhibit the enzyme aromatase, which regulates estrogen production in the adrenal glands, such as, for example, 4(5)-imidazoles, aminoglutethimide, MEGASE® (megestrol acetate), AROMASIN® (exemestane; Pfizer), formestanie, fadrozole, RIVISOR® (vorozole), FEMARA® (letrozole; Novartis), and ARIMIDEX® (anastrozole; AstraZeneca); (iii) anti-androgens such as flutamide, nilutamide, bicalutamide, leuprolide, and goserelin; as well as troxacitabine (a 1,3-dioxolane nucleoside cytosine analog); (iv) protein kinase inhibitors such as MEK inhibitors (WO 2007/044515); (v) lipid kinase inhibitors; (vi) antisense oligonucleotides, particularly those which inhibit expression of genes in signaling pathways implicated in aberrant cell proliferation, for example, PKC-alpha, Raf and H-Ras, such as oblimersen (GENASENSE®, Genta Inc.); (vii) ribozymes such as VEGF expression inhibitors (e.g., ANGIOZYME®) and HER2 expression inhibitors; (viii) vaccines such as gene therapy vaccines, for example, ALLOVECTIN®, LEUVECTIN®, and VAXID®; PROLEUKIN® rIL-2; topoisomerase 1 inhibitors such as LURTOTECAN®; ABARELIX® rmRH; (ix) anti-angiogenic agents such as bevacizumab (AVASTIN®, Genentech); and pharmaceutically acceptable salts, acids and derivatives of any of the above.
Also included in the definition of "chemotherapeutic agent" are therapeutic antibodies such as alemtuzumab (Campath), bevacizumab (AVASTIN®, Genentech); cetuximab (ERBITUX®, Imclone); panitumumab (VECTIBIX®, Amgen), rituximab (RITUXAN®, Genentech/Biogen Idec), pertuzumab (OMNITARG™, 2C4, Genentech), trastuzumab (HERCEPTIN®, Genentech), tositumomab (Bexxar, Corixia), and the antibody drug conjugate, gemtuzumab ozogamicin (MYLOTARG®, Wyeth).

Humanized monoclonal antibodies with therapeutic potential as chemotherapeutic agents in combination with the conjugates of the invention include: alemtuzumab, apolizumab, aselizumab, atlizumab, bapineuzumab, bevacizumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nolovizumab, numavizumab, ocrelizumab, omalizumab, palivizumab, pascolizumab, pecfusituzumab, pectuzumab, pertuzumab, pexelizumab, ralivizumab, ranibizumab, reslivizumab, reslizumab, resyvizumab, rovelizumab, ruplizumab, sibrotuzumab, siplizumab, sontuzumab, tacatuzumab tetraxetan, tadocizumab, talizumab, tefibazumab, tocilizumab, toralizumab, trastuzumab, tucotuzumab celmoleukin, tucusituzumab, umavizumab, urtoxazumab, and visilizumab.

Pharmaceutical compositions according to the present invention, and for use in accordance with the present invention, may comprise, in addition to the active ingredient, i.e. a conjugate compound, a pharmaceutically acceptable excipient, carrier, buffer, stabiliser or other materials well known to those skilled in the art. Such materials should be non-toxic and should not interfere with the efficacy of the active ingredient. The precise nature of the carrier or other material will depend on the route of administration, which may be oral, or by injection, e.g. cutaneous, subcutaneous, or intravenous.

Pharmaceutical compositions for oral administration may be in tablet, capsule, powder or liquid form. A tablet may comprise a solid carrier or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included. A capsule may comprise a solid carrier such a gelatin.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

### Formulations

While it is possible for the conjugate compound to be used (e.g., administered) alone, it is often preferable to present it as a composition or formulation.

In one embodiment, the composition is a pharmaceutical composition (e.g., formulation, preparation, medicament) comprising a conjugate compound, as described herein, and a pharmaceutically acceptable carrier, diluent, or excipient.

In one embodiment, the composition is a pharmaceutical composition comprising at least one conjugate compound, as described herein, together with one or more other pharmaceutically acceptable ingredients well known to those skilled in the art, including, but not limited to, pharmaceutically acceptable carriers, diluents, excipients, adjuvants, fillers, buffers, preservatives, anti-oxidants, lubricants, stabilisers, solubilisers, surfactants (e.g., wetting agents), masking agents, colouring agents, flavouring agents, and sweetening agents.

In one embodiment, the composition further comprises other active agents, for example, other therapeutic or prophylactic agents.

Suitable carriers, diluents, excipients, etc. can be found in standard pharmaceutical texts. See, for example, Handbook of Pharmaceutical Additives, 2nd Edition (eds. M. Ash and I. Ash), 2001 (Synapse Information Resources, Inc., Endicott, New York, USA), Remington's Pharmaceutical Sciences, 20th edition, pub. Lippincott, Williams & Wilkins, 2000; and Handbook of Pharmaceutical Excipients, 2nd edition, 1994.

Another aspect of the present invention pertains to methods of making a pharmaceutical composition comprising admixing at least one [¹¹C]-radiolabelled conjugate or conjugate-like compound, as defined herein, together with one or more other pharmaceutically acceptable ingredients well known to those skilled in the art, e.g., carriers, diluents, excipients, etc. If formulated as discrete units (e.g., tablets, etc.), each unit contains a predetermined amount (dosage) of the active compound.

The term "pharmaceutically acceptable," as used herein, pertains to compounds, ingredients, materials, compositions, dosage forms, etc., which are, within the scope of sound medical judgment, suitable for use in contact with the tissues of the subject in question (e.g., human) without excessive toxicity, irritation, allergic response, or other problem or complication, commensurate with a reasonable benefit/risk ratio. Each carrier, diluent, excipient, etc. must also be "acceptable" in the sense of being compatible with the other ingredients of the formulation.

The formulations may be prepared by any methods well known in the art of pharmacy. Such methods include the step of bringing into association the active compound with a carrier which constitutes one or more accessory ingredients. In general, the formulations are prepared by uniformly and intimately bringing into association the active compound with carriers (e.g., liquid carriers, finely divided solid carrier, etc.), and then shaping the product, if necessary.

The formulation may be prepared to provide for rapid or slow release; immediate, delayed, timed, or sustained release; or a combination thereof.

Formulations suitable for parenteral administration (e.g., by injection), include aqueous or non-aqueous, isotonic, pyrogen-free, sterile liquids (e.g., solutions, suspensions), in which the active ingredient is dissolved, suspended, or otherwise provided (e.g., in a liposome or other microparticulate). Such liquids may additional contain other pharmaceutically acceptable ingredients, such as anti-oxidants, buffers, preservatives, stabilisers, bacteriostats, suspending agents, thickening agents, and solutes which render the formulation isotonic with the blood (or other relevant bodily fluid) of the intended recipient. Examples of excipients include, for example, water, alcohols, polyols, glycerol, vegetable oils, and the like. Examples of suitable isotonic carriers for use in such formulations include Sodium Chloride Injection, Ringer's Solution, or Lactated Ringer's Injection. Typically, the concentration of the active ingredient in the liquid is from about 1 ng/ml to about 10 µg/ml, for example from about 10 ng/ml to about 1 µg/ml. The formulations may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules, and tablets.

### Dosage

It will be appreciated by one of skill in the art that appropriate dosages of the conjugate compound, and compositions comprising the conjugate compound, can vary from patient to patient. Determining the optimal dosage will generally involve the balancing of the level of therapeutic benefit against any risk or deleterious side effects. The selected dosage level will depend on a variety of factors including, but not limited to, the activity of the particular compound, the route of administration, the time of administration, the rate of excretion of the compound, the duration of the treatment, other drugs, compounds, and/or materials used in combination, the severity of the condition, and the species, sex, age, weight, condition, general health, and prior medical history of the patient. The amount of compound and route of administration will ultimately be at the discretion of the physician, veterinarian, or clinician, although generally the dosage will be selected to achieve local concentrations at the site of action which achieve the desired effect without causing substantial harmful or deleterious side-effects.

Administration can be effected in one dose, continuously or intermittently (e.g., in divided doses at appropriate intervals) throughout the course of treatment. Methods of determining the most effective means and dosage of administration are well known to those of skill in the art and will vary with the formulation used for therapy, the purpose of the therapy, the target cell(s) being treated, and the subject being treated. Single or multiple administrations can be carried out with the dose level and pattern being selected by the treating physician, veterinarian, or clinician.

In general, a suitable dose of the active compound is in the range of about 100 ng to about 25 mg (more typically about 1 µg to about 10 mg) per kilogram body weight of the subject per day. Where the active compound is a salt, an ester, an amide, a prodrug, or the like, the amount administered is calculated on the basis of the parent compound and so the actual weight to be used is increased proportionately.

In one embodiment, the active compound is administered to a human patient according to the following dosage regime: about 100 mg, 3 times daily.

In one embodiment, the active compound is administered to a human patient according to the following dosage regime: about 150 mg, 2 times daily.

In one embodiment, the active compound is administered to a human patient according to the following dosage regime: about 200 mg, 2 times daily.

However in one embodiment, the conjugate compound is administered to a human patient according to the following dosage regime: about 50 or about 75 mg, 3 or 4 times daily.

In one embodiment, the conjugate compound is administered to a human patient according to the following dosage regime: about 100 or about 125 mg, 2 times daily.

The dosage amounts described above may apply to the conjugate (including the PBD moiety and the linker to the antibody) or to the effective amount of PBD compound provided, for example the amount of compound that is releasable after cleavage of the linker.

For the prevention or treatment of disease, the appropriate dosage of an ADC of the invention will depend on the type of disease to be treated, as defined above, the severity and course of the disease, whether the molecule is administered for preventive or therapeutic purposes, previous therapy, the patient's clinical history and response to the antibody, and the discretion of the attending physician. The molecule is suitably administered to the patient at one time or over a series of treatments. Depending on the type and severity of the disease, about 1 µg/kg to 15 mg/kg (e.g. 0.1-20 mg/kg) of molecule is an initial candidate dosage for administration to the patient, whether, for example, by one or more separate administrations, or by continuous infusion. A typical daily dosage might range from about 1 µg/kg to 100 mg/kg or more, depending on the factors mentioned above. An exemplary dosage of ADC to be administered to a patient is in the range of about 0.1 to about 10 mg/kg of patient weight. For repeated administrations over several days or longer, depending on the condition, the treatment is sustained until a desired suppression of disease symptoms occurs. An exemplary dosing regimen comprises a course of administering an initial loading dose of about 4 mg/kg, followed by additional doses every week, two weeks, or three weeks of an ADC. Other dosage regimens may be useful. The progress of this therapy is easily monitored by conventional techniques and assays.

### Treatment

The term "treatment," as used herein in the context of treating a condition, pertains generally to treatment and therapy, whether of a human or an animal (e.g., in veterinary applications), in which some desired therapeutic effect is achieved, for example, the inhibition of the progress of the condition, and includes a reduction in the rate of progress, a halt in the rate of progress, regression of the condition, amelioration of the condition, and cure of the condition. Treatment as a prophylactic measure (i.e., prophylaxis, prevention) is also included.

The term "therapeutically-effective amount," as used herein, pertains to that amount of an active compound, or a material, composition or dosage from comprising an active compound, which is effective for producing some desired therapeutic effect, commensurate with a reasonable benefit/risk ratio, when administered in accordance with a desired treatment regimen.

Similarly, the term "prophylactically-effective amount," as used herein, pertains to that amount of an active compound, or a material, composition or dosage from comprising an active compound, which is effective for producing some desired prophylactic effect, commensurate with a reasonable benefit/risk ratio, when administered in accordance with a desired treatment regimen.

### Preparation of Antibody drug conjugates

Antibody drug conjugates may be prepared by several routes, employing organic chemistry reactions, conditions, and reagents known to those skilled in the art, including: (1) reaction of a nucleophilic group of an antibody with a bivalent linker reagent, to form antibody-linker intermediate Ab-L, via a covalent bond, followed by reaction with an activated drug moiety reagent; and (2) reaction of a drug moiety reagent with a linker reagent, to form drug-linker reagent D-L, via a covalent bond, followed by reaction with the nucleophilic of an antibody. Conjugation methods (1) and (2) may be employed with a variety of antibodies, and linkers to prepare the antibody-drug conjugates of the invention.

Nucleophilic groups on antibodies include, but are not limited to side chain thiol groups, e.g. cysteine. Thiol groups are nucleophilic and capable of reacting to form covalent bonds with electrophilic groups on linker moieties such as those of the present invention. Certain antibodies have reducible interchain disulfides, i.e. cysteine bridges. Antibodies may be made reactive for conjugation with linker reagents by treatment with a reducing agent such as DTT (Cleland's reagent, dithiothreitol) or TCEP (tris(2-carboxyethyl)phosphine hydrochloride; Getz et al (1999) Anal. Biochem. Vol 273:73-80; Soltec Ventures, Beverly, MA). Each cysteine disulfide bridge will thus form, theoretically, two reactive thiol nucleophiles. Additional nucleophilic groups can be introduced into antibodies through the reaction of lysines with 2-iminothiolane (Traut's reagent) resulting in conversion of an amine into a thiol.

### The Subject/Patient

The subject/patient may be an animal, mammal, a placental mammal, a marsupial (e.g., kangaroo, wombat), a monotreme (e.g., duckbilled platypus), a rodent (e.g., a guinea pig, a hamster, a rat, a mouse), murine (e.g., a mouse), a lagomorph (e.g., a rabbit), avian (e.g., a bird), canine (e.g., a dog), feline (e.g., a cat), equine (e.g., a horse), porcine (e.g., a pig), ovine (e.g., a sheep), bovine (e.g., a cow), a primate, simian (e.g., a monkey or ape), a monkey (e.g., marmoset, baboon), an ape (e.g., gorilla, chimpanzee, orangutang, gibbon), or a human.

Furthermore, the subject/patient may be any of its forms of development, for example, a foetus. In one preferred embodiment, the subject/patient is a human.

In one embodiment, the patient is a population where each patient has a tumour having αᵥβ₆ integrin on the surface of the cell.

### Synthesis

Conjugates of formula A may be synthesised from corresponding drug-linker compounds of formula B by reacting them with cell binding agents under appropriate conditions. Thus, conjugates where Y is of formula A1 may be synthesied from drug-linker compounds where Y^{L} is of formula B1. Conjugates where Y is of formula A2 may be synthesied from drug-linker compounds where Y^{L} is of formula B2. Conjugates where Y is of formula A3 may be synthesied from drug-linker compounds where Y^{L} is of formula B3.

The conditions, as described above, will depend on the type of bond being formed between the drug-linker compound and the cell binding agent, which itself will reflect the nature of the binding site on the cell binding agent.

Drug-linker compounds of formula B may be synthesised from corresponding compounds of formula C.

Drug-linker compounds where Y^{L} is of formula B1 may be synthesied from compounds where Y^{C} is of formula C1, by reaction with a compound of formula E1:

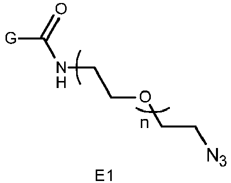

in an appropriate solvent. Compounds of formula E1 may be formed *in situ* by reaction of compounds of formulae F1 and F2:

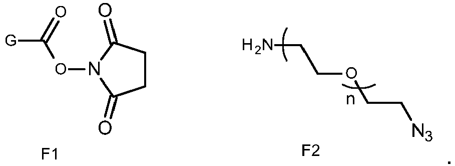

Drug-linker compounds where Y^{L} is of formula B2 may be synthesied from compounds where Y^{C} is of formula C2, by reaction with a compound of formula E2: 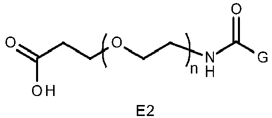 in an appropriate solvent, in the presence of an amide coupling reagent.

Drug-linker compounds where Y^{L} is of formula B3 may be synthesied from compounds where Y^{C} is of formula C3, reaction with a compound of formula E2: 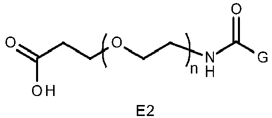 in an appropriate solvent, in the presence of an amide coupling reagent.

Compound of formula C can be made from the corresponding compound of formula G:

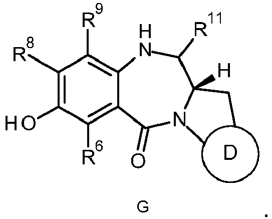

Compounds of formula C where Y^{C} is of formula C1 may be synthesised by reacting a compound of formula G with a compound of formula H1:

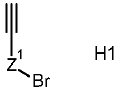

in the presence of tetrabutylammonium iodide and potassium carbonate.

Compounds of formula C where Y^{C} is of formula C2 may be synthesised by reacting a compound of formula G with a compound of formula H2: 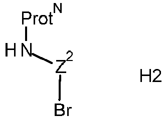 where Prot^{N} is an amine protecting group, such as Alloc, in the presence of tetrabutylammonium iodide and potassium carbonate, followed by deprotection of the amine under standard conditions. The protecting group used should be orthogonal to any other protecting groups in the compound.

Compounds of formula C where Y^{C} is of formula C3 may be synthesised by reacting a compound of formula G with a compound of formula H3: 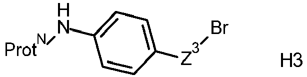 where Prot^{N} is an amine protecting group, such as Alloc, in the presence of tetrabutylammonium iodide and potassium carbonate, followed by deprotection of the amine under standard conditions. The protecting group used should be orthogonal to any other protecting groups in the compound.

Alternativelty, compounds of formula C where Y^{C} is of formula C3 may be synthesised by reacting a compound of formula G with a compound of formula H4: 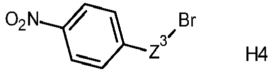 in the presence of tetrabutylammonium iodide and potassium carbonate, followed by reduction of the nitro group under standard conditions.

Compound of formula G containing a single PBD moiety can be synthesised according to the disclosure of WO 2005/085259, and in particular the discussion from pages 31 to 39, which is incorporated herein be reference. Reference is also made to the teaching of co-pending application PCT/EP2012/070232, filed on 12 October 2012.

The synthesis of PBD compounds containing two imine moieties is extensively discussed in the following references, which discussions are incorporated herein by reference:
a) WO 00/12508 (pages 14 to 30);
b) WO 2005/023814 (pages 3 to 10);
c) WO 2004/043963 (pages 28 to 29);
d) WO 2005/085251 (pages 30 to 39); and
e) WO 2011/130598 (pages 126 to 150).

The disclosure of WO 2005/085259 discussed above is also relevant to the synthesis of compounds of formula G which comprise two PBD moieties. The synthesis methods disclosed therein may be modified to include an orthogonally protected hydroxyl group at C7 (i.e.group R^{A} in Scheme 4).

Alternatively, compounds of formula G may be synthesised as described in the above references, but starting from a dimer core of formula J: 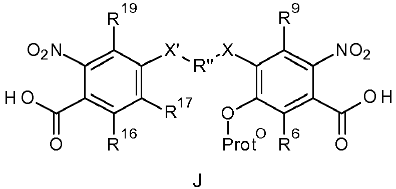 where Prot^{O} is a hydroxyl protecting group. Such compounds of formula J may be made by methods analogous to those in the examples of the present application.

### Amine protecting groups

Amine protecting groups are well-known to those skilled in the art. Particular reference is made to the disclosure of suitable protecting groups in Greene's Protecting Groups in Organic Synthesis, Fourth Edition, John Wiley & Sons, 2007 (ISBN 978-0-471-69754-1), pages 696-871.

### Hydroxyl protecting groups

Hydroxyl protecting groups are well-known to those skilled in the art. Particular reference is made to the disclosure of suitable protecting groups in Greene's Protecting Groups in Organic Synthesis, Fourth Edition, John Wiley & Sons, 2007 (ISBN 978-0-471-69754-1), pages 16-298.

### General Conditions

Reaction progress was monitored by thin-layer chromatography (TLC) using Merck Kieselgel 60 F254 silica gel, with fluorescent indicator on aluminium plates. Visualisation of TLC was achieved with UV light or iodine vapour unless otherwise stated. Flash chromatography was performed using Merck Kieselgel 60 F254 silica gel. Extraction and chromatography solvents were bought and used without further purification from Fisher Scientific, U.K. All chemicals were purchased from Aldrich, VWR, and Combi Blocks.
¹H and ¹³C NMR spectra were obtained on a Bruker Avance 400 spectrometer. Coupling constants are quoted in hertz (Hz). Chemical shifts are recorded in parts per million (ppm) downfield from tetramethylsilane. Spin multiplicities are described as s (singlet), bs (broad singlet), d (doublet), t (triplet), q (quartet), p (pentuplet) and m (multiplet).

### LCMS Method 1 (default when not specified)

The HPLC (Waters Alliance 2695) was run using a mobile phase of water (A) (formic acid 0.1%) and acetonitrile (B) (formic acid 0.1%). Gradient: initial composition 5% B held over 1.0 min, then increase from 5% B to 95% B over a 3 min period. The composition was held for 0.1 min at 95% B, then returned to 5% B in 0.03 minutes and hold there for 0.87 min. Total gradient run time equals 5 min.

Flow rate 3.0 mL/min, 400µL was split *via* a zero dead volume tee piece which passes into the mass spectrometer. Wavelength detection range: 220 to 400 nm. Function type: diode array (535 scans). Column: Phenomenex Onyx Monolithic C18 50 x 4.60 mm

### LCMS Method 2

The HPLC (Waters Alliance 2695) was run using a mobile phase of water (A) (formic acid 0.1%) and acetonitrile (B) (formic acid 0.1%). Gradient: initial composition 5% B held over 1.0 min, then increase from 5% B to 95% B over a 2.5 min period. The composition was held for 0.5 min at 95% B, then returned to 5% B in 0.1 minutes and hold there for 0.9 min. Total gradient run time equals 5 min.

Flow rate 3.0 mL/min, 400µL was split *via* a zero dead volume tee piece which passes into the mass spectrometer. Wavelength detection range: 220 to 400 nm. Function type: diode array (535 scans). Column: Phenomenex Onyx Monolithic C18 50 x 4.60 mm."

### LCMS Method 3

The HPLC (Shimazu LCMS-2020) was run using a mobile phase of water (A) (formic acid 0.1%) and acetonitrile (B) (formic acid 0.1%). Gradient: initial composition 5% B held over 0.25 min, then increase from 5% B to 100% B over a 2 min period. The composition was held for 0.50 min at 100% B, then returned to 5% B in 0.05 minutes and hold there for 0.05 min. Total gradient run time equals 3 minutes.

Flow rate 0.8 mL/min. Wavelength detection range: 220 to 400 nm. Column: Waters Acquity UPLC BEH Shield RP18 1.7µm 2.1x50mm.

### Synthesis of Key Intermediates

### (a) 5-(benzyloxy)-4-((5-(4-carboxy-2-methoxy-5-nitrophenoxy)pentyl)oxy)-2-nitrobenzoic acid (I7)

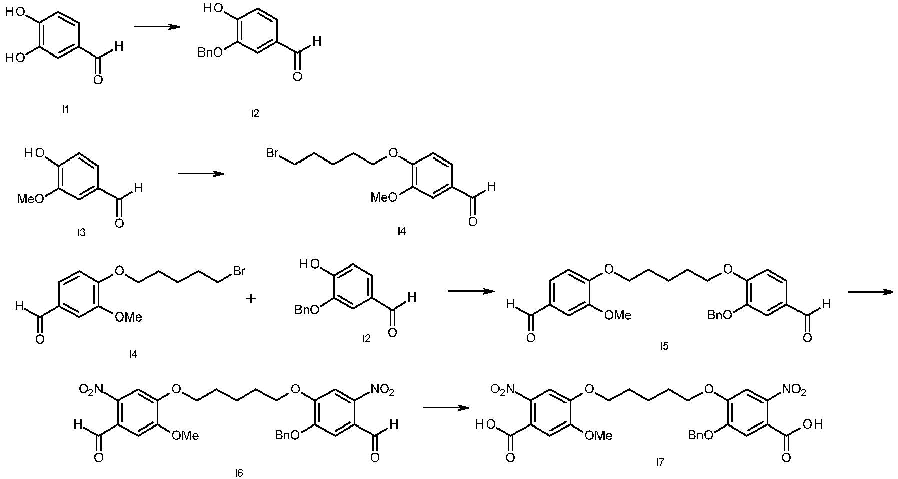

### (i) 3-(benzyloxy)-4-hydroxybenzaldehyde (12)

Sodium hydride (51.2 g, 1.27 mol, 2.2 eq) was rinsed twice with hexane in a three-neck flask and anhydrous DMSO (800 mL) was added. The flask was placed in a water bath at room temperature. A solution of 3,4-dihydroxybenzaldehyde I1 (80 g, 579.2 mmol) in dry DMSO (160 mL) was added dropwise, with an addition funnel, over 40 minutes and the reaction mixture was stirred 30 minutes under Argon. During the addition a lot of hydrogen gas is formed, so an exit with cotton wool and calcium chloride is placed in one of the neck. Benzyl bromide (68.8 mL, 579.2 mmol, 1 eq) was then added dropwise and the reaction was stirred overnight. The reaction mixture was poured into ice and quenched with HCI (1M) until pH acid and extracted with EtOAc. The organic phase was then washed with brine and was dried over magnesium sulphate, filtered and excess solvent was removed by rotary evaporation under reduced pressure. The resulting residue was subjected to a pad of Silica with pure dichloromethane. The resulting material was precipitated with a minimum of dichloromethane in hexane. The resulting white precipitate was filtered and dried to afford the desired compound (80.7 g, 60 % yield). LC/MS (Method 3) 1.43 min (no ionisation). ¹H NMR (400 MHz, CDCl₃) δ 9.81 (s, 1H), 7.51 (d, *J* = 1.8 Hz, 1H), 7.45 - 7.34 (m, 7H), 7.06 (d, *J* = 8.1 Hz, 1H), 6.24 (s, 1H), 5.17 (s, 2H).

### (ii) 4-((5-bromopentyl)oxy)-3-methoxybenzaldehyde (14)

Vanillin I3 (50 g, 328 mmol) was dissolved in acetone (1 L). Dibromopentane (227 g, 985 mmol, 3 eq) and potassium carbonate (68 g, 492 mmol, 1.5 eq) were added. The slurry was warmed to 65°C and stirred for 2 hours, and then 80°C for 30 minutes. The resulting potassium carbonate was filtered and the excess solvent was removed by rotary evaporation under reduced pressure. The resulting residue was subjected to a pad of Silica: 7.5% EtOAc in Hexane (4 L) then 10% EtOAc in Hexane (1 L) and 25% EtOAc in Hexane to afford a white solid (53.7 g, 54 % yield). LC/MS (Method 3) 1.63 min (ES+) *m*/*z* (relative intensity) 302.53 ([M + H]^{+.}, 100). ¹H NMR (400 MHz, CDCl₃) δ 9.83 (s, 1H), 7.42 (dd, *J* = 8.1, 1.9 Hz, 1H), 7.40 (d, *J* = 1.9 Hz, 1H), 6.95 (d, *J* = 8.1 Hz, 1H), 4.10 (t, *J* = 6.6 Hz, 2H), 3.91 (s, 3H), 3.43 (t, *J* = 6.7 Hz, 2H), 1.92 (m, 4H), 1.64 (tt, *J* = 9.7, 6.2 Hz, 2H).

### (iii) 3-(benzyloxy)-4-((5-(4-formyl-2-ethoxyphenoxy)pentyl)oxy) benzaldehyde (15)

4-((5-bromopentyl)oxy)-3-methoxybenzaldehyde I4 (40.0 g, 132.81 mmol, 1 eq) and 3-(benzyloxy)-4-hydroxybenzaldehyde I2 (30.3 g, 132.81 mmol, 1 eq) were dissolved in dimethylformamide (200 mL). Potassium carbonate (13.8 g, 99.61 mmol, 0.75 eq) and tetrabutylammonium iodide (4.9 g, 13.28 mmol, 0.1 eq) were added. The reaction mixture was warmed to 80°C and stirred for 12 hours. The resulting potassium carbonate was filtered and the excess solvent was removed by rotary evaporation under reduced pressure. The resulting residue was dissolved in ethyl acetate and washed subsequently with water, 1N NaOH, 1N HCl, water and brine. The organic phase was dried over magnesium sulphate, filtered and excess solvent was removed by rotary evaporation under reduced pressure to afford the desired compound (75 g, quantitative yield) as a light yellow oil. LC/MS (Method 3) 1.77 min (ES+) *m*/*z* (relative intensity) 449.15 [M + H]⁺, 471.25 [M + Na]⁺. ¹H NMR (400 MHz, CDCl₃) δ 9.83 (s, 1H), 9.81 (s, 1H), 7.46 (dd, *J* = 3.1, 1.4 Hz, 2H), 7.43 (d, *J* = 1.7 Hz, 2H), 7.41 (dd, *J* = 8.1, 1.9 Hz, 1H), 7.39 (d, *J* = 1.8 Hz, 1H), 7.37 - 7.32 (m, 2H), 7.30 (dd, *J* = 5.0, 3.6 Hz, 1H), 6.97 (d, *J* = 8.1 Hz, 1H), 6.92 (d, *J* = 8.1 Hz, 1H), 5.15 (s, 2H), 4.13 (t, *J* = 6.4 Hz, 2H), 4.09 (t, *J* = 6.6 Hz, 2H), 3.88 (s, 3H), 2.01 - 1.89 (m, 4H), 1.77 - 1.64 (m, 2H).

### (iv) 5-(benzyloxy)-4-((5-(4-formyl-2-methoxy-5-nitrophenoxy)pentyl)oxy)-2-nitrobenzaldehyde (16)

3-(benzyloxy)-4-((5-(4-formyl-2-ethoxyphenoxy)pentyl)oxy) benzaldehyde I5 (30 g, 66.40 mmol) was dissolved in dichloromethane (60 mL) and added to nitric acid (68%, 60 mL) at 0°C. The reaction mixture was stirred at 0°C for 20 minutes and at room temperature for 3 hours and then cold water was added. The precipitate formed was filtered and washed with water. The white solid was dried by vacuum. 42g of white solid was obtained (>100% yield due to water remaining). LC/MS (Method 3) 1.88 min, no ionisation. ¹H NMR (400 MHz, CDCl₃) δ 10.44 (s, 1H), 10.41 (s, 1H), 7.61 (s, 1H), 7.56 (s, 1H), 7.48 (s, 1H), 7.46 - 7.30 (m, 6H), 5.25 (s, 2H), 4.20 (dd, *J* = 12.2, 5.9 Hz, 2H), 4.13 (dd, *J* = 15.1, 8.7 Hz, 2H), 3.96 (s, 3H), 2.01 (dt, *J* = 14.1, 6.4 Hz, 4H), 1.80 - 1.65 (m, 2H).

### (v) 5-(benzyloxy)-4-((5-(4-carboxy-2-methoxy-5-nitrophenoxy)pentyl)oxy)-2-nitrobenzoic acid (I7)

A solution of sodium chlorite (35.3 g, 390 mmol, 5 eq) and sodium phosphate (26.2 g, 218.4 mmol, 2.8 eq) in water (250 mL) was added to a solution of 5-(benzyloxy)-4-((5-(4-formyl-2-methoxy-5-nitrophenoxy)pentyl)oxy)-2 nitrobenzaldehyde I6 (42 g, 78 mmol) in THF (200 mL). Hydrogen peroxide (60%, 103 mL, 2.18 mol, 28 eq) was then added quickly. After 30 min, the reaction wasquenched with 1N HCI and extracted with ethyl acetate. The organic layer was washed two times with brine, dried over magnesium sulphate; filtered and excess solvent was removed by rotary evaporation under reduced pressure. The resulting residue was dissolved in a minimum of dichloromethane and precipitated out with ether. Pale yellow solid (26 g, 58%) was filtered and washed with ether and used as crude for the next reaction. LC/MS (Method 3) 1.69 min (ES+) *m*/*z* (relative intensity) 569.35 [M + H]⁺.

### (b) (2R,11aS)-2-((tert-butyldimethylsilyl)oxy)-8-((5-(((2R,11aS)-2-((tert-butyldimethylsilyl)oxy)-5,11-dioxo-7-((triisopropylsilyl)oxy)-10-((2-(trimethylsilyl)ethoxy)methyl)-2,3,5,10,11,11a-hexahydro-1H-benzo[e]pyrrolo[1,2-a][1,4]diazepin-8-yl)oxy)pentyl)oxy)-7-methoxy-10-((2-(trimethylsilyl)ethoxy)methyl)-2,3-dihydro-1H-benzo[e]pyrrolo[1,2-a][1,4]diazepine-5,11(10H,11aH)-dione (113)

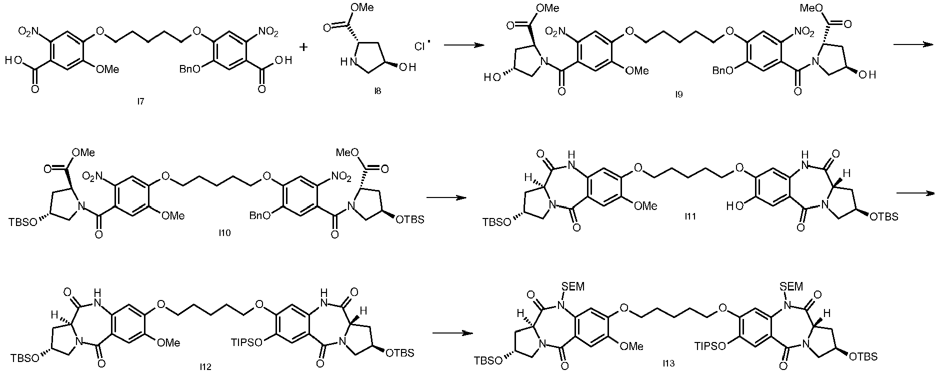

### (i) (2S,4R)-methyl 1-(4-((5-(2-(benzyloxy)-4-((2S,4R)-4-hydroxy-2-(methoxycarbonyl)pyrrolidine-1-carbonyl)-5-nitrophenoxy)pentyl)oxy)-5-methoxy- 2-nitrobenzoyl)-4-hydroxypyrrolidine-2-carboxylate (19)

Oxalyl chloride (7.6 mL, 89.92 mmol, 3 eq) was added to a solution of I7 (17.1 g, 29.97 mmol) in dichloromethane (150 mL) and DMF (2 mL). After 20 min the solvent was removed by rotary evaporation under reduced pressure and minimum of dichloromethane was added to dissolve the crude and triturated with diethyl ether. The yellow solid formed was filtered and added portion wise to a solution of I8 (13.6 g, 74.93 mmol, 2.5 eq) and triethylamine (20.92 mL, 149.87 mmol, 5eq) in dichloromethane (100 mL) at -40°C. The reaction was complete after few minutes. The solvent was removed by rotary evaporation under reduced pressure and the resulting residue was subjected to flash column chromatography (silica gel; 50% ethyl acetate in hexane to 100% ethyl acetate to collect the mono addition product and then 5% to 20% methanol in dichloromethane). Pure fractions were collected and combined and excess eluent was removed by rotary evaporation under reduced pressure to give the product (15 g, 61% over 3 steps). LC/MS (Method 3) 1.47 min (ES+) *m*/*z* (relative intensity) 825.45 ([M + H]^{+.}, 100). ¹H NMR (400 MHz, CDCl₃) δ 7.65 (s, 1H), 7.61 (s, 1H), 7.45 - 7.27 (m, 5H), 6.89 (s, 1H), 6.81 (s, 1H), 5.20 (s, 2H), 4.85 - 4.74 (m, 2H), 4.40 (d, *J* = 20.3 Hz, 2H), 4.21 - 4.02 (m, 4H), 3.91 (s, 3H), 3.79 (s, 6H), 3.53 - 3.41 (m, 2H), 3.13 (d, *J* = 11.1 Hz, 1H), 3.04 (d, *J* = 11.0 Hz, 1H), 2.79 - 2.72 (m, *J* = 4.3 Hz, 1H), 2.63 (s, 1H), 2.42 - 2.32 (m, 2H), 2.21 - 2.06 (m, *J* = 11.3 Hz, 2H), 2.02 - 1.89 (m, 4H), 1.76 - 1.65 (m, 2H).

### (ii) (2S,4R)-methyl 1-(4-((5-(2-(benzyloxy)-4-((2S,4R)-4-((tert-butyldimethylsilyl)oxy)-2-(methoxycarbonyl)pyrrolidine-1-carbonyl)-5-nitrophenoxy)pentyl)oxy)-5-methoxy- 2-nitrobenzoyl)-4-((tert-butyldimethylsilyl)oxy)pyrrolidine-2-carboxylate (110)

I9 (14 g, 16.97 mmol), tert-butyldimethylsilyl chloride (12.8 g, 84.87 mmol, 5 eq) and imidazole (13.9 g, 203.64 mmol, 12 eq) were melted together at 120°C.The reaction was complete after 30 minutes. The resulting residue was subjected to flash column chromatography (silica gel; 10% ethyl acetate in hexane to 100% ethyl acetate). Pure fractions were collected and combined and excess eluent was removed by rotary evaporation under reduced pressure to give the product (17 g, quantitative).
LC/MS (Method 3) 2.17 min. ¹H NMR (400 MHz, CDCl₃) δ 7.69 (s, 1H), 7.65 (s, 1H), 7.43 - 7.27 (m, 5H), 6.87 (s, 1H), 6.78 (s, 1H), 5.17 (d, J = 5.6 Hz, 2H), 4.73 - 7.79 (m, 2H), 4.45 - 4.34 (m, 2H), 4.32 - 4.10 (m, 2H), 4.09 - 4.00 (m, 2H), 3.90 (s, 3H), 3.80 (s, 3H), 3.79 (s, 3H), 3.48 - 3.41 (m, 1H), 3.29 (dd, *J* = 10.3, 4.6 Hz, 1H), 3.03 (dd, *J* = 10.4, 2.4 Hz, 2H), 2.96 (dd, *J* = 10.2, 2.8 Hz, 2H), 2.31 - 2.21 (m, 2H), 2.19 - 2.05 (m, 2H), 2.00 - 1.88 (m, 4H), 1.74 - 1.66 (m, 2H), 0.82 (s, 18H), 0.02 (d, *J* = 1.1 Hz, 6H), -0.03 (d, *J* = 5.5 Hz, 6H).

### (iii) (2R,11aS)-2-((tert-butyldimethylsilyl)oxy)-8-((5-(((2R,11aS)-2-((tert-butyldimethylsilyl)oxy)-7-hydroxy-5,11-dioxo-2,3,5,10,11,11a-hexahydro-1H-benzo[e]pyrrolo[1,2-a][1,4]diazepin-8-yl)oxy)pentyl)oxy)-7-methoxy-2,3-dihydro-1H-benzo[e]pyrrolo[1,2-a][1,4]diazepine-5,11(10H,11aH)-dione (I11)

Ammonium formate (106 g, 168 mmol, 10 eq) was added to a solution of I10 (17.7 g, 16.80 mmol) in ethanol (500 mL). Palladium on carbon (1.8 g, 10%) was wetted with ethyl acetate and added to the reaction mixture. The solution was warmed to 70°C. After 20 min, the reaction mixture was then filtered through celite and washed with ethyl acetate. The solvent was removed by rotary evaporation. The residue was dissolved in ethyl acetate and washed with saturated aqueous ammonium chloride and brine, dried over magnesium sulphate; filtered and excess solvent was removed by rotary evaporation under reduced pressure to give the desired compound (13.9 g, 98%) as a yellow gum.
LC/MS (Method 3) 1.91 min, (ES+) *m*/*z* (relative intensity) 839.35 ([M + H]^{+.}, 100).

### (iv) (2R,11aS)-2-((tert-butyldimethylsilyl)oxy)-8-((5-(((2R,11aS)-2-((tert-butyldimethylsilyl)oxy)-5,11-dioxo-7-((triisopropylsilyl)oxy)-2,3,5,10,11,11a-hexahydro-1H-benzo[e]pyrrolo[1,2-a][1,4]diazepin-8-yl)oxy)pentyl)oxy)-7-methoxy-2,3-dihydro-1H-benzo[e]pyrrolo[1,2-a][1,4]diazepine-5,11(10H,11aH)-dione (112)

I11 (13.9 g, 16.56 mmol), triisopropylsilyl chloride (3.6 mL, 18.36 mmol, 1.1 eq) and imidazole (3.4 g, 49.94 mmol, 3 eq) were melted together at 120°C. The reaction was complete after 30 minutes. The resulting residue was subjected to flash column chromatography (silica gel; 10% ethyl acetate in hexane to 100% ethyl acetate). Pure fractions were collected and combined and excess eluent was removed by rotary evaporation under reduced pressure to give the product (15.4 g, 93%). LC/MS (Method 3) 2.31 min, ¹H NMR (400 MHz, CDCl₃) δ 9.81 (s, 1H), 8.70 (s, 1H), 8.65 (s, 1H), 7.43 (s, 2H), 6.47 (s, 1H), 6.42 (s, 1H), 4.54 - 4.43 (m, 2H), 4.17 (dt, *J* = 7.6, 3.8 Hz, 2H), 4.00 (t, *J* = 6.4 Hz, 2H), 3.95 (t, *J* = 6.2 Hz, 2H), 3.87 (s, 3H), 3.73 - 3.58 (m, 4H), 2.84 - 2.76 (m, 2H), 2.09 - 1.96 (m, 1H), 1.92 - 1.85 (m, 4H), 1.68 - 1.62 (m, 2H), 1.31 - 1.17 (m, 3H), 1.08 (d, *J* = 2.5 Hz, 9H), 1.06 (d, *J* = 2.5 Hz, 9H), 0.85 (s, 9H), 0.84 (s, 9H), 0.06 (s, 6H), 0.07 (s, 6H).

### (v) (2R,11aS)-2-((tert-butyldimethylsilyl)oxy)-8-((5-(((2R,11aS)-2-((tert-butyldimethylsilyl)oxy)-5,11-dioxo-7-((triisopropylsilyl)oxy)-10-((2-(trimethylsilyl)ethoxy)methyl)-2,3,5,10,11,11a-hexahydro-1H-benzo[e]pyrrolo[1,2-a][1,4]diazepin-8-yl)oxy)pentyl)oxy)-7-methoxy-10-((2-(trimethylsilyl)ethoxy)methyl)-2,3-dihydro-1H-benzo[e]pyrrolo[1,2-a][1,4]diazepine-5,11(10H,11aH)-dione (113)

112 (15.4 g, 15.74 mmol) was dissolved in dry tetrahydrofuran (250 mL) and cooled to -30°C (dry ice / acetone). n-Butyllithium (29 mL, 46.41 mmol, 3 eq) was then added dropwise and the reaction mixture was stirred for 1 hour at -30°C. 2-(Trimethylsilyl)ethoxymethyl chloride (8.2 mL, 46.41 mmol, 3 eq) was then added dropwise and the cold bath was removed. The reaction mixture was stirred at ambient temperature for 12 hours and the solvent was removed by rotary evaporation. The residue was dissolved in ethyl acetate and washed with water and brine, dried over magnesium sulphate; filtered and excess solvent was removed by rotary evaporation under reduced pressure to give the desired compound as yellow oil used as crude for the next reaction. ¹H NMR (400 MHz, CDCl₃) δ 7.34 (s, 1H), 7.33 (s, 1H), 7.20 (s, 1H), 7.15 (s, 1H), 5.49 (dd, *J* = 10.0, 1.8 Hz, 2H), 4.64 (dd, *J* = 9.9, 7.5 Hz, 2H), 4.56 (dt, *J* = 8.9, 5.7 Hz, 2H), 4.21 (dt, *J* = 8.6, 4.4 Hz, 2H), 4.09 - 3.93 (m, 4H), 3.91 (s, 3H), 3.82 - 3.61 (m, 6H), 3.61 - 3.50 (m, 2H), 2.90 - 2.76 (m, 2H), 2.03 - 1.97 (m, 2H), 1.97 - 1.86 (m, 4H), 1.75 - 1.64 (m, 2H), 1.34 - 1.19 (m, 3H), 1.10 (d, *J* = 2.7 Hz, 9H), 1.08 (d, *J* = 2.7 Hz, 9H), 0.96 (ddd, *J* = 9.1, 6.9, 2.0 Hz, 4H), 0.86 (d, *J* = 2.9 Hz, 9H), 0.85 (d, *J* = 2.9 Hz, 9H), 0.09 (s, 6H), 0.07 (s, 6H), 0.01 (d, *J* = 3.2 Hz, 18H).

### (c) (S)-7-hydroxy-8-((5-(((S)-7-methoxy-2-methyl-5,11-dioxo-10-((2-(trimethylsilyl)ethoxy)methyl)-5,10,11,11a-tetrahydro-1H-benzo[e]pyrrolo[1,2-a][1,4]diazepin-8-yl)oxy)pentyl)oxy)-2-methyl-10-((2-(trimethylsilyl)ethoxy)methyl)-1H-benzo[e]pyrrolo[1,2-a][1,4]diazepine-5,11(10H,11aH)-dione (118)

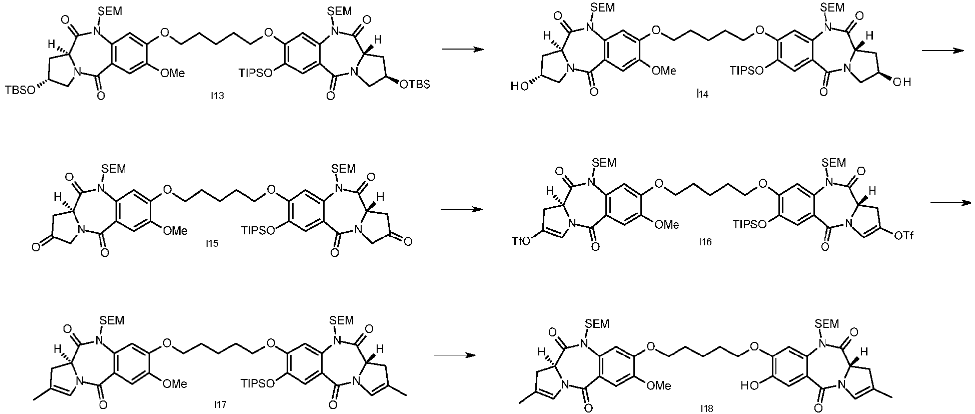

### (i) (2R,11aS)-2-hydroxy-8-((5-(((2R,11aS)-2-hydroxy-5,11-dioxo-7-((triisopropylsilyl)oxy)-10-((2-(trimethylsilyl)ethoxy)methyl)-2,3,5,10,11,11a-hexahydro-1H-benzo[e]pyrrolo[1,2-a][1,4]diazepin-8-yl)oxy)pentyl)oxy)-7-methoxy-10-((2-(trimethylsilyl)ethoxy)methyl)-2,3-dihydro-1H-benzo[e]pyrrolo[1,2-a][1,4]diazepine-5,11(10H,11aH)-dione (114)

Crude 113 (15.74 mmol) was dissolved in dry tetrahydrofuran (40 mL) and a solution of 1% *v*/*v* conc. HCI in methanol (120 mL). The reaction mixture was stirred 2 hours at ambient temperature; diluted with ethyl acetate and washed with water (two times), saturated aqueous sodium bicarbonate and brine, dried over magnesium sulphate; filtered and excess solvent was removed by rotary evaporation under reduced pressure to give the desired compound as yellow oil used as crude for the next reaction. LC/MS (Method 3) 2.08 min, (ES+) *m*/*z* (relative intensity) 1027.40 [M + H]⁺, ¹H NMR (400 MHz, CDCl₃) δ 7.32 (s, 1H), 7.32 (s, 1H), 7.20 (s, 1H), 7.14 (s, 1H), 5.49 (dd, *J* = 10.0, 3.2 Hz, 2H), 4.69 - 4.57 (m, 3H), 4.33 - 4.24 (m, 2H), 4.16 - 3.93 (m, 4H), 3.88 (s, 3H), 3.89 - 3.55 (m, 6H), 3.00 - 2.88 (m, 2H), 2.53 (br, 1H), 2.17 - 2.05 (m, 2H), 2.00 - 1.86 (m, 4H), 1.78 - 1.63 (m, 3H), 1.46 - 1.39 (m, 2H), 1.36 - 1.19 (m, 4H), 1.09 (s, 9H), 1.07 (s, 9H), 1.01 - 0.92 (m, 4H), 0.02 (s, 18H).

### (ii) (S)-7-methoxy-10-((2-(trimethylsilyl)ethoxy)methyl)-8-((5-(((S)-2,5,11-trioxo-7-((triisopropylsilyl)oxy)-10-((2-(trimethylsilyl)ethoxy)methyl)-2,3,5,10,11,11a-hexahydro-1H-benzo[e]pyrrolo[1,2-a][1,4]diazepin-8-yl)oxy)pentyl)oxy)-1H-benzo[e]pyrrolo[1,2-a][1,4]diazepine-2,5,11(3H,10H,11aH)-trione (115)

TCCA (1.62 g, 7.00 mmol, 1.2 eq) was added to a solution of crude 114 (5.84 mmol), TEMPO (90 mg, 0.58 mmol, 0.1 eq) and sodium acetate (1.14 g, 14.00 mmol, 2.4 eq) in dichloromethane (120 mL) at -10°C (acetone / ice). The reaction was stirred for 30 min and filtered through celite. The reaction mixture was then quenched with saturated aqueous sodium bicarbonate. The organic phase washed with sodium thiosulfate and brine, dried over magnesium sulphate; filtered and excess solvent was removed by rotary evaporation under reduced pressure. The resulting residue was subjected to flash column chromatography (silica gel; 20% to 70% ethyl acetate in hexane). Pure fractions were collected and combined and excess eluent was removed by rotary evaporation under reduced pressure to give the product (3.21 g, 54% over 3 steps). LC/MS (Method 3) 2.17 min, (ES+) *m*/*z* (relative intensity) 1023.75 [M + H]⁺, ¹H NMR (400 MHz, CDCl₃) δ 7.32 (s, 2H), 7.23 (s, 1H), 7.18 (s, 1H), 5.53 (dd, *J* = 10.0, 1.6 Hz, 2H), 4.70 (t, *J* = 9.6 Hz, 2H), 4.66 - 4.58 (m, 2H), 4.22 (d, *J* = 20.1 Hz, 2H), 4.13 - 3.95 (m, 4H), 3.91 (s, 3H), 3.89 - 3.74 (m, 4H), 3.72 - 3.62 (m, 2H), 3.56 (ddd, *J* = 19.2, 5.8, 3.1 Hz, 2H), 2.78 (dd, *J* = 19.3, 9.9 Hz, 2H), 1.95 (dd, *J* = 14.6, 7.3 Hz, 4H), 1.76 - 1.63 (m, 2H), 1.33 - 1.23 (m, 3H), 1.10 (s, 9H), 1.09 (s, 9H), 1.02 - 0.90 (m, 4H), 0.02 (s, 18H).

### (iii) (S)-8-((5-(((S)-5,11-dioxo-2-(((trifluoromethyl)sulfonyl)oxy)-7-((triisopropylsilyl)oxy)-10-((2-(trimethylsilyl)ethoxy)methyl)-5,10,11,11a-tetrahydro-1H-benzo[e]pyrrolo[1,2-a][1,4]diazepin-8-yl)oxy)pentyl)oxy)-7-methoxy-5,11-dioxo-10-((2-(trimethylsilyl)ethoxy)methyl)-5,10,11,11a-tetrahydro-1H-benzo[e]pyrrolo[1,2-a][1,4]diazepin-2-yl trifluoromethanesulfonate (116)

Anhydrous 2,6-lutidine (2.12 mL, 18.17 mmol, 6.2 eq) was injected in one portion to a solution of 115 (3 g, 2.93 mmol) in dry dichloromethane (40 mL) at -50°C (acetone / dry ice). Triflic anhydride (2.12 mL, 18.17 mmol, 6 eq) was then added slowly whilst monitoring the temperature. After 20 minutescold water was added to the still cold reaction mixture and the organic layer was separated and washed with cold saturated sodium bicarbonate, brine, dried over magnesium sulphate; filtered and excess solvent was removed by rotary evaporation under reduced pressure. The resulting residue was subjected to flash column chromatography (silica gel; 5% to 20% ethyl acetate in hexane). Pure fractions were collected and combined and excess eluent was removed by rotary evaporation under reduced pressure to give the product (3.1 g, 82%). LC/MS (Method 3) 2.37 min, no ionisation, ¹H NMR (400 MHz, CDCl₃) δ 7.32 (s, 2H), 7.23 (s, 1H), 7.19 (s, 1H), 7.14 (s, 1H), 7.11 (s, 1H), 5.54 (d, *J* = 10.0 Hz, 2H), 4.74 - 4.66 (m, 2H), 4.63 (d, *J* = 11.0 Hz, 2H), 4.09 - 3.96 (m, 4H), 3.94 - 3.87 (m, 2H), 3.90 (s, 3H), 3.82 - 3.76 (m, 2H), 3.69 - 3.65 (m, 2H), 3.22 - 3.08 (m, 2H), 1.99 - 1.90 (m, 4H), 1.75 - 1.64 (m, 2H), 1.33 - 1.21 (m, 3H), 1.11 (d, *J* = 1.1 Hz, 9H), 1.09 (d, *J* = 1.1 Hz, 9H), 1.01 - 0.91 (m, 4H), 0.02 (s, 18H).

### (iv) (S)-7-methoxy-2-methyl-8-((5-(((S)-2-methyl-5,11-dioxo-7-((triisopropylsilyl)oxy)-10-((2-(trimethylsilyl)ethoxy)methyl)-5,10,11,11a-tetrahydro-1H-benzo[e]pyrrolo[1,2-a][1,4jdiazepin-8-yl)oxy)pentyl)oxy)-10-((2-(trimethylsilyl)ethoxy)methyl)-1H-benzo[e]pyrrolo[1,2-a][1,4]diazepine-5,11(10H,11aH)-dione (117)

Triphenylarsine (588 mg, 1.92 mmol, 0.8 eq) was added to a mixture of triflate 116 (3.1 g, 2.4 mmol, 1eq), methylboronic acid (1.0 g, 16.8 mmol, 7 eq), silver oxide (4.45 g, 19.2 mmol, 8 eq) and potassium phosphate tribasic (6.1 g, 28.8 mmol, 12 eq) in dry dioxane (40 mL) under an argon atmosphere. The reaction was flushed with argon 3 times and bis(benzonitrile)palladium(II) chloride (184 mg, 0.48 mmol, 0.2 eq) was added. The reaction was flushed with argon 3 more times before being stirred at 70 °C. After 1 hour the reaction was observed to be complete by TLC and filtered through a pad celite. The solvent was removed by rotary evaporation under reduced pressure. The resulting residue was subjected to column flash chromatography (silica gel; 20% to 50% ethyl acetate / hexane). Pure fractions were collected and combined, and excess eluent was removed by rotary evaporation under reduced pressure afforded the product (1.1 g, 36 %). LC/MS (Method 3) 2.34 min, no ionisation, ¹H NMR (400 MHz, CDCl₃) δ 7.35 (s, 2H), 7.20 (s, 1H), 7.16 (s, 1H), 6.68 (d, *J* = 1.3 Hz, 1H), 6.64 (d, *J* = 1.4 Hz, 1H), 5.53 (s, 1H), 5.51 (s, 1H), 4.67 (t, *J* = 10.1 Hz, 2H), 4.45 (dt, *J* = 10.5, 3.2 Hz, 2H), 4.08 - 3.94 (m, 4H), 3.90 (s, 3H), 3.77 (dd, *J* = 8.9, 7.5 Hz, 2H), 3.68 (dt, *J* = 10.0, 5.2 Hz, 2H), 3.43 (d, *J* = 16.5 Hz, 2H), 2.78 (d, *J* = 10.4 Hz, 2H), 2.00 - 1.86 (m, 4H), 1.83 (s, 3H), 1.82 (s, 3H), 1.72 - 1.68 (m, 2H), 1.30 - 1.25 (m, 3H), 1.09 (s, 9H), 1.06 (s, 9H), 0.99 - 0.94 (m, 4H), 0.02 (s, 18H).

### (v) (S)-7-hydroxy-8-((5-(((S)-7-methoxy-2-methyl-5,11-dioxo-10-((2-(trimethylsilyl)ethoxy)methyl)-5,10,11,11a-tetrahydro-1H-benzo[e]pyrrolo[1,2-a][1,4]diazepin-8-yl)oxy)pentyl)oxy)-2-methyl-10-((2-(trimethylsilyl)ethoxy)methyl)-1H-benzo[e]pyrrolo[1,2-a][1,4]diazepine-5,11(10H,11aH)-dione (118)

Lithium acetate (110 mg, 1.08 mmol, 1 eq) was added to a solution of compound 117 (1.1 g, 1.08 mmol) in wet dimethylformamide (20 mL, 50:1 DMF/water). The reaction was stirred for 12 hours at ambient temperature and diluted with ethyl acetate and washed with citric acid (pH ∼ 3), brine, dried over magnesium sulphate; filtered and excess solvent was removed by rotary evaporation under reduced pressure to provide the product (1.03 g, quantitative). LC/MS (Method 3) 1.98 min, (ES+) *m*/*z* (relative intensity) 863.35 [M + H]⁺, ¹H NMR (400 MHz, CDCl₃) δ 7.44 (s, 1H), 7.35 (s, 1H), 7.20 (s, 1H), 7.19 (s, 1H), 6.70 - 6.62 (m, 2H), 6.21 (br, 1H), 5.51 (dd, *J* = 10.0, 5.3 Hz, 2H), 4.66 (dd, *J* = 11.3, 10.1 Hz, 2H), 4.46 (dd, *J* = 10.4, 3.2 Hz, 2H), 4.18 - 3.99 (m, 4H), 3.90 (s, 3H), 3.78 (td, *J* = 9.7, 6.9 Hz, 2H), 3.67 (td, *J* = 9.7, 6.8 Hz, 2H), 3.49 - 3.35 (m, 2H), 2.81 - 2.68 (m, 2H), 2.00 - 1.89 (m, 4H), 1.82 (s, 3H), 1.81 (s, 3H), 1.75 - 1.63 (m, 2H), 0.97 (ddd, *J* = 9.9, 6.6, 3.3 Hz, 4H), 0.01 (s, 18H).

### (d) (S)-7-hydroxy-2-methylene-10-((2-(trimethylsilyl)ethoxy)methyl)-2,3-dihydro-1H-benzo[e]pyrrolo[1,2-a][1,4]diazepine-5,11(10H,11aH)-dione (123)

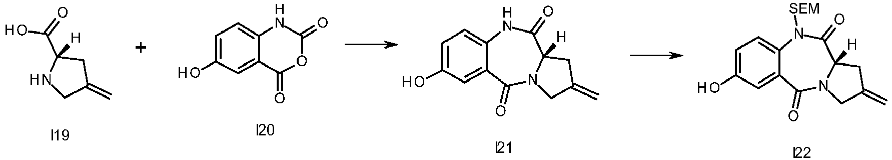

### (i) (S)-7-hydroxy-2-methylene-2,3-dihydro-1H-benzo[e]pyrrolo[1,2-a][1,4]diazepine-5,11(10H,11aH)-dione (121)

Phenol isatoic anhydride I20 (1.59 g, 8.88 mmol, 1 eq), hydrochloride 119 (1.60 g, 9.7 mmol, 1.1 eq), diisopropylethylamine (2.06 mL, 11.8 mmol, 1.3 eq) were suspended in DMSO (8mL) and heated at 120°C for 15 minutes. LC/MS monitoring showed total consumption of starting material. The reaction mixture was diluted with water and ice and the resulting precipitate was collected by filtration (1g). The product was recrystallized in acetonitrile to yield the desired product (600 mg). LC/MS (1.97 min (ES-) *m*/*z* (relative intensity) 242.7 ([*M* - H]^{-.}, 100)); ¹H NMR (400 MHz, DMSO) δ 10.24 (s, 1H), 9.64 (s, 1H), 7.15 (d, *J* = 2.5 Hz, 1H), 7.01 - 6.90 (m, 2H), 5.08 (s, 2H), 4.31 - 4.16 (m, 2H), 4.03 (dd, *J* = 16.2, 1.3 Hz, 1H), 3.20 (d, *J* = 16.1 Hz, 1H), 2.77 (dd, *J* = 15.9, 9.4 Hz, 1H).

### (ii) (S)-7-hydroxy-2-methylene-10-((2-(trimethylsilyl)ethoxy)methyl)-2,3-dihydro-1H-benzo[e]pyrrolo[1,2-a][1,4]diazepine-5,11(10H,11aH)-dione (122)

n-Buli (2.51 mL, 4.02 mmol, 2.2 eq) was added to a suspension of phenol dilactam 121(447 mg, 1.83 mmol, 1eq) in anhydrous THF at -78°C. SEM chloride (670 mg, 711µL, 4.02 mmol, 2.2 eq) was injected slowly under vigorous stirring. The mixture was allowed to return to room temperature and was treated with dilute HCI in methanol (3 drops of concentrated hydrochloric acid / 50 mL methanol). The mixture was heated at 40°C to accelerate the hydrolysis of phenolic SEM ethers and the reaction was monitored by LC/MS and TLC (ethyl acetate). The mixture was partitioned between ethyl acetate and water, followed by a wash with brine. Flash column chromatography (80/20 Ethyl acetate / hexane) gave the product in 73% yield (502 mg). LC/MS (3.10 min (ES-) *m*/*z* (relative intensity) 372.9 ([M - H]^{-.}, 100)).

### (e) (S)-8-methoxy-2-(6-methoxynaphthalen-2-yl)-7-((triisopropylsilyl)oxy)-10-((2-(trimethylsilyl)ethoxy)methyl)-1H-benzo[e]pyrrolo[1,2-a][1,4]diazepine-5,11(10H,11aH)-dione (133)

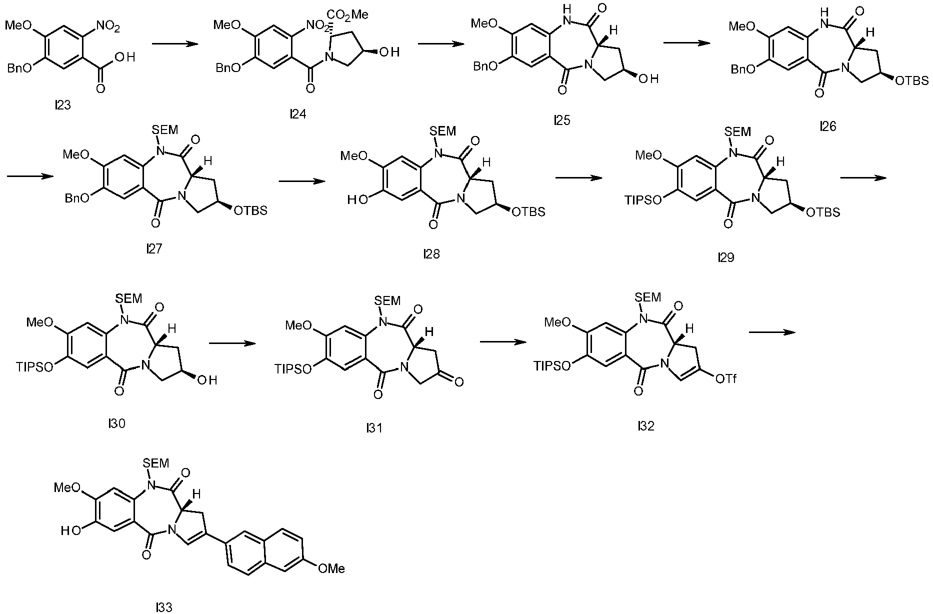

### (i) (2S,4R)-methyl 1-(5-(benzyloxy)-4-methoxy-2-nitrobenzoyl)-4-hydroxypyrrolidine-2-carboxylate (124)

Oxalyl chloride (1.19 mL, 1.73 g, 13.6 mmol) was added to a stirred suspension of the nitrobenzoic acid I23 (2.77 g, 9.1 mmol) and DMF (3 drops) in anhydrous DCM (50 mL). Following initial effervescence the reaction suspension became a solution and the mixture was allowed to stir at room temperature for 16 hours. Conversion to the acid chloride was confirmed by treating a sample of the reaction mixture with MeOH and the resulting methyl ester was observed by LC/MS (3.27 min). The majority of solvent was removed by evaporation under reduced pressure; the resulting concentrated solution was re-dissolved in a minimum amount of dry DCM and triturated with diethyl ether. The resulting yellow precipitate was collected by filtration, washed with cold diethyl ether and dried for 1 hour in a vacuum oven at 40 °C. The solid acid chloride was added portionwise over a period of 5 min to a stirred suspension of (2S,4R)-methyl-4-hydroxypyrrolidine-2-carboxylate hydrochloride (1.86 g, 10.2 mmol, 1.15 eq) and TEA (3.10 mL, 2.25 g, 22.2 mmol, 2.5 eq) in DCM (40mL) at -40 °C (dry ice/CH₃CN). Immediately, the reaction was complete as judged by LC/MS (2.70 min (ES+) *m*/*z* (relative intensity) 431.01 ([M + H]^{+.}, 100)) and by TLC (ethyl acetate). The mixture was diluted with DCM (20 mL) and washed with 1N HCI (30 mL), saturated NaHCO₃ (30 mL), brine (40 mL), dried (MgSO₄), filtered and the solvent evaporated *in vacuo* to give the pure product as an orange solid (3.7 g, 94%). LC/MS (2.70 min (ES+) *m*/*z* (relative intensity) 431.01 ([*M* + H]^{+.}, 100)); ¹H NMR (400 MHz, CDCl₃) δ 7.70 (d, *J* = 1.8 Hz, 1H), 7.48 - 7.30 (m, 5H), 6.89 (s, 1H), 5.29 (d, *J* = 5.5 Hz, 1H), 5.25 (d, *J* = 7.2 Hz, 1H), 4.84 (t, *J* = 8.1 Hz, 1H), 4.40 (s, 1H), 3.99 (d, *J* = 10.0 Hz, 3H), 3.82 (s, 3H), 3.46 (s, 1H), 3.33 (dd, *J* = 11.3, 4.2 Hz, 1H), 3.12 - 2.96 (m, 1H), 2.51 - 2.29 (m, 1H), 2.22 - 2.07 (m, 1H).

### (ii) (2R,11aS)-7-(benzyloxy)-2-hydroxy-8-methoxy-2,3-dihydro-1H-benzo[e]pyrrolo[1,2-a][1,4]diazepine-5,11(10H,11aH)-dione (125)

Zinc dust (10 g, 153 mmol, 19 eq (excess)) was added to a solution of nitro-ester 124(3.5 g, 8.1 mmol) in 5% formic acid in methanol (70 mL). An exotherm was observed, followed by reduction of the nitro to the aniline. LC/MS (2.47 min (ES+) *m*/*z* (relative intensity) 401.03 ([M + H]^{+.}, 100)). The resulting suspension was filtered through celite and washed with methanol (30 mL) to give a clear filtrate. The solvent and residual formic acid were removed by evaporation. The residue was redisolved in methanol and hydrazine hydrate (380 µL, 12.2 mmol) was added to the solution and the reaction mixture was heated at 60°C until completion was observed. LC/MS (2.45 min (ES+) *m*/*z* (relative intensity) 369.01 ([*M* + H]^{+.}, 100)). The mixture was allowed to cool down to room temperature, the solvent removed under vacuum, the residue triturated with diethyl ether and the precipitate retrieved by filtration and dried in a vacuum desiccator to provide the desired product as a white powder (2.77 g, 92%). LC/MS (2.45 min (ES+) *m*/*z* (relative intensity) 369.01 ([*M* + H]^{+.}, 100)). ¹H NMR (400 MHz, DMSO) δ 7.95 (s, 1H), 7.52 - 7.34 (m, 6H), 6.77 (s, 1H), 5.14 (s, 2H), 4.33 (dd, *J* = 8.9, 4.4 Hz, 1H), 4.19 (dd, *J* = 8.0, 6.0 Hz, 1H), 3.83 (s, 3H), 3.65 (dd, *J* = 11.8, 3.3 Hz, 1H), 3.50 - 3.42 (m, 1H), 2.70 - 2.60 (m, 1H), 2.03 - 1.91 (m, 1H).

### (iii) (2R,11aS)-7-(benzyloxy)-2-((tert-butyldimethylsilyl)oxy)-8-methoxy-2,3-dihydro-1H-benzo[e]pyrrolo[1,2-a][1,4]diazepine-5,11(10H,11aH)-dione (126)

TBSCI (5.32g, 35.3 mmol) and imidazole (5.76 g, 84.6 mmol) were added to a solution of the dilactam 125 (2.6 g, 7.1 mmol) in anhydrous DMF (30 mL) at 0°C (ice/acetone). The mixture was allowed to stir under a nitrogen atmosphere for 3 hours after which time the reaction was deemed complete as judged by LC/MS (3.60 min (ES+) *m*/*z* (relative intensity) 483.09 ([M + H]^{+.}, 100)). The reaction mixture was poured onto ice (∼ 200 mL) and allowed to warm to room temperature with stirring. The resulting white precipitate was collected by vacuum filtration, washed with H₂O, redisolved in ethyl acetate, and dried over magnesium sulphate followed by rotoevaporation under vacuum. The residue was purified by flash column chromatography (50 /50 ethyl acetate / hexane) to provide the desired compound (2.42 g, 71%). LC/MS (3.60 min (ES+) *m*/*z* (relative intensity) 483.09 ([*M* + H]^{+.}, 100)). ¹H NMR (400 MHz, CDCl₃) δ 8.26 (s, 1H), 7.53 (s, 1H), 7.49 - 7.28 (m, 5H), 6.47 (s, 1H), 5.16 (q, *J* = 11.9 Hz, 2H), 4.51 (p, *J* = 5.5 Hz, 1H), 4.19 (dd, *J* = 8.2, 4.3 Hz, 1H), 3.89 (s, 3H), 3.68 (ddd, *J* = 27.8, 11.9, 5.4 Hz, 2H), 2.82 (dt, *J* = 12.7, 4.9 Hz, 1H), 2.17 - 1.94 (m, 1H), 0.92 - 0.81 (m, 9H), 0.08 (d, *J* = 3.0 Hz, 6H).

### (iv) (2R,11aS)-7-(benzyloxy)-2-((tert-butyldimethylsilyl)oxy)-8-methoxy-10-((2-(trimethylsilyl)ethoxy)methyl)-2,3-dihydro-1H-benzo[e]pyrrolo[1,2-a][1,4]diazepine-5,11(10H,11aH)-dione (127)

A solution of n-BuLi (4.7 mL of a 1.6 M solution in hexane, 7.52 mmol) was added dropwise to a stirred suspension of the dilactam I26 (2.42 g, 5.01 mmol) in anhydrous THF (30 mL) at -60 °C under a nitrogen atmosphere. The reaction mixture was allowed to stir at this temperature for 1 hour at which point neat SEMCI (1.33 mL, 1.25 g, 7.51 mmol) was added dropwise. The reaction mixture was allowed to slowly warm to room temperature and was stirred for 2 hours under a nitrogen atmosphere. The reaction was deemed complete as judged by TLC (EtOAc) and LC/MS (4.30 min (ES+) *m*/*z* (relative intensity) 613.16 ([*M* + H]^{+.}, 100)). The THF was removed by evaporation *in vacuo* and the resulting residue dissolved in EtOAc (100 mL), washed with H₂O (100 mL), brine (30 mL), dried (MgSO₄) filtered and evaporated *in vacuo* to provide the crude N10-SEM-protected dilactam (2.23 g, 72%). Product carried through to next step without purification. LC/MS (4.30 min (ES+) *m*/*z* (relative intensity) 613.16 ([M + H]^{+.}, 100)). ¹H NMR (400 MHz, CDCl₃) δ 7.49 - 7.28 (m, 6H), 7.24 (s, 1H), 5.55 - 5.49 (m, 1H), 5.18 (q, *J* = 11.9 Hz, 2H), 4.68 - 4.61 (m, 1H), 4.56 (p, *J =* 5.8 Hz, 1H), 4.22 (dd, *J* = 8.2, 3.8 Hz, 1H), 3.89 (s, 3H), 3.83 - 3.58 (m, 4H), 3.58 - 3.50 (m, 1H), 2.84 (ddd, *J* = 12.8, 5.5, 4.0 Hz, 1H), 2.05 - 1.96 (m, 1H), 1.04 - 0.89 (m, 6H), 0.89 - 0.84 (m, 9H), 0.05 - 0.00 (m, 9H).

### (v) (2R,11aS)-2-((tert-butyldimethylsilyl)oxy)-7-hydroxy-8-methoxy-10-((2-(trimethylsilyl)ethoxy)methyl)-2,3-dihydro-1H-benzo[e]pyrrolo[1,2-a][1,4]diazepine-5,11(10H,11aH)-dione (128)

Benzyl ether 127 (2.23g, 3.63 mmol) was hydrogenated in ethyl acetate (40 mL) at 40 PSI overnight in Parr hydrogenator in the presence of 10% Pd/C (220 mg, 10% w/w) after which completion was observed by LC/MS. The solids were removed by filtration over celite and the resulting filtrate was concentrated under vacuum. The resulting residue was found to be of satisfactory purity and was carried through to the next steps without further purification. (1.90 g, 100%). LC/MS (3.87 min (ES+) *m*/*z* (relative intensity) 522.81 ([*M* + H]^{+.}, 100)). ¹H NMR (400 MHz, CDCl₃) δ 7.46 (s, 1H), 7.22 (s, 1H), 6.11 (s, 1H), 5.51 (d, *J* = 9.9 Hz, 1H), 4.66 (d, *J* = 8.1 Hz, 1H), 4.57 (p, *J* = 5.8 Hz, 1H), 4.23 (dd, *J* = 8.2, 3.8 Hz, 1H), 3.92 (s, 3H), 3.84 - 3.50 (m, 4H), 2.92 - 2.74 (m, 1H), 2.10 - 1.90 (m, 1H), 1.06 - 0.89 (m, 2H), 0.89 - 0.82 (m, 9H), 0.09 (d, *J* = 0.9 Hz, 6H), 0.03 (d, *J* = 3.2 Hz, 9H).

### (vi) (2R,11aS)-2-((tert-butyldimethylsilyl)oxy)-8-methoxy-7-((triisopropylsilyl)oxy)-10-((2-(trimethylsilyl)ethoxy)methyl)-2,3-dihydro-1H-benzo[e]pyrrolo[1,2-a][1,4]diazepine-5,11(10H,11aH)-dione (129)

Neat triisopropylsilylchloride (1.55 mL, 1.39 g, 7.24 mmol) was added to a mixture of imidazole (743 mg, 10.9 mmol) and the previously prepared phenol I28 (1.90 g, 3.64 mmol) (ground together). The mixture was heated until the phenol and imidazole melted and went into solution (100 °C). The reaction mixture was allowed to stir for 5 mins and was then allowed to cool, whereupon a solid was observed to form at the bottom of the flask. The reaction mixture was diluted with 5% EtOAc/ hexanes and loaded directly onto silica gel and the column was eluted with 5% EtOAc/ hexanes, followed by 10% EtOAc/hexanes. Excess eluent was removed by rotary evaporation under reduced pressure, followed by drying under high vacuum to afford as an oil (2.5 g, 100 %). LC/MS (4.50 min (ES+) *m*/*z* (relative intensity) 679.49 ([*M* + H]^{+.}, 100)). ¹H NMR (400 MHz, CDCl₃) δ 7.36 (s, 1H), 7.18 (s, 1H), 5.51 (d, *J* = 9.9 Hz, 1H), 4.63 (d, *J* = 9.9 Hz, 1H), 4.55 (p, *J* = 5.6 Hz, 1H), 4.21 (dd, *J* = 8.1, 4.1 Hz, 1H), 3.83 (s, 3H), 3.72 - 3.51 (m, 4H), 2.91 - 2.75 (m, 1H), 2.10 - 1.94 (m, 1H), 1.33 - 1.21 (m, 3H), 1.09 (dd, *J* = 7.4, 2.0 Hz, 18H), 1.00 - 0.89 (m, 2H), 0.89 - 0.81 (m, 9H), 0.08 (s, 6H), 0.03 (s, 9H).

### (vii) (2R,11aS)-2-hydroxy-8-methoxy-7-((triisopropylsilyl)oxy)-10-((2-(trimethylsilyl)ethoxy)methyl)-2,3-dihydro-1H-benzo[e]pyrrolo[1,2-a][1,4]diazepine-5,11(10H,11aH)-dione (130)

The secondary TBS ether 129 (2.5 g, 3.68 mmol) was dissolved in a mixture of 1% v/v conc aq HCI in methanol (20 mL) and THF (5 mL). After stirring for 6h at 20°C, analysis of the reaction mixture by TLC (50:50 v/v EtOAc/Hexane) revealed that the reaction was almost complete. The reaction mixture was dissolved in EtOAc (100 mL) and washed with water (2 x 100 mL). The combined organic layers were washed with brine (60 mL), dried (MgSO₄), filtered and evaporated under reduced pressure to provide the crude product which was taken directly to the next step as a crude. (2.08 g, 3.68 mmol, 100%). LC/MS 3.77 min (ES+) *m*/*z* (relative intensity) 565.29 ([*M* + H]^{+.}, 100).

### (viii) (S)-8-methoxy-7-((triisopropylsilyl)oxy)-10-((2-(trimethylsilyl)ethoxy)methyl)-1H-benzo[e]pyrrolo[1,2-a][1,4]diazepine-2,5,11(3H,10H,11aH)-trione (I31)

TCCA (600 mg, 2.58 mmol, 0.7 eq) was added to a stirred solution of I30 (2.08 g, 3.68 mmol, 1 eq) and TEMPO (57 mg, 0.36 mmol, 0.1 eq) in dry dichloromethane (50 mL) at -10 °C (ice/acetone bath). The reaction mixture was vigorously stirred for 20 minutes, at which point TLC (25/75 ethyl acetate/hexane) revealed complete consumption of the starting material. The reaction mixture was filtered through celite and the filtrate washed with aqueous saturated sodium bicarbonate (50 mL), sodium thiosulphate (1.5 g in 50 mL), brine (100 mL) and dried over magnesium sulphate. Rotary evaporation under reduced pressure followed by flash column chromatography (gradient elution: 90:10 v/v Hexane/EtOAc to 80:20 v/v Hexane/EtOAc) afforded the desired product (1.40 g, 67%). LC/MS 3.87 min (ES+) *m*/*z* (relative intensity) 563.05 ([*M* + H]^{+.}, 100); ¹H NMR (400 MHz, CDCl₃) δ 7.35 (s, 1H), 7.22 (s, 1H), 5.55 (d, *J* = 9.9 Hz, 1H), 4.69 (d, *J* = 9.9 Hz, 1H), 4.63 (dd, *J* = 9.9, 3.1 Hz, 1H), 4.23 (d, *J* = 20.1 Hz, 1H), 3.91 - 3.83 (m, 4H), 3.79 (td, *J* = 9.7, 6.7 Hz, 1H), 3.68 (td, *J* = 9.7, 6.7 Hz, 1H), 3.56 (dd, *J* = 19.2, 3.1 Hz, 1H), 2.78 (dd, *J* = 18.2, 9.9 Hz, 1H), 1.34 - 1.21 (m, 3H), 1.10 (d, *J* = 7.3 Hz, 18H), 0.98 (ddd, *J* = 9.6, 6.5, 4.1 Hz, 2H), 0.05 - 0.01 (m, 9H).

### (ix) (S)-8-methoxy-5,11-dioxo-7-((triisopropylsilyl)oxy)-10-((2-(trimethylsilyl)ethoxy)methyl)-5,10,11,11a-tetrahydro-1H-benzo[e]pyrrolo[1,2-a][1,4]diazepin-2-yl trifluoromethanesulfonate (132)

Triflic anhydride (1.24 mL, 2.08 g, 7.37 mmol, 3 eq) was injected (temperature controlled) to a vigorously stirred suspension of ketone 131 (1.40 g, 2.49 mmol, 1 eq) in dry dichloromethane (50 mL) in the presence of 2,6-lutidine (1.16 mL, 1.06 g, 9.96 mmol, 4 eq, dried over sieves) at -50°C (acetone/dry ice bath). The reaction mixture was allowed to stir for 1.5 hours when LC/MS, following a mini work-up (water/dichloromethane), revealed the reaction to be complete. Water was added to the still cold reaction mixture and the organic layer was separated and washed with saturated sodium bicarbonate, brine and magnesium sulphate. The organic phase was filtered and excess solvent was removed by rotary evaporation under reduced pressure. The residue was subjected to column flash chromatography (gradient elution: 95:5 v/v Hexane/EtOAc to 80:20 v/v Hexane/EtOAc) afforded the desired product (1.34 g, 77%). LC/MS (Method 2) 4.08 min (ES+) *m*/*z* (relative intensity) 716.82 ([*M* + Na]^{+.}, 100). ¹H NMR (400 MHz, CDCl₃) δ 7.34 (s, 1H), 7.22 (s, 1H), 7.11 (t, *J* = 2.0 Hz, 1H), 5.55 (d, *J* = 9.9 Hz, 1H), 4.69 (d, *J* = 9.9 Hz, 1H), 4.63 (dd, *J* = 11.0, 3.7 Hz, 1H), 3.91 (ddd, *J* = 16.3, 3.7, 1.8 Hz, 1H), 3.86 (s, 3H), 3.80 (td, *J* = 9.5, 7.1 Hz, 1H), 3.69 (td, *J* = 9.5, 7.1 Hz, 1H), 3.16 (ddd, *J* = 16.3, 11.1, 2.4 Hz, 1H), 1.34 - 1.20 (m, 3H), 1.10 (d, *J* = 7.2 Hz, 18H), 1.02 - 0.94 (m, 2H), 0.05 - 0.01 (m, 9H).

### (x) (S)-8-methoxy-2-(6-methoxynaphthalen-2-yl)-7-((triisopropylsilyl)oxy)-10-((2-(trimethylsilyl)ethoxy)methyl)-1H-benzo[e]pyrrolo[1,2-a][1,4]diazepine-5,11(10H,11aH)-dione (133)

Pd(PPh₃)₄ (112 mg, 95.2 µmol, 0.05 eq) was added to a stirred mixture of enol triflate I32 (1.35 g, 1.94 mmol), 6-methoxy-2-naphthylboronic acid (1.02 g, 5.05 mmol, 2.6 eq), K₃PO₄ (1.07 g, 5.04 mmol), dioxane (15 mL). The reaction mixture was allowed to stir under a argon atmosphere for 2 hours at 35°C after which time the complete consumption of starting material was observed by TLC (EtOAc/Hexane) and LC/MS (4.22 min (ES+) *m*/*z* (relative intensity) 702.88 ([*M* + H]^{+.}, 100)). The reaction mixture was diluted with EtOAc (100 mL) and washed with brine (200 mL), dried (MgSO₄), filtered and evaporated under reduced pressure to provide the crude product. Purification by flash chromatography (gradient elution: 90:10 v/v Hexane/EtOAc to 70:30 v/v Hexane/EtOAc) afforded the TIPS protected C2-aryl which was immediately redissolved in wet DMF (50/1 DMF/water v/v). Solid lithium acetate (198 mg, 1.94 mmol, 1 eq) was added and the reaction allowed to proceed for 3 hours at 40°C followed by three days at -20°C. The reaction mixture was diluted with ethyl acetate and washed with citric acid (pH ∼ 3), water and brine. The organic layer was dried over magnesium sulphate filtered and excess ethyl acetate was removed by rotary evaporation under reduced pressure to provide the deprotected material (1.17g, Quantitative). : LC/MS (3.33 min (ES+) *m*/*z* (relative intensity) 546.77 ([*M* + H]^{+.}, 100)).

### Example 1

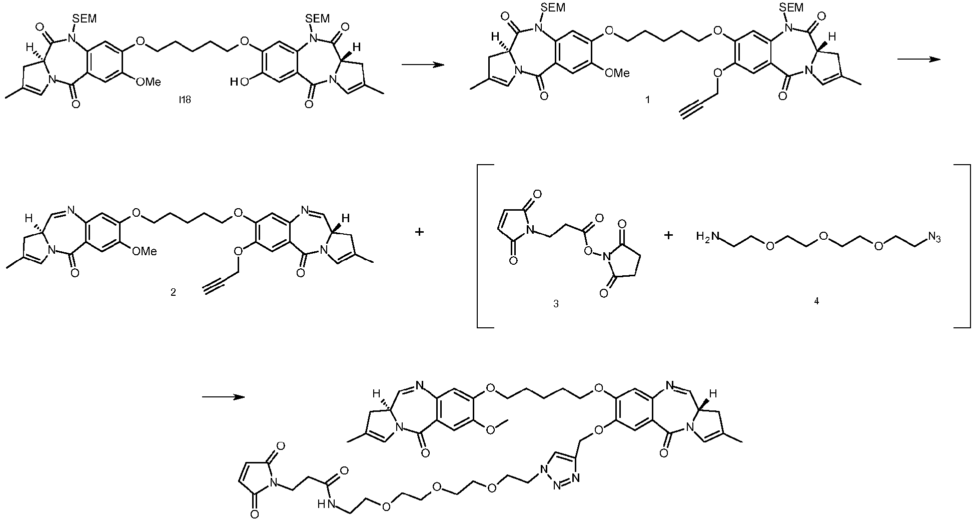

5

### (a) (S)-7-methoxy-2-methyl-8-((5-(((S)-2-methyl-5,11-dioxo-7-(prop-2-yn-1-yloxy)-10-((2-(trimethylsilyl)ethoxy)methyl)-5,10,11,11a-tetrahydro-1H-benzo[e]pyrrolo[1,2-a][1,4]diazepin-8-yl)oxy)pentyl)oxy)-10-((2-(trimethylsilyl)ethoxy)methyl)-1H-benzo[e]pyrrolo[1,2-a][1,4]diazepine-5,11(10H,11aH)-dione (1)

Tetrabutylammonium iodide (34 mg, 0.093 mmol, 0.2 eq), potassium carbonate (96 mg, 0.694 mmol, 1.5 eq) and propargyl bromide (0.1 mL, 0.973 mmol, 2.1 eq) were added to a solution of alcohol 118 (400 mg, 0.463 mmol) of dimethylformamide (10 mL). The reaction mixture was stirred for 1 hour at 70°C after when the reaction was observed to be complete by LC/MS. The reaction mixture was diluted with ethyl acetate and washed with water three times, brine, dried over magnesium sulphate; filtered and excess solvent was removed by rotary evaporation under reduced pressure to provide the desired product (448 mg, quantitative). LC/MS (Method 3) 2.04 min, no ionisation, ¹H NMR (400 MHz, CDCl₃) δ 7.52 (s, 1H), 7.35 (s, 1H), 7.23 (s, 1H), 7.20 (s, 1H), 6.67 (s, 2H), 5.52 (dd, *J* = 10.0, 4.2 Hz, 2H), 4.78 (t, *J* = 2.5 Hz, 2H), 4.68 (dd, *J* = 10.0, 7.8 Hz, 2H), 4.46 (dt, *J* = 10.4, 3.4 Hz, 2H), 4.14 - 4.00 (m, 4H), 3.90 (s, 3H), 3.84 - 3.73 (m, 2H), 3.73 - 3.62 (m, 2H), 3.44 (d, *J* = 16.6 Hz, 2H), 2.80 - 2.72 (m, 2H), 2.52 (t, *J* = 2.4 Hz, 1H), 1.99 - 1.92 (m, 4H), 1.83 (s, 3H), 1.83 (s, 3H), 1.75 - 1.68 (m, 2H), 0.97 (ddd, *J* = 9.7, 6.7, 3.0 Hz, 4H), 0.01 (s, 18H).

### (b) (S)-7-methoxy-2-methyl-8-((5-(((S)-2-methyl-5-oxo-7-(prop-2-yn-1-yloxy)-5,11a-dihydro-1H-benzo[e]pyrrolo[1,2-a][1,4]diazepin-8-yl)oxy)pentyl)oxy)-1H-benzo[e]pyrrolo[1,2-a][1,4jdiazepin-5(11aH)-one (2)

Compound 1 (500 mg, 0.55 mmol) was dissolved in dry THF (15 mL) and cooled to -78 °C. Lithium triethylborohydride (1.39 mL, 1.39 mmol, 2.5 eq) was then added dropwise. The reaction was stirred under argon at -78°C. After 30 minutes, the cold bath was removed and water added. The reaction mixture was extracted with ethyl acetate and washed with brine, dried over magnesium sulphate; filtered and excess solvent was removed by rotary evaporation under reduced pressure. The resulting residue was dissolved with a mixture of dichloromethane / methanol / water (3 / 6 / 1, 6 mL / 12 mL / 2 mL). Silica gel was added until the solution gets thick and left stirring at ambient temperature for 5 days. The reaction mixture was filtered and washed with brine, dried over magnesium sulphate; filtered and excess solvent was removed by rotary evaporation under reduced pressure. The resulting residue was subjected to column flash chromatography (silica gel; 5% methanol / chloroform). Pure fractions were collected and combined, and excess eluent was removed by rotary evaporation under reduced pressure afforded the desired product (240 mg, 72 %). LC/MS (Method 3) 1.88 min, (ES+) *m*/*z* (relative intensity) 608.68 [M + H]⁺, ¹H NMR (400 MHz, CDCl₃) δ 7.80 (d, *J* = 4.1 Hz, 1H), 7.79 (d, *J* = 4.1 Hz, 1H), 7.63 (s, 1H), 7.48 (s, 1H), 6.78 (s, 1H), 6.77 (s, 1H), 6.72 (br, 2H), 4.81 - 4.76 (m, 2H), 4.28 - 4.18 (m, 2H), 4.12 - 4.01 (m, 4H), 3.91 (s, 3H), 3.24 - 3.09 (m, 2H), 2.94 (dd, *J* = 16.8, 5.0 Hz, 2H), 2.52 (t, *J* = 2.3 Hz, 1H), 1.99 - 1.85 (m, 4H), 1.81 (s, 6H), 1.71 - 1.60 (m, 2H).

### (c) 3-(2,5-dioxo-2,5-dihydro-1H-pyrrol-1-yl)-N-(2-(2-(2-(2-(4-((((S)-8-((5-(((S)-7-methoxy-2-methyl-5-oxo-5,11a-dihydro-1H-benzo[e]pyrrolo[1,2-a][1,4]diazepin-8-yl)oxy)pentyl)oxy)-2-methyl-5-oxo-5,11a-dihydro-1H-benzo[e]pyrrolo[1,2-a][1,4]diazepin-7-yl)oxy)methyl)-1H-1,2,3-triazol-1-yl)ethoxy)ethoxy)ethoxy)ethyl)propanamide (5)

Azide 4 (64 µL, 0.328 mmol, 1.2 eq) was added to a solution of succinimide 3 (100 mg, 0.375 mmol, 1.3 eq) in dry DMSO (2 mL) and the reaction mixture was stirred at ambient temperature for 12 hours. In another round bottom flask, compound 2 (170 mg, 0.279 mmol) was dissolved in tert-butanol (2 mL). Water (2 mL) was added followed by sodium ascorbate (11 mg, 0.056 mmol, 0.2 eq) and copper sulphate (4 mg, 0.014 mmol, 0.05 eq). The reaction mixture (with reacted 4 and3) in DMSO was then added to the reaction mixture in water / tert-butanol. The reaction mixture was degassed with argon and stirred at ambient temperature. The reaction was complete after 1 hour and then diluted with dichloromethane and washed with water two times, brine, dried over magnesium sulphate; filtered and excess solvent was removed by rotary evaporation under reduced pressure. The resulting residue was subjected to column flash chromatography (silica gel; 2% to 10% methanol / chloroform). Pure fractions were collected and combined, and excess eluent was removed by rotary evaporation under reduced pressure afforded the desired product (40 mg, 15 %). LC/MS (Method 3) 1.29 min, (ES+) *m*/*z* (relative intensity) 978.40 [M + H]⁺

### Example 2

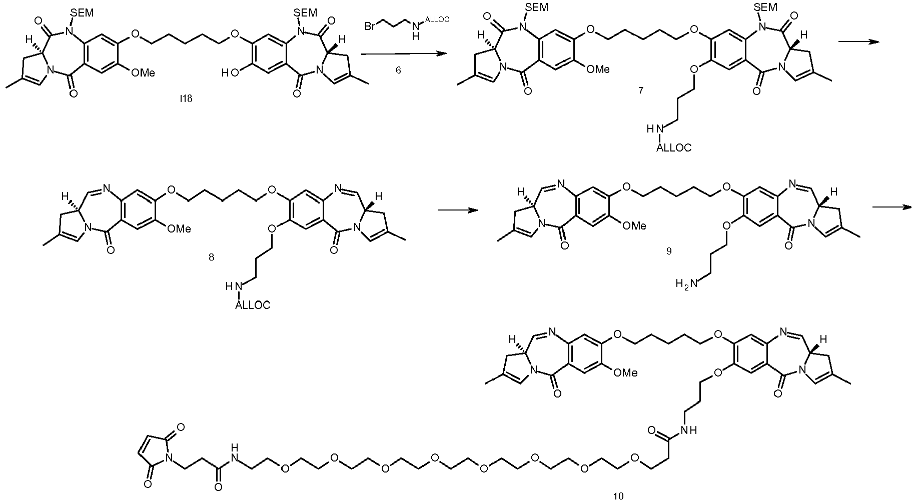

### (a) allyl (3-(((S)-8-((5-(((S)-7-methoxy-2-methyl-5,11-dioxo-10-((2-(trimethylsilyl)ethoxy)methyl)-5,10,11,11a-tetrahydro-1H-benzo[e]pyrrolo[1,2-a][1,4]diazepin-8-yl)oxy)pentyl)oxy)-2-methyl-5,11-dioxo-10-((2-(trimethylsilyl)ethoxy)methyl)-5,10,11,11a-tetrahydro-1H-benzo[e]pyrrolo[1,2-a][1,4]diazepin-7-yl)oxy)propyl)carbamate (7)

Tetrabutylammonium iodide (34 mg, 0.093 mmol, 0.2 eq), potassium carbonate (96 mg, 0.694 mmol, 1.5 eq) and 6 (154 mg, 0.695 mmol, 1.5 eq) were added to a solution of alcohol 118 (400 mg, 0.463 mmol) in dimethylformamide (5 mL). The reaction mixture was stirred for 1 hour at 70°C after when the reaction was observed to be complete by LC/MS. The reaction mixture was diluted with ethyl acetate and washed with water three times, brine, dried over magnesium sulphate; filtered and excess solvent was removed by rotary evaporation under reduced pressure to provide the desired product (420 mg, 90%).
LC/MS (Method 3) 2.05 min, (ES+) *m*/*z* (relative intensity) 863.35 [M + H]⁺, ¹H NMR (400 MHz, CDCl₃) δ 7.33 (s, 1H), 7.31 (s, 1H), 7.18 (s, 1H), 7.17 (s, 1H), 6.64 (br, 2H), 5.87 (ddd, *J* = 22.7, 10.9, 5.7 Hz, 1H), 5.57 (br, 1H), 5.48 (dd, *J* = 10.0, 3.0 Hz, 2H), 5.22 (dd, *J* = 18.3, 1.4 Hz, 1H), 5.15 (dd, *J* = 10.4, 1.2 Hz, 1H), 4.67 (t, *J* = 9.9 Hz, 2H), 4.51 (d, *J* = 5.5 Hz, 2H), 4.44 (ddd, *J* = 10.4, 5.2, 3.3 Hz, 2H), 4.16 - 3.99 (m, 6H), 3.87 (s, 3H), 3.79 - 3.72 (tdd, 2H), 3.70 - 3.59 (m, 2H), 3.43 (br, 1H), 3.42 - 3.34 (m, 3H), 2.73 (dd, *J* = 16.6, 10.5 Hz, 2H), 2.04 - 1.88 (m, 6H), 1.80 (s, 6H), 1.66 (d, *J* = 7.0 Hz, 2H), 0.94 (ddt, *J* = 9.4, 6.6, 2.5 Hz, 4H), 0.01 (s, 18H).

### (b) Allyl (3-(((S)-8-((5-(((S)-7-methoxy-2-methyl-5-oxo-5,11a-dihydro-1H-benzo[e]pyrrolo[1,2-a][1,4]diazepin-8-yl)oxy)pentyl)oxy)-2-methyl-5-oxo-5,11a-dihydro-1H-benzo[e]pyrrolo[1,2-a][1,4]diazepin-7-yl)oxy)propyl)carbamate (8)

Compound 7 (580 mg, 0.55 mmol) was dissolved in dry THF (15 mL) and cooled to -78°C. Lithium triethylborohydride (1.44 mL, 1.44 mmol, 2.5 eq) was then added dropwise. The reaction was stirred under argon at -78°C. After 30 minutes, the cold bath was removed and water added. The reaction mixture was extracted with ethyl acetate and washed with brine, dried over magnesium sulphate; filtered and excess solvent was removed by rotary evaporation under reduced pressure. The resulting residue was dissolved with a mixture of dichloromethane / methanol / water (3 / 6 / 1, 6 mL / 12 mL / 2 mL). Silica gel was added until the solution gets thick and left stirring at ambient temperature for 5 days. The reaction mixture was filtered and washed with brine, dried over magnesium sulphate; filtered and excess solvent was removed by rotary evaporation under reduced pressure. The resulting residue was subjected to column flash chromatography (silica gel; 5% methanol / chloroform). Pure fractions were collected and combined, and excess eluent was removed by rotary evaporation under reduced pressure afforded the desired product (220 mg, 54 %). LC/MS (Method 3) 1.41 min, (ES+) *m*/*z* (relative intensity) 712.40 [M + H]⁺, ¹H NMR (400 MHz, CDCl₃) δ 7.80 (dd, *J* = 3.9, 1.0 Hz, 2H), 7.49 (s, 1H), 7.47 (s, 1H), 6.78 (s, 2H), 6.73 (d, *J* = 1.3 Hz, 2H), 5.96 - 5.83 (m, 1H), 5.68 (br, 1H), 5.27 (d, *J* = 17.2 Hz, 1H), 5.17 (dd, *J* = 10.4, 1.2 Hz, 1H), 4.54 (d, *J* = 5.1 Hz, 2H), 4.28 - 4.16 (m, 2H), 4.16 - 4.00 (m, 6H), 3.91 (s, 3H), 3.48 (br, 1H), 3.50 - 3.38 (m, 2H), 3.36 - 3.34 (m, 1H), 3.24 - 3.11 (m, 2H), 2.95 (dd, *J* = 16.8, 4.8 Hz, 2H), 2.05 - 2.00 (m, 2H), 1.99 - 1.89 (m, 4H), 1.83 (s, 6H).

### (c) (S)-7-(3-aminopropoxy)-8-((5-(((S)-7-methoxy-2-methyl-5-oxo-5,11a-dihydro-1H-benzo[e]pyrrolo[1,2-a][1,4]diazepin-8-yl)oxy)pentyl)oxy)-2-methyl-1H-benzo[e]pyrrolo[1,2-a][1,4]diazepin-5(11aH)-one (9)

*Tetrakis*(triphenylphosphine)palladium(0) (7 mg, 0.006 mmol, 0.06 eq) was added to a solution of 8 (80 mg, 0.112 mmol) and pyrrolidine (23 µL, 0.28 mmol, 2.5 eq) in dry dichloromethane (2 mL). The reaction was flushed with argon three times and stirred 3 hours at room temperature. Then the reaction was diluted with dichloromethane and washed sequentially with saturated aqueous ammonium chloride and brine. The organic phase was dried over magnesium sulphate filtered and excess dichloromethane removed by rotary evaporation under reduced pressure. The resulting residue was used as a crude mixture for the next reaction. LC/MS (Method 3) 1.05 min, (ES+) *m*/*z* (relative intensity) 628.35 [M + H]⁺.

### (d) 1-(3-(2,5-dioxo-2,5-dihydro-1H-pyrrol-1-yl)propanamido)-N-(3-(((S)-8-((5-(((S)-7-methoxy-2-methyl-5-oxo-5,11a-dihydro-1H-benzo[e]pyrrolo[1,2-a][1,4]diazepin-8-yl)oxy)pentyl)oxy)-2-methyl-5-oxo-5,11a-dihydro-1H-benzo[e]pyrrolo[1,2-a][1,4]diazepin-7-yl)oxy)propyl)-3,6,9,12,15,18,21,24-octaoxaheptacosan-27-amide (10)

1-ethyl-3-(3'-dimethylaminopropyl)carbodiimide (EDCI, 11 mg, 0.060 mmol, 1.1 eq) was added to a solution of crude 9 (0.056 mmol) and Mal-(PEG)₈-acid (35 mg, 0.060 mmol, 1.1 eq) in dry dichloromethane (2 mL). The reaction was degassed three times with Argon and stirred for 1 hours and the presence of starting material was no longer observed by LC/MS. The reaction was diluted with dichloromethane and washed sequentially with water and brine. The organic phase was dried over magnesium sulphate filtered and excess dichloromethane removed by rotary evaporation under reduced pressure. The resulting residue was subjected to flash column chromatography (silica gel; 100% chloroform to 10% methanol in chloroform). Pure fractions were collected and combined and excess eluent was removed by rotary evaporation under reduced pressure to give the desired product (19 mg, 28% over 2 steps). LC/MS (Method 3) 1.30 min, (ES+) *m*/*z* (relative intensity) 1202.55 [M + H]⁺. ¹H NMR (400 MHz, CDCl₃) δ 7.79 (d, *J* = 4.0 Hz, 1H), 7.48 (d, *J* = 5.1 Hz, 1H), 6.77 (d, *J* = 1.8 Hz, 1H), 6.72 (d, *J* = 3.5 Hz, 1H), 6.68 (s, 2H), 6.45 - 6.34 (m, 1H), 4.59 - 4.48 (m, 1H), 4.28 - 4.20 (m, 1H), 4.18 - 3.95 (m, 6H), 3.91 (s, 2H), 3.86 - 3.75 (m, 4H), 3.75 - 3.67 (m, 2H), 3.63 - 3.58 (m, 28H), 3.54 - 3.49 (m, 2H), 3.49 - 3.36 (m, 6H), 3.35 - 3.34 (m, 1H), 3.20 - 3.13 (m, 2H), 2.94 (d, *J* = 16.8 Hz, 2H), 2.50 (t, *J* = 7.2 Hz, 2H), 2.46 - 2.37 (m, 2H), 2.07 - 1.99 (m, 2H), 1.97 - 1.91 (m, 4H), 1.82 (s, 3H), 1.77 (s, 3H), 1.69 - 1.63 (m, 2H), 0.90 - 0.77 (m, 1H).

### Example 3

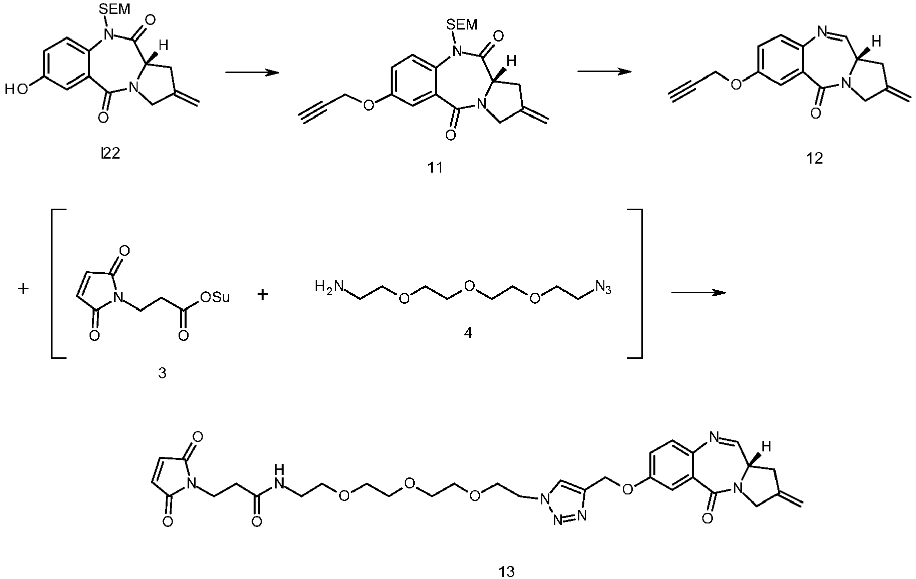

### (a) (S)-2-methylene-7-(prop-2-yn-1-yloxy)-10-((2-(trimethylsilyl)ethoxy)methyl)-2,3-dihydro-1H-benzo[e]pyrrolo[1,2-a][1,4]diazepine-5,11(10H,11aH)-dione (11)

Propargyl bromide (149 µL, 158 mg, 1.33 mmol, 1.05 eq) was added to a suspension of phenol I22 (477 mg, 1.27 mmol, 1eq), TBAI (47 mg, 0.127 mmol, 0.1eq), and potassium carbonate (132 mg, 0.96 mmol, 0.75 eq) in dry DMF (10 mL), and stirred at 75°C for 1 hour when completion was observed. The reaction mixture was partitioned between ethyl acetate (100 mL) and water (100mL). The organics were further washed with water (2 x 100 mL), brine (50 mL) and dried over magnesium sulphate. Yield = 420 mg (80%). LC/MS (3.43 min (ES+) *m*/*z* (relative intensity) 413.07 ([*M* + H]^{+.}, 100)); ¹H NMR (400 MHz, CDCl₃) δ 7.63 (dd, *J* = 9.0, 1.7 Hz, 1H), 7.44 (d, *J* = 3.1 Hz, 1H), 7.17 - 7.11 (m, 1H), 5.48 (dd, *J* = 9.9, 1.5 Hz, 1H), 5.22 - 5.07 (m, 2H), 4.74 (d, *J* = 2.7 Hz, 2H), 4.68 (d, *J* = 9.2 Hz, 1H), 4.40 - 4.14 (m, 3H), 3.69 (dtd, *J* = 16.8, 9.6, 7.7 Hz, 2H), 3.43 (d, *J* = 15.9 Hz, 1H), 2.88 - 2.71 (m, 1H), 2.60 - 2.47 (m, 1H), 1.04 - 0.88 (m, 2H), 0.08 - -0.06 (m, 9H).

### (b) (S)-2-methylene-7-(prop-2-yn-1-yloxy)-2,3-dihydro-1H-benzo[e]pyrrolo[1,2-a][1,4]diazepin-5(11aH)-one (12)

1M superhydride solution in THF (1.32 mL, 1.3 eq) was injected slowly to a solution of dilactam 11 (420 mg, 1.02 mmol, 1eq) in THF at -78°C. The reaction was monitored for 1 hour after which time the complete conversion of starting material directly was observed by LC/MS (3.02 min (ES+) *m*/*z* (relative intensity) 267.10 ([M + H]^{+.}, 100)). The reaction mixture was carefully diluted with H₂O (500 mL) and extracted with ethyl acetate (50 mL). The combined organic layers were washed with water (1 x 50 mL), brine (1 x 20 mL), dried over MgSO₄, filtered and evaporated under reduced pressure at 35°C to provide the intermediate SEM-carbinolamine. The white solid were immediately dissolved in EtOH (40 mL), DCM (15mL) and H₂O (5 mL) and treated with flash silica gel (30 g). The thick suspension was allowed to stir at room temperature for 72 hours after which time the formation of a significant quantity of desired product was observed by TLC (95:5 v/v CHCl₃/MeOH). The reaction mixture was filtered through a very wide porosity 3 sinter funnel and the pad rinsed slowly and thoroughly with 90:10 v/v CHCl₃/MeOH until no further product eluted (checked by TLC). The filtrate was washed with brine (300 mL), dried (MgSO₄), filtered and evaporated *in vacuo,* followed by high vacuum drying, to provide the crude product. Purification by flash chromatography (gradient elution: 100% HPLC grade CHCl₃ to 98:2 v/v CHCl₃/MeOH) gave the desired product as a mixture of carbinolamine ethers and imine (155 mg, 57%). In order to obtain an NMR sample, material (10 mg) was treated with HPLC grade CHCl₃ (50 mL) and allowed to stand overnight to promote the formation of the imine form. The solvent was removed by evaporation under reduced pressure, and the residue was again treated with HPLC grade CHCl₃ (50 mL) and allowed to stand for 4 hours. LC/MS (2.28 min (ES+) *m*/*z* (relative intensity) 267.10 ([*M* + H]^{+.}, 100)); ¹H NMR (400 MHz, CDCl₃) δ 7.71 (d, *J* = 4.4 Hz, 1H), 7.60 (d, *J* = 3.0 Hz, 1H), 7.29 (d, *J* = 8.8 Hz, 1H), 7.17 (dd, *J* = 8.8, 3.0 Hz, 1H), 5.23 - 5.19 (m, 1H), 5.18 (s, 1H), 4.79 - 4.75 (m, 2H), 4.32 - 4.26 (m, 1H), 3.89 (s, 1H), 3.78 - 3.69 (m, 1H), 3.49 (d, *J* = 5.5 Hz, 1H), 3.12 (dd, *J* = 16.1, 9.1 Hz, 1H), 2.96 (dd, *J* = 3.0, 1.4 Hz, 1H).

### (c) (S)-3-(2,5-dioxo-2,5-dihydro-1H-pyrrol-1-yl)-N-(2-(2-(2-(2-(4-(((2-methylene-5-oxo-2,3,5,11a-tetrahydro-1H-benzo[e]pyrrolo[1,2-a][1,4]diazepin-7-yl)oxy)methyl)-1H-1,2,3-triazol-1-yl)ethoxy)ethoxy)ethoxy)ethyl)propanamide (13)

Amino-(Peg)₃-azide 4 (86.1 mg, 78µL,0.39 mmol 1.05 eq) was added to a solution of maleimide succinimide 3 (100 mg, 0.38 mmol, 1eq) in DMSO (0.5 mL) at 25°C and reacted for 45 minutes. This solution was added to a mixture of propargyl-PBD 12 (100 mg, 0.38 mmol, 1eq), sodium ascorbate (15 mg, 0.076 mmol, 0.2 eq), and copper sulphate (4.69 mg, 0.018 mmol, 0.05 eq) in *tert*-butanol/water 1/1 v/v (1.2 mL). The reaction was degazed and allowed to proceed under argon. After 2 hours, the LC/MS profile of the reaction was judged encouraging with significant formation of product alongside the amino-(peg)₃-PBD. When allowed to proceed overnight, the LC/MS profile became poorer. The reaction mixture was partitioned between chloroform/methanol (90/10, v/v) and water. The organics were washed with water, followed by brine and dried over magnesium sulphate. The volatiles were removed by rotoevaporation under vacuum. The residue was purified by flash column chromatography (gradient methanol/chloroform 1/99 to 20/80). The product (dark spot under UV) came as mixed fractions with the amino-(peg)₃-PBD (blue glow under UV) and was further purified by preparative TLC (25 mg, 10%). LC/MS (2.28 min (ES+) *m*/*z* (relative intensity) 636.16 ([M + H]^{+.}, 100)); ¹H NMR (400 MHz, CDCl₃) δ 7.90 (s, 1H), 7.71 (d, *J* = 4.4 Hz, 1H), 7.61 (d, *J* = 2.9 Hz, 1H), 7.29 (d, *J* = 8.8 Hz, 1H), 7.18 (dd, *J* = 8.8, 3.0 Hz, 1H), 6.67 (s, 2H), 6.46 (s, 1H), 5.26 (d, *J* = 7.1 Hz, 1H), 5.23 - 5.16 (m, 2H), 4.61 - 4.53 (m, 2H), 4.32 - 4.25 (m, 1H), 3.94 - 3.87 (m, 2H), 3.82 (t, *J* = 7.2 Hz, 2H), 3.65 - 3.53 (m, 10H), 3.52 - 3.46 (m, 3H), 3.43 - 3.35 (m, 2H), 3.14 (dd, *J* = 16.0, 9.0 Hz, 1H), 2.95 (dd, *J* = 16.0, 1.5 Hz, 1H), 2.49 (t, *J* = 7.2 Hz, 2H).

### Example 4

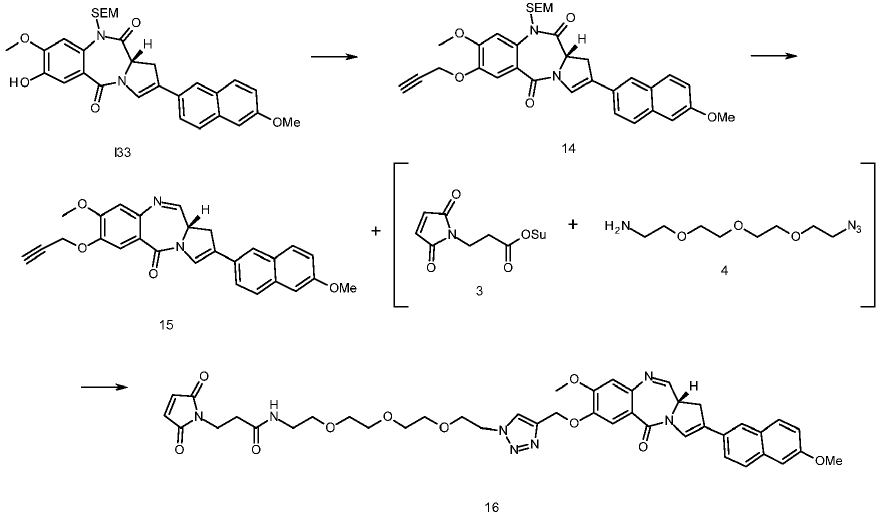

### (a) (S)-8-methoxy-2-(6-methoxynaphthalen-2-yl)-7-(prop-2-yn-1-yloxy)-10-((2-(trimethylsilyl)ethoxy)methyl)-1H-benzo[e]pyrrolo[1,2-a][1,4]diazepine-5,11(10H,11aH)-dione (14)

Propargyl bromide (251µL, 2.24 mmol 1.05 eq) was added to a suspension of crude phenol I33 (1.17g, 2.14 mmol, 1 eq), TBAI (79mg, 0.21 mmol, 1 eq) and potassium carbonate (221mg, 1.60 mmol, 0.75 eq) in dry DMF (10 mL), and stirred at 75°C for 1 hour when completion was observed. The reaction mixture was partitioned between ethyl acetate (100 mL) and water (100mL). The organics were further washed with water (2 x 100 mL), brine (50 mL) and dried over magnesium sulphate. The residue was subjected to column flash chromatography (gradient elution: 35:65 v/v Hexane/Et2O to 0:100 v/v Hexane/Et₂O) afforded the desired product (594 mg, 47% from crude, 52% over three steps). LC/MS (3.85 min (ES+) *m*/*z* (relative intensity) 584.92 ([*M* + H]^{+.}, 100)); [α]²¹_{D} = +345° (*c* = 0.229, Chloroform); ¹H NMR (400 MHz, CDCl₃) δ 7.75 - 7.64 (m, 3H), 7.64 - 7.55 (m, 2H), 7.52 (s, 1H), 7.32 (s, 1H), 7.19 - 7.08 (m, 2H), 5.59 (d, *J* = 10.0 Hz, 1H), 4.92 - 4.77 (m, 2H), 4.71 (dd, *J* = 10.4, 3.8 Hz, 2H), 4.08 (ddd, *J* = 16.0, 3.3, 1.7 Hz, 1H), 3.97 - 3.89 (m, 6H), 3.83 (td, *J* = 9.4, 7.2 Hz, 1H), 3.72 (td, *J* = 9.4, 7.1 Hz, 1H), 3.27 (ddd, *J* = 15.9, 10.6, 2.1 Hz, 1H), 2.56 (t, *J* = 2.4 Hz, 1H), 1.05 - 0.95 (m, 2H), 0.07 - 0.02 (m, 9H).

### (b) (S)-8-methoxy-2-(6-methoxynaphthalen-2-yl)-7-(prop-2-yn-1-yloxy)-1H-benzo[e]pyrrolo[1,2-a][1,4]diazepin-5(11aH)-one (15)

1M superhydride solution in THF (1.27 mL, 1.27 mmol, 1.3 eq) was injected slowly to a solution of dilactam 14 (573 mg, 0.98 mmol, 1 eq) in THF (10 mL) at -78°C. The reaction was monitored for 1 hour after which time the complete conversion of starting material directly was observed by LC/MS (3.03 min (ES+) *m*/*z* (relative intensity) 456.88 ([M + H]^{+.}, 100)). The reaction mixture was carefully diluted with H₂O (500 mL) and extracted with chlorofom (50 mL). The combined organic layers were washed with water (1 x 50 mL), brine (1 x 20 mL), dried over MgSO₄, filtered and evaporated under reduced pressure at 35°C to provide the intermediate SEM-carbinolamine. The white solid were immediately dissolved in EtOH (40 mL), DCM (15mL) and H₂O (5 mL) and treated with flash silica gel (30 g). The thick suspension was allowed to stir at room temperature for 72 hours after which time the formation of a significant quantity of desired product was observed by TLC (95:5 v/v CHCl₃/MeOH). The reaction mixture was filtered through a very wide porosity 3 sinter funnel and the pad rinsed slowly and thoroughly with 90:10 v/v CHCl₃/MeOH until no further product eluted (checked by TLC). The filtrate was washed with brine (100 mL), dried (MgSO₄), filtered and evaporated *in vacuo,* followed by high vacuum drying, to provide the crude product. Purification by flash chromatography (Ethyl acetate) gave the desired product (100 mg, 23%). In order to obtain an NMR sample, material (10 mg) was treated with HPLC grade CHCl₃ (50 mL) and allowed to stand overnight to promote the formation of the imine form. The solvent was removed by evaporation under reduced pressure, and the residue was again treated with HPLC grade CHCl₃ (50 mL) and allowed to stand for 4 hours. LC/MS (3.03 min (ES+) *m*/*z* (relative intensity) 456.88 ([M + H₂O]^{+.}, 100)); ¹H NMR (500 MHz, CDCl₃) δ 7.96 (d, *J* = 4.0 Hz, 1H), 7.74 - 7.68 (m, 3H), 7.63 - 7.57 (m, 3H), 7.19 - 7.09 (m, 2H), 6.87 (s, 1H), 4.87 (dd, *J* = 4.6, 2.4 Hz, 2H), 4.49 (ddd, *J* = 11.6, 5.1, 4.1 Hz, 1H), 3.99 - 3.91 (m, 6H), 3.72 (ddd, *J* = 16.1, 11.5, 2.0 Hz, 1H), 3.53 (ddd, *J* = 16.2, 5.1, 1.7 Hz, 1H), 2.56 (t, *J* = 2.4 Hz, 1H).

### (c) (S)-3-(2,5-dioxo-2,5-dihydro-1H-pyrrol-1-yl)-N-(2-(2-(2-(2-(4-(((8-methoxy-2-(6-methoxynaphthalen-2-yl)-5-oxo-5,11a-dihydro-1H-benzo[e]pyrrolo[1,2-a][1,4]diazepin-7-yl)oxy)methyl)-1H-1,2,3-triazol-1-yl)ethoxy)ethoxy)ethoxy)ethyl)propanamide (16)

Amino-(Peg)₃-azide 4 (34.2µL, 37.6 mg, 0.17 mmol, 0.9 eq) was added to a solution of maleimide succinimide 3 (56.1 mg, 0.21 mmol, 1.1 eq) in DMSO (0.5 mL) at 25°C and reacted for 45 minutes. This solution was added to a mixture of propargyl-PBD 15 (84 mg, 0.19 mmol, 1 eq), sodium ascorbate (7.6 mg, 0.038 mmol, 0.2 eq), and copper sulphate (2.4 mg, 0.010 mmol, 0.05 eq) in *tert*-butanol/water 1/1 v/v (1 mL). DMSO (2.5 mL) to improve solubility. The reaction was degazed and allowed to proceed under argon. After 5 hours, the LC/MS profile of the reaction showed significant formation of product. The reaction mixture was partitioned between chloroform and water. The organics were washed with water, followed by brine and dried over magnesium sulphate. The volatiles were removed by rotoevaporation under vacuum. The residue was purified by flash column chromatography (gradient methanol/chloroform 1/99 to 5/95) to give the desired product. Yield: 64 mg (41%). LC/MS (2.85 min (ES+) *m*/*z* (relative intensity) 808.61 ([*M* + H]^{+.}, 100)); ¹H NMR (500 MHz, CDCl₃) δ 7.96 (d, *J* = 4.0 Hz, 1H), 7.94 (s, 1H), 7.74 - 7.68 (m, 3H), 7.64 - 7.56 (m, 3H), 7.19 - 7.10 (m, 2H), 6.86 (s, 1H), 6.65 (s, 2H), 6.44 (s, 1H), 5.33 (q, *J* = 11.9 Hz, 2H), 4.60 - 4.54 (m, 2H), 4.50 (dt, *J* = 9.1, 5.0 Hz, 1H), 3.95 - 3.88 (m, 8H), 3.82 (t, *J* = 7.3 Hz, 2H), 3.77 - 3.65 (m, 2H), 3.65 - 3.58 (m, 5H), 3.58 - 3.51 (m, 3H), 3.52 - 3.47 (m, 2H), 3.39 (dd, *J* = 10.6, 5.3 Hz, 2H), 2.49 (td, *J* = 7.0, 0.8 Hz, 2H).

### Example 5

### General antibody conjugation procedure

Antibodies are diluted to 1-5 mg/mL in a reduction buffer (examples: phosphate buffered saline PBS, histidine buffer, sodium borate buffer,TRIS buffer). A freshly prepared solution of TCEP (tris(2-carboxyethyl)phosphine hydrochloride) is added to selectively reduce cysteine disulfide bridges. The amount of TCEP is proportional to the target level of reduction, within 1 to 4 molar equivalents per antibody, generating 2 to 8 reactive thiols. After reduction for several hours at 37°C, the mixture is cooled down to room temperature and excess drug-linker (**5, 10, 13, 16**) added as a diluted DMSO solution (final DMSO content of up to 10% volume/volume of reaction mixture). The mixture was gently shaken at either 4°C or room temperature for the appropriate time, generally 1-3 hours. Excess reactive thiols can be reacted with a 'thiol capping reagent' like N-ethyl maleimide (NEM) at the end of the conjugation. Antibody-drug conjugates are concentrated using centrifugal spin-filters with a molecular weight cut-off of 10 kDa or higher, then purified by tangential flow filtration (TFF) or Fast Protein Liquid Chromatography (FPLC). Corresponding antibody-drug conjugates can be determined by analysis by High-Performance Liquid Chromatography (HPLC) or Ultra-High-Performance Liquid Chromatography (UHPLC) to assess drug-per-antibody ratio (DAR) using reverse-phase chromatography (RP) or Hydrophobic-Interaction Chromatography (HIC), coupled with UV-Visible, Fluorescence or Mass-Spectrometer detection; aggregate level and monomer purity can be analysed by HPLC or UHPLC using size-exclusion chromatography coupled with UV-Visible, Fluorescence or Mass-Spectrometer detection. Final conjugate concentration is determined by a combination of spectroscopic (absorbance at 280, 214 and 330 nm) and biochemical assay (bicinchonic acid assay BCA; Smith, P.K., et al. (1985) Anal. Biochem. 150 (1): 76-85; using a known-concentration IgG antibody as reference). Antibody-drug conjugates are generally sterile filtered using 0.2 µm filters under aseptic conditions, and stored at +4°C, -20°C or -80°C.

Examples of particular conjugations are described below.

### Conjugate A1 (Herceptin-10, ConjA)

Herceptin™ (2.0 mg, 13.3 nanomoles) was diluted into 1.8 mL of a reduction buffer containing 10 mM sodium borate pH 8.4, 2.5 mM EDTA and a final antibody concentration of 1.11 mg/mL. A 10 mM solution of TCEP was added (2 molar equivalent/antibody, 26.6 nanomoles, 2.66 µL) and the reduction mixture was heated at +37°C for 2.5 hours in a heating block. After cooling down to room temperature, compound **10** was added as a DMSO solution (3.5 molar equivalent/antibody, 45 nanomoles, in 0.2 mL DMSO). The solution was mixed for 2 hour at room temperature, then the conjugation was quenched by addition of N-ethyl maleimide (1 micromole, 10µL at 100 mM) followed 15 minutes later by N-acetyl cystein (1.5 micromoles, 15µL at 100 mM), then injected into a AKTA™FPLC using a GE Healthcare XK16/70 column packed with Superdex 200 PG, eluting with 1.5 mL/min of sterile-filtered Phosphate-buffered saline (PBS). Fractions corresponding to ConjA1 monomer peak were pooled, concentrated using a 15mL Amicon Ultracell 50KDa MWCO spin filter, analysed and sterile-filtered. BCA assay gives a concentration of final ConjA at 1.49 mg/mL in 1.2 mL, obtained mass of ConjA1 is 1.79 mg (90% yield).

UHPLC analysis on a Shimadzu Prominence system using a Phenomenex Aeris 3.6u XB-C18 150 x 2.1 mm column eluting with a gradient of water and acetonitrile on a reduced sample of ConjA1 at 280 nm and 330 nm (Compound **10** specific) shows a mixture of light and heavy chains attached to several molecules of compound **10,** consistent with a drug-per-antibody ratio (DAR) of 2.7 molecules of compound **10** per antibody.

UHPLC analysis on a Shimadzu Prominence system using a Waters Acquity UPLC BEH200 SEC 1.7 um 4.6 x 150 mm column eluting with sterile-filtered Phosphate-buffered saline (PBS) containing 5% isopropanol (v/v) on a sample of ConjA at 280 nm shows a monomer purity of over 99% with no impurity detected.

### Conjugate A2 (Herceptin-10, ConjA2)

Herceptin™ (2.0 mg, 13.3 nanomoles) was diluted into 1.8 mL of a reduction buffer containing 10 mM sodium borate pH 8.4, 2.5 mM EDTA and a final antibody concentration of 1.11 mg/mL. A 10 mM solution of TCEP was added (2 molar equivalent/antibody, 26.6 nanomoles, 2.66 µL) and the reduction mixture was heated at +37°C for 2.5 hours in a heating block. After cooling down to room temperature, compound **10** was added as a DMSO solution (3.5 molar equivalent/antibody, 45 nanomoles, in 0.2 mL DMSO). The solution was mixed for 2 hours at room temperature, then the conjugation was quenched by addition of *N*-ethyl maleimide (1 micromole, 10 µL at 100 mM) followed 15 minutes later by *N*-acetyl cysteine (1.5 micromoles, 15 µL at 100 mM), then injected into an AKTA™ FPLC using a GE Healthcare XK16/70 column packed with Superdex™ 200 PG, eluting with 1.5 mL/min of sterile-filtered phosphate-buffered saline (PBS). Fractions corresponding to ConjA2 monomer peak were pooled, concentrated using a 15mL Amicon Ultracell 50KDa MWCO spin filter, analysed and sterile-filtered. BCA assay gives a concentration of final ConjA2 at 1.49 mg/mL in 1.2 mL, obtained mass of ConjA2 is 1.79 mg (90% yield).

UHPLC analysis on a Shimadzu Prominence system using a Phenomenex Aeris 3.6u XB-C18 150 x 2.1 mm column eluting with a gradient of water and acetonitrile on a reduced sample of ConjA2 at 280 nm and 330 nm (compound **10** specific) shows a mixture of light and heavy chains attached to several molecules of compound **10,** consistent with a drug-per-antibody ratio (DAR) of 2.7 molecules of compound **10** per antibody.

UHPLC analysis on a Shimadzu Prominence system using a Waters Acquity UPLC BEH200 SEC 1.7 um 4.6 x 150 mm column eluting with sterile-filtered phosphate-buffered saline (PBS) containing 5% isopropanol (v/v) on a sample of ConjA2 at 280 nm shows a monomer purity of over 99% with no impurity detected.

### Conjugate B (Herceptin-5, ConjB)

Herceptin™ (2.0 mg, 13.3 nanomoles) was diluted into 1.8 mL of a reduction buffer containing 10 mM sodium borate pH 8.4, 2.5 mM EDTA and a final antibody concentration of 1.11 mg/mL. A 10 mM solution of TCEP was added (2 molar equivalent/antibody, 26.6 nanomoles, 2.66 µL) and the reduction mixture was heated at +37°C for 2.0 hours in a heating block. After cooling down to room temperature, compound **5** was added as a DMSO solution (10 molar equivalent/antibody, 133 nanomoles, in 0.2 mL DMSO). The solution was mixed for 1 hours at room temperature, then the conjugation was injected into an AKTA™ FPLC using a GE Healthcare XK16/70 column packed with Superdex 200 PG, eluting with 1.5 mL/min of sterile-filtered phosphate-buffered saline (PBS). Fractions corresponding to ConjB monomer peak were pooled, concentrated using a 15mL Amicon Ultracell 50KDa MWCO spin filter, analysed and sterile-filtered. BCA assay gives a concentration of final ConjB at 3.13 mg/mL in 0.65 mL, obtained mass of ConjB is 1.61 mg (80% yield).

UHPLC analysis on a Shimadzu Prominence system using a Phenomenex Aeris 3.6u XB-C18 150 x 2.1 mm column eluting with a gradient of water and acetonitrile on a reduced sample of ConjB at 280 nm and 330 nm (compound **5** specific) shows a mixture of light and heavy chains attached to several molecules of compound **5,** consistent with a drug-per-antibody ratio (DAR) of 4 molecules of compound **5** per antibody.

UHPLC analysis on a Shimadzu Prominence system using a Waters Acquity UPLC BEH200 SEC 1.7 um 4.6 x 150 mm column eluting with sterile-filtered Phosphate-buffered saline (PBS) containing 5% isopropanol (v/v) on a sample of ConjB at 280 nm shows a monomer purity of over 98.6%.

### Conjugate C (Herceptin-13, ConjC)

Herceptin™(1.0 mg, 6.7 nanomoles) was diluted into 0.9 mL of a reduction buffer containing 10 mM sodium borate pH 8.4, 2.5 mM EDTA and a final antibody concentration of 1.11 mg/mL. A 1 mM solution of TCEP was added (3 molar equivalent/antibody, 20 nanomoles, 20 µL) and the reduction mixture was heated at +37°C for 1.5 hours in a heating block. After cooling down to room temperature, compound **13** was added as a DMSO solution (10 molar equivalent/antibody, 67 nanomoles, in 0.1 mL DMSO). The solution was mixed for 1 hour at room temperature, the conjugation mixture was analysed by HPLC and then injected into an AKTA™ FPLC using a GE Healthcare XK16/70 column packed with Superdex™ 200 PG, eluting with 1.5 mL/min of sterile-filtered phosphate-buffered saline (PBS). Fractions corresponding to ConjC monomer peak were pooled, concentrated using a 15mL Amicon Ultracell 50KDa MWCO spin filter, analysed and sterile-filtered. BCA assay gives a concentration of final ConjC at 0.70 mg/mL in 1.0 mL, obtained mass of ConjC is 0.70 mg (70% yield).

UHPLC analysis on a Shimadzu Prominence system using an Agilent Technologies PLRP-S 1000A 5 µm 50 x 2.1 mm column eluting with a gradient of water and acetonitrile on a reduced sample of ConjC at 280 nm and 330 nm (compound **13** specific) shows a mixture of light and heavy chains attached to several molecules of compound **13,** consistent with a drug-per-antibody ratio (DAR) of >2.7 molecules of compound **13** per antibody.

### Conjugate D (Herceptin-16, ConjD)

Herceptin™ was diluted into 0.9 mL of a reduction buffer containing 10 mM sodium borate pH 8.4, 2.5 mM EDTA and a final antibody concentration of 1.11 mg/mL. A 1 mM solution of TCEP was added (3 molar equivalent/antibody, 20 nanomoles, 20 µL) and the reduction mixture was heated at +37°C for 1.5 hours in a heating block. After cooling down to room temperature, compound **16** was added as a DMSO solution (10 molar equivalent/antibody, 67 nanomoles, in 0.1 mL DMSO). The solution was mixed for 1 hour at room temperature, the conjugation mixture was analysed by HPLC and then injected into an AKTA™ FPLC using a GE Healthcare XK16/70 column packed with Superdex™ 200 PG, eluting with 1.5 mL/min of sterile-filtered phosphate-buffered saline (PBS). Fractions corresponding to ConjD monomer peak were pooled, concentrated using a 15mL Amicon Ultracell 50KDa MWCO spin filter, analysed and sterile-filtered. BCA assay gives a concentration of final ConjD at 0.55 mg/mL in 1.0 mL, obtained mass of ConjD is 0.55 mg (55% yield).

UHPLC analysis on a Shimadzu Prominence system using an Agilent Technologies PLRP-S 1000A 5 µm 50 x 2.1 mm column eluting with a gradient of water and acetonitrile on a reduced sample of ConjD at 280 nm and 330 nm (compound **16** specific) shows a mixture of light and heavy chains attached to several molecules of compound **16,** consistent with a drug-per-antibody ratio (DAR) of 3.56 molecules of compound **16** per antibody.

### Example 6: In vivo ADC efficacy studies

CB.17 SCID mice, aged 8-12 weeks, may be injected with 1 mm³ tumour fragments sub cutaneously in the flank. When tumours reach an average size of 100 - 150 mg, treatment may be begun. Mice may be weighed twice a week. Tumour size may be measured twice a week. Animals may be monitored individually. The endpoint of the experiment is a tumour volume of 1000 mm³ or 60 days, whichever comes first. Responders can be followed longer.

Groups of 10 xenografted mice can be injected i.v. with 0.2ml of antibody drug conjugate (ADC), or naked antibody, in phosphate buffered saline (vehicle) or with 0.2ml of vehicle alone. The concentration of ADC can be adjusted to give, for example, 0.3 or 1.0 mg ADC/ kg body weight in a single dose. Three identical doses may be given to each mouse at intervals of, for example, 1 week.

### Example 7: In vitro ADC efficacy studies

Medium from subconfluent (about 80-90% confluency) SK-BR-3 cells in a T75 flask was aspirated and PBS (about 20ml) was added to rinse away the culture medium. The PBS was aspirated and Trypsin-EDTA (5ml) added. The flask was returned to the 37°C gassed incubator for up to about 5 minutes. The flask was rapped sharply to dislodge and dissociate cells from the plastic. The cell suspension was transferred to a sterile 50ml screw-top centrifuge tube. Medium (McCoy's + 10% FCS) was added to a final volume of 15ml, then the tube was centrifuged (400g for 5 min). The supernatant was aspirated and the pellet re-suspended in 10ml culture medium. Repeated aspiration (up and down a 10ml pipette) may be necessary to break up cell clumps and produce monodisperse cell suspensions suitable for counting. Cell suspension (10µl) was mixed with Trypan blue (10µl) and live/dead cells counted with a haemocytometer to determine cell concentration and viability. The cell suspension was diluted to 20x10⁴/ml and 50µl was dispensed into clear 96 well flat bottomed plates. The cells were incubated overnight to allow recovery before use.

A stock solution (1ml) of antibody drug conjugate (ADC) (20µg/ml) was made by dilution of filter-sterilised ADC into cell culture medium. A set of 8x 10-fold dilutions of stock ADC was made in a 24 well plate by serial transfer of 100µl onto 900µl of cell culture medium.

50µl of each ADC dilution is dispensed into 4 replicate wells of the 96 well plate, containing 50µl cell suspension seeded the previous day. Control wells receive 50µl cell culture medium. The 96-well plate containing cells and ADCs was incubated at 37°C in a CO₂-gassed incubator for 4 days. At the end of the incubation period, viable cells were measured by MTS assay. MTS (Promega) was dispensed (20µl per well) into each well and incubated for 4 hours at 37°C in the CO₂-gassed incubator. Well absorbance was measured at 490nm. Percentage cell survival is calculated from the mean absorbance in the 4 ADC-treated wells compared to the mean absorbance in the 4 control wells (100%).

| | |
|---|---|
| ADC | EC₅₀ (µg/ml) |
| ConjA | 0.06187 |

### Abbreviations

- Ac: acetyl
- Acm: acetamidomethyl
- Alloc: allyloxycarbonyl
- Boc: di-*tert*-butyl dicarbonate
- t-Bu: tert-butyl
- Bzl: benzyl, where Bzl-OMe is methoxybenzyl and Bzl-Me is methylbenzene
- Cbz or Z: benzyloxy-carbonyl, where Z-Cl and Z-Br are chloro- and bromobenzyloxy carbonyl respectively
- DMF: *N,N*-dimethylformamide
- Dnp: dinitrophenyl
- DTT: dithiothreitol
- Fmoc: 9*H*-fluoren-9-ylmethoxycarbonyl
- imp: *N*-10 imine protecting group: 3-(2-methoxyethoxy)propanoate-Val-Ala-PAB
- MC-OSu: maleimidocaproyl-*O-N*-succinimide
- Moc: methoxycarbonyl
- MP: maleimidopropanamide
- Mtr: 4-methoxy-2,3,6-trimethtylbenzenesulfonyl
- PAB: para-aminobenzyloxycarbonyl
- PEG: ethyleneoxy
- PNZ: *p*-nitrobenzyl carbamate
- Psec: 2-(phenylsulfonyl)ethoxycarbonyl
- TBDMS: tert-butyldimethylsilyl
- TBDPS: tert-butyldiphenylsilyl
- Teoc: 2-(trimethylsilyl)ethoxycarbonyl
- Tos: tosyl
- Troc: 2,2,2-trichlorethoxycarbonyl chloride
- Trt: trityl
- Xan: xanthyl

### References

The following references are incorporated by reference in their entirety:
EP 0522868
EP 0875569
EP 1295944
EP 1347046
EP 1394274
EP 1394274
EP 1439393
JP 05003790
JP 2004113151
JP 58180487
US 2001/055751
US 2002/034749
US 2002/042366
US 2002/150573
US 2002/193567
US 2003/0228319
US 2003/060612
US 2003/064397
US 2003/065143
US 2003/091580
US 2003/096961
US 2003/105292
US 2003/109676
US 2003/118592
US 2003/119121
US 2003/119122
US 2003/119125
US 2003/119126
US 2003/119128
US 2003/119129
US 2003/119130
US 2003/119131
US 2003/124140
US 2003/124579
US 2003/129192
US 2003/134790-A1
US 2003/143557
US 2003/157089
US 2003/165504
US 2003/185830
US 2003/186372
US 2003/186373
US 2003/194704
US 2003/206918
US 2003/219806
US 2003/224411
US 2003/224454
US 2003/232056
US 2003/232350
US 20030096743
US 20030130189
US 2003096743
US 2003130189
US 2004/0001827
US 2004/005320
US 2004/005538
US 2004/005563
US 2004/005598
US 2004/0101899
US 2004/018553
US 2004/022727
US 2004/044179
US 2004/044180
US 2004/101874
US 2004/197325
US 2004/249130
US 20040018194
US 20040052793
US 20040052793
US 20040121940
US 2005/271615
US 2006/116422
US 4816567
US 5362852
US 5440021
US 5583024
US 5621002
US 5644033
US 5674713
US 5700670
US 5773223
US 5792616
US 5854399
US 5869445
US 5976551
US 6011146
US 6153408
US 6214345
US 6218519
US 6268488
US 6518404
US 6534482
US 6555339
US 6602677
US 6677435
US 6759509
US 6835807
US 7223837
US 7375078
US 7521541
US 7723485
WO 00/012508
WO 00/12507
WO 00/12508
WO 01/16318
WO 01/45746
WO 02/088172
WO 03/026577
WO 03/043583
WO 04/032828
WO 2000/12130
WO 2000/14228
WO 2000/20579
WO 2000/22129
WO 2000/32752
WO 2000/36107
WO 2000/40614
WO 2000/44899
WO 2000/55351
WO 2000/75655
WO 200053216
WO 2001/00244
WO 2001/38490
WO 2001/40269
WO 2001/40309
WO 2001/41787
WO 2001/46232
WO 2001/46261
WO 2001/48204
WO 2001/53463
WO 2001/57188
WO 2001/62794
WO 2001/66689
WO 2001/72830
WO 2001/72962
WO 2001/75177
WO 2001/77172
WO 2001/88133
WO 2001/90304
WO 2001/94641
WO 2001/98351
WO 2002/02587
WO 2002/02624
WO 2002/06317
WO 2002/06339
WO 2002/101075
WO 2002/10187
WO 2002/102235
WO 2002/10382
WO 2002/12341
WO 2002/13847
WO 2002/14503
WO 2002/16413
WO 2002/16429
WO 2002/22153
WO 2002/22636
WO 2002/22660
WO 2002/22808
WO 2002/24909
WO 2002/26822
WO 2002/30268
WO 2002/38766
WO 2002/54940
WO 2002/59377
WO 2002/60317
WO 2002/61087;
WO 2002/64798
WO 2002/71928
WO 2002/72596
WO 2002/78524
WO 2002/81646
WO 2002/83866
WO 2002/86443
WO 2002/88170
WO 2002/89747
WO 2002/92836
WO 2002/94852
WO 2002/98358
WO 2002/99074
WO 2002/99122
WO 2003/000842
WO 2003/002717
WO 2003/003906
WO 2003/003984
WO 2003/004989
WO 2003/008537
WO 2003/009814
WO 2003/014294
WO 2003/016475
WO 2003/016494
WO 2003/018621
WO 2003/022995
WO 2003/023013
WO 2003/024392
WO 2003/025138
WO 2003/025148
WO 2003/025228
WO 2003/026493
WO 2003/029262
WO 2003/029277
WO 2003/029421
WO 2003/034984
WO 2003/035846
WO 2003/042661
WO 2003/045422
WO 2003/048202
WO 2003/054152
WO 2003/055439
WO 2003/055443
WO 2003/062401
WO 2003/062401
WO 2003/072035
WO 2003/072036
WO 2003/077836
WO 2003/081210
WO 2003/083041
WO 2003/083047
WO 2003/083074
WO 2003/087306
WO 2003/087768
WO 2003/088808
WO 2003/089624
WO 2003/089904
WO 2003/093444
WO 2003/097803
WO 2003/101283
WO 2003/101400
WO 2003/104270
WO 2003/104275
WO 2003/105758
WO 2003004529
WO 2003042661
WO 2003104399
WO 2004/000997
WO 2004/001004
WO 2004/009622
WO 2004/011611
WO 2004/015426
WO 2004/016225
WO 2004/020595
WO 2004/022709
WO 2004/022778
WO 2004/027049
WO 2004/031238
WO 2004/032828
WO 2004/032842
WO 2004/040000
WO 2004/043361
WO 2004/043963
WO 2004/044178
WO 2004/045516
WO 2004/045520
WO 2004/045553
WO 2004/046342
WO 2004/047749
WO 2004/048938
WO 2004/053079
WO 2004/063355
WO 2004/063362
WO 2004/063709
WO 2004/065577
WO 2004/074320
WO 2004000221
WO 2004020583
WO 2004042346
WO 2004065576
WO 2005/023814
WO 2005/082023
WO 2005/085251
WO 2006/111759
WO 2007/044515
WO 2007/085930
WO 2009/052249
WO 2010/091150
WO 91/02536
WO 92/07574
WO 92/17497
WO 94/10312
WO 94/28931
WO 9630514
WO 97/07198
WO 97/44452
WO 98/13059
WO 98/37193
WO 98/40403
WO 98/51805
WO 98/51824
WO 99/28468
WO 99/46284
WO 99/58658

Am. J. Hum. Genet. 49 (3):555-565 (1991)
Amiel J., et al Hum. Mol. Genet. 5, 355-357, 1996
Amir et al (2003) Angew. Chem. Int. Ed. 42:4494-4499
Amsberry, et al (1990) J. Org. Chem. 55:5867
Angew Chem. Intl. Ed. Engl. (1994) 33:183-186
Annu. Rev. Neurosci. 21:309-345 (1998)
Arai H., et al J. Biol. Chem. 268, 3463-3470, 1993
Arai H., et al Jpn. Circ. J. 56, 1303-1307, 1992
Arima, et al., J. Antibiotics, 25, 437-444 (1972)
Attie T., et al, Hum. Mol. Genet. 4, 2407-2409, 1995
Auricchio A., et al Hum. Mol. Genet. 5:351-354, 1996
Barel M., et al Mol. Immunol. 35, 1025-1031, 1998
Barella et al (1995) Biochem. J. 309:773-779
Barnett T., et al Genomics 3, 59-66, 1988
Beck et al (1992) J. Mol. Biol. 228:433-441
Beck et al (1996) J. Mol. Biol. 255:1-13
Berge, et al., J. Pharm. Sci., 66, 1-19 (1977)
Biochem. Biophys. Res. Commun. (2000) 275(3):783-788
Biochem. Biophys. Res. Commun. 255 (2), 283-288 (1999)
Blood (2002) 100 (9):3068-3076
Blood 99 (8):2662-2669 (2002)
Blumberg H., et al Cell 104, 9-19, 2001
Bose, et al., Tetrahedron, 48, 751-758 (1992)
Bourgeois C., et al J. Clin. Endocrinol. Metab. 82, 3116-3123, 1997
Brinster et al (1988) Proc. Natl. Acad. Sci. USA 85:836
Buchman and Berg (1988) Mol. Cell. Biol. 8:4395
Cancer Res. 61 (15), 5857-5860 (2001)
Carl et al (1981) J. Med. Chem. 24:479-480
Carlsson et al (1978) Biochem. J. 173:723-737
Carter, P. (2006) Nature Reviews Immunology 6:343-357
Cell 109 (3):397-407 (2002)
CellTiter Glo Luminescent Cell Viability Assay, Promega Corp. Technical Bulletin TB288
Chakravarty et al (1983) J. Med. Chem. 26:638-644
Chan,J. and Watt, V.M., Oncogene 6 (6), 1057-1061 (1991)
Child et al (1999) J. Biol. Chem. 274: 24335-24341
Cho H.-S., et al Nature 421, 756-760, 2003
Ciccodicola, A., et al EMBO J. 8(7):1987-1991 (1989)
Clackson et al (1991) Nature, 352:624-628
Clark H.F., et al Genome Res. 13, 2265-2270, 2003
Corey E, Quinn JE, Buhler KR, et al. LuCap35: a new model of prostate cancer progression to androgen independence. The Prostate 2003;55:239-46
Coussens L., et al Science (1985) 230(4730):1132-1139
Cree et al (1995) AntiCancer Drugs 6:398-404
Crouch et al (1993) J. Immunol. Meth. 160:81-88
Davis et al (2001) Proc. Natl. Acad. Sci USA 98(17):9772-9777
de Groot et al (2001) J. Org. Chem. 66:8815-8830
de Groot et al (2003) Angew. Chem. Int. Ed. 42:4490-4494
Dennis et al. (2002) "Albumin Binding As A General Strategy For Improving The Pharmacokinetics Of Proteins" J Biol Chem. 277:35035-35043
Dobner et al (1992) Eur. J. Immunol. 22:2795-2799
Dornan et al (2009) Blood 114(13):2721-2729
Doronina et al (2006) Bioconj. Chem. 17:114-124
Dubowchik et al. Bioconjugate Chemistry, 2002, 13,855-869
Dubowchik, et al. (1997) Tetrahedron Letters, 38:5257-60
Dumoutier L., et al J. Immunol. 167, 3545-3549, 2001
E. Schroder and K. Lübke, The Peptides, volume 1, pp 76-136 (1965) Academic Press
Ehsani A., et al (1993) Genomics 15, 426-429
Eliel, E. and Wilen, S., "Stereochemistry of Organic Compounds", John Wiley & Sons, Inc., New York, 1994
Elshourbagy N.A., et al J. Biol. Chem. 268, 3873-3879, 1993
Erickson et al (2006) Cancer Res. 66(8): 1-8
Feild, J.A., et al (1999) Biochem. Biophys. Res. Commun. 258 (3):578-582
Fields, G. and Noble, R. (1990) "Solid phase peptide synthesis utilizing 9-fluoroenylmethoxycarbonyl amino acids", Int. J. Peptide Protein Res. 35:161-214
Fuchs S., et al Mol. Med. 7, 115-124, 2001
Fujisaku et al (1989) J. Biol. Chem. 264 (4):2118-2125)
Gary S.C., et al Gene 256, 139-147, 2000
Gaugitsch, H.W., et al (1992) J. Biol. Chem. 267 (16):11267-11273)
Geiser et al "Automation of solid-phase peptide synthesis" in Macromolecular Sequencing and Synthesis, Alan R. Liss, Inc., 1988, pp. 199-218
Genome Res. 13 (10):2265-2270 (2003)
Genomics 62 (2):281-284 (1999)
Geoghegan & Stroh, (1992) Bioconjugate Chem. 3:138-146
Getz et al (1999) Anal. Biochem. Vol 273:73-80
Glynne-Jones et al (2001) Int J Cancer. Oct 15; 94(2):178-84
Gregson et al., Chem. Commun. 1999, 797-798
Gregson et al., J. Med. Chem. 2001, 44, 1161-1174
Gu Z., et al Oncogene 19, 1288-1296, 2000
Ha et al (1992) J. Immunol. 148(5):1526-1531
Haendler B., et al J. Cardiovasc. Pharmacol. 20, s1-S4, 1992
Hamann P. (2005) Expert Opin. Ther. Patents 15(9):1087-1103
Hamblett et al (2004) Clin. Cancer Res. 10:7063-7070
Handbook of Pharmaceutical Additives, 2nd Edition (eds. M. Ash and I. Ash), 2001 (Synapse Information Resources, Inc., Endicott, New York, USA)
Handbook of Pharmaceutical Excipients, 2nd edition, 1994
Hara, et al., J. Antibiotics, 41, 702-704 (1988)
Hashimoto et al (1994) Immunogenetics 40(4):287-295
Hay et al. (1999) Bioorg. Med. Chem. Lett. 9:2237
Herdwijn, P. et al., Canadian Journal of Chemistry. 1982, 60, 2903-7
Hermanson, G.T. (1996) Bioconjugate Techniques; Academic Press: New York, p 234-242
Hochlowski, et al., J. Antibiotics, 40, 145-148 (1987)
Hofstra R.M.W., et al Eur. J. Hum. Genet. 5, 180-185, 1997
Hofstra R.M.W., et al Nat. Genet. 12, 445-447, 1996
Horie et al (2000) Genomics 67:146-152
Hubert, R.S., et al (1999) Proc. Natl. Acad. Sci. U.S.A. 96 (25):14523-14528)
Hurley and Needham-VanDevanter, Acc. Chem. Res., 19, 230-237 (1986) Immunogenetics 54 (2):87-95 (2002)
Int. Rev. Cytol. 196:177-244 (2000)
Itoh, et al., J. Antibiotics, 41, 1281-1284 (1988)
J. Biol. Chem. 270 (37):21984-21990 (1995)
J. Biol. Chem. 276 (29):27371-27375 (2001)
J. Biol. Chem. 277 (22):19665-19672 (2002)
J. Biol. Chem. 278 (33):30813-30820 (2003)
Janeway, C., Travers, P., Walport, M., Shlomchik (2001) Immuno Biology, 5th Ed., Garland Publishing, New York
Jeffrey et al (2005) J. Med. Chem. 48:1344-1358
Jonsson et al (1989) Immunogenetics 29(6):411-413
Junutula, et al., 2008b Nature Biotech., 26(8):925-932
Kang, G-D., et al., Chem. Commun., 2003, 1680-1689
Kasahara et al (1989) Immunogenetics 30(1):66-68
King et al (2002) Tetrahedron Letters 43:1987-1990
Kingsbury et al (1984) J. Med. Chem. 27:1447
Kohler et al (1975) Nature 256:495
Kohn, in Antibiotics III. Springer-Verlag, New York, pp. 3-11 (1975).
Konishi, et al., J. Antibiotics, 37, 200-206 (1984)
Kovtun et al (2006) Cancer Res. 66(6):3214-3121
Kuhns J.J., et al J. Biol. Chem. 274, 36422-36427, 1999
Kuminoto, et al., J. Antibiotics, 33, 665-667 (1980)
Kurebayashi et al (1999) Brit. Jour. Cancer 79(5-6):707-717
Lab. Invest. 82 (11):1573-1582 (2002)
Lambert J. (2005) Current Opin. in Pharmacol. 5:543-549
Langley and Thurston, J. Org. Chem., 52, 91-97 (1987)
Larhammar et al (1985) J. Biol. Chem. 260(26):14111-14119
Law et al (2006) Cancer Res. 66(4):2328-2337
Le et al (1997) FEBS Lett. 418(1-2):195-199
Leber, et al., J. Am. Chem. Soc., 110, 2992-2993 (1988)
Leimgruber, et al., J. Am. Chem. Soc., 87, 5791-5793 (1965)
Leimgruber, et al., J. Am. Chem. Soc., 87, 5793-5795 (1965)
Levenson et al (1997) Cancer Res. 57(15):3071-3078
Liang et al (2000) Cancer Res. 60:4907-12
Manfré, F. et al., J. Org. Chem. 1992, 57, 2060-2065
Marks et al (1991) J. Mol. Biol., 222:581-597
McDonagh (2006) Protein Eng. Design & Sel., 19(7): 299-307
Mendoza et al (2002) Cancer Res. 62:5485-5488
Miller et al (2003) Jour. of Immunology 170:4854-4861
Miura et al (1996) Genomics 38(3):299-304
Miura et al (1998) Blood 92:2815-2822
Moore M., et al Proc. Natl. Acad. Sci. U.S.A. 84, 9194-9198, 1987
Morrison et al (1984) Proc. Natl. Acad. Sci. USA, 81:6851-6855
Muller et al (1992) Eur. J. Immunol. 22 (6):1621-1625
Mungall A.J., et al Nature 425, 805-811, 2003
Nagase T., et al (2000) DNA Res. 7 (2):143-150)
Nakamuta M., et al Biochem. Biophys. Res. Commun. 177, 34-39, 1991
Nakayama et al (2000) Biochem. Biophys. Res. Commun. 277(1):124-127
Naruse et al (2002) Tissue Antigens 59:512-519
Nature 395 (6699):288-291 (1998)
Neuberger and Williams (1988) Nucleic Acids Res. 16:6713
Novabiochem Catalog 2006/2007
Ogawa Y., et al Biochem. Biophys. Res. Commun. 178, 248-255, 1991
Okamoto Y., et al Biol. Chem. 272, 21589-21596, 1997
Oncogene 10 (5):897-905 (1995)
Oncogene 14(11):1377-1382 (1997))
Parrish-Novak J., et al J. Biol. Chem. 277, 47517-47523, 2002
Payne, G. (2003) Cancer Cell 3:207-212
Phillips et al (2008) Cancer Res. 68(22):9280-9290
Pingault V., et al (2002) Hum. Genet. 111, 198-206
Pletnev S., et al (2003) Biochemistry 42:12617-12624
Preud'homme et al (1992) Clin. Exp. Immunol. 90(1):141-146
Proc. Natl. Acad. Sci. U.S.A. (2003) 100 (7):4126-4131
Proc. Natl. Acad. Sci. U.S.A. 93 (1):136-140 (1996)
Proc. Natl. Acad. Sci. U.S.A. 98 (17):9772-9777 (2001)
Proc. Natl. Acad. Sci. U.S.A. 99 (26):16899-16903 (2002)
Proc.Natl. Acad. Sci. U.S.A. 96 (20):11531-11536 (1999) Protective Groups in Organic Synthesis, Greene and Wuts, 3rd Edition, 1999, John Wiley & Sons Inc.
Puffenberger E.G., et al Cell 79, 1257-1266, 1994
Rao et al (1997) Breast Cancer Res. and Treatment 45:149-158
Reiter R.E., et al Proc. Natl. Acad. Sci. U.S.A. 95, 1735-1740, 1998 Remington's Pharmaceutical Sciences, 20th edition, pub. Lippincott, Williams & Wilkins, 2000
Rodrigues et al (1995) Chemistry Biology 2:223
Ross et al (2002) Cancer Res. 62:2546-2553
S. P. Parker, Ed., McGraw-Hill Dictionary of Chemical Terms (1984) McGraw-Hill Book Company, New York
Sakaguchi et al (1988) EMBO J. 7(11):3457-3464
Sakamoto A., Yanagisawa M., et al Biochem. Biophys. Res. Commun. 178, 656-663, 1991
Sanderson et al (2005) Clin. Cancer Res. 11:843-852
Semba K., et al Proc. Natl. Acad. Sci. U.S.A. 82, 6497-6501, 1985
Servenius et al (1987) J. Biol. Chem. 262:8759-8766
Shamis et al (2004) J. Am. Chem. Soc. 126:1726-1731
Sheikh F., et al (2004) J. Immunol. 172, 2006-2010
Shimizu, et al, J. Antibiotics, 29, 2492-2503 (1982)
Sinha S.K., et al (1993) J. Immunol. 150, 5311-5320
Storm et al (1972) J. Amer. Chem. Soc. 94:5815
Strausberg et al (2002) Proc. Natl. Acad. Sci USA 99:16899-16903
Sun et al (2002) Bioorganic & Medicinal Chemistry Letters 12:2213-2215
Sun et al (2003) Bioorganic & Medicinal Chemistry 11:1761-1768
Svensson P.J., et al Hum. Genet. 103, 145-148, 1998
Swiercz J.M., et al J. Cell Biol. 165, 869-880, 2004
Syrigos and Epenetos (1999) Anticancer Research 19:605-614
Takeuchi, et al., J. Antibiotics, 29, 93-96 (1976)
Tawaragi Y., et al Biochem. Biophys. Res. Commun. 150, 89-96, 1988
ten Dijke,P., et al Science 264 (5155):101-104 (1994)
Thompson, J.S., et al Science 293 (5537), 2108-2111 (2001) WO 2004/058309
Thurston, et al., Chem. Brit., 26, 767-772 (1990)
Thurston, et al., Chem. Rev. 1994, 433-465 (1994)
Toki et al (2002) J. Org. Chem. 67:1866-1872
Tonnelle et al (1985) EMBO J. 4(11):2839-2847
Touchman et al (2000) Genome Res. 10:165-173
Trail et al (2003) Cancer Immunol. Immunother. 52:328-337
Tsunakawa, et al., J. Antibiotics, 41, 1366-1373 (1988)
Tsutsumi M., et al Gene 228, 43-49, 1999
Uchida et al (1999) Biochem. Biophys. Res. Commun. 266:593-602
Verheij J.B., et al Am. J. Med. Genet. 108, 223-225, 2002
Von Hoegen et al (1990) J. Immunol. 144(12):4870-4877
Webster et al (1994) Semin. Cancer Biol. 5:69-76
Weis J.J., et al J. Exp. Med. 167, 1047-1066, 1988
Weis J.J., et al Proc. Natl. Acad. Sci. U.S.A. 83, 5639-5643, 1986
Wilson et al (1991) J. Exp. Med. 173:137-146
Wu et al (2005) Nature Biotech. 23(9):1137-1145
Xie et al (2006) Expert. Opin. Biol. Ther. 6(3):281-291
Xu, M.J., et al (2001) Biochem. Biophys. Res. Commun. 280 (3):768-775 WO 2004/016225
Xu, X.Z., et al Proc. Natl. Acad. Sci. U.S.A. 98 (19):10692-10697 (2001)
Yamaguchi, N., et al Biol. Chem. 269 (2), 805-808 (1994)
Yamamoto T., et al Nature 319, 230-234, 1986
Yu et al (1992) J. Immunol. 148(2) 633-637

## Claims

1. A conjugate of formula (A): 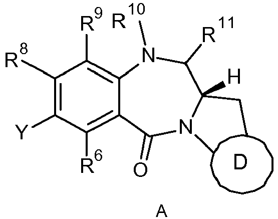 and salts and solvates thereof, wherein:
D represents either group D1 or D2: 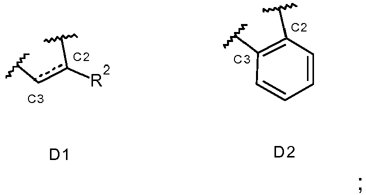 the dotted line indicates the optional presence of a double bond between C2 and C3; when there is a double bond present between C2 and C3, R² is selected from the group consisting of:
(ia) C₅₋₁₀ aryl group, optionally substituted by one or more substituents selected from the group comprising: halo, nitro, cyano, ether, carboxy, ester, C₁₋₇ alkyl, C₃₋₇ heterocyclyl and bis-oxy-C₁₋₃ alkylene;
(ib) C₁₋₅ saturated aliphatic alkyl;
(ic) C₃₋₆ saturated cycloalkyl;
(id) 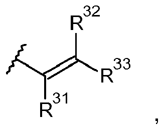 wherein each of R³¹, R³² and R³³ are independently selected from H, C₁₋₃ saturated alkyl, C₂₋₃ alkenyl, C₂₋₃ alkynyl and cyclopropyl, where the total number of carbon atoms in the R² group is no more than 5;
(ie) 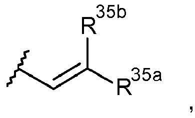 wherein one of R^{35a} and R^{35b} is H and the other is selected from: phenyl, which phenyl is optionally substituted by a group selected from halo, methyl, methoxy; pyridyl; and thiophenyl; and
(if) 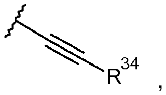 where R³⁴ is selected from: H; C₁₋₃ saturated alkyl; C₂₋₃ alkenyl; C₂₋₃ alkynyl; cyclopropyl; phenyl, which phenyl is optionally substituted by a group selected from halo, methyl, methoxy; pyridyl; and thiophenyl;
(ig) halo;
when there is a single bond present between C2 and C3,
R² is 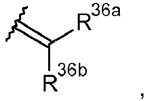 where R^{36a} and R^{36b} are independently selected from H, F, C₁₋₄ saturated alkyl, C₂₋₃ alkenyl, which alkyl and alkenyl groups are optionally substituted by a group selected from C₁₋₄ alkyl amido and C₁₋₄ alkyl ester; or, when one of R^{36a} and R^{36b} is H, the other is selected from nitrile and a C₁₋₄ alkyl ester;
R⁶ and R⁹ are independently selected from H, R, OH, OR, SH, SR, NH₂, NHR, NRR', NO₂, SnMe₃ and halo;
either
(a) R¹⁰ is H, and R¹¹ is OH or OR^{A}, where R^{A} is C₁₋₄ alkyl; or
(b) R¹⁰ and R¹¹ form a nitrogen-carbon double bond between the nitrogen and carbon atoms to which they are bound; or
(c) R¹⁰ is H and R¹¹ is OSO_{z}M, where z is 2 or 3 and M is a monovalent pharmaceutically acceptable cation; or
(d) R¹¹ is OH or OR^{A}, where R^{A} is C₁₋₄ alkyl and R¹⁰ is selected from:
(d-i) 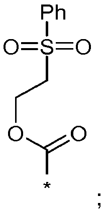
(d-ii) 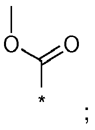
(d-iii) 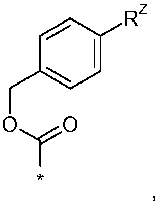
where R^{Z} is selected from:
(z-i) 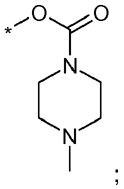
(z-ii) OC(=O)CH₃;
(z-iii) NO₂;
(z-iv) OMe;
(z-v) glucoronide;
(z-vi) -C(=O)-X₁-NHC(=O)X₂-NH-R^{ZC}, where -C(=O)-X₁-NH- and-C(=O)-X₂-NH- represent natural amino acid residues and R^{ZC} is selected from Me, OMe, OCH₂CH₂OMe;
Y is selected from formulae A1 and A2: 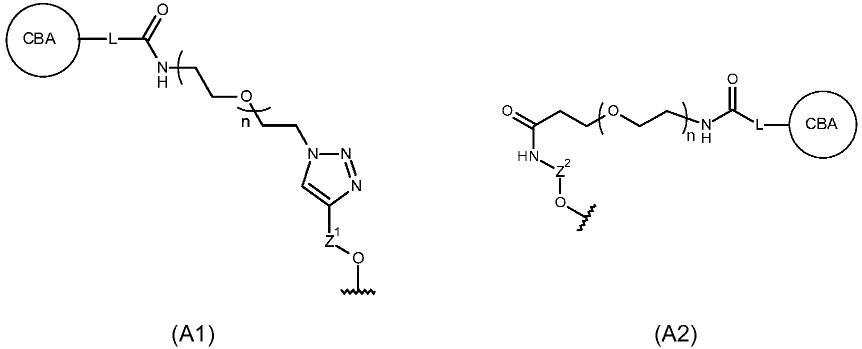
Z¹ is a C₁₋₃ alkylene group;
Z² is a C₁₋₃ alkylene group;
L is a linker connected to a cell binding agent;
CBA is the cell binding agent;
n is an integer between 0 and 48;
R and R' are each independently selected from optionally substituted C₁₋₁₂ alkyl, C₃₋₂₀ heterocyclyl and C₅₋₂₀ aryl groups, and optionally in relation to the group NRR', R and
R' together with the nitrogen atom to which they are attached form an optionally substituted 4-, 5-, 6- or 7-membered heterocyclic ring;
R⁸ is either:
(a) independently selected from H, R, OH, OR, SH, SR, NH₂, NHR, NRR', NO₂, SnMe₃ and halo; or
(b) of formula A*: 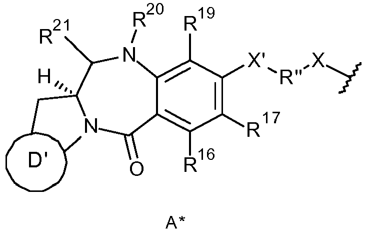 wherein:
D' represents either group D'1 or D'2: 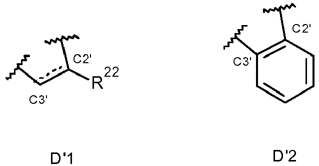 wherein the dotted line indicates the optional presence of a double bond between C2' and C3';
R¹⁷ is independently selected from H, R, OH, OR, SH, SR, NH₂, NHR, NRR', NO₂, SnMe₃ and halo;
R" is a C₃₋₁₂ alkylene group, which chain may be interrupted by one or more heteroatoms, e.g. O, S, N(H), NMe and/or aromatic rings, e.g. benzene or pyridine, which rings are optionally substituted; and
X and X' are independently selected from O, S and N(H); and
R²², R¹⁶, R¹⁹, R²⁰ and R²¹ are as defined for R², R⁶, R⁹, R¹⁰ and R¹¹ respectively.

2. The conjugate according to claim 1, wherein R⁶ and R⁹ are both H.

3. The conjugate according to either claim 1 or claim 2, wherein D is D1, there is a double bond between C2 and C3, and R² is a phenyl, which may bear one to three substituent groups selected from methoxy, ethoxy, fluoro, chloro, cyano, bis-oxy-methylene, methyl-piperazinyl, morpholino and methyl-thiophenyl.

4. The conjugate according to either claim 1 or claim 2, wherein D is D1, there is a double bond between C2 and C3, and R² is selected from:
(a) methyl, ethyl or propyl;
(b) cyclopropyl;
(c) a group of formula: 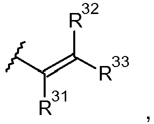 wherein the total number of carbon atoms in the R² group is no more than 3;
(d) the group: 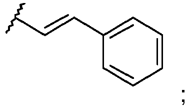
(e) a group of formula: 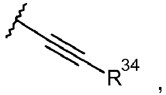 wherein R³⁴ is selected from H and methyl.

5. The conjugate according to either claim 1 or claim 2, wherein D is D1, there is a single bond between C2 and C3, and R² is 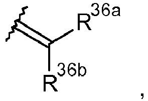 wherein:
(a) R^{36a} and R^{36b} are both H;
(b) R^{36a} and R^{36b} are both methyl;
(c) one of R^{36a} and R^{36b} is H, and the other is selected from methyl and ethyl.

6. The conjugate according to any one of claims 1 to 5, wherein R¹⁰ is H, and R¹¹ is OH.

7. The conjugate according to any one of claims 1 to 6, wherein R¹⁰ and R¹¹ form a nitrogen-carbon double bond between the nitrogen and carbon atoms to which they are bound.

8. The conjugate according to any one of claims 1 to 7, wherein R⁸ is OR^{8A}, where R^{8A} is Me.

9. The conjugate according to any one of claims 1 to 7, wherein R⁸ is of formula A*, X and X' are O, R" is C₃₋₇ alkylene and R¹⁷ is OR^{17A}, where R^{17A} is Me.

10. The conjugate according to claims 9, wherein R¹⁶, R¹⁹, R²⁰, R²¹ and D' are the same as R⁶, R⁹, R¹⁰, R¹¹ and D respectively.

11. The conjugate according to any one of claims 1 to 10, wherein L is of formula:
-L^{A}-(CH₂)ₘ-
where m is from 0 to 6; and
L^{A} is selected from:
| | | | |
|---|---|---|---|
| (L^{A1-1}) | 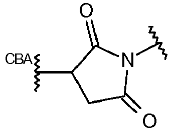 | (L^{A6}) | 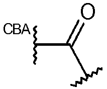 |
| (L^{A1-2}) | 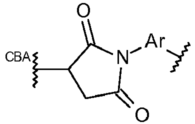 | (L^{A7}) | 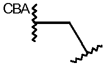 |
| (L^{A2}) | 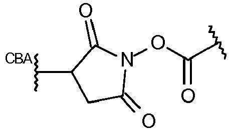 | (L^{A8-1}) | 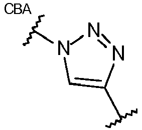 |
| (L^{A3-1}) | 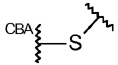 | (L^{A8-2}) | 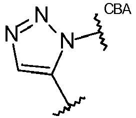 |
| (L^{A3-2}) | 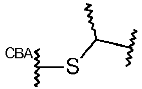 | (L^{A9-1}) | 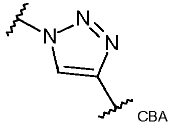 |
| (L^{A4}) | 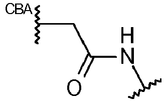 | (L^{A9-2}) | 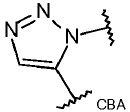 |
| (L^{A5}) | 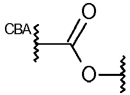 | | |
where Ar represents a C₅₋₆ arylene group.

12. The conjugate according to any one of claims 1 to 11, wherein the cell binding agent is an antibody or an active fragment thereof, wherein the antibody or antibody fragment is an antibody or antibody fragment for a tumour-associated antigen.

13. The conjugate of claim 12 wherein the antibody or antibody fragment is an antibody which binds to one or more tumor-associated antigens or cell-surface receptors selected from (1)-(88):
**(1)** BMPR1B;
**(2)** E16;
**(3)** STEAP1;
**(4)** 0772P;
**(5)** MPF;
**(6)** Napi3b;
**(7)** Sema 5b;
**(8)** PSCA hlg;
**(9)** ETBR;
**(10)** MSG783;
**(11)** STEAP2;
**(12)** TrpM4;
**(13)** CRIPTO;
**(14)** CD21;
**(15)** CD79b;
**(16)** FcRH2;
**(17)** HER2;
**(18)** NCA;
**(19)** MDP;
**(20)** IL20R-alpha;
**(21)** Brevican;
**(22)** EphB2R;
**(23)** ASLG659;
**(24)** PSCA;
**(25)** GEDA;
**(26)** BAFF-R;
**(27)** CD22;
**(28)** CD79a;
**(29)** CXCR5;
**(30)** HLA-DOB;
**(31)** P2X5;
**(32)** CD72;
**(33)** LY64;
**(34)** FcRH1;
**(35)** IRTA2;
**(36)** TENB2;
**(37)** PSMA - FOLH1;
**(38)** SST;
(38.1) SSTR2;
(38.2) SSTR5;
(38.3) SSTR1;
(38.4)SSTR3;
(38.5) SSTR4;
**(39)** ITGAV;
**(40)** ITGB6;
**(41)** CEACAM5;
**(42)** MET;
**(43)** MUC1;
**(44)** CA9;
**(45)** EGFRvIII;
**(46)** CD33;
**(47)** CD19;
**(48)** IL2RA;
**(49)** AXL;
**(50)** CD30 - TNFRSF8;
**(51)** BCMA - TNFRSF17;
**(52)** CT Ags - CTA;
**(53)** CD174 (Lewis Y) - FUT3;
**(54)** CLEC14A;
**(55)** GRP78 - HSPA5;
**(56)** CD70;
**(57)** Stem Cell specific antigens;
**(58)** ASG-5;
**(59)** ENPP3;
**(60)** PRR4;
**(61)** GCC - GUCY2C;
**(62)** Liv-1 - SLC39A6;
**(63)** 5T4;
**(64)** CD56 - NCMA1;
**(65)** CanAg;
**(66)** FOLR1;
**(67)** GPNMB;
**(68)** TIM-1 - HAVCR1;
**(69)** RG-1/Prostate tumor target Mindin - Mindin/RG-1;
**(70)** B7-H4 - VTCN1;
**(71)** PTK7;
**(72)** CD37;
**(73)** CD138 - SDC1;
**(74)** CD74;
**(75)** Claudins - CLs;
**(76)** EGFR;
**(77)** Her3;
**(78)** RON - MST1R;
**(79)** EPHA2;
**(80)** CD20 - MS4A1;
**(81)** Tenascin C - TNC;
**(82)** FAP;
**(83)** DKK-1;
**(84)** CD52;
**(85)** CS1 - SLAMF7;
**(86)** Endoglin - ENG;
**(87)** Annexin A1 - ANXA1;
**(88)** V-CAM (CD106) - VCAM1.

14. The conjugate according to any one of claims 1 to 13, for use in therapy.

15. A pharmaceutical composition comprising the conjugate of any one of claims 1 to 13 a pharmaceutically acceptable diluent, carrier or excipient.

16. The conjugate according to any one of claims 1 to 13 or the pharmaceutical composition according to claim 15, for use in the treatment of cancer in a subject.

17. A compound of formula (B): 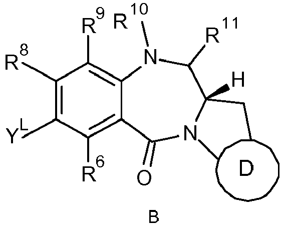 wherein:
Y^{L} is selected from formulae B1 and B2: 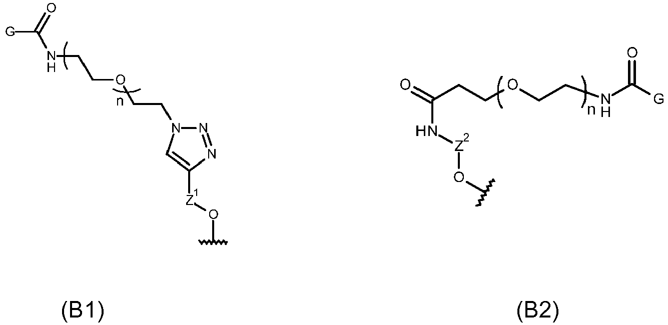
G is a linker for connecting to a cell binding agent; and
D, R⁶, R⁸, R⁹, R¹⁰, R¹¹, Z¹, Z² and n are as defined in any one of claims 1 to 11.

18. The compound according to claim 17, wherein G is of formula:
G^{A}-(CH₂)ₘ-
where m is from 0 to 6; and
G^{A} is selected from:
| | | | |
|---|---|---|---|
| (G^{A1-1}) | 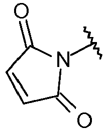 | (G^{A4}) | 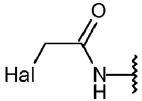 |
| | | | Where Hal = I, Br, Cl |
| (G^{A1-2}) | 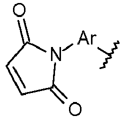 | | |
| (G^{A2}) | 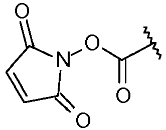 | (G^{A5}) | 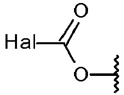 |
| (G^{A3-1}) | 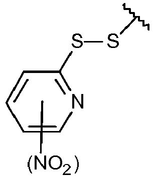 | (G^{A6}) | 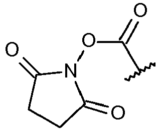 |
| | where the NO₂ group is optional | | |
| (G^{A3-2}) | 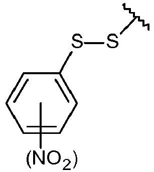 | (G^{A7}) | 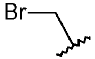 |
| | where the NO₂ group is optional | | |
| (G^{A3-3}) | 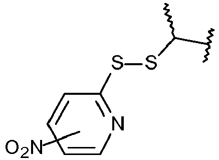 | (G^{A8}) | 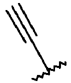 |
| | where the NO₂ group is optional | | |
| (G^{A3-4}) | 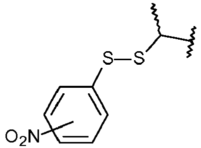 | (G^{A9}) | 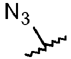 |
| | where the NO₂ group is optional | | |
where Ar represents a C₅₋₆ arylene group.

19. A compound of formula (C): 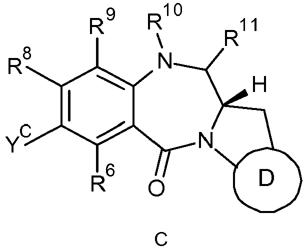
wherein Y^{C} is selected from from formulae C1 and C2: 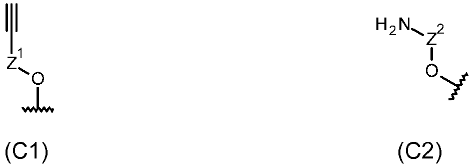 and
D, R⁶, R⁸, R⁹, R¹⁰, R¹¹, Z¹ and Z² are as defined in any one of claims 1 to 11.

## Patentansprüche

1. Konjugat der Formel (A): 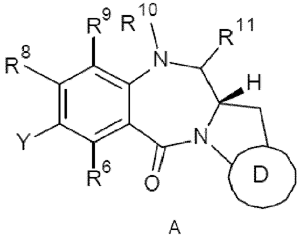 und Salze und Solvate davon, worin:
D entweder für eine Gruppe D1 oder D2 steht: 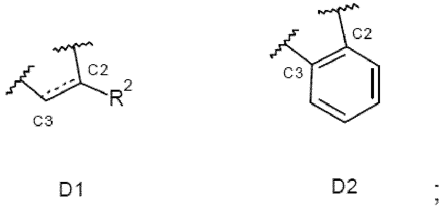 wobei die strichlierte Linie das optionale Vorliegen einer Doppelbindung zwischen C2 und C3 anzeigt;
wenn eine Doppelbindung zwischen C2 und C3 vorliegt, R² aus folgender Gruppe ausgewählt ist:
(ia) C₅₋₁₀-Arylgruppe, die gegebenenfalls mit einem oder mehreren Substituenten substituiert ist, die aus folgender Gruppe ausgewählt sind: Halogen, Nitro, Cyano, Ether, Carboxy, Ester, C₁₋₇-Alkyl, C₃₋₇-Heterocyclyl und Bis-oxy-C₁₋₃-alkylen;
(ib) gesättigtes aliphatisches C₁₋₅-Alkyl;
(ic) gesättigtes C₃₋₆-Cycloalkyl;
(id) 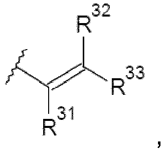 wobei R³¹, R³² und R³³ jeweils unabhängig aus H, gesättigtem C₁₋₃-Alkyl, C₂₋₃-Alkenyl, C₂₋₃-Alkinyl und Cyclopropyl ausgewählt sind, wobei die Gesamtanzahl an Kohlenstoffatomen in der Gruppe R² nicht mehr als 5 beträgt;
(ie) 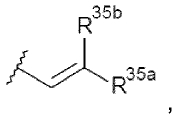 wobei einer von R^{35a} und R^{35b} H ist und der andere aus Phenyl, wobei das Phenyl gegebenenfalls mit einer aus Halogen, Methyl, Methoxy ausgewählten Gruppe substituiert ist; Pyridyl; und Thiophenyl ausgewählt ist; und
(if) 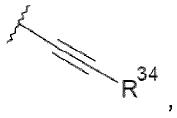 wobei R³⁴ aus H; gesättigtem C₁₋₃-Alkyl; C₂₋₃-Alkenyl; C₂₋₃-Alkinyl, Cyclopropyl; Phenyl, wobei das Phenyl gegebenenfalls mit einer aus Halogen, Methyl und Methoxy ausgewählten Gruppe substituiert ist; Pyridyl; und Thiophenyl ausgewählt ist;
(ig) Halogen;
wenn eine Einfachbindung zwischen C2 und C3 vorliegt,
R² 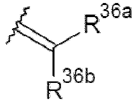 ist, worin R^{36a} und R^{36b} unabhängig voneinander aus H, F, gesättigtem C₁₋₄-Alkyl, C₂₋₃-Alkenyl, wobei Alkyl- und Alkenylgruppen gegebenenfalls mit einer Gruppe substituiert sind, die aus C₁₋₄-Alkylamido und C₁₋₄-Alkylester ausgewählt ist; oder, wenn eines aus R^{36a} und R^{36b} H ist, das andere aus Nitril und einem C₁₋₄-Alkylester ausgewählt ist;
R⁶ und R⁹ unabhängig aus H, R, OH, OR, SH, SR, NH₂, NR, NRR', NO₂, SnMe₃ und Halogen ausgewählt sind;
entweder
(a) R¹⁰ H ist und R¹¹ OH oder OR^{A} ist, worin A C₁₋₄-Alkyl ist; oder
(b) R¹⁰ und R¹¹ eine Stickstoff-Kohlenstoff-Doppelbindung zwischen dem Stickstoff- und dem Kohlenstoffatom bilden, an die sie gebunden sind; oder
(c) R¹⁰ H ist und R¹¹ OSO_{z}M ist, worin z = 2 oder 3 ist und M ein einwertiges, pharmazeutisch annehmbares Kation ist; oder
(d) R¹¹ OH oder OR^{A} ist, worin R^{A} C₁₋₄-Alkyl ist und R¹⁰ ausgewählt ist aus:
(d-i) 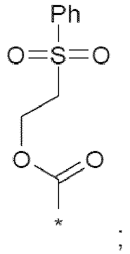
(d-ii) 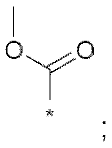
(d-iii) 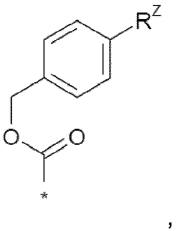
worin R^{z} ausgewählt ist aus:
(z-i) 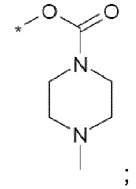
(z-ii) OC(=O)CH₃;
(z-iii) NO₂;
(z-iv) OMe;
(z-v) Glucuronid;
(z-vi) -C(=O)-X₁-NHC(=O)X₂-NH-R^{ZC}, worin -C(=O)-X₁-NH- und -C(=O)-X₂-NH- für natürliche Aminosäurereste stehen und R^{ZC} aus Me, OMe und OCH₂CH₂OMe ausgewählt ist;
Y aus Formel A1 und A2 ausgewählt ist: 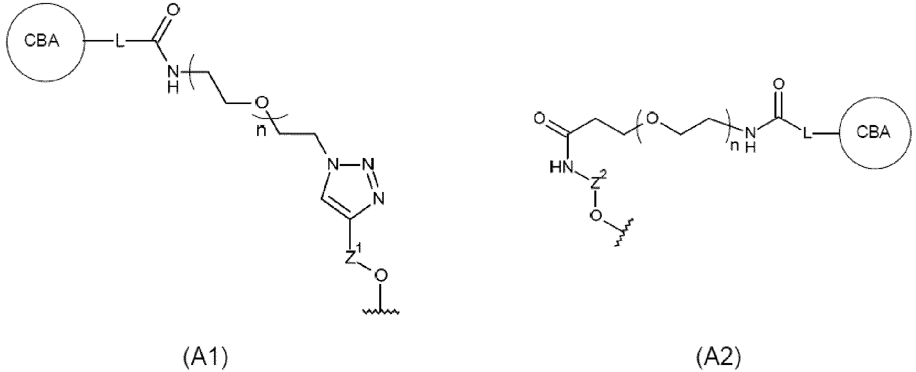
Z¹ eine C₁₋₃-Alkylengruppe ist;
Z² eine C₁₋₃-Alkylengruppe ist;
L ein Linker ist, der mit einem Zellbindungsmittel verbunden ist;
CBA das Zellbindungsmittel ist;
n eine ganze Zahl zwischen 0 und 48 ist;
R und R' jeweils unabhängig voneinander aus gegebenenfalls substituiertem C₁₋₁₂-Alkyl-, C₃₋₂₀-Heterocyclyl- und C₅₋₂₀-Arylgruppen ausgewählt sind und gegebenenfalls in Bezug auf die Gruppe NRR', R und R' zusammen mit dem Stickstoffatom, an das sie gebunden sind, einen gegebenenfalls substituierten, 4-, 5-, 6- oder 7-gliedrigen heterocyclischen Ring bilden;
R⁸ entweder:
(a) unabhängig ausgewählt ist aus H, R, OH, OR, SH, SR, NH₂, NHR, NRR', NO₂, SnMe₃ und Halogen; oder
(b) die Formel A' aufweist: 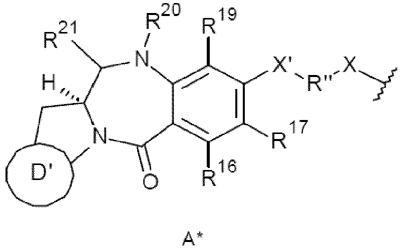 worin:
D' für Gruppe D'1 oder D'2 steht: 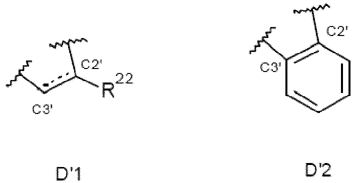 worin die strichlierte Linie das optionale Vorliegen einer Doppelbindung zwischen C2' und C3' anzeigt;
R¹⁷ unabhängig ausgewählt ist aus H, R, OH, OR, SH, SR, NH₂, NHR, NRR', NO₂, SnMe₃ und Halogen;
R" eine C₃₋₁₂-Alkylengruppe ist, deren Kette gegebenenfalls durch ein oder mehrere Heteroatome, z. B. O, S N(H), NMe und/oder aromatische Ringe, z. B. Benzol oder Pyridin, wobei die Ringe gegebenenfalls substituiert sind, unterbrochen ist; und
X und X' unabhängig voneinander aus O, S und N(H) ausgewählt sind; und
R²², R¹⁶, R¹⁹, R²⁰ und R²¹ wie für R², R⁶, R⁹, R¹⁰ bzw. R¹¹ definiert sind.

2. Konjugat nach Anspruch 1, wobei R⁶ und R⁹ beide H sind.

3. Konjugat nach Anspruch 1 oder Anspruch 2, wobei D D1 ist, eine Doppelbindung zwischen C2 und C3 vorliegt und R² Phenyl ist, das gegebenenfalls ein bis drei Substituentengruppen aufweist, die ausgewählt sind aus Methoxy, Ethoxy, Fluor, Chlor, Cyano, Bisoxymethylen, Methylpiperazin, Morpholino und Methylthiopenyl.

4. Konjugat nach Anspruch 1 oder Anspruch 2, wobei D D1 ist, eine Doppelbindung zwischen C2 und C3 vorliegt und R² ausgewählt ist aus:
(a) Methyl, Ethyl oder Propyl;
(b) Cyclopropyl;
(c) einer Gruppe der Formel 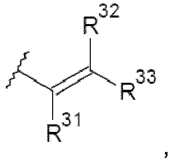 worin die Gesamtanzahl an Kohlenstoffatomen in der Gruppe R² nicht mehr als 3 beträgt;
(d) der Gruppe 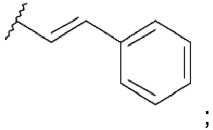
(e) einer Gruppe der Formel: 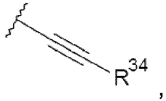 worin R³⁴ aus H und Methyl ausgewählt ist.

5. Konjugat nach Anspruch 1 oder Anspruch 2, wobei D D1 ist, eine Einfachbindung zwischen C2 und C3 vorliegt und R² 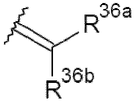 ist, worin:
(a) R^{36a} und R^{36b} beide H sind;
(b) R^{36a} und R^{36b} beide Methyl sind; oder
(c) einer von R^{36a} und R^{36b} H ist und der andere aus Methyl und Ethyl ausgewählt ist.

6. Konjugat nach einem der Ansprüche 1 bis 5, wobei R¹⁰ H ist, und R¹¹ OH ist.

7. Konjugat nach einem der Ansprüche 1 bis 6, wobei R¹⁰ und R¹¹ eine Stickstoff-Kohlenstoff-Doppelbindung zwischen dem Stickstoff und dem Kohlenstoff bilden, an die sie gebunden sind.

8. Konjugat nach einem der Ansprüche 1 bis 7, wobei R⁸ OR^{8A} ist, worin R^{8A} Me ist.

9. Konjugat nach einem der Ansprüche 1 bis 7, wobei R⁸ die Formel A* aufweist, X und X' O sind, R" C₃₋₇-Alkylen ist und R¹⁷ OR^{17A} ist, worin R^{17A} Me ist.

10. Konjugat nach Anspruch 9, wobei R¹⁶, R¹⁹, R²⁰, R²¹ und D' gleich sind wie R⁶, R⁹, R¹⁰, R¹¹ bzw. D.

11. Konjugat nach einem der Ansprüche 1 bis 10, wobei L die Formel -L^{A}-(CH₂)ₘ aufweist,
worin m = 0 bis 6 ist; und
L^{A} ausgewählt ist aus:
| | | | |
|---|---|---|---|
| (L^{A1-1}) | 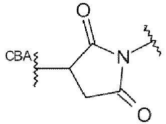 | (L^{A6}) | 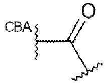 |
| (L^{A1-2}) | 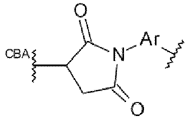 | (L^{A7}) | 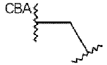 |
| (L^{A2}) | 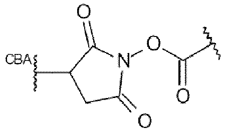 | (L^{A8-1}) | 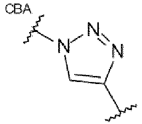 |
| (L^{A3-1}) | 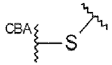 | (L^{A8-2}) | 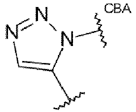 |
| (L^{A3-2}) | 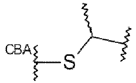 | (L^{A9-1}) | 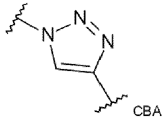 |
| (L^{A4}) | 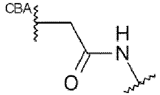 | (L^{A9-2}) | 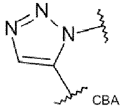 |
| (L^{A5}) | 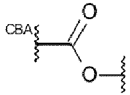 | | |
worin Ar für eine C₅₋₆-Arylengruppe steht.

12. Konjugat nach einem der Ansprüche 1 bis 11, wobei das Zellbindungsmittel ein Antikörper oder ein aktives Fragment davon ist, wobei der Antikörper oder das Antikörperfragment ein Antikörper oder ein Antikörperfragment für ein tumorassoziiertes Antigen ist.

13. Konjugat nach Anspruch 12, wobei der Antikörper oder das Antikörperfragment ein Antikörper ist, der an ein oder mehrere tumorassoziierte Antigene oder Zelloberflächenrezeptoren bindet, die aus (1)-(88) ausgewählt sind:
(1) BMPR1B;
(2) E16;
(3) STEAP1;
(4) 0772P;
(5) MPF;
(6) Napi3b;
(7) Sema 5b;
(8) PSCA hlg;
(9) ETBR;
(10) MSG783;
(11) STEAP2;
(12) TrpM4;
(13) CRIPTO;
(14) CD21;
(15) CD79b;
(16) FcRH2;
(17) HER2;
(18) NCA;
(19) MDP;
(20) IL20R-alpha;
(21) Brevican;
(22) EphB2R;
(23) ASLG659;
(24) PSCA;
(25) GEDA;
(26) BAFF-R;
(27) CD22;
(28) CD79a;
(29) CXCR5;
(30) H LA-DOB;
(31) P2X5;
(32) CD72;
(33) LY64;
(34) FcRH1;
(35) IRTA2;
(36) TENB2;
(37) PSMA - FOLH1;
(38) SST;
(38.1) SSTR2;
(38.2) SSTR5;
(38.3) SSTR1;
(38.4) SSTR3;
(38.5) SSTR4;
(39) ITGAV;
(40) ITGB6;
(41) CEACAM5;
(42) MET;
(43) MUC1;
(44) CA9;
(45) EGFRvIII;
(46) CD33;
(47) CD19;
(48) IL2RA;
(49) AXL;
(50) CD30 - TNFRSF8;
(51) BCMA - TNFRSF17;
(52) CT Ags - CTA;
(53) CD 174 (Lewis Y) - FUT3;
(54) CLEC14A;
(55) GRP78 - HSPA5;
(56) CD70;
(57) Stammzellen-spezifische Antigene;
(58) ASG-5;
(59) ENPP3;
(60) PRR4;
(61) GCC - GUCY2C;
(62) Liv-1 - SLC39A6;
(63) 5T4;
(64) CD56- NCMA1;
(65) CanAg;
(66) FOLR1;
(67) GPNMB;
(68) TIM-1 - HAVCR1;
(69) RG-1/Prostatatumor-Target Mindin - Mindin/RG-1;
(70) B7-H4 - VTCN1;
(71) PTK7;
(72) CD37;
(73) CD138-SDC1;
(74) CD74;
(75) Claudine - CLs;
(76) EGFR;
(77) Her3;
(78) RON - MST1R;
(79) EPHA2;
(80) CD20 - MS4A1;
(81) Tenascin C - TNC;
(82) FAP;
(83) DKK-1;
(84) CD52;
(85) CS1 - SLAMF7;
(86) Endoglin - ENG;
(87) Annexin A1 - ANXA1;
(88) V-CAM (CD106) - VCAM1.

14. Konjugat nach einem der Ansprüche 1 bis 13 zur Verwendung in einer Therapie.

15. Pharmazeutische Zusammensetzung, die ein Konjugat nach einem der Ansprüche 1 bis 13 und einen pharmazeutisch annehmbaren Verdünner, Träger oder Exzipienten umfasst.

16. Konjugat nach einem der Ansprüche 1 bis 13 oder pharmazeutische Zusammensetzung nach Anspruch 15 zur Verwendung bei der Behandlung von Krebs in einem Individuum.

17. Verbindung der Formel (B): 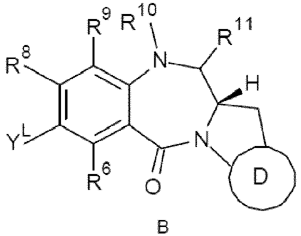 worin:
Y^{L} aus Formel B1 und B2 ausgewählt ist: 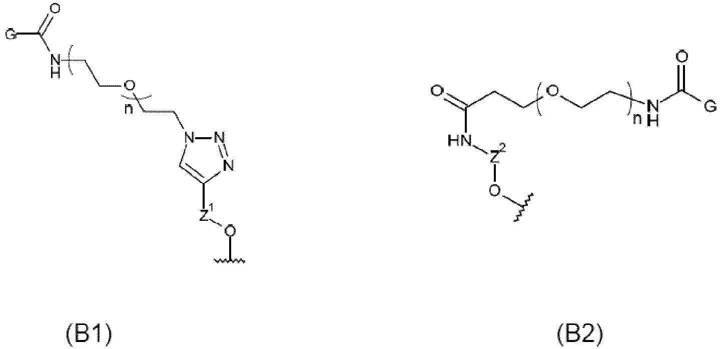
G ein Linker zur Verbindung mit einem Zellbindungsmittel ist; und
D, R⁶, R⁸, R⁹, R¹⁰, R¹¹, Z¹, Z² und n wie in einem der Ansprüche 1 bis 11 definiert sind.

18. Verbindung nach Anspruch 17, wobei G die Formel
G^{A}-(CH₂)ₘ-
aufweist, worin m = 0 bis 6 ist; und
G_{A} ausgewählt ist aus:
| | | | |
|---|---|---|---|
| (G^{A1-1}) | 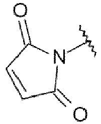 | (G^{A4}) | 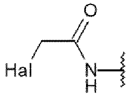 |
| | | | worin Hal = I, Br, Cl |
| (G^{A1-2}) | 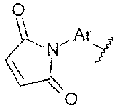 | | |
| (G^{A2}) | 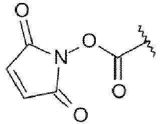 | (G^{A5}) | 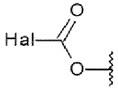 |
| (G^{A3-1}) | 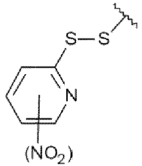 | (G^{A6}) | 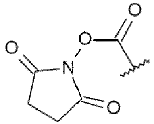 |
| | worin die NO₂-Gruppe optional ist | | |
| (G^{A3-2}) | 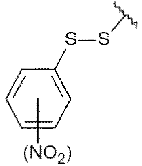 | (G^{A7}) | 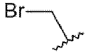 |
| | worin die NO₂-Gruppe optional ist | | |
| | | | |
| (G^{A3-3}) | 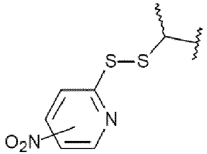 | (G^{A8}) | 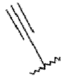 |
| | worin die NO₂-Gruppe optional ist | | |
| (G^{A3-4}) | 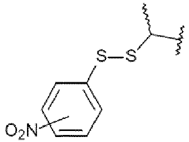 | (G^{A9}) | 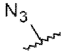 |
| | worin die NO₂-Gruppe optional ist | | |
worin Ar für eine C₅₋₆-Arylengruppe steht.

19. Verbindung der Formel (C): 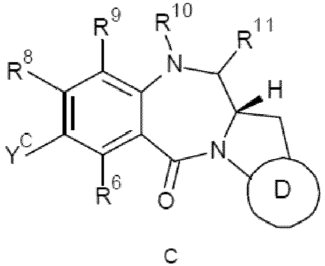
worin Y^{C} aus Formel C1 und C2 ausgewählt ist: 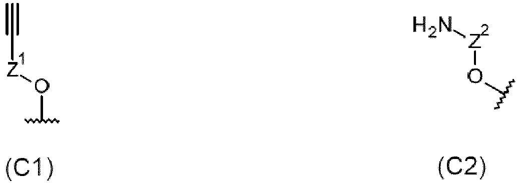 und
D, R⁶, R⁸, R⁹, R¹⁰, R¹¹, Z¹ und Z² wie in einem der Ansprüche 1 bis 11 definiert sind.

## Revendications

1. Conjugué de formule (A) : 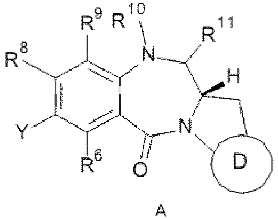 et ses sels et solvates, formule dans laquelle :
D représente le groupe soit D1 soit D2 : 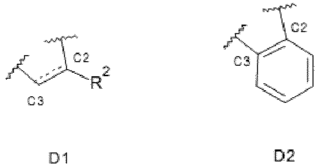 la ligne de tirets indique la présence optionnelle d'une double liaison entre C2 et C3 ;
quand il y a une double liaison présente entre C2 et C3, R² est choisi dans l'ensemble constitué par :
(ia) un groupe aryle en C₅ à C₁₀, éventuellement substitué par un ou plusieurs substituants choisis dans l'ensemble comprenant : halogéno, nitro, cyano, éther, carboxy, ester, alkyle en C₁ à C₇, hétérocyclyle en C₃ à C₇ et bis-oxy-alkylène en C₁ à C₃ ;
(ib) un alkyle aliphatique saturé en C₁ à C₅ ;
(ic) un cycloalkyle saturé en C₃ à C₆ ;
(id) 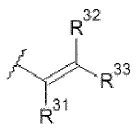 où chacun de R³¹, R³² et R³³ est indépendamment choisi parmi H, alkyle saturé en C₁ à C₃, alcényle en C₂ à C₃, alcynyle en C₂ à C₃ et cyclopropyle, le nombre total d'atomes de carbone dans le groupe R² ne dépassant pas 5 ;
(ie) 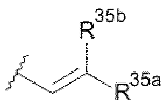 où l'un de R^{35a} et R^{35b} est H et l'autre est choisi parmi : phényle, lequel phényle est éventuellement substitué par un groupe choisi parmi halogéno, méthyle, méthoxy ; pyridyle ; et thiophényle ; et
(if) 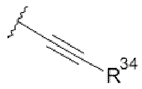 où R³⁴ est choisi parmi : H ; alkyle saturé en C₁ à C₃ ; alcényle en C₂ à C₃ ; alcynyle en C₂ à C₃ ; cyclopropyle ; phényle, lequel phényle est éventuellement substitué par un groupe choisi parmi halogéno, méthyle, méthoxy ; pyridyle ; et thiophényle ;
(ig) halogéno ;
quand il y a une liaison simple présente entre C2 et C3,
R² est 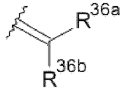 où R^{36a} et R^{36b} sont indépendamment choisis parmi H, F, alkyle saturé en C₁ à C₄, alcényle en C₂ à C₃, lesquels groupes alkyle et alcényle sont éventuellement substitués par un groupe choisi parmi alkylamido en C₁ à C₄ et ester alkylique en C₁ à C₄ ; ou bien, quand l'un de R^{36a} et R^{36b} est H, l'autre est choisi parmi nitrile et un ester alkylique en C₁ à C₄ ;
R⁶ et R⁹ sont indépendamment choisis parmi H, R, OH, OR, SH, SR, NH₂, NHR, NRR', HO₂, SnMe₃ et halogéno ;
soit
(a) R¹⁰ est H, et R¹¹ est OH ou OR^{A}, où R^{A} est un alkyle en C₁ à C₄ ; soit
(b) R¹⁰ et R¹¹ forment une double liaison azote-carbone entre les atomes d'azote et de carbone auxquels ils sont liés ; soit
(c) R¹⁰ est H et R¹¹ est OSO_{z}M, où z vaut 2 ou 3 et M est un cation monovalent pharmaceutiquement acceptable ; soit
(d) R¹¹ est OH ou OR^{A}, où R^{A} est un alkyle en C₁ à C₄ et R¹⁰ est choisi parmi :
(d-i) 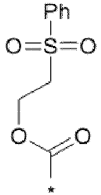
(d-ii) 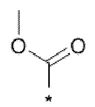
(d-iii) 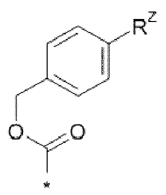
où R^{z} est choisi parmi :
(z-i) 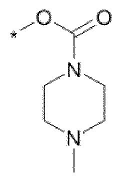
(z-ii) OC(=O)CH₃ ;
(z-iii) NO₂ ;
(z-iv) OMe ;
(z-v) glucuronide ;
(z-vi) -C(=O)-X₁-NHC(=O)X₂-NH-R^{ZC} où -C(=O)-X₁-NH- et -C(=O)-X₂-NH- représentent des résidus d'acides aminés naturels et R^{ZC} est choisi parmi Me, OMe, OCH₂CH₂OMe ;
Y est choisi parmi les formules A1 et A2 : 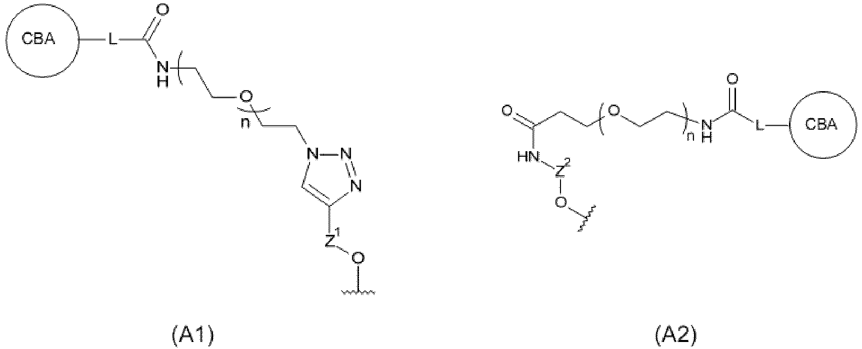
Z¹ est un groupe alkylène en C₁ à C₃ ;
Z² est un groupe alkylène en C₁ à C₃ ;
L est un lieur connecté à un agent de liaison cellulaire; CBA est l'agent de liaison cellulaire ;
n est un entier compris entre 0 et 48 ;
chacun de R et R' est indépendamment choisi parmi les groupes éventuellement substitués alkyle en C₁ à C₁₂, hétérocyclyle en C₃ à C₂₀ et aryle en C₅ à C₂₀, et éventuellement en relation avec le groupe NRR', R et R', conjointement avec l'atome d'azote auquel ils sont rattachés, forment un hétérocycle à 4, 5, 6 ou 7 chaînons éventuellement substitué ;
R⁸ est soit :
(a) indépendamment choisi parmi H, R, OH, OR, SH, SR, NH₂, NHR, NRR', NO₂, SnMe₃ et halogéno ; soit
(b) de formule A* : 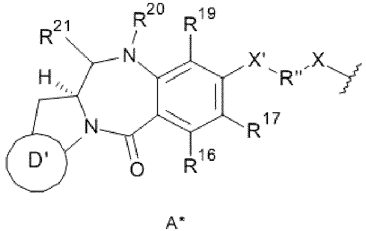 où :
D' représente le groupe soit D'1 soit D'2 : 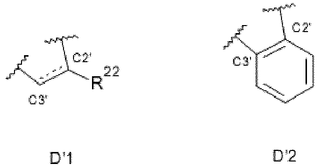
où la ligne de tirets indique la présence optionnelle d'une double liaison entre C2' et C3' ;
R¹⁷ est indépendamment choisi parmi H, R, OH, OR, SH, SR, NH₂, NHR, NRR', NO₂, SnMe₃ et halogéno ;
R" est un groupe alkylène en C₃ à C₁₂ dont la chaîne peut être interrompue par un ou plusieurs hétéroatomes, par exemple O, S, N(H), NMe et/ou des cycles aromatiques, par exemple benzène ou pyridine, lesquels cycles sont éventuellement substitués ; et
X et X' sont indépendamment choisis parmi O, S et N(H) ; et
R²², R¹⁶, R¹⁹, R²⁰ et R²¹ sont tels que définis pour R², R⁶, R⁹, R¹⁰ et R¹¹ respectivement.

2. Conjugué selon la revendication 1, dans lequel R⁶ et R⁹ sont tous deux H.

3. Conjugué selon l'une ou l'autre de la revendication 1 et de la revendication 2, dans lequel D est D1, il y a une double liaison entre C2 et C3, et R² est un phényle, qui peut porter un à trois groupes substituants choisis parmi méthoxy, éthoxy, fluoro, chloro, cyano, bis-oxy-méthylène, méthyl-pipérazinyle, morpholino et méthyl-thiophényle.

4. Conjugué selon l'une ou l'autre de la revendication 1 et de la revendication 2, dans lequel D est D1, il y a une double liaison entre C2 et C3, et R² est choisi parmi :
(a) méthyle, éthyle ou propyle ;
(b) cyclopropyle ;
(c) un groupe de formule : 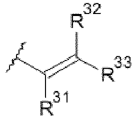 dans laquelle le nombre total d'atomes de carbone dans le groupe R² ne dépasse pas 3 ;
(d) le groupe : 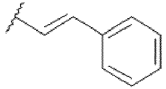
(e) un groupe de formule : 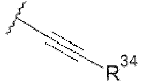 dans laquelle R³⁴ est choisi parmi H et méthyle.

5. Conjugué selon l'une ou l'autre de la revendication 1 et de la revendication 2, dans lequel D est D1, il y a une liaison simple entre C2 et C3, et R² est 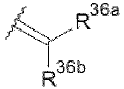 où :
(a) R^{36a} et R^{36b} sont tous deux H ;
(b) R^{36a} et R^{36b} sont tous deux méthyle ;
(c) l'un de R^{36a} et R^{36b} est H, et l'autre est choisi parmi méthyle et éthyle.

6. Conjugué selon l'une quelconque des revendications 1 à 5, dans lequel R¹⁰ est H, et R¹¹ est OH.

7. Conjugué selon l'une quelconque des revendications 1 à 6, dans lequel R¹⁰ et R¹¹ forment une double liaison azote-carbone entre les atomes d'azote et de carbone auxquels ils sont liés.

8. Conjugué selon l'une quelconque des revendications 1 à 7, dans lequel R⁸ est OR^{8A}, où R^{8A} est Me.

9. Conjugué selon l'une quelconque des revendications 1 à 7, dans lequel R⁸ est de formule A*, X et X' sont O, R" est un alkylène en C₃ à C₇ et R¹⁷ est OR^{17A}, où R^{17A} est Me.

10. Conjugué selon la revendication 9, dans lequel R¹⁶, R¹⁹, R²⁰, R²¹ et D' sont identiques à D⁶, R⁹, R¹⁰, R¹¹ et D respectivement.

11. Conjugué selon l'une quelconque des revendications 1 à 10, dans lequel L est de formule :
-L^{A}-(CH₂)ₘ-
dans laquelle M vaut de 0 à 6 ; et
L^{A} est choisi parmi :
| | | | |
|---|---|---|---|
| (L^{A1-1}) | 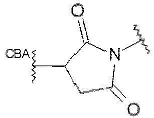 | (L^{A6}) | 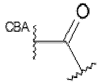 |
| (L^{A1-2}) | 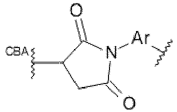 | (L^{A7}) | 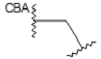 |
| (L^{A2}) | 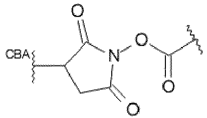 | (L^{A8-1}) | 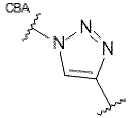 |
| (L^{A3-1}) | 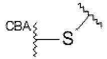 | (L^{A8-2}) | 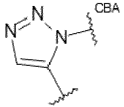 |
| (L^{A3-2}) | 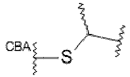 | (L^{A9-1}) | 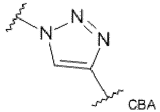 |
| (L^{A4}) | 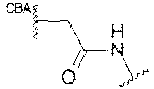 | (L^{A9-2}) | 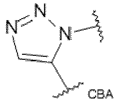 |
| (L^{A5}) | 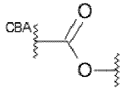 | | |
où Ar représente un groupe arylène en C₅ à C₆.

12. Conjugué selon l'une quelconque des revendications 1 à 11, dans lequel l'agent de liaison cellulaire est un anticorps ou un fragment actif de celui-ci, et dans lequel l'anticorps ou le fragment d'anticorps est un anticorps ou un fragment d'anticorps dirigé contre un antigène associé à une tumeur.

13. Conjugué selon la revendication 12, dans lequel l'anticorps ou le fragment d'anticorps est un anticorps qui se lie à un ou plusieurs antigènes associés à une tumeur ou récepteurs de surface cellulaire choisis parmi (1) à (88) :
(1) BMPR1B ;
(2) E16 ;
(3) STEAP1 ;
(4) 0772P ;
(5) MPF ;
(6) Napi3b ;
(7) Sema 5b ;
(8) PSCA hlg ;
(9) ETBR ;
(10) MSG783 ;
(11) STEAP2 ;
(12) TrpM4 ;
(13) CRIPTO ;
(14) CD21 ;
(15) CD79b ;
(16) FcRH2 ;
(17) HER2 ;
(18) NCA ;
(19) MDP ;
(20) IL20R-alpha ;
(21) Brevican ;
(22) EphB2R ;
(23) ASLG659 ;
(24) PSCA ;
(25) GEDA ;
(26) BAFF-R ;
(27) CD22 ;
(28) CD79a ;
(29) CXCR5 ;
(30) HLA-DOB ;
(31) P2X5 ;
(32) CD72 ;
(33) LY64 ;
(34) FcRH1 ;
(35) IRTA2 ;
(36) TENB2 ;
(37) PSMA - FOLH1 ;
(38) SST ;
(38.1) SSTR2 ;
(38.2) SSTR5 ;
(38.3) SSTR1 ;
(38.4) SSTR3 ;
(38.5) SSTR4 ;
(39) ITGAV ;
(40) ITGB6 ;
(41) CEACAM5 ;
(42) MET ;
(43) MUC1 ;
(44) CA9 ;
(45) EGFRvIII ;
(46) CD33 ;
(47) CD19 ;
(48) IL2RA ;
(49) AXL ;
(50) CD30 - TNFRSF8 ;
(51) BCMA - TNFRSF17 ;
(52) CT Ags - CTA ;
(53) CD174 (Lewis Y) - FUT3 ;
(54) CLEC14A ;
(55) GRP78 - HSPA5 ;
(56) CD70 ;
(57) les antigènes spécifiques des cellules souches ;
(58) ASG-5 ;
(59) ENPP3 ;
(60) PPR4 ;
(61) GCC - GUCY2C ;
(62) Liv-1 - SLC39A6 ;
(63) 5T4 ;
(64) CD56 - NCMA1 ;
(65) CanAg ;
(66) FOLR1 ;
(67) GPNMB ;
(68) TIM1 - HAVCR1 ;
(69) RG-1/Mindin cible de tumeur de la prostate - Mindin/RG-1 ;
(70) B7-H4 - VTCN1 ;
(71) PTK7 ;
(72) CD37 ;
(73) CD138 - SDC1 ;
(74) CD74 ;
(75) Claudins - CLs ;
(76) EGFR ;
(77) Her3 ;
(78) RON - MST1R ;
(79) EPHA2 ;
(80) CD20 - MS4A1 ;
(81) Ténascine C - TNC
(82) FAP ;
(83) DKK-1 ;
(84) CD52 ;
(85) CS1 - SLAMF7 ;
(86) Endogline - ENG ;
(87) Annexine A1 - ANXA1 ;
(88) V-CAM (CD106) - VCAM1.

14. Conjugué selon l'une quelconque des revendications 1 à 13, pour une utilisation en thérapie.

15. Composition pharmaceutique comprenant le conjugué de l'une quelconque des revendications 1 à 13 et un diluant, véhicule ou excipient pharmaceutiquement acceptable.

16. Conjugué selon l'une quelconque des revendications 1 à 13 ou composition pharmaceutique selon la revendication 15, pour une utilisation dans le traitement d'un cancer chez un sujet.

17. Composé de formule (B) : 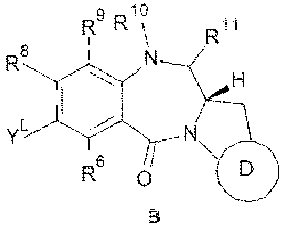 dans laquelle :
Y^{L} est choisi parmi les formules B1 et B2 : 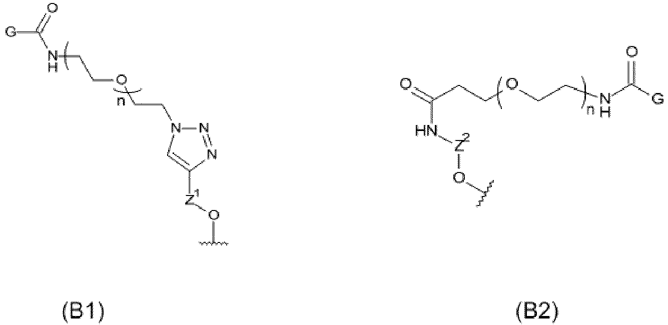
G est un lieur pour une connexion à un agent de liaison cellulaire ; et
D, R⁶, R⁸, R⁹, R¹⁰, R¹¹, Z¹, Z² et n sont tels que définis dans l'une quelconque des revendications 1 à 11.

18. Composé selon la revendication 17, dans lequel G est de formule :
G^{A}-(CH₂)ₘ-
dans laquelle m vaut de 0 à 6 ; et
G^{A} est choisi parmi :
| | | | |
|---|---|---|---|
| (G^{A1-1}) | 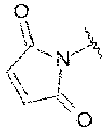 | (G^{A4}) | 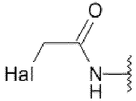 |
| | | | où Hal = I, Br, Cl |
| (G^{A1-2}) | 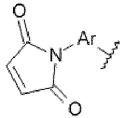 | | |
| (G^{A2}) | 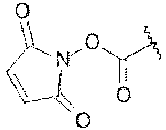 | (G^{A5}) | 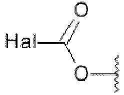 |
| (G^{A3-1}) | 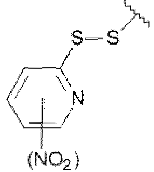 | (G^{A6}) | 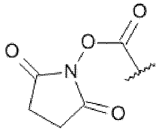 |
| | où le groupe NO₂ est optionnel | | |
| (G^{A3-2}) | 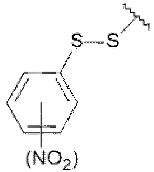 | (G^{A7}) | 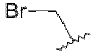 |
| | où le groupe NO₂ est optionnel | | |
| (G^{A3-3}) | 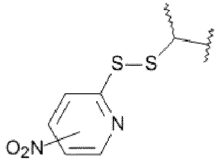 | (G^{A8}) | 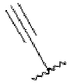 |
| | où le groupe NO₂ est optionnel | | |
| (G^{A3-4}) | 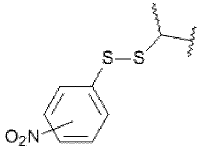 | (G^{A9}) | 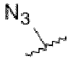 |
| | où le groupe NO₂ est optionnel | | |
où Ar représente un groupe arylène en C₅ à C₆.

19. Composé de formule (C) : 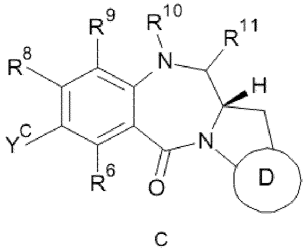 dans laquelle Y^{C} est choisi parmi les formules C1 et C2 : 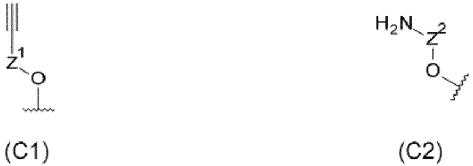 et
D, R⁶, R⁸, R⁹, R¹⁰, R¹¹, Z¹ et Z² sont tels que définis dans l'une quelconque des revendications 1 à 11.
